# EUROPEAN PATENT SPECIFICATION

(11) **EP 1 756 135 B1**
(45) Date of publication and mention of the grant of the patent: **12.08.2009**
(21) Application number: 05739739.0
(22) Date of filing: 02.05.2005
(51) Int. Cl.: C07H 17/08, A61K 31/7048

(54) **ESTER LINKED MACROLIDES USEFUL FOR THE TREATMENT OF MICROBIAL INFECTIONS**
ESTERGEBUNDENE MAKROLIDE, DIE SICH FÜR DIE BEHANDLUNG VON MIKROBIELLEN INFEKTIONEN EIGNEN
MACROLIDES A LIASON ESTER UTILES POUR LE TRAITEMENT D'INFECTIONS MICROBIENNES

(30) Priority: 06.05.2004 US 569402 P; 18.06.2004 US 581118 P
(43) Date of publication of application: 28.02.2007
(73) Proprietor: GlaxoSmithKline istrazivacki centar Zagreb d.o.o., 10000 Zagreb (HR)
(72) Inventor: ALIHODZIC, Sulejman, 10000 Zagreb (HR); MUTAK, Stjepan, 10000 Zagreb (HR); PAVLOVIC, Drazen, 10000 Zagreb (HR); PALEJ, Ivana, 10000 Zagreb (HR); STIMAC, Vlado, 10000 Zagreb (HR); KAPIC, Samra, 10000 Zagreb (HR); Vinter, Adrijana, 10000 Zagreb (HR); MATANOVIC SKUGOR, Maja, 10000 Zagreb (HR)
(74) Representative: Crawley, Karen Anne
(86) International application number: PCT/IB2005/001186
(87) International publication number: WO 2005/108412

(56) References cited:
- WO-A-03/042228
- WO-A-20/04039822
- WO-A-20/04101585
- WO-A-20/04101587
- WO-A-20/04101589

## Description

### FIELD OF THE INVENTION

The present invention relates to novel semi-synthetic macrolides having antimicrobial activity, in particular antibacterial activity. More particularly, the invention relates to 14- and 15-membered macrolides substituted at the 4" position, to processes for their preparation, to compositions containing them and to their use in medicine.

### BACKGROUND OF THE INVENTION

Macrolide antibacterial agents are known to be useful in the treatment or prevention of bacterial infections. However, the emergence of macrolide-resistant bacterial strains has resulted in the need to develop new macrolide compounds. For example, EP 0 895 999 describes derivatives modified at the 4" position of the macrolide ring having antibacterial activity.

International patent application publication WO03/042228 discloses 14- or 15-membered macrolides substituted at the 4"-position by a two heteroatom containing linker group and nitrogen containing bicyclic heterocycle, where said heterocycle has to be attached via terminal heteroatom of the linker, for use in therapy or prophylaxis of systemic or topical bacterial infections in human or animal body.

According to the present invention, we have now found novel 14- and 15-membered macrolides substituted at the 4" position which also have antimicrobial activity.

### SUMMARY OF THE INVENTION

Thus, the present invention provides compounds of general formula (I) 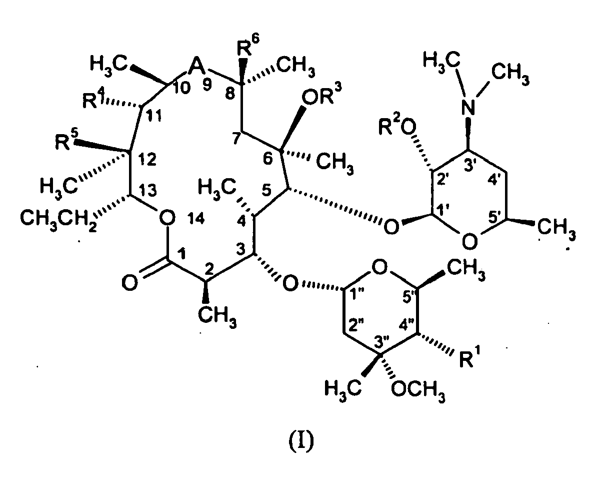 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)(CH₂)_{d}XR¹¹;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxyl, C₂₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹²,
R⁵ is hydroxyl, or
R⁴ and R⁵ taken together with the intervening atoms form a cyclic group having the following structure: 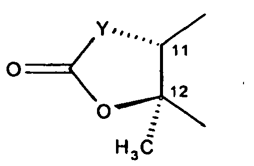
wherein Y is a bivalent radical selected from -CH₂-, -CH(CN)-, -O-, -N(R¹³)- and -CH(SR¹³)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁴R¹⁵ or -C(O)R¹⁴, or
R⁸ and R⁹ together form =CH(CR¹⁴R¹⁵)_{f}aryl, =CH(CR¹⁴R¹⁵)_{f}heterocyclyl, =CR¹⁴R¹⁵ or =C(R¹⁴)C(O)OR¹⁴, wherein the alkyl, aryl and heterocyclyl groups are optionally substituted by up to three groups independently selected from R¹⁶;
R¹⁰ is -OR¹⁷, C₁₋₆alkyl, -(CH₂)_{g}aryl, -(CH₂)_{g}heterocyclyl or -(CH₂)ₕO(CH₂)ᵢOR⁷,
wherein each R¹⁰ group is optionally substituted by up to three groups independently selected from R¹⁶;
R¹¹ is a heterocyclic group having the following structure: 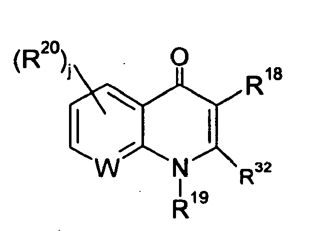 or 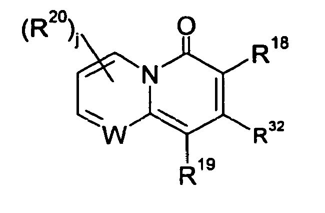
R¹² is hydrogen or C₁₋₆alkyl;
R¹³ is hydrogen or C₁₋₄alkyl substituted by a group selected from optionally substituted phenyl, optionally substituted 5 or 6 membered heteroaryl and optionally substituted 9 to 10 membered fused bicyclic heteroaryl;
R¹⁴ and R¹⁵ are each independently hydrogen or C₁₋₆alkyl;
R¹⁶ is halogen, cyano, nitro, trifluoromethyl, azido, -C(O)R²¹, -C(O)OR²¹, -OC(O)R²¹, -OC(O)OR²¹, -NR²²C(O)R²³, -C(O)NR²²R²³, -NR²²R²³, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁴R¹⁵, halogen and -OR¹⁴, and the aryl and heteroaryl groups are optionally substituted by up to five groups independently selected from halogen, cyano, nitro, trifluoromethyl, azido, -C(O)R²⁴, -C(O)OR²⁴, -OC(O)OR²⁴, -NR²⁵C(O)R²⁶, -C(O)NR²⁵R²⁶, -NR²⁵R²⁶, hydroxy, C₁₋₆alkyl and C₁₋₆alkoxy;
R¹⁷ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁷, -S(O)ₙR²⁷, -NR²⁷R²⁸, -CONR²⁷R²⁸, halogen and cyano;
R¹⁸ is hydrogen, -C(O)OR²⁹, -C(O)NHR²⁹, -C(O)CH₂NO₂, or -C(O)CH₂SO₂R⁷;
R¹⁹ is hydrogen, C₁₋₄alkyl optionally substituted by hydroxyl, cyano, NH₂, -NH(C₁₋₄alkyl) or -N(C₁₋₄alkyl)₂; C₂₋₄alkenyl optionally substituted by hydroxyl, cyano, NH₂, -NH(C₁₋₄alkyl) or -N(C₁₋₄alkyl)₂; C₁₋₄alkoxy, C₃₋₇cycloalkyl, -NH₂, -NH(C₁₋₄alkyl) or -N(C₁₋₄alkyl)₂; (C₁₋₄alkyl)OC(O)N(C₁₋₄alkyl) or optionally substituted phenyl or benzyl;
R²⁰ is halogen, C₁₋₄alkyl, C₁₋₄thioalkyl, C₁₋₄alkoxy, -NH₂, -NH(C₁₋₄alkyl) or -N(C ₁₋₄alkyl)₂;
R²¹ is hydrogen, C₁₋₁₀alkyl, -(CH₂)paryl or -(CH₂)pheteroaryl;
R22 and R²³ are each independently hydrogen, -OR¹⁴, C₁₋₆alkyl, -(CH₂)_{q}aryl or -(CH₂)qheterocyclyl;
R²⁴ is hydrogen, C₁₋₁₀alkyl, -(CH₂)ᵣaryl or -(CH₂)ᵣheteroaryl;
R²⁵ and R²⁶ are each independently hydrogen, -OR¹⁴, C₁₋₆alkyl, -(CH₂)ₛaryl or -(CH₂)ₛheterocyclyl;
R²⁷ and R²⁸ are each independently hydrogen, C₁₋₄alkyl or C₁₋₄alkoxyC₁₋₄alkyl;
R²⁹ is hydrogen or C₁₋₆alkyl optionally substituted by up to three groups independently selected from halogen, C₁₋₄alkoxy, -OC(O)C₁₋₆alkyl and -OC(O)OC₁₋₆alkyl, -(CH₂)_{q}heterocyclyl, -(CH₂)_{q}heteroaryl, -(CH₂)_{q}aryl, or -(CH₂)_{q}C₃₋₇cycloalkyl;
R³⁰ is hydrogen, C₁₋₄alkyl, C₃₋₇cycloalkyl, optionally substituted phenyl or benzyl, acetyl or benzoyl;
R³¹ is hydrogen or R²⁰, or R³¹ and R¹⁹ are linked to form the bivalent radical -O(CH₂)₂-, -(CH₂)ₜ-;-NR⁷(CH₂)ₐ-, -OCH₂NR⁷-, -SCH₂NR⁷-, -CH₂NR⁷CH₂-, -CH₂OCH₂-, -CH₂SCH₂-, -(CH₂)ₐNR⁷- ;
R³² is hydrogen, or R³² and R¹⁹ are linked to form the bivalent radical selected from the group, -S(CH₂)_{b}-, -N(R⁷)(CH₂)_{b}-, and -O(CH₂)_{b}-;
R³³ is propyl;
X is -U(CH₂)ᵥB(CH₂)ᵥD-, -U(CH₂)ᵥB(CH₂)ᵥD(CH₂)ᵥE-, -U(CH₂)ᵥB-R³³-, or -U(CH₂)ᵥB(CH₂)ᵥD-R³³-;
or X is a group selected from: 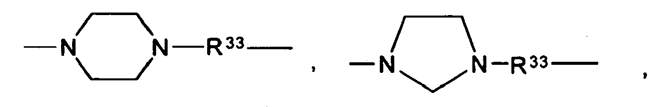 and 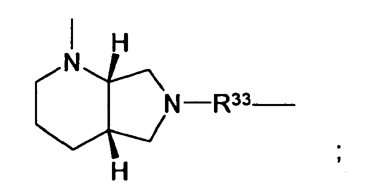
U, B, D and E are independently divalent radicals selected from -N(R³⁰)-, -O-, -S(O)_{z}-, - N(R³⁰)C(O)-, -C(O)N(R³⁰)- and -N[C(O)R³⁰]-;
W is -C(R³¹)- or a nitrogen atom;
a is 1 or 2
b is an integer from 1 to 3;
d is an integer from 1 to 5;
e is an integer from 2 to 4;
f, g, h, m, p, q, r and s are each independently integers from 0 to 4;
i is an integer from 1 to 6;
j, k, n and z are each independently integers from 0 to 2;
t is 2 or 3;
v is an integer from 1 to 8;
or a salt, solvate or ester thereof.

### DETAILED DESCRIPTION OF THE INVENTION

The term "pharmaceutically acceptable" as used herein means a compound which is suitable for pharmaceutical use. Salts and solvates of compounds of the invention which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of the invention and their pharmaceutically acceptable salts and solvates.

The term "pharmaceutically acceptable derivative" as used herein means any pharmaceutically acceptable salt, solvate or prodrug, e.g. ester, of a compound of the invention, which upon administration to the recipient is capable of providing (directly or indirectly) a compound of the invention, or an active metabolite or residue thereof. Such derivatives are recognizable to those skilled in the art, without undue experimentation. Nevertheless, reference is made to the teaching of Burger's Medicinal Chemistry and Drug Discovery, 5th Edition, Vol 1: Principles and Practice. Preferred pharmaceutically acceptable derivatives are salts, solvates, esters, carbamates and phosphate esters. Particularly preferred pharmaceutically acceptable derivatives are salts, solvates and esters. Most preferred pharmaceutically acceptable derivatives are salts and esters.

The compounds of the present invention may be in the form of and/or may be administered as a pharmaceutically acceptable salt. For a review on suitable salts see Berge et al., J. Pharm. Sci., 1977, 66,1-19.

Typically, a pharmaceutical acceptable salt may be readily prepared by using a desired acid or base as appropriate. The salt may precipitate from solution and be collected by filtration or may be recovered by evaporation of the solvent. For example, an aqueous solution of an acid such as hydrochloric acid may be added to an aqueous suspension of a compound of formula (I) and the resulting mixture evaporated to dryness (lyophilised) to obtain the acid addition salt as a solid. Alternatively, a compound of formula (I) may be dissolved in a suitable solvent, for example an alcohol such as isopropanol, and the acid may be added in the same solvent or another suitable solvent. The resulting acid addition salt may then be precipitated directly, or by addition of a less polar solvent such as diisopropyl ether or hexane, and isolated by filtration.

Suitable addition salts are formed from inorganic or organic acids which form non-toxic salts and examples are hydrochloride, hydrobromide, hydroiodide, sulphate, bisulphate, nitrate, phosphate, hydrogen phosphate, acetate, trifluoroacetate, maleate, malate, fumarate, lactate, tartrate, citrate, formate, gluconate, succinate, pyruvate, oxalate, oxaloacetate, trifluoroacetate, saccharate, benzoate, alkyl or aryl sulphonates (eg methanesulphonate, ethanesulphonate, benzenesulphonate or p-toluenesulphonate) and isethionate. Representative examples include trifluoroacetate and formate salts, for example the bis or tris trifluoroacetate salts and the mono or diformate salts, in particular the tris or bis trifluoroacetate salt and the monoformate salt.

Pharmaceutically acceptable base salts include ammonium salts, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine.

Compounds of the invention may have both a basic and an acidic centre may therefore be in the form of zwitterions.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of the invention are within the scope of the invention. The salts of the compound of formula (I) may form solvates (e.g. hydrates) and the invention also includes all such solvates.

The term "prodrug" as used herein means a compound which is converted within the body, e.g. by hydrolysis in the blood, into its active form that has medical effects. Pharmaceutically acceptable prodrugs are described in T. Higuchi and V. Stella, "Prodrugs as Novel Delivery Systems", Vol. 14 of the A.C.S. Symposium Series, Edward B. Roche, ed., "Bioreversible Carriers in Drug Design", American Pharmaceutical Association and Pergamon Press, 1987**,** and in D. Fleisher, S. Ramon and H. Barbra "Improved oral drug delivery: solubility limitations overcome by the use of prodrugs", Advanced Drug Delivery Reviews, 1996, 19(2), 115-130.

Prodrugs are any covalently bonded carriers that release a compound of structure (I) *in vivo* when such prodrug is administered to a patient. Prodrugs are generally prepared by modifying functional groups in a way such that the modification is cleaved, either by routine manipulation or *in vivo,* yielding the parent compound. Prodrugs include, for example, compounds of this invention wherein hydroxy, amine or sulfhydryl groups are bonded to any group that, when administered to a patient, cleaves to form the hydroxy, amine or sulfhydryl groups. Thus, representative examples of prodrugs include (but are not limited to) acetate, formate and benzoate derivatives of alcohol, sulfhydryl and amine functional groups of the compounds of structure (I). Further, in the case of a carboxylic acid (-COOH), esters may be employed, such as methyl esters, ethyl esters, and the like. Esters may be active in their own right and/or be hydrolysable under *in vivo* conditions in the human body. Suitable pharmaceutically acceptable *in vivo* hydrolysable ester groups include those which break down readily in the human body to leave the parent acid or its salt.

References hereinafter to a compound according to the invention include both compounds of formula (I) and their pharmaceutically acceptable derivatives.

With regard to stereoisomers, the compounds of structure (I) have more than one asymmetric carbon atom. In the general formula (I) as drawn, the solid wedge shaped bond indicates that the bond is above the plane of the paper. The broken bond indicates that the bond is below the plane of the paper.

It will be appreciated that the substituents on the macrolide may also have one or more asymmetric carbon atoms. Thus, the compounds of structure (I) may occur as individual enantiomers or diastereomers. All such isomeric forms are included within the present invention, including mixtures thereof.

Where a compound of the invention contains an alkenyl group, cis (Z) and trans (E) isomerism may also occur. The present invention includes the individual stereoisomers of the compound of the invention and, where appropriate, the individual tautomeric forms thereof, together with mixtures thereof.

Separation of diastereoisomers or cis and trans isomers may be achieved by conventional techniques, e.g. by fractional crystallisation, chromatography or HPLC. A stereoisomeric mixture of the agent may also be prepared from a corresponding optically pure intermediate or by resolution, such as HPLC of the corresponding mixture using a suitable chiral support or by fractional crystallisation of the diastereoisomeric salts formed by reaction of the corresponding mixture with a suitable optically active acid or base, as appropriate.

The compounds of structure (I) may be in crystalline or amorphous form. Furthermore, some of the crystalline forms of the compounds of structure (I) may exist as polymorphs, which are included in the present invention.

Compounds wherein R² represents a hydroxyl protecting group are in general intermediates for the preparation of other compounds of formula (I).

When the group OR² is a protected hydroxyl group this is conveniently an ether or an acyloxy group. Examples of particularly suitable ether groups include those in which R² is a trialkylsilyl (i.e. trimethylsilyl). When the group OR² represents an acyloxy group, then examples of suitable groups R² include acetyl or benzoyl.

R⁶ is hydrogen or fluorine. However, it will be appreciated that when A is -C(O)NH- or -CH₂-N(R⁷)-, R⁶ is hydrogen.

When R¹¹ is a heterocyclic group having the following structure: 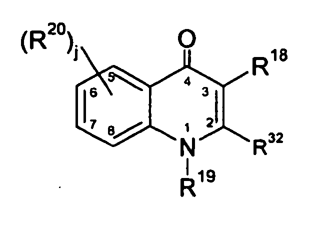 said heterocyclic is linked in the 6 or 7 position to the X group as above defined. When present, the R²⁰ group or groups may be attached at any position on the ring. In one embodiment, an R²⁰ group is attached at the 6 or 7 position.

When R¹¹ is a heterocyclic group having the following structure: 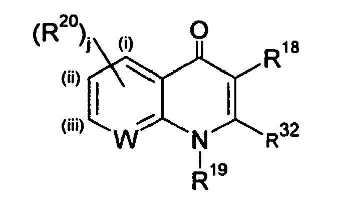 wherein W is -C(R³¹)- where R³¹ is R²⁰ or R³¹ and R¹⁹ are linked to form the bivalent radical -O(CH₂)₂-, -(CH₂)ₜ-;-NR⁷(CH₂)ₐ-, -OCH₂NR⁷-, -SCH₂NR⁷-, -CH₂NR⁷CH₂-, -CH₂OCH₂-, -CH₂SCH₂-, -(CH₂)ₐNR⁷-, said heterocyclic is linked in the (ii) or (iii) position to the X group as above defined.

When R¹¹ is a heterocyclic group having the following structure: 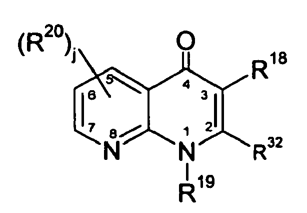 said heterocyclic is linked in the 6 or 7 position to the X group as defined above.

When R¹¹ is a heterocyclic group having the following structure: 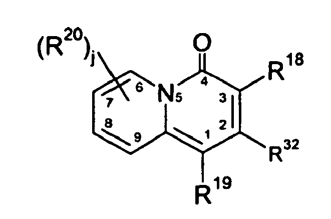 said heterocyclic is linked in the 7 or 8 position to the X group as above defined.

When R¹¹ is a heterocyclic group having the following structure: 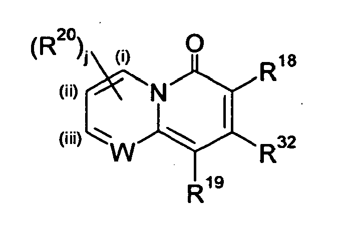 wherein W is -C(R³¹)- where R³¹ is R²⁰ or R³¹ and R¹⁹ are linked to form the bivalent radical -O(CH₂)₂-, -(CH₂)ₜ-;-NR⁷(CH₂)ₐ-, -OCH₂NR⁷-, -SCH₂NR⁷-, -CH₂NR⁷CH₂-, -CH₂OCH₂-, -CH₂SCH₂-, -(CH₂)ₐNR⁷-, said heterocyclic is linked in the (i), (ii) or (iii) position to the X group as above defined. In one embodiment, the heterocyclic is linked to the (i) position. In another embodiment, the heterocyclic is linked in the (ii) or (iii) position.

When R¹¹ is a heterocyclic group having the following structure: 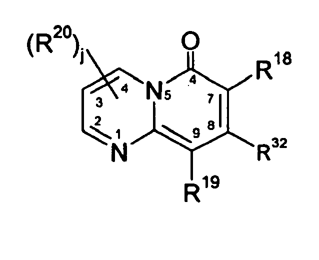 said heterocyclic is linked in the 2 or 3 position to the X group as above defined. In one embodiment, the heterocyclic is linked in the 2 or 3 position. In another embodiment, the heterocyclic is linked in the 4 position.

The term "alkyl" as used herein as a group or a part of a group refers to a straight or branched hydrocarbon chain containing the specified number of carbon atoms. For example, C₁₋₁₀alkyl means a straight or branched alkyl containing at least 1, and at most 10, carbon atoms. Examples of "alkyl" as used herein include, but are not limited to, methyl, ethyl, n-propyl, n-butyl, n-pentyl, isobutyl, isopropyl, t-butyl, hexyl, heptyl, octyl, nonyl and decyl. A C₁₋₄alkyl group is preferred, for example methyl, ethyl, n-propyl, isopropyl, n-butyl, isobutyl or t-butyl.

The term "C₃₋₇cycloalkyl" group as used herein refers to a non-aromatic monocyclic hydrocarbon ring of 3 to 7 carbon atoms such as, for example, cyclopropyl, cyclobutyl, cyclopentyl, cyclohexyl or cycloheptyl.

The term "alkoxy" as used herein refers to a straight or branched chain alkoxy group containing the specified number of carbon atoms. For example, C₁₋₆alkoxy means a straight or branched alkoxy containing at least 1, and at most 6, carbon atoms. Examples of "alkoxy" as used herein include, but are not limited to, methoxy, ethoxy, propoxy, prop-2-oxy, butoxy, but-2-oxy, 2-methylprop-1-oxy, 2-methylprop-2-oxy, pentoxy and hexyloxy. A C₁₋₄alkoxy group is preferred, for example methoxy, ethoxy, propoxy, prop-2-oxy, butoxy, but-2-oxy or 2-methylprop-2-oxy.

The term "alkenyl" as used herein as a group or a part of a group refers to a straight or branched hydrocarbon chain containing the specified number of carbon atoms and containing at least one double bond. For example, the term "C₂₋₆alkenyl" means a straight or branched alkenyl containing at least 2, and at most 6, carbon atoms and containing at least one double bond. Examples of "alkenyl" as used herein include, but are not limited to, ethenyl, 2-propenyl, 3-butenyl, 2-butenyl, 2-pentenyl, 3-pentenyl, 3-methyl-2-butenyl, 3-methylbut-2-enyl, 3-hexenyl and 1,1-dimethylbut-2-enyl. It will be appreciated that in groups of the form -O-C₂₋₆alkenyl, the double bond is preferably not adjacent to the oxygen.

The term "alkynyl" as used herein as a group or a part of a group refers to a straight or branched hydrocarbon chain containing the specified number of carbon atoms and containing at least one triple bond. For example, the term "C₂₋₆alkynyl" means a straight or branched alkynyl containing at least 2, and at most 6, carbon atoms and containing at least one triple bond. Examples of "alkynyl" as used herein include, but are not limited to, ethynyl, 2-propynyl, 3-butynyl, 2-butynyl, 2-pentynyl, 3-pentynyl, 3-methyl-2-butynyl, 3-methylbut-2-ynyl, 3-hexynyl and 1,1-dimethylbut-2-ynyl. It will be appreciated that in groups of the form -O-C₂₋₆alkynyl, the triple bond is preferably not adjacent to the oxygen.

The term "aryl" as used herein refers to an aromatic carbocyclic moiety such as phenyl, biphenyl or naphthyl.

The term "heteroaryl" as used herein, unless otherwise defined, refers to an aromatic heterocycle of 5 to 10 members, having at least one heteroatom selected from nitrogen, oxygen and sulfur, and containing at least 1 carbon atom, including both mono and bicyclic ring systems. Examples of heteroaryl rings include, but are not limited to, furanyl, thiophenyl, pyrrolyl, pyrazolyl, imidazolyl, oxazolyl, isoxazolyl, thiazolyl, isothiazolyl, triazolyl, oxadiazolyl, tetrazolyl, thiadiazolyl, pyridyl, pyridazinyl, pyrazinyl, pyrimidinyl, triazinyl, quinolinyl, isoquinolinyl, 1,2,3,4-tetrahydroisoquinolinyl, benzofuranyl, benzimidazolyl, benzothienyl, benzoxazolyl, 1,3-benzodioxazolyl, indolyl, benzothiazolyl, furylpyridine, oxazolopyridyl and benzothiophenyl.

The term "5 or 6 membered heteroaryl" as used herein as a group or a part of a group refers to a monocyclic 5 or 6 membered aromatic heterocycle containing at least one heteroatom independently selected from oxygen, nitrogen and sulfur. Examples include, but are not limited to, furanyl, thiophenyl, pyrrolyl, pyrazolyl, imidazolyl, oxazolyl, isoxazolyl, thiazolyl, isothiazolyl, triazolyl, oxadiazolyl, tetrazolyl, pyridyl, pyridazinyl, pyrazinyl, pyrimidinyl and triazinyl.

The term "9 to 10 membered fused bicyclic heteroaryl" as used herein as a group or a part of a group refers to quinolinyl, isoquinolinyl, 1,2,3,4-tetrahydroisoquinolinyl, benzofuranyl, benzimidazolyl, benzothienyl, benzoxazolyl, 1,3-benzodioxazolyl, indolyl, benzothiazolyl, furylpyridine, oxazolopyridyl or benzothiophenyl.

The term "heterocyclyl" as used herein, unless otherwise defined, refers to a monocyclic or bicyclic three- to ten-membered saturated or non-aromatic, unsaturated hydrocarbon ring containing at least one heteroatom selected from oxygen, nitrogen and sulfur. Preferably, the heterocyclyl ring has five or six ring atoms. Examples of heterocyclyl groups include, but are not limited to, pyrrolidinyl, tetrahydrofuranyl, tetrahydrothiophenyl, imidazolidinyl, pyrazolidinyl, piperidyl, piperazinyl, morpholino, tetrahydropyranyl and thiomorpholino.

The term "5 or 6 membered heterocyclic group" as used herein as a group or part of a group refers to a monocyclic 5 or 6 membered saturated hydrocarbon ring containing at least one heteroatom independently selected from oxygen, nitrogen and sulfur. Examples of such heterocyclyl groups include, but are not limited to, pyrrolidinyl, tetrahydrofuranyl, tetrahydrothiophenyl, imidazolidinyl, pyrazolidinyl, piperidyl, piperazinyl, morpholino, tetrahydropyranyl and thiomorpholino.

The term "halogen" refers to a fluorine, chlorine, bromine or iodine atom.

The terms "optionally substituted phenyl", "optionally substituted phenyl or benzyl", "optionally substituted 5 or 6 membered heteroaryl", "optionally substituted 9 to 10 membered fused bicyclic heteroaryl" or "optionally substituted 5 or 6 membered heterocyclic group" as used herein refer to a group which is substituted by 1 to 3 groups selected from halogen, C₁₋₄alkyl, C₁₋₄alkoxy, hydroxy, nitro, cyano, amino, C₁₋₄alkylamino or diC₁₋₄alkylamino, phenyl and 5 or 6 membered heteroaryl.

In one embodiment, A is -C(O)-, -C(O)NH-, -NHC(O)-, -N(R⁷)-CH₂-, -CH₂-N(R⁷)- or -CH(NR⁸R⁹)-. In another embodiment, A is -C(O)-, -C(O)NH-, -NHC(O)-, -CH₂-N(R⁷)-, -CH(NR⁸R⁹)- or -C(=NR¹⁰)-. In a further embodiment, A is -C(O)-, -C(O)NH-, -NHC(O)-, -CH₂-NR⁷- or -CH(NR⁸R⁹)-. Representative examples of A include -C(O)- and -N(R⁷)-CH₂-. In one preferred embodiment, A is -N(R⁷)-CH₂-.

A representative example of R² is hydrogen.

Representative examples of R³ include hydrogen and C₁₋₄alkyl, in particular hydrogen and methyl.

In one embodiment, R⁴ is hydroxyl or C₁₋₆alkoxy, in particular hydroxyl or methoxy. In a preferred embodiment, R⁴ is hydroxyl. In another embodiment, R⁵ is hydroxyl. Alternatively, R⁴ and R⁵ taken together with the intervening atoms form a cyclic group having the following structure: 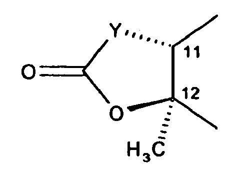 wherein Y is a bivalent radical selected from -O- and -N(R¹³)-.

A representative example of R⁶ is hydrogen.

A representative example of R⁷ is C₁₋₆alkyl, for example C₁₋₄alkyl, in particular methyl.

Representative examples of R¹¹ include heterocyclic groups having the following structures: 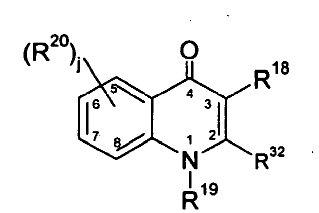 and 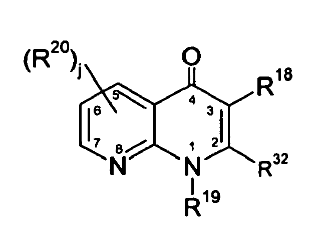 wherein the heterocyclic is linked in the 6 or 7 position to the X group as above defined, and heterocyclic groups having the following structure: 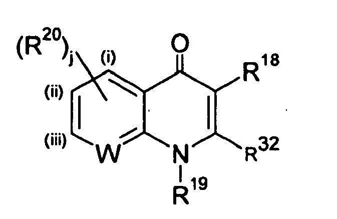 wherein W is -C(R³¹)- and R³¹ and R¹⁹ are linked to form the bivalent radical -(CH₂)ₜ-, and the heterocylic is linked in the (ii) or (iii) position to the X group as above defined.

In one preferred embodiment, R¹¹ is a heterocyclic group having the following structure:

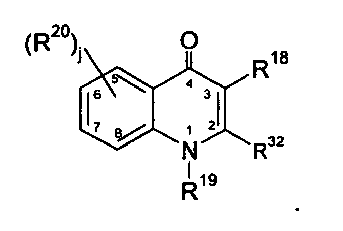

A representative example of R¹³ is hydrogen.

In one embodiment, R¹⁸ is -C(O)OR²⁹, -C(O)NHR²⁹, -C(O)CH₂NO₂.or -C(O)CH₂SO₂R⁷.

A representative example of R¹⁸ is -C(O)OR²⁹. In one preferred embodiment, R¹⁸ is -C(O)OR²⁹ wherein R²⁹ is hydrogen.

Representative examples of R¹⁹ include C₁₋₄alkyl, in particular ethyl, and C₃₋₇cycloalkyl, in particular cyclopropyl.

In one embodiment, R²⁰ is halogen, in particular chlorine or fluorine, or methoxy.

In one embodiment, R³⁰ is hydrogen or C₁₋₄alkyl. A representative example of R³⁰ is hydrogen or methyl.

A representative example of R³¹ is hydrogen, or R³¹ and R¹⁹ are linked to form the bivalent radical -(CH₂)ₜ-.

A representative example of X is -U(CH₂)ᵥB(CH₂)ᵥD-, -U(CH₂)ᵥB(CH₂)ᵥD(CH₂)ᵥE-, -U(CH₂)ᵥB-R³³-, or -U(CH₂)ᵥB(CH₂)ᵥD-R³³-.

Representative examples of U, B, D and E include the divalent radicals -N(R³⁰)-, -O-, S(O)_{z}-, -N(R³⁰)C(O)- and -C(O)N(R³⁰)-.

R³³ is propyl.

A representative example of d is 1 to 4, for example 2 to 4. A particularly preferred example of d is 2.

A representative example of v is 1 to 4, for example 2 or 3. A particularly preferred example is when each v independently is 2.

In one embodiment, X is -U(CH₂)ᵥB(CH₂)ᵥD- or -U(CH₂)ᵥB-R³³- wherein U is -O-, B is -O-, and D is -N-. Particulalry preferred X groups are -O(CH₂)₂O(CH₂)₂N- and -O(CH₂)₂O-(CH₂)₃-.

In one embodiment, j is 0 to 2. A representative example of j is 0 or 1.

A representative example of t is 3.

A representative example of z is 0.

Particularly preferred compounds of the invention are:
4"-*O*-(3-{4-[3-(3-Ethoxycarbonyl-1-ethyl-4-oxo-1,4-dihydro-quinolin-6-yl)-propyl]-piperazin-1-yl}-propionyl)-azithromycin,
4"-*O*-(3-{4-[3-(3-Ethoxycarbonyl-1-ethyl-4-oxo-1,4-dihydro-quinolin-6-yl)-propyl]-piperazin-1-yl}-propionyl)-11-*O*-methyl-azithromycin,
4"-*O*-(3-{2-[2-(3-Carboxy-7-chloro-1-cyclopropyl-4-oxo-1,4-dihydio-quinolin-6-ylamino)-ethoxy]-ethoxy}-propionyl)-roxythromycin,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-6-*O*-methyl-erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-azithromycin,
4"-O-(3-{2-[2-(3-carboxy-7-chloro-1-cyclopropyl-4-oxo-1,4-dihydro-quinolin-6-ylamino)-ethoxy]-ethoxy}-propionyl)-11-O-methyl-azithromycin,
4"-*O*-(3-{2-[2-(3-Carboxy-1-cyclopropyl-4-oxo-1,4-dihydro-quinolin-6-ylamino)-ethoxy]-ethoxy}-propionyl)-azithromycin,
4"-*O*-(2-{3-[2-(3-Carboxy-7-chloro-1-cyclopropyl-4-oxo-1,4-dihydro-quinolin-6-yloxy)-ethoxy]-propionylamino}-acetyl)-azithromycin 11,12-cyclic carbonate,
4"-*O*-(2-{3-[2-(3-Carboxy-7-chloro-1-cyclopropyl-4-oxo-1,4-dihydro-quinolin-6-ylamino)-ethoxy]-propionylamino}-acetyl)-azithromycin 11,12-cyclic carbonate,
4"-*O*-(2-{3-[2-(3-Carboxy-6-fluoro-1-cyclopropyl-8-methoxy-4-oxo-1,4-dihydro-quinolin-7-ylamino)-ethoxy]-propionylamino}-acetyl)-azithromycin 11,12-cyclic carbonate,
4"-*O*-[2-(3-{2-[2-(3-Carboxy-6-fluoro-1-cyclopropyl-4-oxo-1,4-dihydro-quinolin-7-ylamino)-ethoxy]-ethoxy}-propionylamino)-acetyl]-azithromycin 11,12-cyclic carbonate,
4"-*O*-[2-(3-{2-[2-(3-Carboxy-7-chloro-1-cyclopropyl-4-oxo-1,4-dihydro-quinolin-6-ylamino)-ethoxy]-ethoxy}-propionylamino)-acetyl]-azithromycin 11,12-cyclic carbonate,
4"-*O*-[2-(3-{2-[2-(3-Carboxy-7-chloro-1-cyclopropyl-4-oxo-1,4-dihydro-quinolin-6-ylamino)-ethoxy]-ethoxy}-propionylamino)-acetyl]-azithromycin,
4"*O*-{2-[3-({2-[3-(6-Ethoxycarbonyl-7-oxo-2,3-dihydro-1H,7H-pyrido[3,2,1-ij]quinolin-9-yl)-propylamino]-ethyl}-propyl-amino)-propionylamino]-acetyl}-azithromycin,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-9(*E*)-ethoxyimino-erythromycin A,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-6-*O*-methyl-erythromycin A,
4"-*O*-[3-(2-{2-[2-(3-Carboxy-6-fluoro-1-cyclopropyl-4-oxo-1,4-dihydro-quinolin-7-ylamino)-ethoxy]-ethoxy}-ethylamino)-propionyl]-6-*O*-methyl-erythromycin A,
4"-*O*-(3-{2-[2-(3-Carboxy-7-chloro-1-cyclopropyl-4-oxo-1,4-dihydro-quinolin-6-ylamino)-ethoxy]-ethylamino}-propionyl)-6-*O*-methyl-erythromycin A,
4"-*O*-(3-{2-[2-(3-Carboxy-6-fluoro-1-cyclopropyl-4-oxo-1,4-dihydro-quinolin-7-ylamino)-ethoxy]-ethylamino}-propionyl)-6-*O*-methyl-erythromycin A,
4"-*O*-(3-{2-[2-(10-Carboxy-9-oxo-3,4-dihydro-2H,9H-1-oxa-4a-aza-phenanthren-6-ylamino)-ethoxy]-ethoxy}-propionyl)-azithromycin,
11-O-Methyl-4"-O-(3-{2-[3-(3-Carboxy-1-ethyl-4-oxo-1,4-dihydro-quinolin-6-yl)-propoxy]-ethylamino}-propionyl)-azithromycin, and
4"-*O*-(3-{2-[2-(3-Carboxy-6-fluoro-1-cyclopropyl-4-oxo-1,4-dihydro-quinolin-7-ylamino)-ethoxy]-ethoxy}-propionyl)-azithromycin,
or a salt, solvate or ester thereof.

Further particularly preferred compounds of the invention are:
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-6-*O*-methyl-erythromycin A 11,12-cyclic carbamate,
4"-*O*-(3-{2-[2-(3-Carboxy-6-fluoro-1-cyclopropyl-4-oxo-1,4-dihydro-quinolin-7-ylamino)-ethoxy]-ethoxy}-propionyl)-6-*O*-methyl-erythromycin A,
4"-O-(3-{2-[2-(3-Carboxy-7-chloro-1-ethyl-4-oxo-1,4-dihydro-quinoline-6-ylamino)-ethoxy]-ethoxy}-propionyl)-azithromycin,
4"-O-(3-{2-[2-(3-Carboxy-6-fluoro-1-ethyl-4-oxo-1,4-dihydro-quinoline-7-ylamino)-ethoxy]-ethoxy}-propionyl)-azithromycin,
4"-O-(3-{2-[2-(3-Carboxy-1-ethyl-4-oxo-1,4-dihydro-quinoline-6-ylamino)-ethoxy]-ethoxy}-propionyl)-azithromycin,
4"-O-(3-{2-[2-(3-carboxy-7-chloro-1-isopropyl-4-oxo-1,4-dihydro-quinoline-6-ylamino)-ethoxy]-ethoxy}-propionyl)-azithromycin,
4"-O-(3-{2-[2-(6-Carboxy-7-oxo-2,3-dihydro-1H,7H-pyrido[3,2,1-ij]quinolin-9-ylamino)-ethoxy]-ethoxy}-propionyl)-azithromycin,
4"-O-(3-{2-[2-(6-Carboxy-7-oxo-2,3-dihydro-1H,7H-pyrido[3,2,1-ij]quinolin-9-ylamino)-ethoxy]-ethoxy}-propionyl)-6-*O*-methyl-erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-6-*O*-propyl-erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-azithromycin,
4"-*O*-(3-{2-[3-(3-Carboxy-1-ethyl-4-oxo-1,4-dihydro-quinolin-6-yl)-propoxy]-ethylamino}-propionyl)-6-*O*-methyl-erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-6-*O*-methyl-erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-9-ethyloximino-6-*O*-methyl-erythromycin A,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-9-(1-isopropoxy-cyclohexyl)oximino-erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-9-(1-isopropoxy-cyclohexyl)oximino-erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-9-(1-isopropoxy-cyclohexyl)oximino-erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-9-oxime erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-9-oxime erythromycin A,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-9-oxime erythromycin A,
4"-*O*-(3-{2-[2-(3-Carboxy-1-cyclopropyl-7-methoxy-4-oxo-1,4-dihydro-quinolin-6-ylamino)-ethoxy]-ethoxy}-propionyl)-azithromycin,
4"-*O*-(3-{2-[2-(3-Carboxy-1-cyclopropyl-7-dimethylamino-4-oxo-1,4-dihydro-quinolin-6-ylamino)-ethoxy]-ethoxy}-propionyl) azithromycin,
4"-O-(3-{2-[3-(3-carboxy-1-ethyl-4-oxo-1,4-dihydro-quinolin-6-yl)-propoxy]-ethoxy}-propionyl)-azithromycin,
4"-O-(3-{2-[3-(3-carboxy-1-ethyl-4-oxo-1,4-dihydro-quinolin-6-yl)-propoxy]-ethoxy}-propionyl)-6-O-methyl erythromycin A,
9-Ethyloximino-4"-O-(3-{2-[3-(3-carboxy-1-ethyl-4-oxo-1,4-dihydro-quinolin-6-yl)-propoxy]-ethoxy}-propionyl)-erythromycin A,
4"-O-(3-{2-[3-(3-Carboxy-1-ethyl-4-oxo-1,4-dihydro-quinolin-6-yl)-propoxy]-ethoxy}-propionyl)-6-O-methyl-8a-aza-8a-homoerythromycin A,
4"-O-(3-{2-[3-(3-Carboxy-1-ethyl-4-oxo-1,4-dihydro-quinolin-6-yl)-propoxy]-ethoxy}-propionyl)-roxythromycin,
4"-*O*-(3-{2-[2-(3-Carboxy-7-chloro-1-cyclopropyl-4-oxo-1,4-dihydro-quinolin-6-yloxy)-ethoxy]-ethoxy}-propionyl)-azithromycin,
4"-O-(3-{2-[3-(3-Carboxy-1-ethyl-4-oxo-1,4-dihydro-quinolin-6-yl)-propoxy]-ethoxy}-propionyl)-6-O-methyl-11-desoxy-11-(R)-methylamino-erythromycin A 11,12-carbamate,
4"-O-(3-{2-[3-(3-Carboxy-1-cyclopropyl-4-oxo-1,4-dihydro-quinolin-6-yl)-propoxy]-ethoxy}-propionyl)-azithromycin,
4"-O-(3-{2-[3-(3-Carboxy-4-oxo-1,4-dihydro-quinolin-6-yl)-propoxy]-ethoxy}-propionyl)-azithromycin,
4"-O-(3-{2-[3-(3-Carboxy-4-oxo-1-propyl-1,4-dihydro-quinolin-6-yl)-propoxy]-ethoxy}-propionyl)-azithromycin,
4"-O-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-azithromycin,
4"-O-[3-(2-{2-[2-(3-Carboxy-6-fluoro-1-cyclopropyl-4-oxo-1,4-dihydro-quinolin-7-ylamino)-ethoxy]-ethoxy}-ethylamino)-propionyl]-azithromycin,
4"-O-(3-{2-[2-(3-Carboxy-7-chloro-1-cyclopropyl-4-oxo-1,4-dihydro-quinolin-6-ylamino)-ethoxy]-ethylamino}-propionyl)-azithromycin,
4"-O-(3-{2-[2-(3-Carboxy-6-fluoro-1-cyclopropyl-4-oxo-1,4-dihydro-quinolin-7-ylamino)-ethoxy]-ethylamino}-propionyl)-azithromycin,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-6-*O*-methyl erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-6-*O*-methyl-8a-aza-8a-homoerythromycin A,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-azithromycin,
4"-*O*-[3-(2-{[2-(3-Carboxy-7-chloro-1-cyclopropyl-4-oxo-1,4-dihydro-quinolin-6-ylamino)-ethyl]-methyl-amino}-ethoxy)-propionyl]-azithromycin,
4"-*O*-(3-{2-[2-(3-Carboxy-1-cyclopropyl-4-oxo-1,4-dihydro-quinolin-6-yloxy)-ethoxy]-ethoxy}-propionyl)-azithromycin,
4"-*O*-(3-{2-[2-(3-Carboxy-7-chloro-4-oxo-1,4-dihydro-quinolin-6-yloxy)-ethoxy]-ethoxy}-propionyl)-azithromycin,
4"-*O*-(3-{2-[2-(3-Carboxy-7-chloro-4-oxo-1,4-dihydro-quinolin-6-ylamino)-ethoxy]-ethoxy}-propionyl)-azithromycin,
4"-O-{[6-({2-[(2-Aminoethyl)(methyl)amino]ethyl}thio)-1-ethyl-4-oxo-1,4-dihydro-3-quinolinecarboxylic acid]propionyl}-6-*O*-methylerythromycin A,
4"-O-{[6-({2-[(2-Aminoethyl)(methyl)amino]ethyl}thio)-1-ethyl-4-oxo-1,4-dihydro-3-quinolinecarboxylic acid]propionyl}-azithromycin,
4"-O-{[6-({2-[(2-Aminoethyl)thio]ethyl}oxy)-1-ethyl-4-oxo-1,4-dihydro-3-quinolinecarboxylic acid]propionyl}-6-O-methylerythromycin A,
4"-O-{[6-({2-[(2-Aminoethyl)thio]ethyl}oxy)-1-ethyl-4-oxo-1,4-dihydro-3-quinolinecarboxylic acid]propionyl}-O-(9E)-methoxymethyloximino erythromycin A,
4"-O-{[6-({2-[(2-Aminoethyl)thio]ethyl}oxy)-1-ethyl-4-oxo-1,4-dihydro-3-quinolinecarboxylic acid]propionyl}-O-(9E)-hydroximino erythromycin A,
4"-O-{[1-Ethyl-6-(3-{[2-Aminoethyl]oxy}propyl)-4-oxo-1,4-dihydro-3-quinolinecarboxylic acid] ]propionyl}-O-(9E)-hydroximino erythromycin A, and
4"-O-{[1-Ethyl-6-(3-{[2-(methylamino)ethyl]oxy}propyl)-4-oxo-1,4-dihydro-3-quinolinecarboxylic acid]]propionyl}-O-(9E)-hydroximino erythromycin A,
or a salt, solvate or ester thereof.

Compounds according to the invention also exhibit a broad spectrum of antimicrobial activity, in particular antibacterial activity, against a wide range of clinical pathogenic microorganisms. Using a standard microtiter broth serial dilution test, compounds of the invention have been found to exhibit useful levels of activity against a wide range of pathogenic microorganisims. In particular, the compounds of the invention may be active against strains of Staphylococcus aureus, Streptopococcus pneumoniae, Moraxella catarrhalis, Streptococcus pyogenes, Haemophilus influenzae, Enterococcus faecalis, Chlamydia pneumoniae, Mycoplasma pneumoniae and Legionella pneumophila. The compounds of the invention may also be active against resistant strains, for example erythromycin resistant strains. In particular, the compounds of the invention may be active against erythromycin resistant strains of Streptococcus pneumoniae, Streptococcus pyogenes and Staphylococcus aureus.

The compounds of the invention may therefore be used for treating a variety of diseases caused by pathogenic microorganisms, in particular bacteria, in human beings and animals. It will be appreciated that reference to treatment includes acute treatment or prophylaxis as well as the alleviation of established symptoms.

Thus, according to another aspect of the present invention we provide a compound of formula (I) or a pharmaceutically acceptable salt, solvate or ester thereof for use in therapy.

According to a further aspect of the invention we provide a compound of formula (I) or a pharmaceutically acceptable salt, solvate or ester thereof for use in the therapy or prophylaxis of systemic or topical microbial infections in a human or animal subject.

According to a further aspect of the invention we provide the use of a compound of formula (I) or a pharmaceutically acceptable salt, solvate or ester thereof in the manufacture of a medicament for use in the treatment or prophylaxis of systemic or topical microbial infections in a human or animal body.

While it is possible that, for use in therapy, a compound of the invention may be administered as the raw chemical it is preferable to present the active ingredient as a pharmaceutical formulation eg when the agent is in admixture with a suitable pharmaceutical excipient, diluent or carrier selected with regard to the intended route of administration and standard pharmaceutical practice.

Accordingly, in one aspect, the present invention provides a pharmaceutical composition or formulation comprising at least one compound of the invention or a pharmaceutically acceptable salt, solvate or ester thereof in association with a pharmaceutically acceptable excipient, diluent and/or carrier. The excipient, diluent and/or carrier must be "acceptable" in the sense of being compatible with the other ingredients of the formulation and not deleterious to the recipient thereof.

In another aspect, the invention provides a pharmaceutical composition comprising, as active ingredient, at least one compound of the invention or a pharmaceutically acceptable salt, solvate or ester thereof in association with a pharmaceutically acceptable excipient, diluent and/or carrier for use in therapy, and in particular, in the treatment of human or animal subjects suffering from a condition susceptible to amelioration by an antimicrobial compound.

In another aspect, the invention provides a pharmaceutical composition comprising a therapeutically effective amount of the compounds of the present invention and a pharmaceutically acceptable excipient, diluent and/or carrier (including combinations thereof).

There is further provided by the present invention a process of preparing a pharmaceutical composition, which process comprises mixing at least one compound of the invention or a pharmaceutically acceptable salt, solvate or ester thereof, together with a pharmaceutically acceptable excipient, diluent and/or carrier.

The compounds of the invention may be formulated for administration in any convenient way for use in human or veterinary medicine and the invention therefore includes within its scope pharmaceutical compositions comprising a compound of the invention adapted for use in human or veterinary medicine. Such compositions may be presented for use in a conventional manner with the aid of one or more suitable excipients, diluents and/or carriers. Acceptable excipients, diluents and carriers for therapetic use are well known in the pharmaceutical art, and are described, for example, in Remington's Pharmaceutical Sciences, Mack Publishing Co. (A. R. Gennaro edit. 1985). The choice of pharmaceutical excipient, diluent and/or carrier can be selected with regard to the intended route of administration and standard pharmaceutical practice. The pharmaceutical compositions may comprise as - or in addition to - the excipient, diluent and/or carrier any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

For some embodiments, the agents of the present invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e. g. as a carrier, diluent or solubiliser. Alpha-, beta- and gamma-cyclodextrins are most commonly used and suitable examples are described in WO 91/11172, WO 94/02518 and WO 98/55148.

The compounds of the invention may be milled using known milling procedures such as wet milling to obtain a particle size appropriate for tablet formation and for other formulation types. Finely divided (nanoparticulate) preparations of the compounds of the invention may be prepared by processes known in the art, for example see International Patent Application No. WO 02/00196 (SmithKline Beecham).

The routes for administration (delivery) include, but are not limited to, one or more of: oral (e. g. as a tablet, capsule, or as an ingestable solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual.

There may be different composition/formulation requirements depending on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose; or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution, which may contain other substances, for example enough salts or monosaccharides to make the solution isotonic with blood. For buccal or sublingual administration the compositions may be administered in the form of tablets or lozenges, which can be formulated in a conventional manner.

It is to be understood that not all of the compounds need be administered by the same route. Likewise, if the composition comprises more than one active component, then those components may be administered by different routes.

The compositions of the invention include those in a form especially formulated for parenteral, oral, buccal, rectal, topical, implant, ophthalmic, nasal or genito-urinary use. For some applications, the agents of the present invention are delivered systemically (such as orally, buccally, sublingually), more preferably orally. Hence, preferably the agent is in a form that is suitable for oral delivery.

If the compound of the present invention is administered parenterally, then examples of such administration include one or more of intravenously, intraarterially, intraperitoneally, intrathecally, intraventricularly, intraurethrally, intrasternally, intracranially, intramuscularly or subcutaneously administering the agent; and/or by using infusion techniques.

For parenteral administration, the compound is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The compounds according to the invention may be formulated for use in human or veterinary medicine by injection (e.g. by intravenous bolus injection or infusion or via intramuscular, subcutaneous or intrathecal routes) and may be presented in unit dose form, in ampoules, or other unit-dose containers, or in multi-dose containers, if necessary with an added preservative. The compositions for injection may be in the form of suspensions, solutions, or emulsions, in oily or aqueous vehicles, and may contain formulatory agents such as suspending, stabilising, solubilising and/or dispersing agents. Alternatively the active ingredient may be in sterile powder form for reconstitution with a suitable vehicle, e.g. sterile, pyrogen-free water, before use.

The compounds of the invention can be administered (e. g. orally or topically) in the form of tablets, capsules, ovules, elixirs, solutions or suspensions, which may contain flavouring or colouring agents, for immediate-, delayed-, modified-, sustained-, pulsed-or controlled-release applications.

The compounds of the invention may also be presented for human or veterinary use in a form suitable for oral or buccal administration, for example in the form of solutions, gels, syrups, mouth washes or suspensions, or a dry powder for constitution with water or other suitable vehicle before use, optionally with flavouring and colouring agents. Solid compositions such as tablets, capsules, lozenges, pastilles, pills, boluses, powder, pastes, granules, bullets or premix preparations may also be used. Solid and liquid compositions for oral use may be prepared according to methods well known in the art. Such compositions may also contain one or more pharmaceutically acceptable carriers and excipients which may be in solid or liquid form.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia.

Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The compounds of the invention may also be administered orally in veterinary medicine in the form of a liquid drench such as a solution, suspension or dispersion of the active ingredient together with a pharmaceutically acceptable carrier or excipient.

The compounds of the invention may also, for example, be formulated as suppositories e.g. containing conventional suppository bases for use in human or veterinary medicine or as pessaries e.g. containing conventional pessary bases.

The compounds according to the invention may be formulated for topical administration, for use in human and veterinary medicine, in the form of ointments, creams, gels, hydrogels, lotions, solutions, shampoos, powders (including spray or dusting powders), pessaries, tampons, sprays, dips, aerosols, drops (e.g. eye ear or nose drops) or pour-ons.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water.

Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds may also be dermally or transdermally administered, for example, by use of a skin patch.

For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

As indicated, the compound of the present invention can be administered intranasally or by inhalation and is conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134AT"") or 1,1,1,2,3,3,3-heptafluoropropane (HFA 227EA), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e. g. using a mixture of ethanol and the propellant as the solvent, which may additionally contain a lubricant, e. g. sorbitan trioleate.

Capsules and cartridges (made, for example, from gelatin) for use in an inhaler or insufflator may be formulated to contain a powder mix of the compound and a suitable powder base such as lactose or starch.

For topical administration by inhalation the compounds according to the invention may be delivered for use in human or veterinary medicine via a nebuliser.

The compounds of the invention may also be used in combination with other therapeutic agents. The invention thus provides, in a further aspect, a combination comprising a compound of the invention or a pharmaceutically acceptable salt, solvate or ester thereof together with a further therapeutic agent.

When a compound of the invention or a pharmaceutically acceptable salt, solvate or ester thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian. The compounds of the present invention may for example be used for topical administration with other active ingredients such as corticosteroids or antifungals as appropriate.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations by any convenient route.

When administration is sequential, either the compound of the invention or the second therapeutic agent may be administered first. When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

The compositions may contain from 0.01-99% of the active material. For topical administration, for example, the composition will generally contain from 0.01-10%, more preferably 0.01-1% of the active material.

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

For oral and parenteral administration to humans, the daily dosage level of the agent may be in single or divided doses.

For systemic administration the daily dose as employed for adult human treatment it will range from 2-100mg/kg body weight, preferably 5-60mg/kg body weight, which may be administered in 1 to 4 daily doses, for example, depending on the route of administration and the condition of the patient. When the composition comprises dosage units, each unit will preferably contain 200mg to 1g of active ingredient. The duration of treatment will be dictated by the rate of response rather than by arbitrary numbers of days.

Compounds of general formula (I) and salts thereof may be prepared by the general methods outlined hereinafter, said methods constituting a further aspect of the invention. In the following description, the groups R¹ to R³³, A, B, D, E, X, Y, U, W, a, b, d, e, f, g, h, i, j, k, m, n, p, q, r, s, t, v and z have the meaning defined for the compounds of formula (1) unless otherwise stated.

The group X^{a}R^{11a} is XR¹¹ as defined for formula (I) or a group convertible to XR¹¹. Conversion of a group X^{a}R^{11a} to a XR¹¹ group typically arises if a protecting group is needed during the reactions described below. A comprehensive discussion of the ways in which such groups may be protected and methods for cleaving the resulting protected derivatives is given by for example T.W. Greene and P.G.M Wuts in Protective Groups in Organic Synthesis 2nd ed., John Wiley & Son, Inc 1991 and by P.J. Kocienski in Protecting Groups, Georg Thieme Verlag 1994. Examples of suitable amino protecting groups include acyl type protecting groups (e.g. formyl, trifluoroacetyl and acetyl), aromatic urethane type protecting groups (e.g. benzyloxycarbonyl (Cbz) and substituted Cbz, and 9-fluorenylmethoxycarbonyl (Fmoc)), aliphatic urethane protecting groups (e.g. t-butyloxycarbonyl (Boc), isopropyloxycarbonyl and cyclohexyloxycarbonyl) and alkyl type protecting groups (e.g. benzyl, trityl and chlorotrityl). Examples of suitable oxygen protecting groups may include for example alkyl silyl groups, such as trimethylsilyl or tertbutyldimethylsilyl; alkyl ethers such as tetrahydropyranyl or tert-butyl; or esters such as acetate. Hydroxyl groups may be protected by reaction of for example acetic anhydride, benzoic anhydride or a trialkylsilyl chloride in an aprotic solvent. Examples of aprotic solvents are dichloromethane, N,N-dimethylformamide, dimethylsulfoxide, tetrahydrofuran and the like.

Compounds of formula (I) wherein d is an integer from 1 to 5, may be prepared by reaction of a 4" hydroxy compound of formula (II) wherein R² is a hydroxyl protecting group with a suitable activated and protected derivative of the carboxylic acid (III), followed where necessary by subsequent removal of the hydroxyl protecting group R² and conversion of the XaR^{11a} group to XR¹¹.

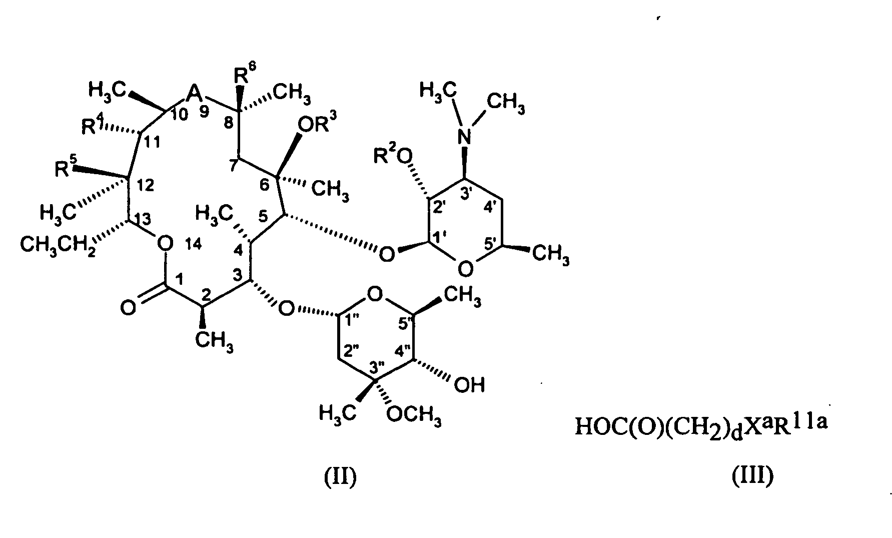

Suitable activated derivatives of the carboxyl group include the corresponding acyl halide, mixed anhydride or activated ester such as a thioester. The reaction is preferably carried out in a suitable aprotic solvent such as a halohydrocarbon (e.g. dichloromethane) or N,N-dimethylformamide optionally in the presence of a tertiary organic base such as dimethylaminopyridine or triethylamine or in the presence of inorganic base (eg sodium hydroxide) and at a temperature within the range of 0° to 120°C. The compounds of formula (II) and (III) may also be reacted in the presence of a carbodiimide such as dicyclohexylcarbodiimide (DCC).

In a further embodiment of the invention, compounds of formula (I) wherein d is an integer from 1 to 5 and U is -N(R³⁰)-, may be prepared by reaction of compounds of formula (V), 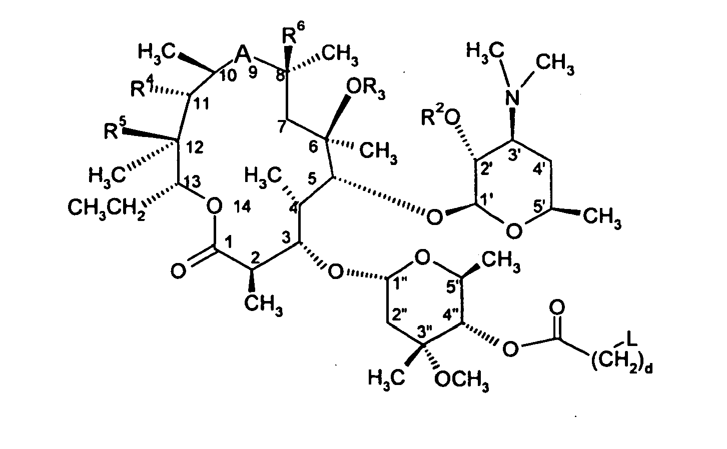 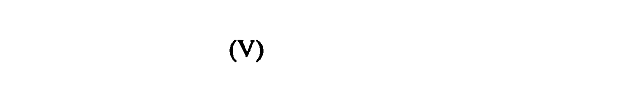 wherein d is an integer from 1 to 5 and L is a suitable leaving group, with X^{a}R^{11a} (IV) in which U is -N(R³⁰)-. The reaction is preferably carried out in a solvent such as a halohydrocarbon (e.g. dichloromethane), an ether (e.g. tetrahydrofuran or dimethoxyethane), acetonitrile or ethyl acetate and the like, dimethylsulfoxide, N,N-dimethylformamide or 1-methyl-pyrrolidone and in the presence of a base, followed, if desired, by removal of the hydroxyl protecting group R² and conversion of the X^{a}R^{11a} group to XR¹¹. Examples of the bases which may be used include organic bases such as diisopropylethylamine, triethylamine and 1,8-diazabicyclo[5.4.0]undec-7-ene, and inorganic bases such as potassium hydroxide, cesium hydroxide, tetraalkylammonium hydroxide, sodium hydride, potassium hydride and the like. Suitable leaving groups for this reaction include halides (e.g. chloride, bromide or iodide) and sulfonyloxy groups (e.g. tosyloxy or methanesulfonyloxy).

Compounds of formula (V) may be prepared by reaction of a compound of formula (II), wherein R² is a hydroxyl protecting group, with a suitable activated derivative of the carboxylic acid HOC(O)(CH₂)_{d}L (VI), wherein L is a suitable leaving group as above defined. Suitable activated derivatives of the carboxyl group are those defined above for carboxylic acid (III). The reaction is carried out using the conditions described above for the reaction of a compound of formula (II) with carboxylic acid (III).

In a preferred embodiment of the invention, compounds of formula (I) wherein d is 2 and U is -N(R³⁰)-, may be prepared by Michael reaction of a compound of formula (VII) wherein R² is optionally a hydroxyl protecting group 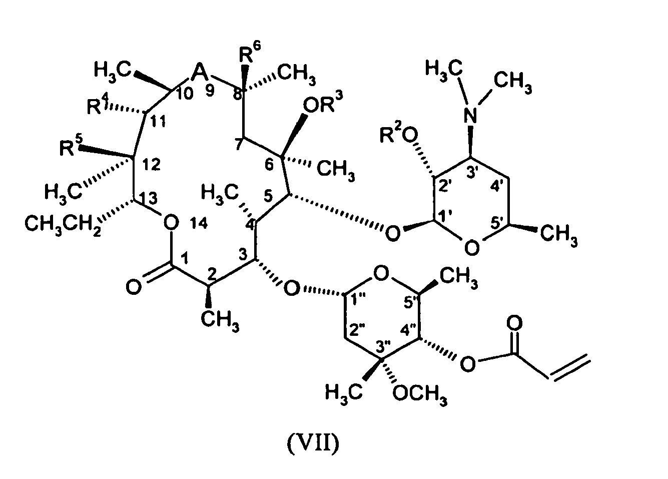 with a compound of formula X^{a}R^{11a} (IV). The reaction is suitably carried out in a solvent such as dimethylsulfoxide, N,N-dimethylformamide, 1-methyl-pyrrolidone, a halohydrocarbon (e.g. dichloromethane), an ether (e.g. tetrahydrofuran or dimethoxyethane), acetonitrile or alcohol (e.g methanol or isopropanol) and the like, and in the presence of a base, followed, if desired, by removal of hydroxyl protecting group R² and conversion of the X^{a}R^{11a} group to XR¹¹.

Compound of formula R^{11a}L (VIII), wherein L is a suitable leaving group such as chlorine, fluorine or bromine, and R³¹ and R¹⁹ are linked to form the bivalent radical -O(CH₂)₂-, -(CH₂)ₜ-;-NR⁷(CH₂)ₐ-, -OCH₂NR⁷-, -SCH₂NR⁷-, -CH₂NR⁷CH₂-, -CH₂OCH₂-, -CH₂SCH₂- or -(CH₂)ₐNR⁷- are known compounds or they may be prepared by analogous methods to those known in the art. Thus, they can be prepared according to the procedures described in US 2002/0025959 A1.

Compounds of formula (III) wherein X is -U(CH₂)ᵥB(CH₂)ᵥD-, -U(CH₂)ᵥB-R³³-, or X is a group selected from: 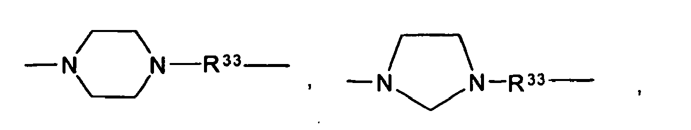 and 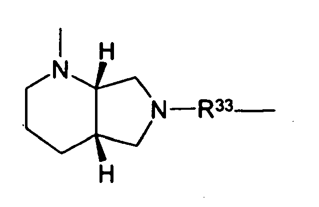 may be prepared by reaction of X^{a}R^{11a} (V), wherein X has the meaning defined above with R³⁴OC(O)CH=CH₂ (VII) wherein R³⁴ is carboxyl protecting group, followed by removal of R³⁴. Suitable R³⁴ carboxyl protecting group include t-butyl, allyl or benzyl.

Compounds of formula (III) may also be prepared by reaction of X^{a}R^{11a} (V) with acrylonitrile followed by hydrolysis of the nitrile to the acid.

Compounds of formula (IV) wherein X is - U(CH₂)ᵥB(CH₂)ᵥD- in which D is -N(R³⁰)-, -O- or -S-, or wherein X is -U(CH₂)ᵥB(CH₂)ᵥD(CH₂)ᵥE- in which E is -N(R³⁰)-, -O- or -S- or X is a group selected from: 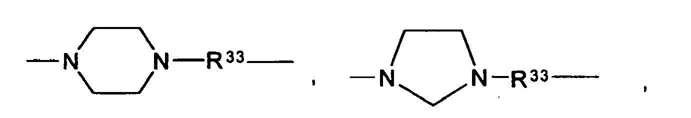 and 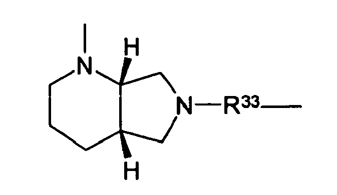 may be prepared by reaction of a compound of formula R^{11a}L (VIII), wherein L is a suitable leaving group such as chlorine, fluorine or bromine, with a compound of formula -U(CH₂)ᵥB(CH₂)D- (IX) in which D is -N(R³⁰)-, -O- or -S-, or with compound of formula -U(CH₂)ᵥB(CH₂)ᵥD(CH₂)ᵥE- (X) in which E is -N(R³⁰)-, -O- or -S- or with piperazine or with imidazolidin or with 1H-octahydro-pyrrolo[3,4-*b*]pyridine.

Compound of formula R^{11a}L (VIII), wherein L is a suitable leaving group such as chlorine, fluorine or bromine, and R³² and R¹⁹ are linked to form the bivalent radical selected from the group -S(CH₂)_{b}-, -N(R⁷)(CH₂)_{b}- or -O(CH₂)_{b}- are known compounds or they may be prepared by analogous methods to those known in the art. Thus, they can be prepared according to the procedures described in Arch. Pharm. Pharm. Med Chem. 1997, 330, 63.

Compounds of formula (I) may be converted into other compounds of formula (I). Thus compounds of formula (I) wherein B is -S(O)_{z}- and z is 1 or 2 may be prepared by oxidation of the corresponding compound of formula (I) wherein z is 0. The oxidation is preferably carried out using a peracid, e.g. peroxybenzoic acid, followed by treatment with a phosphine, such as triphenylphosphine. The reaction is suitably carried out in an organic solvent such as methylene chloride. Compounds of formula (I) wherein U or B is -N(R³⁰)- and R³⁰ is C₁₋₄alkyl can be prepared from compounds wherein R³⁰ is hydrogen by reductive alkylation.

Compounds of formula (II) wherein A is -C(O)NH- or -NHC(O)-, R⁴ or R⁵ are hydroxy, R³ is hydrogen and R⁶ is hydrogen are known compounds or they may be prepared by analogous methods to those known in the art. Thus they can be prepared according to the procedures described in EP 507595 and EP 503932.

Compounds of formula (II), wherein A is -C(O)NH- or -NHC(O)-, R⁴ or R⁵ are hydroxyl and R³ is C₁₋₄alkyl or C₃₋₆alkenyl optionally substituted by 9 to 10 membered fused bicyclic heteroaryl and R⁶ is hydrogen are known compounds or they may be prepared by analogous methods to those known in the art. Thus they can be prepared according to the procedures described in WO 9951616 and WO 0063223.

Compounds of formula (II), wherein A is -C(O)NH-, R⁴ and R⁵ taken together with the intervening atoms form a cyclic group having the following structure:

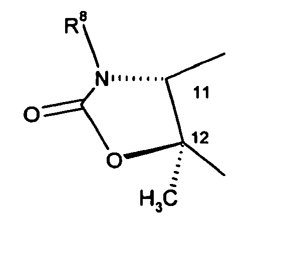

R³ is C₁₋₄alkyl, or C₃₋₆alkenyl optionally substituted by 9 to 10 membered fused bicyclic heteroaryl and R⁶ is hydrogen are known compounds or they may be prepared by analogous methods to those known in the art. Thus they can be prepared according to the procedures described in US 6262030.

Compounds of formula (II), wherein A is -C(O)-, -C(O)NH-, -NHC(O)-, -N(R⁷)-CH₂-, -CH₂-N(R⁷)- or -CH(NR⁸R⁹)-, R⁴ or R⁵ are hydroxyl or R⁴ and R⁵ taken together with the intervening atoms form a cyclic group having the following structure: 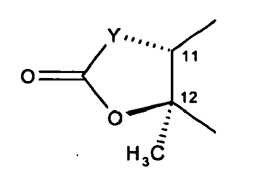 wherein Y is a bivalent radical selected from -O- and -N(R¹³)-, and R³ is C₁₋₄alkyl, or C₃₋₆alkenyl optionally substituted by 9 to 10 membered fused bicyclic heteroaryl are known compounds or they may be prepared by analogous methods to those known in the art. Thus they can be prepared according to the procedures described in EP 307177, EP 248279, WO 0078773, WO 9742204.

Compounds of formula (II), wherein A is -C(O)NH-, -NHC(O)-, -N(CH₃)-CH₂- or -CH₂-N(CH₃)-, R⁴ or R⁵ are hydroxyl or R⁴ and R⁵ taken together with the intervening atoms form a cyclic group having the following structure: 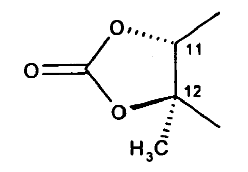 and R⁶ is hydrogen are known compounds or they may be prepared by analogous methods to those known in the art. Thus they can be prepared according to the procedures described in EP 508699 and J. Chem. Res. Synop., 1988, pages 152-153, US Patent 6262030.

Compounds of formula (II), wherein A is -C(=NR¹⁰)-, R⁴ or R⁵ are hydroxyl or R⁴ and R⁵ taken together with the intervening atoms form a cyclic group having the following structure: 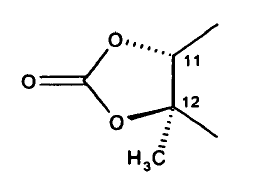 and R⁶ is hydrogen, are known compounds or they may be prepared by analogous methods to those known in the art. Thus they can be prepared according to the procedures described in EP 284203.

Compounds of formula (II), wherein A is -C(O)-, R⁴ and R⁵ taken together with the intervening atoms form a cyclic group having the following structure:

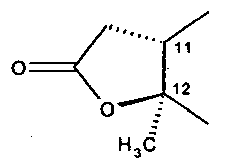

R⁶ is hydrogen and R³ is C₁₋₄ alkyl may be prepared by decarboxylation of a compound of formula (XI), wherein R³⁵ is hydroxyl protecting group followed, if required, by removal of the protecting group R² or R³⁵.

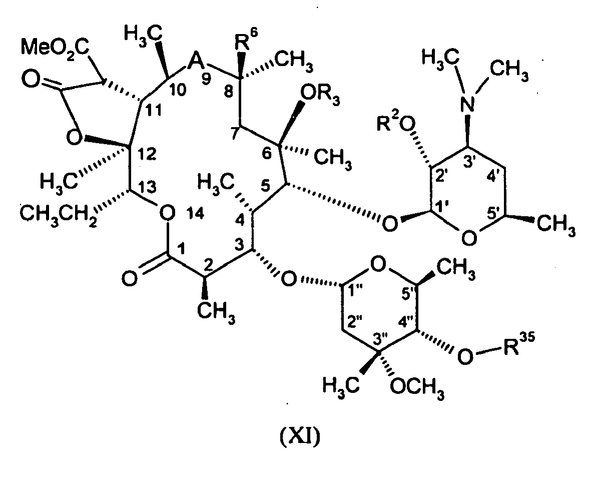

The decarboxylation may be carried out in the presence of a lithium salt such as lithium chloride, preferably in an organic solvent such as dimethylsulfoxide.

Compounds of formula (II), wherein A is -C(O)-, R⁴ and R⁵ taken together with the intervening atoms form a cyclic group having the following structure: 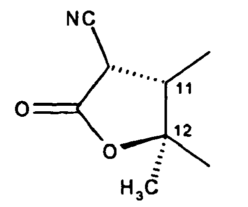 and R³ is C₁₋₄ alkyl may be prepared according to the procedures described in WO 02/50091 and WO 02/50092.

The following abbreviations are used in the text: DBU for 1,8-diazabicyclo[5.4.0]undec-7-ene, DCM for dichloromethane, DMAP for 4-dimethylaminopyridine, DMF for N,N-dimethylformamide, DMSO for dimethyl sulfoxide, EDAC.HCl for 1-(3-dimethylaminopropyl)-3-ethylcarbodiimide hydrochloride, EtOAc for ethyl acetate, KO-t-Bu for potassium *tert*-butoxide, MeOH for methanol, TEA for triethylamine and THF for tetrahydrofuran, MIBK for methyl iso-buthyl ketone.

In order that the invention may be more fully understood the following examples are given by way of illustration only.

### Examples

2'-O-Acetyl-6-O methyl-erythromycin A may be prepared by the procedure described by W. R. Baker et al. in J. Org. Chem. 1988, 53, 2340, 2'-*O*-acetyl-azithromycin and 2'-*O*-acetyl-azithromycin-11,12-carbonate may be prepared by the procedures described by S. Djokic et al. in J. Chem. Res. (S) 1988, 152 and 11-*O*-methyl-azithromycin may be prepared by the procedure described by G.Kobrehel et al. in J. Antibiotics 1992, 45,527-532. 9(*E*)-Ethoxyimino-erythromycin A may be prepared by the procedures described in EP 1 167 375. 6-*O*-Ethyl erythromycin A, 6-*O*-propyl erythromycin A and 9-(1-isopropoxycyclohexyl)oximino-erythromycin A may be prepared by procedure described in US Patent 4,990,602 and Bioorg.Med.Chem.Lett. 2000, 10, 815-819. 6-O-Methyl-8a-aza-8a-homoerythromycin A may be prepared by procedure described in US patent_6,110,965. 8,9-Anhydro-9-deoxo-erythromycin A 6,9-cyclic ether may be prepared by procedure described in Experientia 1971, 27, 362. 2'-*O*-Acetyl-*O*-(9*E*)-acetylhydroximino erythromycin A may be prepared by procedure described in WO2004/039822. 4"-O-Propenoyl-azithromycin may be prepared by procedure described in WO03/042228. 7-[2-(2-Carboxy-ethoxy)-ethylamino]-6-fluoro-1-cyclopropyl-8-methoxy-4-oxo-1,4-dihydro-quinoline-3-carboxylic acid my be prepared according to the procedure described in WO2004/101585. 1-Cyclopropyl-6-iodo-4-oxo-1,4-dihydro-qunoline-3-carboxylic acid ethyl ester, 7-chloro-1-isopropyl-6-fluoro-4-oxo-1,4-dihydro-quinoline-3-carboxylic acid and 7-chloro-1-tert-butyl-6-fluoro-4-oxo-1,4-dihydro-quinoline-3-carboxylic acid may be prepared by the procedure described in J.Med.Chem., 1995, 38, 973. 1-Ethyl-6-iodo-4-oxo-1,4-dihydro-qunoline-3-carboxylic acid ethyl ester my be prepared by procedure described in Aust. J. Chem., 1973, 26, 907. Ethyl 1-ethyl-6-hydroxy-4-oxo-1,4-dihydro-3-quinolinecarboxylate may be prepared by the procedure described in GB 1433774.

### Intermediate 1:

### 7-{2-[2-(2-Carboxy-ethoxy)-ethoxy]-ethylamino}-1,2,3,6-tetrahydro-6-oxo-[1,3]-oxazino-[3,2a]-quinoline-5-carboxylic acid

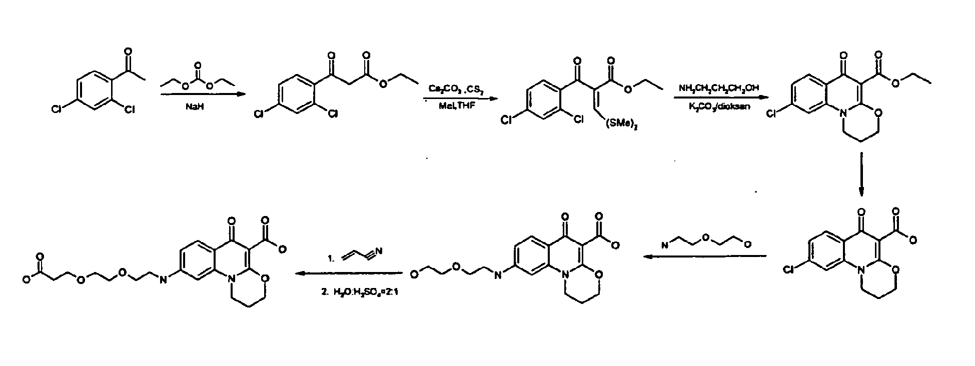

### a) 3-(2,4-Dichlorophenyl)-3-oxo-propionic acid ethyl ester

Synthesis of **Intermediate 1a** was done by standard procedure starting from 2,4-dichloroacetophenone, diethylcarbonate (25 eq ) and NaH (2 eq ) at 80°C for 60 minutes. MS (ES+) m/z: [MH]⁺ = 262

### b) 2-[Bis(methylthio)methylene]-3-(2,4-dichlorophenyl)-3-oxo-propionic acid ethyl ester

To a mixture of **Intermediate 1a** (15.7 g) and Cs₂CO₃ (2.5 eq) in THF (230 mL) was added CS₂ (4.6 eq) with stirring at -10°C. After 5 minutes CH₃I (2.5 eq) was added in one portion and reaction was stirred at room temperature overnight. The reaction was diluted with ether (50 mL) and filtered. Filtrate was concentrated in vacuo.
MS (ES+) m/z: [MH]⁺ = 366

### c) 7-Chloro-1,2,3,6-tetrahydro-6-oxo-[1,3]oxazino[3,2a]quinoline-5-carboxylic acid ethyl ester

A mixture of **Intermediate 1b** (18.08 g), 3-amino-1-propanole (1.2 eq) and K₂CO₃ (2.4 eq) in dioxane (500 mL) was stirred at room temperature for 1 hour and refluxed overnight. The reaction mixture was filtrated and filtrate was concentrated to dryness under reduced pressure. The crude product was precipitated from MeOH affording the title compound (2.6 g).
MS (ES+) m/z: [MH]⁺ = 308

### d) 7-Chloro-1,2,3,6-tetrahydro-6-oxo-[1,3]oxazino[3,2a]quinoline-5-carboxylic acid

To a solution of **Intermediate 1c** (1.4 g) in THF (15 mL) solution of NaOH (4.6 eq ) in water (15 mL) was added and the reaction mixture was stirred at 80°C overnight. THF was evaporated, HCl (0.6 M) was added to reach pH value about 4 and extracted with 3x10 mL of DCM. The organic layers were washed with brine, dried over Na₂SO₄, filtered and DCM was evaporated under reduced pressure affording the title compound (1.16 g).
MS (ES+) m/z : [MH]⁺ = 280

### e) 7-[2-(2-Hydroxy-ethoxy)-ethylamino]-1,2,3,6-tetrahydro-6-oxo-[1,3]oxazino[3,2a]-quinoline-5-carboxylic acid

**Intermediate 1d** (1 g) was dilluted in 5 mL of methyl-pyrrolidone, 1.8 mL (5 eq) of 2-(2-aminoetoxy)ethanol was added and stirred at 110°C for 24 hours. To the reaction mixture was added EtOAc, pH adjusted to 6 and extracted with 3x15 mL of H₂O The organic layers were washed with brine, dried over Na₂SO₄, filtered and EtOAc was evaporated under reduced pressure affording the title compound (600 mg).
MS (ES+) m/z : [MH]⁺ = 349

### f) 7-{2-[2-(2-Carboxy-ethoxy)-ethoxy]-ethylamino}-1,2,3,6-tetrahydro-6-oxo-[1,3]-oxazino-[3,2a]-quinoline-5-carboxylic acid

**Intermediate 1e** (600 mg) was diluted in 7.4 mL of C₃H₃N, 0.515 mL of DBU was added and the mixture stirred at 80°C for 24 hours. C₃H₃N was evaporated under reduced pressure, residue dissolved in EtOAc, pH was adjusted to 3 and extracted with 3x15 mL of H₂O. EtOAc was evaporated under reduced pressure affording 650 mg of cyano derivative. The cyano derivative was dissolved in 40 mL of H₂O/H₂SO₄ (2:1) and stirred for 24 h at 75°C affording the title compound.
MS (ES+) m/z : [MH]⁺ = 421

### Intermediate 2

### 1-Cyclopropyl-6-fluoro-7-chloro-4-oxo-1,4-dihvdro-quinoline-3-(2-nitroacetyl)

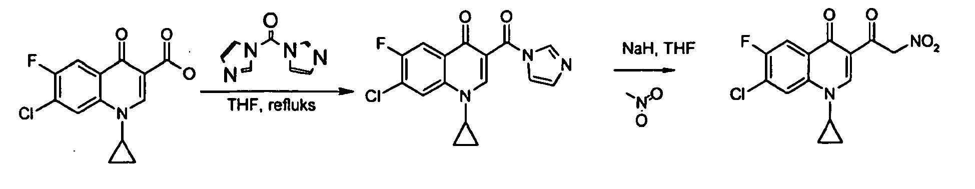

A mixture of 7-chloro-1-cyclopropyl-6-fluoro-4-oxo-1,4-dihydro-quinoline-3-carboxylic acid (1 g, 3,55 mmol) and of 1,1-carbonyldiimidazole (2.88 g, 17.75 mmol) in 15 ml CCl₃ was heated to reflux over the night. the mixture was cooled and the solvent was removed under reduced pressure. To the resudue a small amount of diethyl ether was added and the resulting solid was collected by filtration and washed with diethyl ether to give a imidazolide intermediate in a quantitative yield.
To the mixture of NaH (0.26 g, 0.0108 mol, 60 % disperse oil) and of nitromethane (0,58 mL 0.0108 mol) in 20 mL of anhydrous THF a solution of imidazolide intermediate (0.9 g, 0.289 mmol) in 20 mL of anhydrous THF was added dropwise and heated to reflux for 18 h. The mixture was cooled and 20 mL of H₂O was slowly added and neutralized by HCl, and then extracted with CH₂Cl₂. The organic layer was washed with H₂O and brine, dried by anhydrous Na₂SO₄ and evaporated. The product was precipitated and filtrated off yielding 0.4g of title compound. (90.6 % pure compound according to LC-MS).
MS (ES+) m/z: [MH]⁺ = 325.1

### Intermediate 3

### 1-Cyclopropyl-6,7-difluoro-8-methoxy-4-oxo-1,4-dihydro-quinoline-3-(2-nitroacetyl)

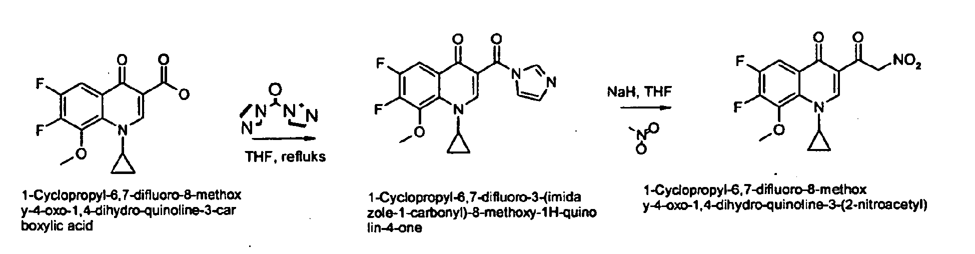

A mixture of 1-cyclopropyl-6,7-difluoro-8-methoxy-4-oxo-1,4-dihydro-quinoline-3-carboxylic acid (1 g, 3.38 mmol) and 1,1-carbonyldiimidazole (2.19 g, 13.54 mmol) in 15 mL CCl₃ was heated to reflux over the night. The mixture was cooled and the solvent was removed under reduced pressure. To the resudue a small amount of diethyl ether was added and the resulting solid was collected by filtration and washed with diethyl ether to give a imidazolide intermediate in a quantitative yield.
To the mixture of NaH (0.28 g, 0.0116 mmol, 60 % disperse oil) and nitromethane (0.62 mL, 0.01158 mol) in 20 mL of anhydrous THF a solution of imidazolide intermediate (1 g, 2.89 mmol) in 20 mL of anhydrous THF was added dropwise and heated to reflux for 18 h. The mixture was cooled and 20 mL of H₂O was slowly added and neutralized by HCl, and then extracted with CH₂Cl₂. The organic layer was washed with H₂O and brine, dried by anhydrous Na₂SO₄ and evaporated. The product was precipitated and filtrated off yielding 0.56g of title product. (93.46 % pure compound according to LC-MS).
MS (ES+) m/z : [MH]⁺ = 339.1

### Intermediate 4

### 7-[2-(2-Cyano-ethoxy)-ethylamino]-1-cyclopropyl-6-fluoro-8-methoxy-4-oxo-1,4-dihydro-quinoline-3-(2-nitroacetyl)

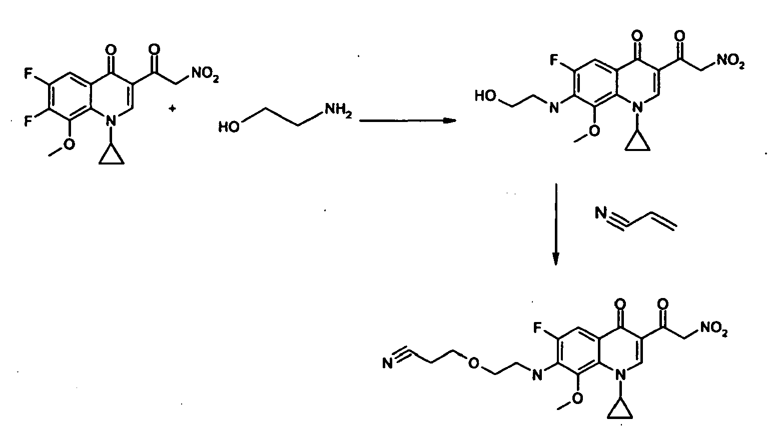

To a solution of **Intermediate 3** (250 mg) in DMSO (15 mL) ethanolamine (0.425 ml) was added and the reaction mixture was stirred at 90°C for 1.5 hours. pH Value of mixture was adjusted to 4.5 and product was precipitated. After filtration, 190mg of 1-cyclopropyl-6-fluoro-7-(2-hydroxy-ethylamino)-8-methoxy-4-oxo-1,4-dihydro-quinoline-3-(2-nitroacetyl) was obtained. A solution of 1-cyclopropyl-6-fluoro-7-(2-hydroxy-ethylamino)-8-methoxy-4-oxo-1,4-dihydro-quinoline-3-(2-nitroacetyl) (180 mg) in acrylonitrile and DBU was stirred at 80°C under N₂ for 5 hours. CH₃CN was evaporated under reduced pressure yielding oily title product.

### Intermediate 5

### 6-[3-Piperazin-1-yl)-propyl]-1-ethyl-4-oxo-1,4-dihydro-quinoline-3-carboxylic acid ethyl ester

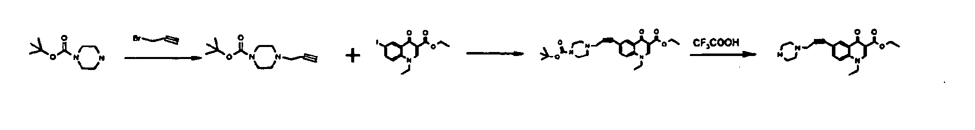

### a) 4-Prop-2-ynyl-piperazine-1-carboxylic acid tert-butyl ester

To the degassed solution of piperazine-1-carboxylic acid tert-butiyl ester (1.0 g, 5.37 mmol) in acetonitrile (10 mL) were added Na₂CO₃ (1.708 g, 16.11 mmol) and mixture was stirred for 20 min. The suspension was heated to 50 °C and 3-bromo-propyne (0.9 mL, 8.055 mmol) was added. The solvent was evaporated and the residue was extracted with EtAc and water (2x50 mL). Organic layer was washed with NaCl and NaHC03 (2x50 mL). The organic layer was dried over K₂CO₃ and evaporated in vacuum yielding (0.70 g) oil title intermediate.
MS (ES+) m/z : [MH]⁺ = 225.1

### b) 6-[3-(4-tert-Butoxycarbinyl-piperazin-1-yl)-prop-1-ynyl]-1-ethyl-4-oxo-1,4-dihydroquinoline-3-carboxylic acid ethyl ester

1-Ethyl-6-iodo-4-oxo-1,4-dihydro-qunoline-3-carboxylic acid ethyl ester (0.7g, 3.125 mmol), copper (I) iodide (42.47 mg, 0.223 mmol) and triethylamine (10.809 mL, 78.05 mmol) were suspended in dry acetonitrile (20 mL). The suspension was heated to 50 °C and N₂ bubbled through. After 20 min, dichlorobis (triphenylposphine) palladium (II) (46.96 mg, 0.0669 mmol) and **Intermediate 5a** (0.7 g 3.125 mmol) were added and dark red suspension was heated at 50 °C for 3 hours. The solvent was evaporated and the residue was extracted with EtOAc and water (2x50 mL). Organic layer was washed with NaCl and NaHCO₃ (2x50 mL), dried over K₂CO₃ and evaporated in vacuum yielding (1,24 g) oil red title product.
MS (ES+) m/z : [MH]⁺ = 468.3

### c) 6-[3-Piperazin-1-yl)-propyl]-1-ethyl-4-oxo-1,4-dihydro-quinoline-3-carboxylic acid ethyl ester

To the solution of **Intermediate 5b** (1.2 g, 2.57 mmol) in DCM (1.2 mL) was added CF₃COOH (1.2 mL) and mixture was stirred at room temp. for 48 h. To the reaction mixture was added water (pH=1.2) and layers were separated (pH=9.6). The organic layer was dried over K₂CO₃ and evaporated in vacuum yielding (1.7 g) oil red title product.
MS (ES+) m/z : [MH]⁺ = 368.3

### Intermediate 6

### 1-Cyclopropyl-6-fluoro-7-[2-(2-hydroxy-ethoxy)-ethylamino]4-oxo-1,4-dihydroquinoline-3-carboxylic acid (A) and

### 7-Chloro-1-cyclopropyl-6-[2-(2-hydroxy-ethoxy)-ethyl amino]-4-oxo-1,4-dihydro-quinoline-3-carboxylic acid (B)

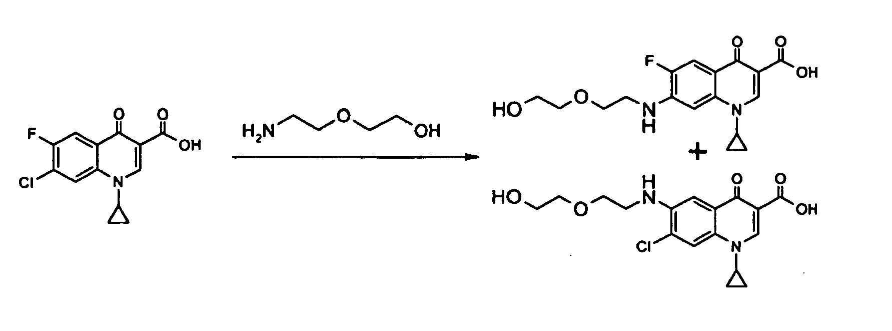

To a mixture of 7-chloro-1-cyclopropyl-6-fluoro-4-oxo-1,4-dihydro-quinoline-3-carboxylic acid (10g, 0.035 mol) in 1-methyl-2-pirolidone (70 mL) 2-(2-amino-ethoxy)-ethanol (18 mL, 0.18 mol, 5 eq.) was added, the reaction mixture was stirred at 110°C for 24 hours.Then was diluted with water (200 mL) and CH₂Cl₂ (60 mL) and pH was adjusted to 10. The aqueous layer was extracted with CH₂Cl₂ (5x50 mL) and then pH was adjusted to 6.7. After 10 minutes first product precipitated. Filtrated off yielding 2.7g of crude 7-chloro-1-cyclopropyl-6-[2-(2-hydroxy-ethoxy)-ethylamino]-4-oxo-1,4-dihydro-quinoline-3-carboxylic acid. (according to LC-MS 100 % pure **Intermediate 6B**) Over night second product precipitated. Filtrated off yielding 7.7g of yellow product (according to LC-MS a mixture of **Intermediate 6A** and **Intermediate 6B** in a 1:1 ratio).

### Intermediate 7

### 6-{2-[2-(2-carboxy-ethoxy)ethoxy]ethylamino}-1-cyclouropyl-7-chloro-4-oxo-1,4-dihydro-quinoline-3-carboxylic acid

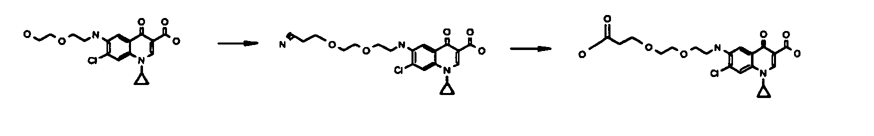

**Intermediate 6B** (2 g, 5.45 mmol) was dilluted in 25 mL of acrylonitrile, DBU (2.0 mL) was added and stirred at 80°C for 24 hours. Acrylonitrile was evaporated under reduced presure, residue was dissolved in DCM, pH was adjusted to pH 3 and extracted with 3x20 mL H₂O. The organic layers were washed with brine, dried over Na₂SO₄, filtered and DCM was evaporated under reduced pressure affording 1.9 g of 6-{2-[2-(2-cyanoethoxy)ethoxy]ethylamino}-1-cyclopropyl-7-chloro-4-oxo-1,4-dihydro-quinoline-3-carboxylic acid. This product was dissolved in 40 mL of mixture H₂O/H₂SO₄ (1:1) and stirred for 24 hours at 75°C. The obtained precipitate was filtered and dried under reduced pressure for 1h affording 1.7g of title product.

### Intermediate 8

### 1-Cyclopropyl-6-fluoro-7-chloro-4-oxo-1,4-dihydro-3-[(2-methanesulfonyl)acetyl]-quinoline.

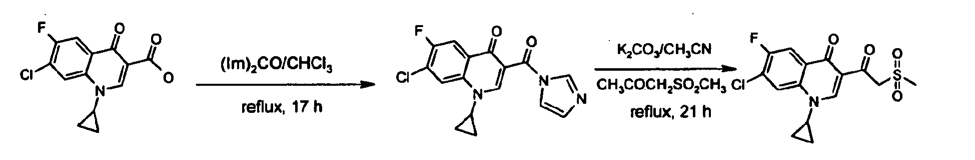

A mixture of 1-cyclopropyl-6-fluoro-7-chloro-4-oxo-1,4-dihydro-quinoline-3-carboxylic acid (2 g, 0.0071 mol) and 1,1'-carbonyldiimidazole (5.76 g, 0.035 mol) in 15 mL CHCl₃ was heated to reflux for 17 hours. The solvent was removed by reduced pressure. To the residue ether was added and then stirred at room temperature for 30 min. The solid was filtered and dried affording 1.64 g of 3-imidazolide derivative. Imidazolide derivative (1 g, 0.003mol) was dissolved in 40 mL acetonitrile, then methanesulphonylacetone (2 g, 0.015 mol) and K₂CO₃ were added and the mixture was heated to reflux for 21 hours. The solvent was removed under reduced pressure and 120 mL of H₂O was added. The solution was acidified by 2N HCl (pH ∼ 3) and extracted with EtOAc. The organic layer was dried and concentrated to give a crude solid product. The crude product was purified by column chromatography (DCM-EtOH-NH₄OH = 90:9:1.5) to give pure product 1-cyclopropyl-6-fluoro-7-chloro-4-oxo-1,4-dihydro-3-[(2-methanesulfonyl)acetyl]-quinoline.
MS (ES+) m/z : [MH]⁺ = 358.1
¹H NMR (500 MHz, DMSO) δ 8.58, 8.37, 8.13, 5.22, 3.78, 3.13, 1.31 and 1.16.

### Intermediate 9

### 6-[3-(2-Amino-ethoxy)-prop-1-ynyl]-1-ethyl-4-oxo-1,4-dihydro-quinoline-3-carboxylic acid trifluoroacetate salt

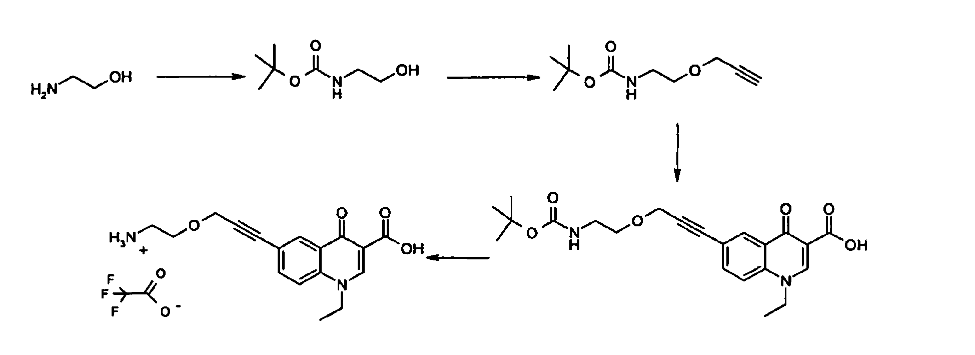

### a) (2-Hydroxy-ethyl)-carbamic acid tert-butyl ester

To a stirring solution of ethanolamine (1.96 mL, 32.7 mmol) in dioxane (40 mL) and water (20 mL) saturated solution of NaHCO₃ (20 mL) was added. The solution was cooled in ice bath and di-*t*-butyl dicarbonate (8.0 g) was added portionwise. After 1 hour TLC showed no starting material. EtOAc (50 mL) and water (20 mL) were added, organic layer was separated and evaporated yielding 4.20 g of the oily title compound.

### b) (2-Prop-2-ynyloxy-ethyl)-carbamic acid tert-butyl ester

To a stirring solution of **Intermediate 9a** (1.16 g) in THF (30 mL) at room temperature *t*-butylammonium iodide (0.15 g), sodium iodide (0.15 g) and propargyl bromide (80 % in toluene, 1.20 mL) were added. KOH (0.40 g) was added portionwise during 30 minutes and the suspension was stirred at room temperature for 24 hours. The solvent was evaporated, EtOAc (30 mL) and water (30 mL) were added, organic layer was washed with 10 % Na₂S₂O₅ solution and evaporated yielding 1.21 g of the title compound.

### c) 6-[3-(2-tert-Butoxycarbonylamino-ethoxy)-prop-1-ynyl]-1-ethyl-4-oxo-1,4-dihydro-quinoline-3-carboxylic acid

CuI (55 mg) and triethylamine (14.06 mL) were added into a solution of 1-ethyl-6-iodo-4-oxo-1,4-dihydro-quinoline-3-carboxylic acid (1.0 g) in MeCN (20 mL). The mixture has been stirring at room temperature for 20 minutes. Pd(PPh3)₂Cl₂ (61 mg) and **Intermediate 9b** (0.70 g) were added and the mixture has been stirring at 50 °C for 4 hours. The solvents were evaporated, EtOAc (30 mL) and water (30 mL) were added, organic layer was washed with water (30 mL) and brine (30 ml) and evaporated yielding 1.0 g of the title compound.
MS (ES+) m/z: [MH]⁺ = 415.24

### d) 6-[3-(2-Amino-ethoxy)-prop-1-ynyl]-1-ethyl-4-oxo-1,4-dihydro-quinoline-3-carboxylic acid trifluoroacetate salt

Trifluoroacetic acid (0.386 mL) was added into solution of **Intermediate 9c** (0.42 g) in MeCN (5 mL) at room temperature. The solution has been stirring at room temperature for 48 hours and evaporated yielding 0.80 g of the title compound.

### Intermediate 10

### 9-(2-hydroxy-ethylamino)-1-oxo-6,7-dihydro-1H,5H-pyrido[3,2,1-ij]quinoline-2-carboxylic acid

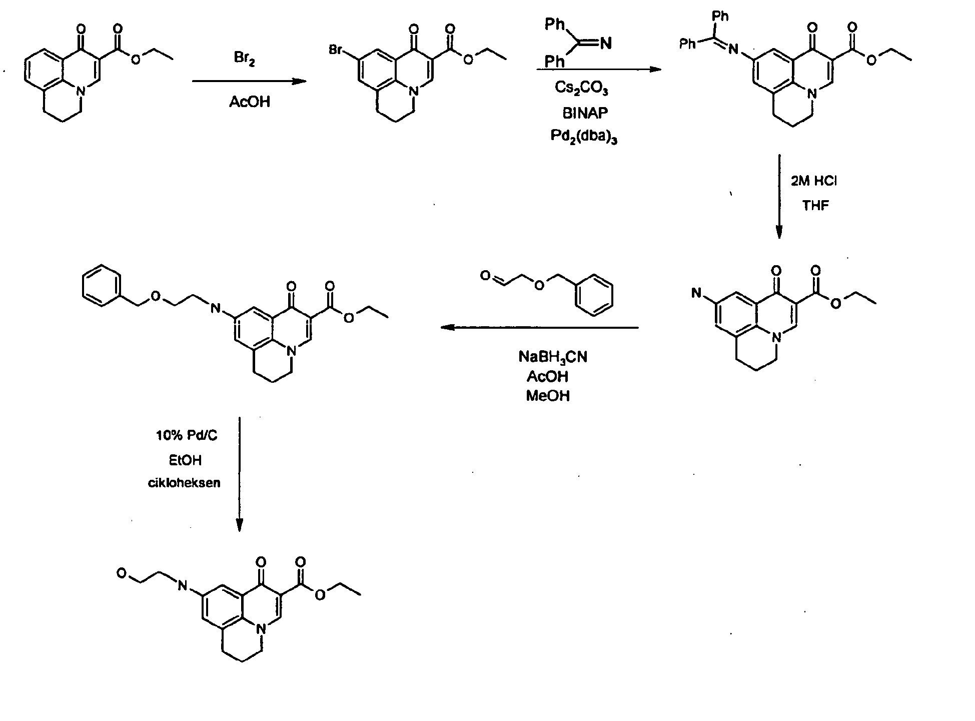

### a) 9-Bromo-1-oxo-6,7-dihydro-1H,5H-pyrido[3,2,1-ij] quinoline-2-carboxylic acid ethyl ester

To the solution of 1-oxo-6,7-dihydro-1H,5H-pyrido[3,2, 1-ij]quinoline-2-carboxylic acid ethyl ester (7.5g, 29mmol) in glacial acetic acid (120mL) bromine was added (1.6mL, 32mmol). The mixture was stirred over night at room temperature, and new portion of bromine (1.6mL), 32mmol) was added. After 24 h, reaction mixture was diluted with 100mL of H₂O and pH was adjusted to 2.9. Precipitate was filtered and dried. The crude product was precipitated from CH₂Cl₂ / Diisoprophylether and dried in vacuum drier yielding 13.07g of the crude title product.
MS (ES+) m/z : [MH]⁺ = 338.0

### b) 9-(Benzhydrylidene-amino)-1-oxo-6,7-dihydro-1H,5H-pyrido[3,2,1-ij]quinoline-2-carboxylic acid ethyl ester

Tris(dibenzylideneacetone)dipalladium chloroform complex (50mg, 0.05mmol), rac-2,2'-bis(diphenylphosphino)-1,1'-binaphthyl (100mg, 0.16mmol), **Intermediate 10a** (3g, 8.9mmol) and benzophenone imine (1.2ml) were diluted in THF (45ml). The air of atmosphere was replaced with N₂, and Cs₂CO₃ (2.5g) was added. The mixture was stirred under reflux. Another two portions of Tris(dibenzylideneacetone)dipalladium chloroform complex (50mg, 0.05mmol), rac-2,2'-bis(diphenylphosphino)-1,1'-binaphthyl (100mg, 0.16mmol), benzophenone imine (1.2L) and Cs₂CO₃ (2.5g) was added every 2.5h. The mixture was stirred under reflux over night and then cooled to room temperature and filtered. HPLC/MS indicated the presents of product **10b**.
MS (ES+) m/z : [MH]⁺ = 437.3

### c) 9-Amino-1-oxo-6,7-dihydro-1H,5H-pyrido[3,2,1-ij]quinoline-2-carboxylic acid ethyl ester

To the mixture of **Intermediate 10b** 5% HCl was added dropwise until appearance of precipitate. Precipitate was filtered and dried in vacuum drier yielding 2g of the crude title product.
MS (ES+) m/z : [MH]⁺ = 273.2
**¹³C-NMR(125 MHz, DMSO)** δ: 13.81, 19.90, 25.57, 51.37, 59.24, 108.39, 115.66, 124.99, 128.06, 129.06, 129.91, 130.51, 133.95, 147.54, 163.98, 171.63.

### d) 9-(2-Benzyloxy-ethylamino)-1-oxo-6,7-dihydro-1H,5H-pyrido[3,2,1-ij]quinoline-2-carboxylic acid ethyl ester

To the solution of **Intermediate 10c** (200mg, 0.73mmol) in MeOH (75mL), benzyloxyacetaldehide (110mg, 0.73mmol), NaBH₃CN (137mg, 2.2mmol) and AcOH (250µl) was added. Reaction mixture was stirred for 20 minutes and evaporated in vacuum. Oil product was purified by column chromatography in system CH₂Cl₂ -(MeOH-NH₄OH= 9:1.5) = 9:(1.5) yielding 159mg of the title product.
MS (ES+) m/z : [MH]⁺ = 407.2

### e) 9-(2-hydroxy-ethylamino)-1-oxo-6,7-dihydro-1H,5H-pyrido[3,2,1-ij]quinoline-2-carboxylic acid

To the solution of **Intermediate 10d** (159mg, 0.39mmol) in EtOH (41.6mL) cyclohexene (12.8mL) and 10% Pd/C (243mg) were added. The mixture was stirred under reflux over night, filtered through celite and evaporated in vacuum yielding 80mg of the title product.

### Intermediate 11

### 4"-Glycyl azithromycin-11,12-cyclic carbonate

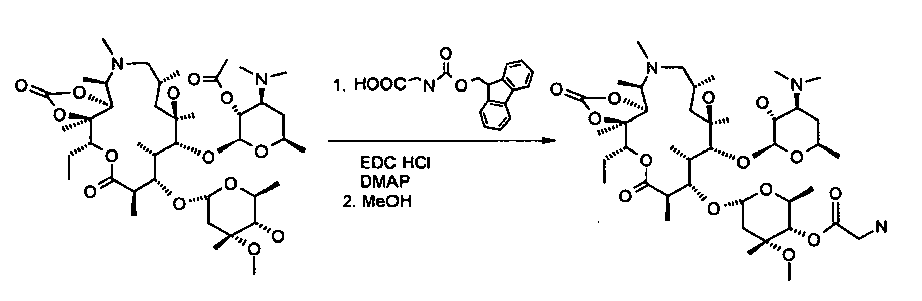

To a solution of 2'-O-Acetyl azithromycin-11,12-cyclic carbonate (8.16g, 9.8mmol) in CH₂Cl₂ (50 mL) were added DMAP (0.61 g, 5mmol), N-(9-Fluorenylmethoxy-carbonyl) glycine (Fmoc Gly, 4.46g, 15mmol), and 1-[3-(dimethylamino)propyl]-3-ethylcarbodiimide hydrochloride (EDC HCl, 3.83g, 20mmol). The mixture was stirred overnight at room temperature. The solvent was evaporated and the crude product dissolved in 200 mL of methanol. The mixture was stirred under reflux overnight at 65°C and then concentrated under reduced pressure. After column chromatography in system (CH₂Cl₂-MeOH-NH₄OH = 90:9:0,5) and rechromatography in the same system, 480mg of title compound was afforded.
MS (ES+) m/z : [MH]⁺ = 833.5
**¹³C-NMR(125 MHz, CDCl₃)** δ**:** 5.55, 10.45, 10.84, 14.19, 14.83, 17.82, 21.34, 21.58, 22.03, 22.15, 22.69, 26.27, 26.89, 29.15, 29.69, 34.40, 34.93, 35.26, 39.27, 40.39, 41.99, 42.92, 43.23, 43.92, 45.25, 49.50, 49.60, 61.26, 62.86, 65.39, 67.68, 68.18, 68.30, 68.93, 70.76, 73.02, 73.35, 74.30, 76.29, 76.82, 77.07, 77.33, 77.90, 78.07, 79.54, 85.12, 85.96, 95.27, 102.90, 106.59, 147.48, 153.36, 154.93, 158.06, 171.09, 174.23, 177.18

### Intermediate 12

### 4"-Glycyl azithromycin

Using the similar procedure to that described in **Intermediate 11, Intermediate 12** was prepared starting from 2'-O-Acetyl azithromycin. 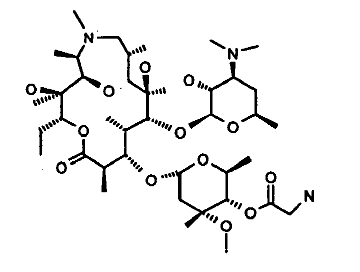 MS (ES+) m/z : [MH]⁺ = 806.5
**¹³C-NMR(125 MHz, CDCl₃)** δ**:** 7.45, 9.12, 11.27, 14.12, 14.69, 15.50, 16.24, 17.89, 21.29, 21.39, 21.79, 21.99, 22.66, 26.55, 26.78, 27.54, 28.98, 29.70, 31.59, 35.04, 35.29, 36.35, 38.42, 40.38, 42.00, 42.28, 43.76, 44.42, 45.15, 45.32, 49.48, 62.45, 62.88, 65.61, 67.87, 70.15, 70.98, 72.94, 73.60, 73.83, 74.31, 76.79, 77.05, 77.30, 77.45, 77.87, 79.52, 80.15, 83.16, 94.73, 102.34, 174.12, 178.81

### Intermediate 13

### 6-{2-[2-(2-Carboxy-ethoxy)-ethoxy]-ethoxy}-7-chloro-1-cyclopropyl-4-oxo-1,4-dihydroquinolone-3-carboxylic acid

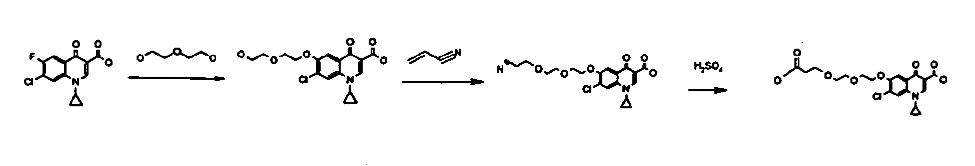

Mixture of 50 mL diethylene glycole and 50 mL DMSO was prepared and heated at 70°C. Into mixture 8 g of KO-t-Bu was added portionwise. Then, 5 g of fluoro-chloro quinolonic acid (17.8 mmol) was added portionwise. The temperature was increased to 105°C. After 5 hours, the 25 mL of H₂O was added and the mixture was extracted with 2x20 mL of DCM. Water layer was adjusted to pH 4. The obtained precipitate was filtered off and dried under reduced pressure affording 500 mg of 7-chloro-1-cyclopropyl-6-[2-(2-hydroxy-ethoxy)-ethoxy]-4-oxo-1,4-dihydro-quinolone-3-carboxylic acid.
7-Chloro-1-cyclopropyl-6-[2-(2-hydroxy-ethoxy)-ethoxy]-4-oxo-1,4-dihydro-quinolone-3-carboxylic acid (500 mg) was dissolved in 12.5 mL of acrylonitrile, then 1 mL of DBU was added and the mixture stirred for 24 hours at 80°C. Acrylonitrile was evaporated under reduced pressure, residue was dissolved in 300 mL of 2-propanol and the pH of the mixture was adjusted to pH 3.5. The precipitate was obtained after 12 hours, filtered off and washed with water (pH 3.5). The precipitate was dissolved in 20 mL H₂O:H₂SO₄ (1:1) and stirred for 24 hours at room temperature. The obtained precipitate was filtered off and dried under reduced pressure affording 300 mg of the title compound.

### Intermediate 14

### 4"-Propenoyl-11-O-methyl-azithromycin

### a) 4"-Propenoyl-2'-O-Ac-11-O-methyl-azithromycin

2'-O-Ac 11-*O*-Methyl-azithromycin was dissolved in toluene under N₂ atmosphere and TEA (3 eqv) and 3-Chloropropionyl-chloride (1 eqv) were added. Reaction mixture was stirred 5 minutes and then the same amount of TEA and 3-Chloropropionyl-chloride was added. After 10 minutes the reaction was finished, extracted with Na₂HCO₃ and toluene. The organic layer was washed with H₂O and brine, dried by anhydrous Na₂SO₄ and evaporated.

### b) 4"-Propenoyl-11-O-methyl-azithromycin

A solution of **Intermediate 14a** in MeOH was stirred at room temperature for 48 hours. The solvent was evaporated under reduced pressure affording the title compound.

### Intermediate 15

### 1-Oxo-9-(3-piperazin-1-yl)-prop-1-ynyl)-6,7-dihydro-1H,5H-pyrido[3,2,1-ij]quinoline-2-carboxylic acid ethyl ester

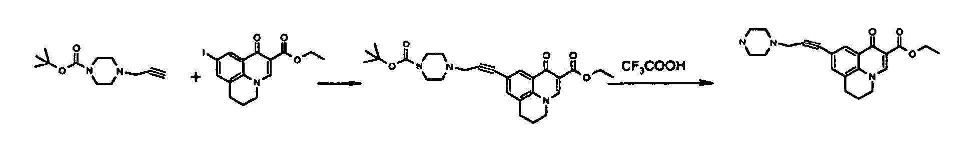

### a) 4-Prop-2-ynyl-piperazine-1-carboxylic acid tert-butyl ester

To the degassed solution of piperazine-1-carboxylic acid tert-butyl ester (0.5 g, 2.69 mmol) in acetonitrile (5 mL) was added Na₂CO₃ (0.854 g, 8.05 mmol) and mixture was stirred for 20 min. The suspension was heated to 50 °C and 3-bromo-propyne (448.65 µL, 4.03 mmol) was added. The solvent was evaporated and the residue was extracted with EtOAc and water. Organic layer was washed with NaCl and NaHCO₃ (2x20 mL), dried over K₂CO₃ and evaporated in vacuum yielding 0.45 g of the title product as yellowish oil.
MS (ES+) m/z : [MH]⁺ = 247.2

### b) 9-[3-(4-tert-Butoxycarbinyl-piperazin-1-yl)-prop-1-ynyl]-1-oxo-6,7-dihydro-1H,5H-pyrido[3,2,1-ij]quinoline-2-carboxylic acid ethyl ester

**Intermediate 17** (0.2 g, 0.524 mmol), copper (I) iodide (9.98 mg, 0.0524 mmol) and triethylamine (2.54 mL, 18.34 mmol) were suspended in dry acetonitrile (10 mL). The suspension was heated to 50 °C and N₂ bubbled through. After 20 min, dichlorobis (triphenylposphine) palladium (II) (11.03 mg, 0.0157 mmol) and **Intermediate 15a** (0.164 g 0.733 mmol) were added and dark red suspension was heated for 3 hours at 50 °C. The solvent was evaporated and the residue was extracted with EtOAc and water (2x20 mL). Organic layer was washed with NaCl and NaHCO₃ (2x20 mL), dried over K₂CO₃ and evaporated in vacuum yielding 0.34 g of the title product as red oil.

### c) 1-Oxo-9-(3-piperazin-1-yl)-prop-1-ynyl)-6,7-dihydro-1H,5H-pyrido[3,2,1-ij]quinoline-2-carboxylic acid ethyl ester

To the solution of **Intermediate 15b** (0.34 g, 0.71 mmol) in DCM (3.4 mL) was added CF₃COOH (3.4 mL) and mixture was stirred for 48 hours at room temp. To the reaction mixture was added water (pH 1.2) and layers were separated (pH 9.6). The organic layer was dried over K₂CO₃ and evaporated in vacuum yielding 0.22 g of the title product as red oil.
MS (ES+) m/z : [MH]⁺ = 380.2

### Intermediate 16

### 10-Amino-1-Oxo-6,7-dihydro-1H,5H-pyrido[3,2,1-ij]quinoline-2-carboxylic acid ethyl ester

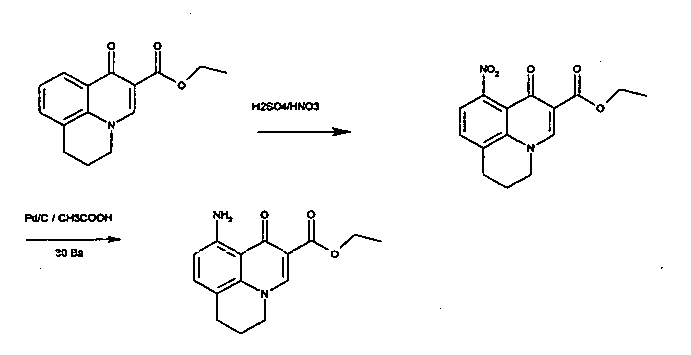

### a) 10-Nitro-1-Oxo-6,7-dihydro-1H,5H-pyrido[3,2,1-ij]quinoline-2-carboxylic acid ethyl ester

1-Oxo-6,7-dihydro-1H,5H-pyrido[3,2,1-ij]quinoline-2-carboxylic acid ethyl ester (1.0 g) was placed in round bottom flask and to that, mixture of H₂SO₄ / HNO₃ (1:1) was added and stirred for 3 hours at 0°C. The reaction mixture was poured on ice and precipitate was filtered off affording 900 mg of title product (LC/MS : 95%).

### b) 10-Amino-1-Oxo-6,7-dihydro-1H,5H-pyrido[3,2,1-ij]quinoline-2-carboxylic acid ethyl ester

**Intermediate 16a** (900 mg) was diluted in 35 mL of acetic acid and to this mixture 800 mg of 10 %Pd/C was added and stirred for 15 h at room temperature and at 30 Ba. The reaction mixture was filtered to remove catalyst and then acetic acid was evaporated under reduced pressure affording 700 mg of the title product. (LC/MS : 95%).

### Intermediate 17

### 9-Iodo-1-oxo-6,7-dihydro-1H,5H-pyrido[3,2,1-ij]quinoline-2-carboxylic acid ethyl ester

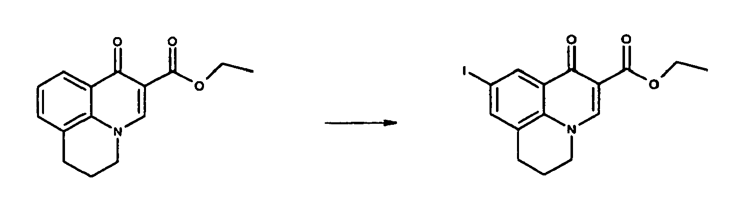

To a 0°C cooled trifluoromethansulfonic acid (3 mL, 33.31 mmol) 1-Oxo-6,7-dihydro-1H,5H-pyrido[3,2,1-ij]quinoline-2-carboxylic acid ethyl ester (1.53 g, 5.95 mmol) was added and to that solution N-Iodosuccinimide (1.6 g, 714 mmol) was added. The mixture was allowed to warm from 0 °C to room temperature while stirring. Reaction mixture was poured in ice and precipitate was filtered off affording 1 g of the title product (LC/MS : 57%).

### Intermediate 18

### 6-Amino-1-ethyl-4-oxo-1,4-dihydro-quinoline-3-carboxylic acid ethyl ester

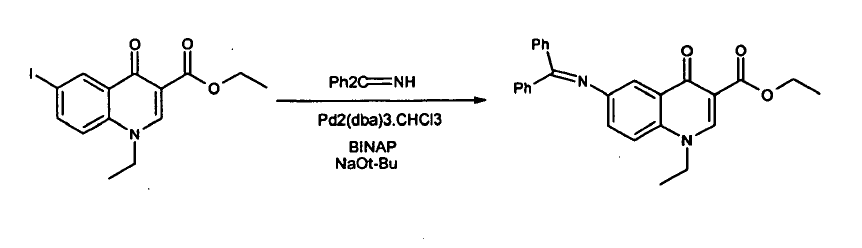

### a) 6-(Benzhydrylidene-amino)-1-ethyl-4-oxo-1,4-dihydro-quinoline-3-carboxylic acid ethyl ester

A Pyrex tube was charged with sodium tert-butoxide (1.4 mmol), Pd₂(dba)₃ (0.00125 mmol), and BINAP (0.00375 mmol). The Pyrex tube was fitted with a septum and after the air atmosphere was replaced with argon, toluene (4 mL), 1-ethyl-6-iodo-4-oxo-1,4-dihydroqunoline-3-carboxylic acid ethyl ester (1.0 mmol), and benzophenone imine (1.2 mmol) were added by syringe. The reaction was sealed and heated to 80°C with stirring until starting material was consumed as judged by GC analysis. The reaction mixture was cooled to room temperature, diluted with ether (40 mL), filtered, and concentrated. The crude reaction mixture was then recrystallized from MeOH to furnish the desired product in 90% yield.

### b) 6-Amino-1-ethyl-4-oxo-1,4-dihydro-quinoline-3-carboxylic acid ethyl ester

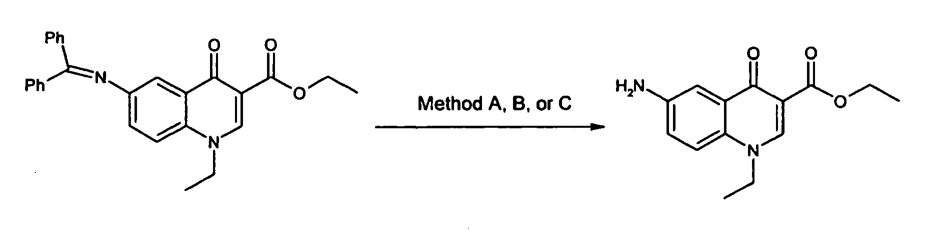

### Method A: Transamination with Hydroxylamine

To a solution of the imine adduct in MeOH (0.1 M) at RT was added NaOAc (2.4 eq) and hydroxylamine hydrochloride (1.8 eq). Oxime formation was usually complete in 15 to 30 minutes. The solution was then partitioned between 0.1 M NaOH and CH₂Cl₂. The organic layer was dried over anhydrous Na₂SO₄ and concentrated in vacuo. The product was purified by chromatography on silica gel.

### Method B: Hydrogenolysis

A solution of the imine adduct, ammonium formate (15eq) and 5% Pd/C (10 mol%) were heated to 60°C in MeOH (0.2 M in imine). After 2hours reduction was usually complete. The solution was cooled to room temperature and diluted with CH₂Cl₂ (5x volume of MeOH) to be passed through a plug of celite. The organic solution was washed with 0.1 M NaOH, dried over anhydrous Na₂SO₄ and concentrated in vacuo. The product was purified by chromatography on silica gel.

### Method C: Acidic Hydrolysis

To a solution of the imine adduct in THF (0.3 M) was added aqueous 2.0 M HCl (added 5% by volume of THF). After 5-20 minutes hydrolysis was complete and the reaction mixture was partitioned betwen 0.5 M HCl and 2:1 hexane/EtOAc. The aqueous layer was separated and made alkaline. The product aniline was extracted with CH₂Cl₂, dried over anhydrous Na₂SO₄ and concentrated in vacuo.

### Intermediate 19

### 2'-O-Acetyl-4"-O-propenoyl-6-O-methyl-erythromycin A

To a solution of 2'-*O*-acetyl-6-*O*-methyl-erythromycin A (1.1 g) in DCM (20 mL) pyridine (1.7 mL) and acryloyl chloride (1.1 mL) were added at 0°C. After 2 hours a further addition of pyridine (1.7 mL) and of acryloyl chloride (1.1 mL) was performed. The reaction mixture was quenched with a saturated solution of NH₄Cl (10 mL) and extracted with DCM (3x20 mL). The organic phase was washed with a saturated solution of NaHCO₃ (10 mL), water (10 mL), dried over Na₂SO₄, filtered and evaporated under reduced pressure. The crude product was purified by flash-chromatography (DCM-MeOH-NH₃ 95:5:0.5) affording the title compound (470 mg).
MS (ES+) m/z : [MH]⁺ = 844

### Intermediate 20

### 4"-O-Propenoyl-6-O-methylerythromycin A

**Intermediate 19** (1.82 g) was dissolved in MeOH (100 mL) and stirred at 60°C for 4 hours, then at room temperature for 16 hours. The solvent was evaporated under reduced pressure and the crude product was purified by flash chromatography (eluent: MeOH-DCM-NH₄OH 5:90:0.5) affording the title compound (1.4 g).
MS (ES+) m/z : [MH]⁺ = 802
¹H-NMR (500 MHz) δ: 6.44 (d, 1H), 6.13 (dd, 1H), 5.89 (d, 1H), 5.07 (d, 1H), 5.00 (d, 1H), 4.75 (d, 1H), 4.60 (d, 1H), 4.38 (m, 1H), 3.97 (s, 1H), 3.80-3.73 (m, 2H), 3.66 (d, 1H), 3.46 (s, 1H), 3.32 (s, 3H), 3.21-3.18 (m, 2H), 3.04 (s, 3 H), 3.00 (m, 1H), 2.92 (m, 1H), 2.56 (m, 2H), 2.43 (d, 1H), 2.31 (s, 6H).
¹³C-NMR (75 MHz) δ: 221.0; 175.7; 165.8; 131.5; 128.0; 102.1; 96.0; 80.5, 78.8, 78.3; 78.0; 76.6; 74.3, 72.7; 71.1; 69.1; 67.8; 65.3; 63.2: 50.7; 49.5; 45.3; 44.9; 40.3; 39.2; 38.8; 37.2; 35.2; 28.9; 21.7, 21.1; 19.7, 18.3, 18.0, 15.9; 12.3; 10.6; 9.1.

### Intermediate 21

### 2'-O-Acetyl-4"-O-propenoyl-azithromycin-11,12-carbonate

A solution of 2'-*O*-acetyl-azithromycin-11,12-carbonate (10.9 g) in toluene (300 mL) was stirred at room temperature under argon atmosphere. To this solution TEA (12.66 mL) and 3-chloro-propionyl chloride (1.94 mL) were added in two portions over a period of 10 minutes. After 20 minutes the solution was diluted with a saturated aqueous solution of NaHCO₃ (300 mL) and extracted with toluene (4x80 mL). The collected organic phase was dried, filtered and concentrated under reduced pressure affording the title compound (11.0 g).
MS (ES+) m/z : [MH]⁺ = 872

### Intermediate 22

### 4"-O-Propenoyl-azithromycin-11,12-carbonate

A solution of **Intermediate 21** (11.0 g) in MeOH (200 mL) was stirred at room temperature for 48 hours. The solvent was evaporated under reduced pressure affording the title compound (9.81 g).
MS (ES+) m/z : [MH]⁺ = 829.1
¹H-NMR (500 MHz,) δ: 6.45 (d, 1H), 6.17 (dd, 1H), 5.87 (d, 1H), 5.11 (d, 1H), 4.88 (dd, 1H), 4.77 (d, 1H), 4.53 (d, 1H), 4.47-4.40 (m, 3H), 3.72 (m, 1H), 3.60 (d, 1H), 3.33 (s, 3H), 3.25 (dd, 1H), 2.87-2.85 (m, 2H), 2.58 (m, 1H), 2.44-2.38 (m, 2H), 2.32 (s, 6H), 2.21 (s, 3H), 2.06 (m, 1H), 2.00 (m, 1H), 1.92 (m, 1H), 1.84 (m, 1H), 170-1.56 (m, 4H), 1.45 (s, 3H), 1.40 (dd, 1H), 1.29 (s, 3H), 1.25 (m, 1H), 1.22 (d, 3H), 1.18 (d, 6H), 1.12 (s, 3H), 108-1.06 (2d, 6H), 0.93 (m, 6H).

### Intermediate 23

### [2-(2-Hydroxy-ethoxy)-ethyl]-carbamic acid tert-butyl ester

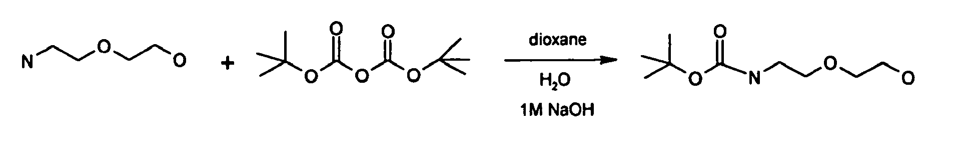

To the solution of dioxane (40 mL), H₂O (20 mL) and NaOH (20 mL; 1 M) was added 2-(2-aminoetoxy) ethanol. The reaction mixture was cooled to 0°C and di-t-Bu dicarbonate (4.8 g) was added. The mixture was stirred for 30 min at 0°C, and then the stirring was continued for 2 hours at room temperature. In next 3 hours two portion of di-t-Bu dicarbonate (2x 0.22 g) were added. The mixture was stirred over night at room temperature and then concentrated (20-30 mL). EtOAc (60 mL) was added to the solution and pH was adjusted to 2.5. Aqueous layer was extracted with EtOAc (3x20 mL). Organic layers was washed with H₂O (3x30 mL), dried over K₂CO₃ and evaporated in vacuum to give 3.7 g of the title product as oil.

### Intermediate 24:

### 7-Chloro-1-cyclopropyl-6-(2-hydroxy-ethoxy)-4-oxo-1,4-dihydro-quinoline-3-carboxylic acid (A) and

### 1-Cyclopropyl-6-fluoro-7-(2-hydroxy-ethoxy)-4-oxo-1,4-dihydro-quinoline-3-carboxylic acid (B)

To a mixture of DMSO (5 mL) and ethyleneglycol (6 mL), KO-t-Bu (1.6 g, 14.23 mmol) was added portionwise over 10 min, and then heated to 90 °C. To the mixture, 7-chloro-1-cyclopropyl-6-fluoro-4-oxo-1,4-dihydro-quinoline-3-carboxylic acid (1.0 g) was added portionwise over 20 min, the temperature was increased to 105 °C and the mixture was stirred for 6 h. Water (30 mL) was added to the reaction solution and the pH of the solution was adjusted to pH=5. The resulting solution was left in the refrigerator overnight. The precipitate obtained was filtered, washed with cold water, and dried affording a 2:1 mixture of **Intermediate 24A** and **Intermediate 24B** (1.0 g).

Part of the crude product (700 mg) was dissolved in EtOH (15 mL) by heating to the reflux. The resulting solution was cooled to 30°C and a first precipitation occurred. The precipitate was filtered, washed with cold EtOH and dried under reduced pressure. **Intermediate 24A** (204 mg) was obtained as a white solid;
**¹H-NMR (500 MHz, DMSO-d6)** δ**:** 15.06 (s, 1H), 8.71 (s, 1H), 8.40 (s, 1H), 7.86 (s, 1H), 4.97 (t, 1H), 4.25 (t, 2H), 3.87 (m, 1H), 3.82 (q, 2H), 1.32 (m, 2H), 1.20 (m, 2H); ¹³C-NMR (75 MHz, DMSO-d6) δ: 176.61, 165.67, 152.47, 147.54, 135.34, 129.48, 124.95, 120.02, 106.90, 106.66, 71.22, 59.15, 35.99, 7.46;

### Intermediate 25

### 7-Chloro-6-[2-(2-cyano-ethoxy)-ethoxy]-1-cyclopropyl-4-oxo-1,4-dihydro-quinoline-3-carboxylic acid

To a suspension of **Intermediate 24A** (2 g) in acrylonitrile (40 mL) was added DBU (2.3 mL). The reaction mixture was stirred at 80°C for 24 h. The acrylonitrile was evaporated under reduced pressure. Isopropanol (30 mL) was added to the residue and the pH of the solution was adjusted to pH=5 by adding 2M HCl, during which the product precipitated. The precipitate was filtered, washed with water, and dried affording **Intermediate 25** (1.7 g) as a white solid.
MS (ES+) m/z : [MH]⁺ = 377.0
**¹H-NMR (500 MHz, DMSO-d6)** δ**:** 8.68 (s, 1H), 8.38 (s, 1H), 7.84 (s, 1H), 4.38 (t, 2H), 3.91 (t, 2H), 3.86 (m, 1H), 3.75 (t, 2H), 2.79 (t, 2H), 1.32 (m, 2H), 1.20 (m, 2H); ¹³C-NMR (75 MHz, DMSO-d6) δ: 176.63, 165.65, 152.18, 147.61, 135.50, 129.44, 124.97, 120.04, 119.11, 106.96, 106.80, 69.02, 68.30, 65.49, 35.99, 18.06, 7.46;

### Intermediate 26

### 6-[2-(2-Carboxy-ethoxy)-ethoxy]-7-chloro-1-cyclopropyl-4-oxo-1,4-dihydro-quinoline-3-carboxylic acid

A solution of **Intermediate 25** (1.10 g) in a mixture of conc. H₂SO₄ (10 mL) and H₂O (20 mL) was stirred at 75 °C for 24 h. The pH of the reaction mixture was adjusted to 10.2 with 40% NaOH, during which the product precipitated. The precipitate was filtered, washed with water, and dried affording **Intermediate 26** (0.8 g) as a white solid. MS (ES+) m/z : [MH]⁺ = 396.0
**¹H-NMR (300 MHz, DMSO-d6)** δ**:** 15.0 (s, 1H), 11.8 (s, 1H), 8.69 (s, 1H), 8.38 (s, 1H), 7.85 (s, 1H), 4.35 (m, 2H), 3.91-3.82 (m, 3H), 3.74 (dt, 2H), 2.49 (m, 2H), 1.31 (m, 2H), 1.19 (m, 2H);

### Intermediate 27

### 7-{2-[2-(2-carboxy-ethoxy)ethoxy]ethylamino}-1-cyclopropyl-6-fluoro-4-oxo-1,4-dihydro-quinoline-3-carboxylic acid (A) and

### 6-{2-[2-(2-carboxy-ethoxy)ethoxy]ethylamino}-1-cyclopropyl-7-chloro-4-oxo-1,4-dihydro-quinoline-3-carboxylic acid (B)

Using the similar procedure to that described in **Intermediate 7**, starting from a mixture of **Intermediate 6A** and **Intermediate 6B**, **Intermediate 27** was prepared as a mixture of **Intermediate 27A** and **Intermediate 27B** in a 1:1 ratio.

### Intermediate 28

### 6-{2-[2-(2-amino-ethoxy)-ethoxy]-ethylamino}-1-cyclopropyl-7-chloro-4-oxo-1,4-dihydro-quinoline-3-carboxylic acid (A) and

### 7-{2-[2-(2-amino-ethoxy)-ethoxy]-ethylamino}-1-cyclopropyl-6-fluoro-4-oxo-1,4-dihydro-quinoline-3-carboxylic acid (B)

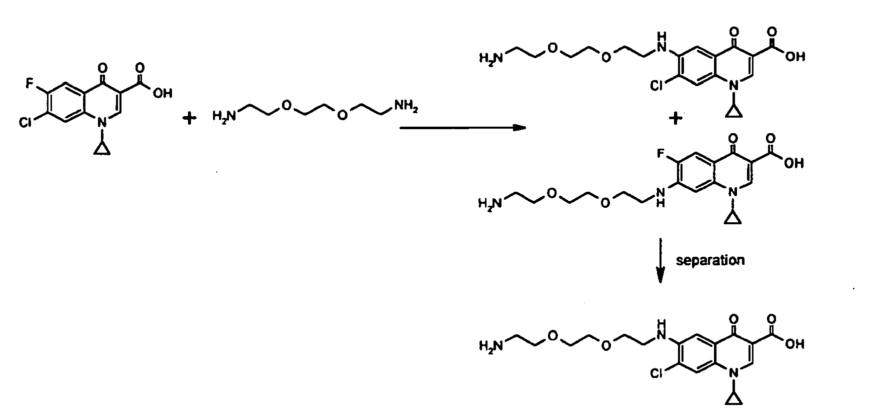

A mixture of 7-chloro-1-cyclopropyl-6-fluoro-4-oxo-1,4-dihydro-quinoline-3-carboxylic acid (5g, 0.018 mol), 2,2'-(ethylenedioxy)bis-(ethylamine) (26 mL, 0.18 mol, 10eq.) in 1-methyl-2-pyrrolidone was heated at 110°C for 24 hours. Reaction mixture was diluted with water (70 mL) pH was adjusted to 11 and extracted with CH₂Cl₂ (9x40 mL). Water layer was then acified to pH 6.8 with H₂SO₄, extracted with CH₂Cl₂ (50 mL) and evaporated. 2-Propanol was added (200 mL) and stirred at 82 °C for 30 minutes. The reaction mixture was then filtered and 2-propanol was evaporated in vacuum yielding 8 g of oily product, according to LC-MS 50% of chloro derivative **(A)** and 30% of fluoro derivative. Product was purified by column chromatography (eluent CH₂Cl₂-2-propanol = 1:1) yielding pure chloro derivative **(A).**
MS (ES+) m/z : [MH]⁺ = 409.9 **(A)**
MS (ES+) m/z : [MH]⁺ = 393.4 **(B)**

### Intermediate 29

### 3-(2-tert-butoxycarbonylethyl)-imidazolidine-1-carboxylic acid tert-butyl ester

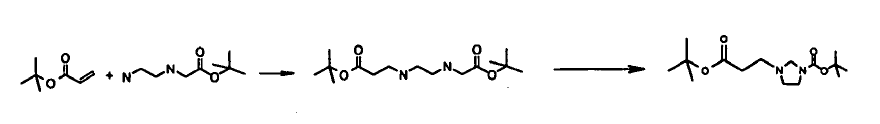

### a) 3-[2-(tert-butoxycarbonylmethyl-amino)-ethylamino]-propionic acid tert-butyl ester

To the solution of (2-amino-ethylamino)-acetic acid tert-butyl ester (1.0 mL, 6.32 mmol) in i-PrOH (50 mL) was added acrylic acid tert-butyl ester (309.1 µL, 2.11 mmol). The suspension was heated for 48 hours at 60 °C. The solvent was evaporated and product was purificated by column chromatography (DCM-MeOH-NH₃ = 90:3:0.5) yielded the title product as colorless oil (0.45 mg).
MS (ES+) m/z : [MH]⁺ = 289.2

### b) 3-(2-tert-butoxycarbonylethyl)-imidazolidine-1-carboxylic acid tert-butyl ester

To the solution of **Intermediate 29a** (0.45 mg, 1.56 mmol) in chloroform (20 mL) were added HCOOH (0.218 mL, 5.78 mmol) and HCHO (0.24 mL, 8.69 mmol) and stirred at room temperature for 2 hours. To the reaction mixture was added water (pH 1.3) and layers were separated (pH 2.5). The organic layer was dried over K₂CO₃ and evaporated in vacuum yielding 034 g of oil colorless product.
MS (ES+) m/z : [MH]⁺ = 301.2

### Intermediate 30

### 6-[2-(2-Amino-ethoxy)-ethylamino]-7-chloro-1-cyclopropyl-4-oxo-1,4-dihydroquinoline-3-carboxylic acid (A) and

### 7-[2-(2-Amino-ethoxy)-ethylamino]-1-cyclopropyl-6-fluoro-4-oxo-1,4-dihydro-quinoline-3-carboxylic acid (B)

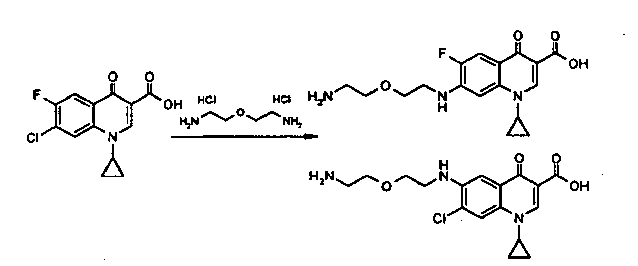

To a solution of 7-chloro-1-cyclopropyl-6-fluoro-4-oxo-1,4-dihydro-quinoline-3-carboxylic acid (0.55g, 1.95 mmol) in 1-methyl-2-pyrrolidone (40 mL) bis-(2-aminoethyl)-ether dihydrochlorid (2.1g, 11.9 mmol, 6eq.) and DBU (3.49 mL, 23.4 mmol, 12 eq.) added and the reaction mixture was stirred at 110°C for 18 hours. The reaction mixture was then diluted with water (70 mL), pH was adjusted to 11 and extracted with CH₂Cl₂ (9x40 mL). Water layer was then acified with H₂SO₄ to pH 6.8, extracted with 50 mL of CH₂Cl₂ and then evaporated in vacuum. Crude product was diluted in 2-propanol (60 mL), stirred at 82°C for 20 minutes and filtrated. Precipitate was pure salt (Na₂SO₄). 2-Propanol was evaporated in vacuum and product was purified by column chromatography (fraction, eluent: CH₂Cl₂-MeOH-NH₃-CH₃CN=4:4:2:1) yielding 0.5g of title compounds as a mixture of chloro and fluoro derivatives in ratio 3:1
MS (ES+) m/z : [MH]⁺ = 365.8 **(A)** (75 %)
MS (ES+) m/z : [MH]⁺ = 349.4 **(B)** (25 %)

### Intermediate 31

### 7-Chloro-1-ethyl-6-[2-(2-hydroxy-ethoxy)-ethyl amino]-4-oxo-1,4-dihydro-quinoline-3-carboxylic acid (A) and

### 1-Ethyl-6-fluoro-7-[2-(2-hydroxy-ethoxy)-ethylamino]-4-oxo-1,4-dihydro-quinoline-3-carboxylic acid (B)

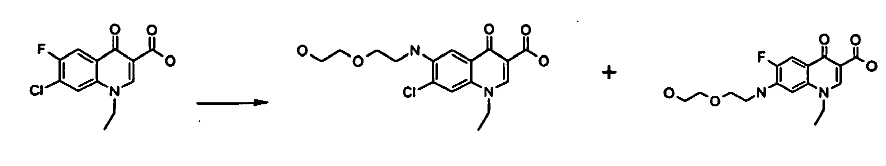

Starting from 7-chloro-1-ethyl-6-fluoro-4-oxo-1,4-dihydro-quinoline-3-carboxylic acid (2.5 g, 9.27 mmol) using the procedure of Intermediate 6, the mixture of **Intermediates A** and **B** (1.0 g) was obtained
According to HPLC/MS product contains **Intermediate A** and **Intermediate B** in a ratio 1:1 HPLC/MS (ES) m/z: [MH]⁺ = 355.81 (Intermediate A)
[MH]⁺ = 339.38 (Intermediate B).

### Intermediate 32

### 7-Chloro-6-2-(2-(2-cyano-ethoxy)-ethoxy)ethylamino)-1-ethyl-4-oxo-1,4-dihydro-quinolin-3-carboxylic acid (A) and

### 6-Fluoro-7-2-(2-(2-cyano-ethoxy)-ethoxy)ethylamino)-1-ethyl-4-oxo-1,4-dihydro-quinolin-3-carboxylic acid (B)

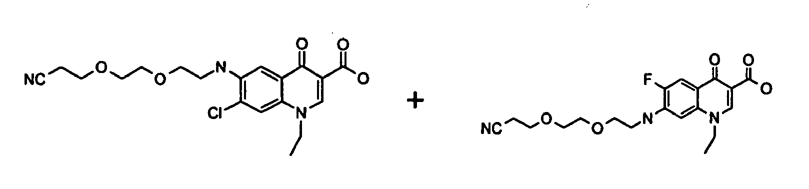

Mixture of **Intermediates 31A and 31 B** (600 mg, 1.13 mmol) was dissolved in 10 %NaOH solution (5 mL) and cooled to 0°C and then acrylonitrile (370 µL, 5.65 mmol) was added portionwise and stirred at R.T. for 2h. To reaction mixture water (50 mL) was added and extracted with MIBK (3x50 mL). H₂O layer was filtered with charcoal and pH was adjusted to pH 4. The obtained precipitate was filtered and dried affording 600 mg of product.
According to HPLC/MS product contains Intermediates **(A):(B)** in a ratio 1:1
HPLC/MS (ES) m/z: [MH]⁺ = 408.78 (Intermediate A)
[MH]⁺ = 391.39 (Intermediate B).

### Intermediate 33

### 7-Chloro-6-2-(2-(2-carboxy-ethoxy)-ethoxy)ethylamino)-1-ethyl-4-oxo-1,4- dihydro-quinolin-3-carboxylic acid(A) and

### 6-Fluoro-7-2-(2-(2-carboxy-ethoxy)-ethoxy)ethylamino)-1-ethyl-4-oxo-1,4- dihydro-quinolin-3-carboxylic acid(B)

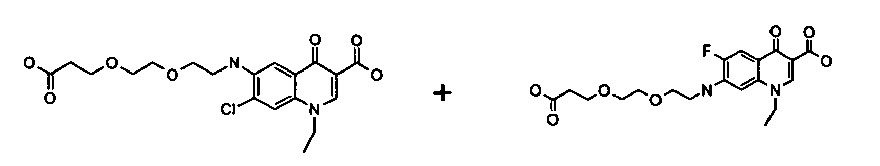

Mixture of **Intermediates 32A and 32 B** (600 mg, 1.47 mmol) was dissolved in H₂SO₄/H₂O =1/1.5 (15 mL) and stirred at 70°C for 24 h. Reaction mixture was poured on cold water, precipitate was filtered and dried affording 400 mg of product.
According to HPLC/MS product contains Intermediates **(A):(B)** in a ratio 1:1
HPLC/MS (ES) m/z: [MH]⁺ = 427.85 (Intermediate A)
[MH]⁺ = 411.36 (Intermediate B)

### Intermediate 34

### 7-Chloro-6-2-(2-(2-carboxy-ethoxy)-ethoxy)ethylamino)-1-isopropyl-4-oxo-1,4-dihydro-quinolin-3-carboxylic acid

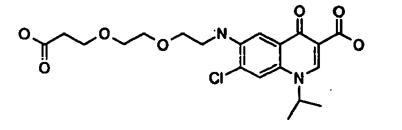

### a) 7-Chloro-6-{2-(2-(2-hydroxy-ethoxy)-ethoxy)ethylamino}-1-isopropyl-4-oxo-1,4-dihydro-quinolin-3-carboxylic acid

To a mixture of 7-chloro-1-isopropyl-6-fluoro-4-oxo-1,4-dihydro-quinoline-3-carboxylic acid (3 g, 10.57 mmol) in 1-methyl-2-pirolidone (30 mL) 2-(2-amino-ethoxy)-ethanol (5.3 mL, 52.85 mmol, 5 eq.) was added, the reaction mixture was stirred at 110°C for 24 hours. Then was diluted with water (50 mL) and extracted with CH₂Cl₂ (3 x 150 mL). After separation water layer was acidified to pH 4 and left overnight at 4 °C. The obtained precipitate was filtered off and dried under reduced pressure for 1 h affording 974 mg of title product.
HPLC/MS (ES) m/z: [MH]⁺ = 370.1

### b) 7-Chloro-6-{2-(2-(2-cyanoethoxy)-ethoxy)ethylamino}-1-ispropyl-4-oxo-1,4-dihydro-quinolin-3-carboxylic acid

To the solution of **Intermediate 34a** (1.82 g, 4.94 mmol) in MIBK (50 mL) 40% aqueos solution of NaOH (1.03 mL, 14.8 mmol) was added and then acrylonitrile (6.5 mL, 98.7 mmol) was added portionvise and sirred at 0-10°C for 2 h. Than DBU (2 mL) was added and stirred at R.T. for 1 h. The reaction mixture was then diluted with cold MIBK (20 mL) and H₂O (20 mL), extracted with H₂O (2x15 mL). The combined water layers were filtrated with charcoal and pH was adjusted to pH 5. The precipitate was filtered and dried under reduced pressure yielding 1.2g of the title product.
HPLC/MS (ES) m/z: [MH]⁺ = 422.78.

### c) 7-Chloro-6-2-(2-(2-carboxy-ethoxy)-ethoxy)ethylamino)-1-isopropyl-4-oxo-1,4-dihydro-quinolin-3-carboxylic acid

The **Intermediate 34b** (1.2 mg, 2.85 mmol) was dissolved in H₂SO₄:H₂O =1:1.5 (50 mL) and stirred at 70°C for 48 h. Than reaction mixture was poured on cold water. The obtained precipitate was filtered off and dried under reduced pressure affording 1 g of the title compound.
HPLC/MS (ES) m/z: [MH]⁺ = 441.85.

### Intermediate 35

### 9-[2-(2-Carboxy-ethoxy)-ethylamino]-1-oxo-6,7-dihydro-1H,5H-pyrido [3,2,1-ij] quinoline-2-carboxylic acid

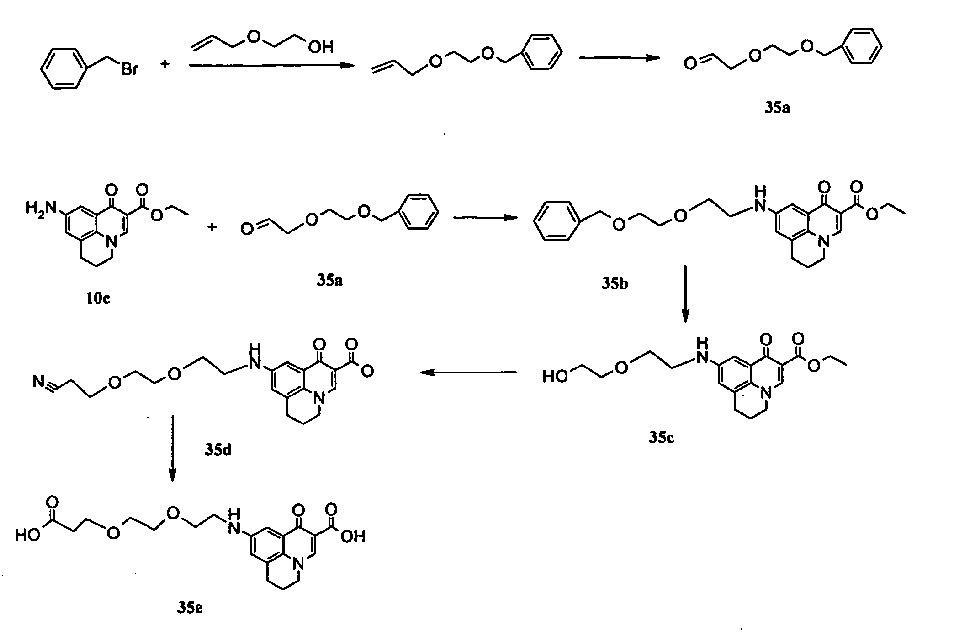

### a) (2-Benzyloxy-ethoxy)-acetaldehyde

THF (40 mL) was cooled to 0°C under N₂ atmosphere for 30 minutes and in this solution 2-allyloxyethanol (3.14 mL, 29.4 mmol) and NaH (1.1 g, 27.5 mmol) were added. The reaction mixture was heated to 35°C and after 45 minutes, this mixture was cooled to 0°C and benzyl bromide (5.24 mL, 44.1 mmol) was added. Reaction mixture was stirred at RT overnight and then extracted with EtOAc/H₂O. The combined organic layers were dried over anhydrous Na₂SO₄ and evaporated under vacuum.

The crude product was purified via flash chromatography (hexane : acetone = 6 : 1) giving (2-allyloxy-ethoxymethyl)-benzene.

To the solution of (2-allyloxy-ethoxymethyl)-benzene (2 g, 10.4 mmol) in THF : H₂O = 1 :1 (50ml), cooled to 0°C, OsO₄ (0.127g; 2.5% solution in THF) and NaIO₄ (12.83 g suspended in 50ml of H₂O) were added. The reaction mixture was stirred at RT for 3h, filtered and then extracted with EtOAc/H₂O. The combined organic layers were dried over anhydrous Na₂SO₄ and evaporated under vacuum giving 2.08 g of the title intermediate.

### b) 9-[2-(2-Benzyloxy-ethoxy)-ethylamino]-1-oxo-6,7-dihydro-1H,5H-pyrido[3,2,1-ij] quinoline-2-carboxylic acid ethyl ester

To the solution of **Intermediate 10c** (400mg, 1.47 mmol) in MeOH (150 mL), **Intermediate 35a** (285 mg, 1.47 mmol), NaBH₃CN (273mg, 2.2mmol) and AcOH (300µL) were added. Reaction mixture was stirred at RT over night, filtered and evaporated in vacuum. Oil product was purified by column chromatography (eluent CH₂Cl₂ : MeOH: NH₄OH= 90 : 15: :1.5 yielding 850 mg of the title product.
MS (ES+) m/z : [MH]⁺ = 451.19.

### c) 9-[2-(2-Hydroxy-ethoxy)-ethylamino]-1-oxo-6,7-dihydro-1H,5H-pyrido[3,2,1-ij] quinoline-2-carboxylic acid ethyl ester

To the solution of **Intermediate 35b** (850mg, 1.89 mmol) in MeOH (30 mL) 10% Pd/C (425mg) and acetic acid (100 µL) were added. The mixture was stirred under pressure of 5 bar on R.T. for 48 hours. The rection mixture was filtered through celite and evaporated in vacuum yielding 685mg of crude product. After purification by spe-chromatography 306mg (assay 97.7%) of the title product was obtained.
**¹H-NMR(500MHz, DMSO-d6)** δ**:** 1.27 (t, 3H), 2.05 (k, 2H), 2.9 (t, 2H), 3.26 (q, 2H), 3.46 (m, 2H), 3.51 (m, 2H), 3.58 (t, 2H), 4.2 (m,4H), 4.6 (t, 1H), 5.9 (t, 1H), 6.9 (s, 1H), 7.13 (s, 1H), 8.35 (s, 1H).
**¹³C-NMR(300MHz, DMSO-d6)** δ**:** 14.30, 20.96, 26.16, 42.65, 51.7, 59.17, 60.15, 68.60, 72.17, 102.39, 106.95, 118.47, 127.58, 128.94, 129.73, 145.05, 145.89, 165.02, 172.41.

### d) 9-{2-[2-(2-Cyano-ethoxy)-ethoxy]-ethylamino}-1-oxo-6,7-dihydro-1H,5H-pyrido [3,2,1-ij]quinoline-2-carboxylic acid

Using the similar procedure to that described in **Intermediate 34b**, starting from **Intermediate 35c,** 274mg (assay 91 %) of **Intermediate 35d** was obtained.
**¹H-NMR(500MHz, DMSO-d6)** δ**:** 2.11 (k, 2H), 2.73 (t, 2H), 2.9 (t, 2H), 3.3 (m, 2H), 3.58 (m, 8H), 4.39 (t,2H), 6.34 (t, 1H), 7.1 (s, 1H), 7.14 (s, 1H), 8.65 (s, 1H), 15.95 (s, 1H). **¹³C-NMR(300MHz, DMSO-d6)** δ**:** 17.99, 20.72, 26.17, 42.43, 52.64, 65.21, 68.66, 69.53, 65.56, 100.19, 105.43, 119.20, 120.44, 126.94, 128.48, 129.96, 143.83, 146.82, 166.86, 176.47.

### e) 9-[2-(2-Carboxy-ethoxy)-ethylamino]-1-oxo-6,7-dihydro-1H,5H-pyrido [3,2,1-ij] quinoline-2-carboxylic acid

Using the similar procedure to that described in **Intermediate 34c,** starting from **Intermediate 35d, Intermediate 35e** (342mg) was obtained.
**¹H-NMR(500MHz, DMSO-d6)** δ**:** 2.09 (m, 2H), 2.42 (t, 2H), 2.97 (t, 2H), 3.29 (q, 2H), 3.52 (m, 4H), 3.58 (m, 4H), 4.40 (t, 2H), 6.35 (t, 1H), 7.13 (s, 1H), 7.21 (s, 1H), 8.64 (s, 1H), 12.20 (s, 1H), 15.90 (s, 1H).
**¹³C-NMR(300MHz, DMSO-d6)** δ**:** 20.70, 26.16, 34.61, 42.41, 52.62, 66.14, 68.56, 69.52, 69.54, 100.12, 105.40, 120.43, 126.90, 128.44, 129.94, 143.79, 146.79, 166.88, 172.55, 176.44.

### Intermediate 36

### 7-{2-[2-(2-Carboxy-ethoxy)-ethoxy]-ethoxy}-1-cyclopropyl-4-oxo-1,4-dihydro-quinoline-3-carboxylic acid

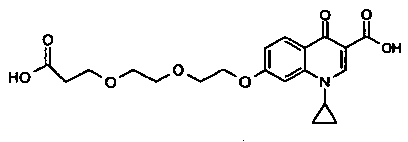

### a) 7-Chloro-1-cyclopropyl-4-oxo-1,4-dihydro-quinoline-3-carboxylic acid

7-Chloro-1-cyclopropyl-4-oxo-1,4-dihydro-quinoline-3-carboxylic acid ethyl ester (1.50 g) was suspended in THF (15 mL) at room temperature. NaOH (411 mg, 2 eq) solution in water (15 mL) was added and the mixture was stirred at 80 °C for 24 hours. THF was evaporated and the solution was acidified to pH 5 using HCl. The obtained precipitate was filtrated off and dried under reduced pressure yielding 1.18 g of the titled **Indermediate 36a.**

### b) 7-{2-[2-(2-Carboxy-ethoxy)-ethoxy]-ethoxy}-1-cyclopropyl-4-oxo-1,4-dihydroquinoline-3-carboxylic acid

Using the similar procedure to that described for Intermediate 13, starting from Intermediate 36a 180 mg of the title compound was obtained.
HPLC/MS (ES+) m/z [MH]⁺ = 416.19

### Intermediate 37

### 2'-O-Acetyl-4"-O-propenoyl-9-(1-isopropoxy-cyclohexyl)oximino-erythromycin A

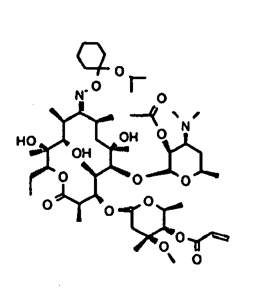

Using the similar procedure to that described in **Intermediate 19,** starting from **2'-*O*-acetyl-9-(1-isopropoxy-cyclohexyl)oximino-erythromycin A** (0.50 g) titled **Intermediate 37** (0.48 g) was obtained.

### Intermediate 38

### 6-{2-[2-(2-Carboxy-ethoxy)-ethoxy]-ethylamino}-7-chloro-1-cyclopropyl-4-oxo-1,4-dihydro-quinoline-3-carboxylic acid

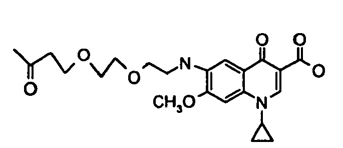

### a) 7-Methoxy-1-cyclopropyl-6-[2-(2-hydroxy-ethoxy)-ethylamino]-4-oxo-1,4-dihydroquinoline-3-carboxylic acid

Sodium (2.5 g, 0.1086 mole) was added portion wise to methanol (60 mL) followed with addition of **Intermediate 6B** (7.00 g, 0.019 mole) at R.T.. Reaction mixture was heated in sealed tube for 100 hours at 130 °C. Mixture was evaporated in vacuum to dryness, solid residue was dissolved in water (50 mL) and acidified with acetic acid to pH about 5.5. After cooling in ice bath yellow crystals precipitated, The precipitate was filtrated, washed with water and dried in vacuum yielding 5.9 g of the titled compound.
HPLC/MS (ES+) m/z [MH]⁺ = 362.

### b) 7-Methoxy-6-[2-(2-cyanopropoxy-ethoxy)-ethylamino]-1-cyclopropyl-4-oxo-1,4-dihydro-quinoline-3-carboxylic acid

Using the similar procedure to that described in **Intermediate 32**, starting from **Intermediate 38a** afforded **Intermediate 38b** (3.18 g).
HPLC/MS (ES+) m/z [MH]⁺ = 415.

### c) 7-Methoxy-6-{2-[2-(2-carboxy-ethoxy)-ethoxy]-ethylamino}-1-cyclopropyl-4-oxo-1,4-dihydro-quinoline-3-carboxylic acid

Using the similar procedure to that described in Intermediate 33, starting from Intermediate 38b afforded **Intermediate 38c** (2.34 g).
HPLC/MS (ES+) m/z [MH]⁺ = 434.5.

### Intermediate 39

### 6-{2-[2-(2-Carboxy-ethoxy)-ethoxy]-ethylamino}-1-cyclopropyl-7-dimethylamino-4-oxo-1,4-dihydro-quinoline-3-carboxylic acid

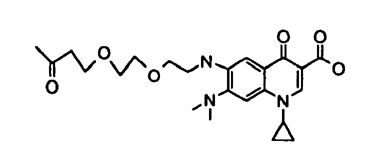

### a) 1-Cyclopropyl-7-dimethylamino-6-[2-(2-hydroxy-ethoxy)-ethylamino]-4-oxo-1,4-dihydro-quinoline-3-carboxylic acid

To a solution of **Intermediate 6B** (2.58 g) in n-butanol (40 mL) 40% water solution of dimethylamine (7 mL) was added and heated in sealed tube for 75 hours at 140 °C. After cooling the reaction mixture was evaporated at reduced pressure to dryness and ethanol (20 mL) was added. After keeping in refrigerator over night precipitate occured. The precipitate was filtered off and washed with ethanol yielding the title product (2.05 g)
HPLC/MS (ES+) m/z [MH]⁺ = 375.

### b) 1-Cyclopropyl-7-dimethylamino-6-[2-(2-cyanopropoxy-ethoxy)-ethylamino]-4-oxo-1,4-dihydro-quinoline-3-carboxylic acid

Using the similar procedure to that described in **Intermediate 32,** starting from **Intermediate 39a** afforded **Intermediate 39b** (1.75 g).
HPLC/MS (ES+) m/z [MH]⁺ = 429.

### c) 6-{2-[2-(2-Carboxy-ethoxy)-ethoxy]-ethylamino}-1-cyclopropyl-7-dimethylamino-4-oxo-1,4-dihydro-quinoline-3-carboxylic acid

Using the similar procedure to that described in **Intermediate 33**, starting from **Intermediate 39b** afforded **Intermediate 39c** (1.33 g).
HPLC/MS (ES+) m/z [MH]⁺ = 444.7.

### Intermediate 40

### (2-Prop-2-ynyloxy-ethoxymethyl)-benzene 2

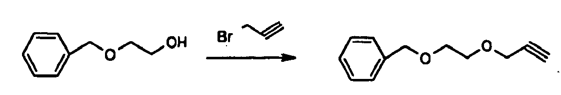

THF (120 mL) was cooled to 0°C under N₂ atmosphere and to this solution 2-benzyloxyethanol (25 mL, 0.154 mol) and NaH (60% min.oil, 7.4 g, 0.184 mol) were added. The reaction mixture was heated at 35 °C for 45 minutes, cooled to 0°C, and than 3-bromo-propyne (27.44 mL, 0.308 mol) was added. Reaction mixture was stirred at RT overnight and then partitioned between EtOAc/H₂O. The combined organic layer was dried over anhydrous Na₂SO₄, evaporated in vacuo to yield 24.8 g of the title product.
HPLC/MS(ES+) m/z [MH]⁺ = 190.48.

### Intermediate 41

### 1-Ethyl-6-iodo-4-oxo-1,4-dihydro-quinoline-3-carboxylic acid

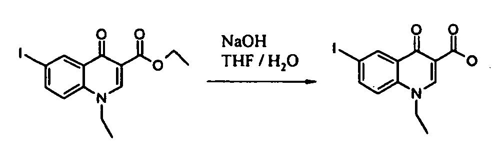

Starting from 1,4-Dihydro-1-ethyl-6-iodo-4-oxo-quinoline-3-carboxylic acid ethyl ester (7.5 g, 0.02 mol) using the procedure of **Intermediate 36a** the title compound was obtained (7.3 g).
LC/MS(ES+) m/z [MH]⁺ = 344.2

### Intermediate 42

### 6-{3-[2-(2-Carboxy-ethoxy)-ethoxy]-propyl}-1-ethyl-4-oxo-1,4-dihydro-quinoline-3-carboxylic acid

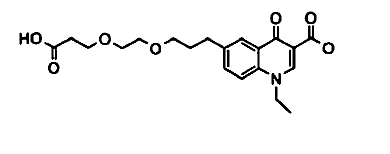

### a) 6-[3-(2-Benzyloxy-ethoxy)-prop-1-ynyl]-1-ethyl-4-oxo-1,4-dihydro-quinoline-3-carboxylic acid

To solution of **Intermediate 41** (10.6 g, 0.03 mol) in MeCN:H₂O=1:1 (250 mL), CuI (0.571 g, 0.003 mol) was added and stirred for 20 minutes at 50 °C. Then solution of Pd(OPh₃)₂Cl₂ (1.05 g, 0.0014 mol ) and **Intermediate 40** (10.26 g, 0.0015 mol ) in MeCN:H₂O=1:1 (40 mL) were added to the reaction mixture and stirred at 50 °C overnight. Organic solvents were evaporated and water (500 mL) was added to the residue, pH was adjusted to 12 and water layer was extracted with diizopropyl-ether (3x150 mL). Charcoal was added to water layer, then stirred for 15 minutes and filtrated over Celite. After pH was adjusted to 6 product precipitated. The precipitate was filtrated off yielding 10.1 g of the title product.
LC/MS(ES+) m/z [MH]⁺= 406.3
**¹H-NMR (300 MHz, CDCl₃)** δ**:** 8.78 (s, 1H); 8.57 (d, 1H); 7.83 (dd, 1H); 7.57 (d, 1H); 7.38-7.27 (m, 5H); 4.60 (s, 2H); 4.48 (s,2H); 4.39 (q, 2H); 3.82-3.80 (m, 2H); 3.72-3.69 (m, 2H); 1.59 (t, 3H)
**¹³C-NMR (75 MHz, CDCl₃)** δ**:** 177.79; 166.76; 148.14; 138.51; 138; 136.67; 130.67; 128.39; 126.49; 121.046; 116.53; 109.36; 87.87; 84.27; 73.36; 69.49; 69.49; 59.12; 49.8; 14.66.

### b) 1-Ethyl-6-[3-(2-hydroxy-ethoxy)-propyl]-4-oxo-1,4-dihydro-quinoline-3-carboxylic acid

Hydrogenation of **Intermediate 42 a** (10 g, 0.025 mol) in MeOH (120 mL) with addition of 10 % Pd/C (3 g) at 5 bar for 48 hours yilded the titled product (6.45 g).
LC/MS(ES+) m/z [MH]⁺= 320.29
**¹H-NMR (300 MHz, DMSO)** δ**:** 9.02 (s, 1H); 8.19 (s, 1H); 7.98 (d, 1H); 7.84 (d, 1H); 4.62 (q, 2H); 3.51 (t, 2H); 3.43-3.39 (m,4H); 2.86 (t, 2H); 1.95-1.85 (m, 2H); 1.43 (t, 3H) **¹³C-NMR (75 MHz, DMSO)** δ**:** 177.62; 166.34; 148.69; 140.52; 137.54; 135.14; 125.65; 124.73; 118.24; 108; 72.18; 69.38; 60.37; 49.10; 31.21; 30.85; 14.76

### c) 6-{3-[2-(2-Cyano-ethoxy)-ethoxy]-propyl}-1-ethyl-4-oxo-1,4-dihydro-quinoline-3-carboxylic acid

Using the similar procedure to that described in **Intermediate 32,** starting from **Intermediate 42b** (6.4 g, 0.02 mol) **Intermediate 42c** (7.2 g) was obtained.
LC/MS(ES+) m/z [MH]⁺= 373.2
**¹H-NMR (300 MHz, DMSO-d6)** δ**:** 8.57 (s, 1H); 8.09 (d, 1H); 7.68 (d, 1H); 7.59 (dd, 1H); 4.34 (q, 2H); 3.62 (t, 2H); 3.57-3.59 (m, 2H); 3.49-3.51 (m, 2H); 3.42 (t, 2H); 2.76 (t, 2H), 2.75 (t, 2H), 1.34 (t, 3H).
**¹³C-NMR (75 MHz, DMSO-d6)** δ**:** 175.38; 167.18; 146.69; 137.15; 136.97; 132.59; 128.09; 125.08; 119.24; 116.53; 110; 69.55; 69.32; 69.29; 65.19; 47.32; 31.01; 30.75; 14.42.

### d) 6-{3-[2-(2-Carboxy-ethoxy)-ethoxy]-propyl}-1-ethyl-4-oxo-1,4-dihydro-quinoline-3-carboxylic acid

Using the similar procedure to that described in **Intermediate 33**, starting from **Intermediate 42c** (7.2 g, 0.019 mol) **Intermediate 42d** (4.2 g) was obtained.
LC/MS(ES+) m/z [MH]⁺= 392.2
**¹H-NMR (300 MHz, DMSO-d6)** δ**:** 9.02 (s, 1H); 8.18 (d, 1H); 7.98 (s, 1H); 7.85 (dd, 1H); 4.60 (q, 2H); 3.47-3.53 (ov, 4H); 3.62 (m, 2H); 3.41 (t, 2H); 2.85 (t, 2H); 2.45 (t, 2H), 1.87 (m, 2H), 1.43 (t, 3H).
**¹³C-NMR (75 MHz, DMSO-d6)** δ**:** 177.41; 172.51; 166.09; 148.43; 140.26; 137.32; 134.94; 125.40; 124.51; 117.99; 107.42; 69.54; 69.34; 69.15; 66.15; 48.87; 34.66; 30.96; 30.52; 14.52.

### Intermediate 43

### 1-Cyclopropyl-6-iodo-4-oxo-1,4-dihydro-quinoline-3-carboxylic acid

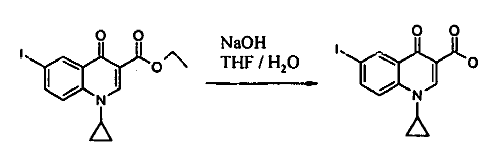

Using the similar procedure to that described in **Intermediate 41,** starting from **1-cyclopropyl-6-iodo-4-oxo-1,4-dihydro-quinoline-3-carboxylic acid ethyl ester** (2.6 g, 6.8 mmol) **Intermediate 43** (2.3 g) was obtained.
LC/MS(ES+) m/z [MH]⁺= 355.93.

### Intermediate 44

### 6-{3-[2-(2-Carboxy-ethoxy)-ethoxy]-propyl}-1-cyclopropyl-4-oxo-1,4-dihydro-quinoline-3-carboxylic acid

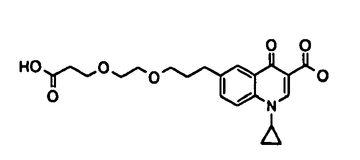

### a) 6-[3-(2-Benzyloxy-ethoxy)-prop-1-ynyl]-1-cyclopropyl-4-oxo-1,4-dihydro-quinoline-3-carboxylic acid

Using the similar procedure to that described in **Intermediate 42a,** using **Intermediate 43** (2.3 g, 6.5 mmol) and **Intermediate 40** (2.47 g, 13 mmol) **Intermediate 44a** (2.1 g) was obtained.
LC/MS(ES+) m/z [MH]⁺= 418.2

### b) 1-Cyclopropyl-6-[3-(2-hydroxy-ethoxy)-propyl]-4-oxo-1,4-dihydro-quinoline-3-carboxylic acid

Using the similar procedure to that described in **Intermediate 42b**, starting from **Intermediate 44a** (2.1 g, 5 mmol) **Intermediate 44b** (1.5 g) was obtained.
LC/MS(ES+) m/z [MH]⁺= 332.08

### c) 6-{3-[2-(2-Cyano-ethoxy)-ethoxy]-propyl}-1-cyclopropyl-4-oxo-1,4-dihydro-quinoline-3-carboxylic acid

Using the similar procedure to that described in **Intermediate 32,** starting from **Intermediate 44b** (1.34 g, 4 mmol) **Intermediate 44c** (1.83 g) was obtained.
LC/MS(ES+) m/z [MH]⁺= 385.03

### d) 6-{3-[2-(2-Carboxy-ethoxy)-ethoxy]-propyl}-1-cyclopropyl-4-oxo-1,4-dihydroquinoline-3-carboxylic acid

Using the similar procedure to that described in **Intermediate 33,** starting from **Intermediate 44c** (1.33 g, 3.5 mmol) **Intermediate 44d** (0.64 g) was obtained.
LC/MS(ES+) m/z [MH]⁺= 404.11

### Intermediate 45

### 6-iodo-4-oxo-1,4-dihydro-quinoline-3-carboxylic acid

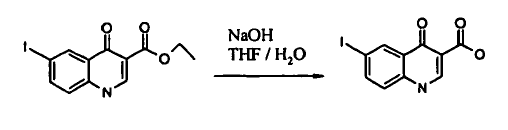

Using the similar procedure to that described in **Intermediate 41,** starting from **6-iodo-4-oxo-1,4-dihydro-quinoline-3-carboxylic acid ethyl ester** (317 mg, 0.85 mmol) **Intermediate 45** (224 mg) was obtained.
LC/MS(ES+) m/z [MH]⁺= 315.97.

### Intermediate 46

### 6-{3-[2-(2-Carboxy-ethoxy)-ethox]-propyl}-4-oxo-1,4-dihydro-quinoline-3-carboxylic acid

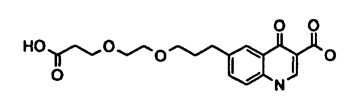

### a) 6-[3-(2-Benzyloxy-ethoxy)-prop-1-ynyl]-4-oxo-1,4-dihydro-quinoline-3-carboxylic acid

Using the similar procedure to that described in **Intermediate 42a,** using **Intermediate 45** (6 g, 0.02 mol) and **Intermediate 40** (7.6 g, 0.04 mol) Intermediate 46a (2.2 g) was obtained.
LC/MS(ES+) m/z [MH]⁺= 378.12.

### b) 6-[3-(2-Hydroxy-ethoxy)-propyl]-4-oxo-1,4-dihydro-quinoline-3-carboxylic acid

Using the similar procedure to that described in **Intermediate 42b,** starting from **Intermediate 46a** (2 g, 5 mmol) **Intermediate 46b** (1 g) was obtained.
LC/MS(ES+) m/z [MH]⁺= 292.01.

### c) 6-{3-[2-(2-Cyano-ethoxy)-ethoxy]-propyl}-4-oxo-1,4-dihydro-quinoline-3-carboxylic acid

Using the similar procedure to that described in **Intermediate 32,** starting from **Intermediate 46b** (0.933 g, 3.2 mmol) **Intermediate 46c** (970 mg) was obtained.
LC/MS(ES+) m/z [MH]⁺= 345.09.

### d) 6-{3-[2-(2-Carboxy-ethoxy)-ethoxy]-propyl}-4-oxo-1,4-dihydro-quinoline-3-carboxylic acid

Using the similar procedure to that described in **Intermediate 33,** starting from **Intermediate 46c** (970 mg, 2.8 mmol) **Intermediate 46d** (0.95 g) was obtained.
LC/MS(ES+) m/z [MH]⁺= 364.09.

### Intermediate 47

### 1-Allyl-6-iodo-4-oxo-1,4-dihydro-quinoline-3-carboxylic acid

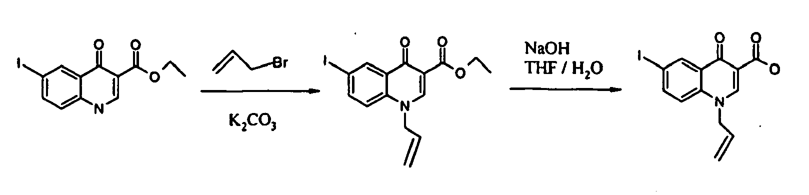

### a) 1-Allyl-6-iodo-4-oxo-l,4-dihydro-quinoline-3-carboxylic acid ethyl ester

In the solution of 6-iodo-4-oxo-1,4-dihydro-quinoline-3-carboxylic acid ethyl ester (4 g, 0.0117 mol) and K₂CO₃ (3.22 g, 0.023 mol) in DMF (40 mL), allyl-bromide (1.48 mL, 0.0176 mol) was added and stirred at 65°C for 1 hour. Reaction mixture was dilluted with water (50 mL) and the **Intermediate 47a** was precipitated yielding 3.3g the title product.
LC/MS(ES+) m/z [MH]⁺= 383.98
**¹H-NMR (300 MHz, DMSO-d6)** δ**:** 8.71 (s, 1H); 8.49 (d, 1H); 8.04 (dd, 1H); 7.53 (d, 1H); 6.00-6.07 (m, 1H); 5.11 (dd, 1H); 5.24 (dd, 1H); 5.03 (d, 2H); 4.23 (q, 2H); 1.29 (t, 3H) **¹³C-NMR (75 MHz, DMSO-d6)** δ**:** 171.60; 164.42; 149.94; 140.71; 138.68; 134.87; 132.65; 129.93; 120.30; 118.06; 110.82; 90.36; 60.02; 54.69; 14.44.

### b) 1-Allyl-6-iodo-4-oxo-1,4-dihydro-quinoline-3-carboxylic acid

Using the similar procedure to that described in **Intermediate 36a,** starting from **Intermediate 47a** (3.3 g, 8.6 mmol) **Intermediate 47b** (2.7 g) was obtained.
LC/MS(ES+) m/z [MH]⁺= 355.92.

### Intermediate 48

### 6-{3-[2-(2-Carboxy-ethoxy)-ethoxy]-propyl}-4-oxo-1-propyl-1,4-dihydro-quinoline-3-carboxylic acid

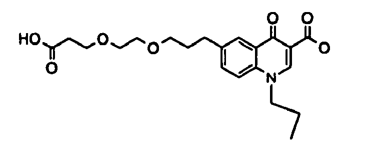

### a) 1-Allyl-6-[3-(2-benzyloxy-ethoxy)-prop-1-ynyl]-4-oxo-1,4-dihydro-quinoline-3-carboxylic acid

Using the similar procedure to that described in **Intermediate 42a**, using **Intermediate 47b** (2.7 g, 76 mmol) and **Intermediate 40** (2.9 g, 15.2 mmol) **Intermediate 48a** (2.7 g) was obtained.
LC/MS(ES+) m/z [MH]⁺= 418.12.

### b) 6-[3-(2-Hydroxy-ethoxy)-propyl]-4-oxo-1-propyl-1,4-dihydro-quinoline-3-carboxylic acid

Using the similar procedure to that described in **Intermediate 42b**, starting from **Intermediate 48a** (1.1 g, 2.6 mmol) **Intermediate 48b** (740 mg) was obtained.
LC/MS(ES+) m/z [MH]⁺= 334.10.

### c) 6-{3-[2-(2-Cyano-ethoxy)-ethoxy]-propyl}-4-oxo-1-propyl-1,4-dihydro-quinoline-3-carboxylic acid

Using the similar procedure to that described in **Intermediate 32,** starting from **Intermediate 48b** (740 mg, 2.2 mmol) **Intermediate 48c** (900 mg) was obtained.
LC/MS(ES+) m/z [MH]⁺= 387.16.

### d) 6-{3-[2-(2-Carboxy-ethoxy)-ethoxy]-propyl}-4-oxo-1-propyl-1,4-dihydro-quinoline-3-carboxylic acid

Using the similar procedure to that described in **Intermediate 33,** starting from **Intermediate 48c** (900 mg, 2.3 mmmol) **Intermediate 48d** (950 mg) was obtained.
LC/MS(ES+) m/z [MH]⁺= 406.16.

### Intermediate 49

### 6-{3-[2-(2-amino-ethoxy)-ethoxy]-prop-1-ynyl}-1-ethyl-4-oxo-1,4-dihydro-quinoline-3-carboxylic acid

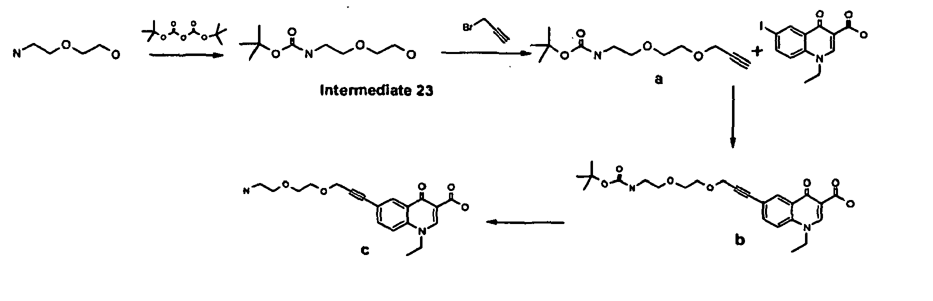

### a) [2-(2-prop-2-ynyloxy-ethoxy)-ethyl]-carbamic acid tert-butyl ester

Using the similar procedure to that described in **Intermediate 9b,** starting from **Intermediate 23** (2.87 g, 14 mmol) **Intermediate 49a** (3 g) was obtained.

### b) 6-{3-[2-(2-Tert-butoxycarbonylamino-ethoxy)-ethoxy]-prop-1-ynyl}-1-ethyl-4-oxo-1,4-dihydro-quinoline-3-carboxylic acid

Using the similar procedure to that described in **Intermediate 9c,** starting from **1-ethyl-6-iodo-4-oxo-1,4-dihyro-quinoline-3-carboxylic acid** (2 g, 5.8 mmol) and **Intermediate 49a** (1.7 g, 6.9 mmol) **Intermediate 49b** (3.9 g) was obtained.
LC/MS(ES+) m/z [MH]⁺= 458.5.

### c) 6-{3-[2-(2Amino-ethoxy)-ethoxy]-prop-1-ynyl}-1-ethyl-4-oxo-1,4-dihydro-quinoline-3-carboxylic acid

Using the similar procedure to that described in **Intermediate 9d,** starting from **Intermediate 49b** (3.9 g, 8.5 mmol) crude **Intermediate 49c** was obtained. Aftre purification by column chromatography (eluent: DCM : MeOH : NH₃ = 90:15:1.5) the title product was obtained (0.9 g)
LC/MS(ES+) m/z [MH]⁺= 359.

### Intermediate 50

### 1-Cyclopropyl-7-chloro-6-[2-(2-hydroxy-ethylamino)-ethylamino]-4-oxo-1,4-dihydroquinoline-3-carboxylic acid (A) and

### 1-Cyclopropyl-6-fluoro-7-[2-(2-hydroxy-ethylamino)-ethylamino]-4-oxo-1,4-dihydroquinoline-3-carboxylic acid (B)

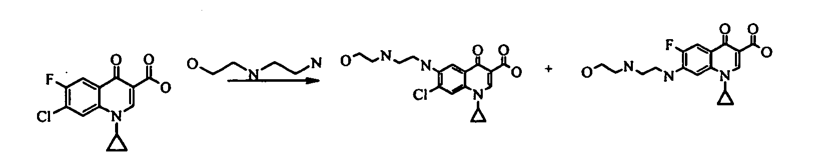

Using a similar procedure as described in **Intermediates 6A** and **6B** starting from 7-chloro-1-cyclopropyl-6-fluoro-4-oxo-1,4-dihydro-quinoline-3-carboxylic acid (8.65 g, 30.8 mmol) and 2-(2-aminoethylamino)ethanol (5 eqv.) mixture of (8.5 g) chloro and fluoro derivative was obtained.

### Intermediate 51

### 7-Chloro-1-cyclopropyl-6-{2-[(2-hydroxy-ethyl)-methyl-amino]-ethylamino}-4-oxo-1,4-dihydro-quinoline-3-carboxylic acid (A) and

### 6-Fluoro-1-cyclopropyl-7-{2-[(2-hydroxy-ethyl)-methyl-amino]-ethylamino}-4-oxo-1,4-dihydro-quinoline-3-carboxylic acid (B)

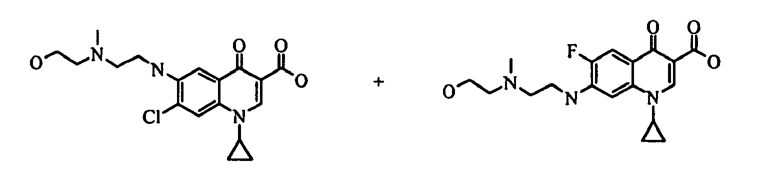

To the mixture of **Intermediates 50A and B** (2.23 g) in CHCl₃ (40 mL) formaldehyde (2eq, 355µL) and formic acid (3.7 eq, 892 µL) were added and heated in a screw cap vial at 60°C for 4 hours. The solvent was evaporated, to the residue water (30 mL) and ethyl acetate (30 mL) were added, pH adjusted to 9 and water layer washed with ethyl acetate. Than pH of water layer was adjusted to pH 6 - 7 with 1N HCl yielding **Intermediate 51A** (389 mg) as a yellow precipitate. LC-MS [ES⁺] *m*/*z*: 380.
**¹H-NMR (300 MHz, DMSO-d6)** δ**:** 8.6; 8.3; 7.4; 6.3; 5.2; 3,8; 3.7; 3.6; 3.3; 3.1; 2.7; 1.3;1.2.
**¹³C-NMR (75 MHz, DMSO-d6)** δ**:** 176.7; 166.3; 146.1; 142.6; 132.7; 126.9; 125.6; 119.4; 106.5; 103.2; 57.8; 53.9; 40.8; 36.0.

**Intermediate 51B** was isolated from a mother liquor as a yellow solid (1.657 g).

### Intermediate 52

### 6-(2-{[2-(2-Carboxy-ethoxy)-ethyl]-methyl-amino}-ethylamino)-7-chloro-1-cyclopropyl-4-oxo-1,4-dihydro-quinoline-3-carboxylic acid

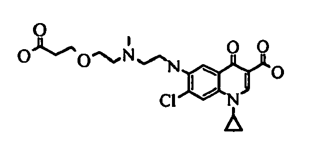

### a) 7-Chloro-6-(2-{[2-(2-cyano-ethoxy)-ethyl]-methyl-amino}-ethylamino)-1-cyclopropyl-4-oxo-1,4-dihydro-quinoline-3-carboxylic acid

Using the similar procedure to that described in **Intermediate 32**, starting from Intermediate **51a** (2.68 g, 7 mmol) **Intermediate 52a** (1.76 mg) was obtained.
LC/MS(ES+) m/z [MH]⁺= 433.

### b) 6-(2-{[2-(2-Carboxy-ethoxy)-ethyl]-methyl-amino}-ethylamino)-7-chloro-1-cyclopropyl-4-oxo-1,4-dihydro-quinoline-3-carboxylic acid

Using the similar procedure to that described in **Intermediate 33,** starting from **Intermediate 52a** (1.7 g, 4 mmmol) the title compound (730 mg) was obtained.
LC/MS(ES+) m/z [MH]⁺= 452.
**¹H-NMR (500 MHz, DMSO-d6)** δ**:** 8.6; 8.3; 7.5; 6.2; 3.8; 3.7; 3.6; 2.9; 2.8; 1.3; 1.2.

### Intermediate 53

### 1-tert-Butyl-7-chloro-6-[2-(2-hydroxy-ethoxy)-ethoxy]-4-oxo-1,4-dihydro-quinoline-3-carboxylic acid

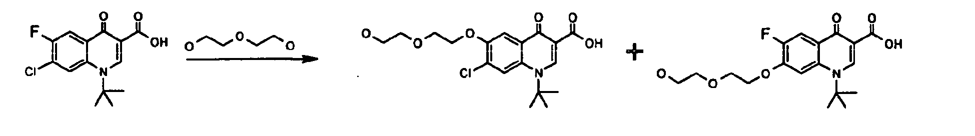

To a solution of diethylene glycol (19 mL, 0.2 mol, 20 eq.) and DMSO (15 mL) slowly KO-t-Bu (4.5g, 0.4 mol, 4 eq.) was added. The reaction mixture was warmed up to 90 °C and 1-tert.-butyl-7-chloro-6-fluoro-4-oxo-1,4-dihydro-quinoline-3-carboxylic acid (3g, 0.01 mol) was added slowly. The mixture was then stirred at 105 °C for 6 hours and then left over night at R.T. Than water (70 mL) was added, pH was adjusted to 3.5 and placed into refrigerator, the product precipitated. The precipitate was filtered off yielding 1.1 g of the title product.
LC/MS(ES+) m/z [MH]⁺= 383.8.

### Intermediate 54

### 6-{2-[2-(2-carboxy-ethoxy)-ethoxy]-ethoxy}-7-chloro-4-oxo-1,4-dihydro-quinoline-3-carboxylic acid

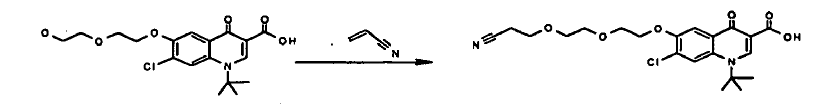

### a) 1-tert-Butyl-7-chloro-6-{2-[2-(2-cyano-ethoxy)-ethoxy]-ethoxy}-4-oxo-1,4-dihydroquinoline-3-carboxylic acid

Using the similar procedure to that described in **Intermediate 32,** starting from **Intermediate 53** (1 g, 2.6 mmol) **Intermediate 54a** (780 mg) was obtained.
LC/MS(ES+) m/z [MH]⁺= 436.9.

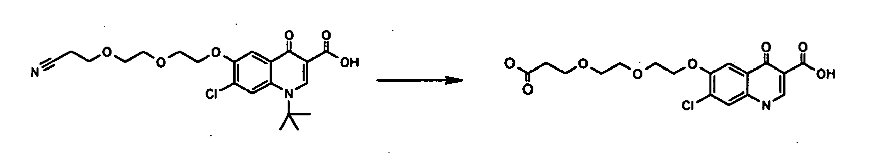

### b) 6-{2-[2-(2-carboxy-ethoxy)-ethoxy]-ethoxy}-7-chloro-4-oxo-1,4-dihydro-quinoline-3-carboxylic acid

Using the similar procedure to that described in **Intermediate 33,** starting from **Intermediate 54a** (700 mg, 1.5 mmmol) the title compound (430 mg) was obtained.
LC/MS(ES+) m/z [MH]⁺= 399.8.

### Intermediate 55

### 1-Tert-butyl-7-chloro-6-[2-(2-hydroxy-ethoxy)-ethylamino]-4-oxo-1,4-dihydroquinoline-3-carboxylic acid (A) and

### 1-Tert-butyl-6-fluoro-7-[2-(2-hydroxy-ethoxy)-ethylamino]-4-oxo-1,4-dihydroquinoline-3-carboxylic acid (B)

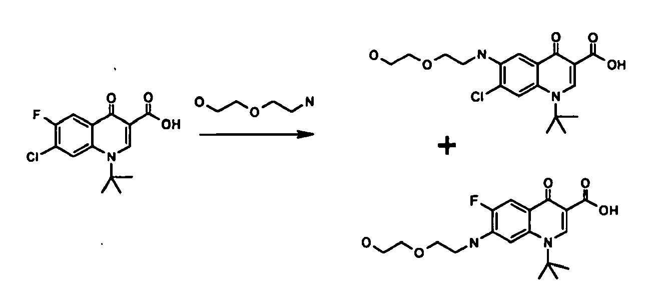

To a mixture of 1-tert.-butyl-7-chloro-6-fluoro-4-oxo-1,4-dihydro-quinoline-3-carboxylic acid (3g, 10 mmol) in 1-methyl-2-pyrrolidone (20 mL) 2-(2-amino-ethoxy)-ethanol (5 mL, 50 mmol, 5 eq) was added and the reaction mixture was stirred at 110 °C for 24 hours. The reaction mixture was diluted with H₂O (20 mL) and CH₂Cl₂ (50 mL) and pH was adjusted to 13. The aqueus layer was extracted with CH₂Cl₂ (2 x 30 mL) and pH was adjusted to 3.5. After 5 minutes in the refrigerator precipitation occurred. The precipitate was filtered off yielding 1.83 g of the title compounds (according to LC-MS a mixture of **Intermediate 55A** and **Intermediate 55B** in a ratio 2: 1). The filtrate was left at 5° C for 1 hour giving additional amount (0.75 g ) of the **Intermediate 55A** and **Intermediate 55B** in a ratio 1:1.
LC/MS(ES+) m/z [MH]⁺= 382.8 **(Intermediate 55A)**
LC/MS(ES+) m/z [MH]⁺= 366.4 **(Intermediate 55B)**

### Intermdiate 56

### 6-{2-[2-(2-carboxy-ethoxy)-ethoxy]-ethylamino}-7-chloro-4-oxo-1,4-dihydro-quinoline-3-carboxylic acid (C) and

### 7-{2-[2-(2-carboxy-ethoxy)-ethoxy]-ethylamino}-6-fluoro-4-oxo-1,4-dihydro-quinoline-3-carboxylic acid (D)

### a) 1-Tert-butyl-7-chloro-6-{2-[2-(2-cyano-ethoxy)-ethoxy]-ethylamino}-4-oxo-1,4-dihydro-quinoline-3-carboxylic acid (A) and

### 1-Tert-butyl -7-{2-[2-(2-cyano-ethoxy)-ethoxy]-ethylamino}-6-fluoro-4-oxo-1,4-dihydroquinoline-3-carboxylic acid (B)

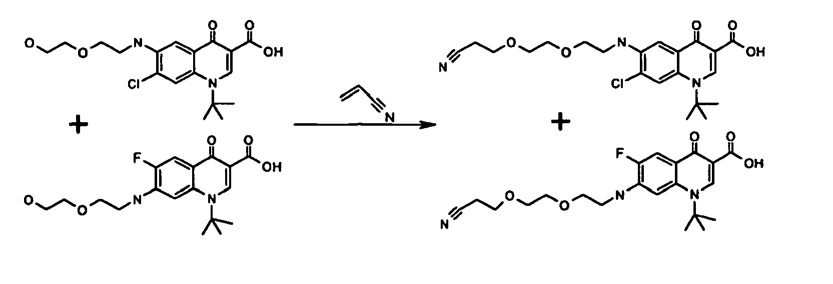

Using the similar procedure to that described in **Intermediate 32**, starting from a mixture of **Intermediate 55A** and **Intermediate 55B** (1.5 g, 3.9 mmol) a mixture of **Intermediate 56A** and **Intermediate 56B** (1.3 g) was obtained in a ratio 2:1.
LC/MS(ES+) m/z [MH]⁺= 435.9 (**Intermediate 56A**)
LC/MS(ES+) m/z [MH]⁺= 419.5 (**Intermediate 56B)**

### b) 6-{2-[2-(2-carboxy-ethoxy)-ethoxy]-ethylamino}-7-chloro-4-oxo-1,4-dihydroquinoline-3-carboxylic acid (C) and

### 7-{2-[2-(2-carboxy-ethoxy)-ethoxy]-ethylamino}-6-fluoro-4-oxo-1,4-dihydro-quinoline-3-carboxylic acid (D)

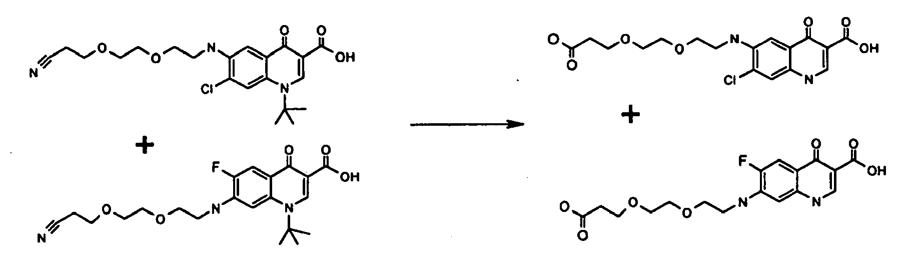

Using the similar procedure to that described in **Intermediate 33**, starting from a mixture of **Intermediate 56A** and **Intermediate 56B** (1.3 g, 2.98 mmol) a mixture of **Intermediate 56C** and **Intermediate 56D** (850 mg) was obtained in a ratio 3:1.
LC/MS(ES+) m/z [MH]⁺= 398.8 (**Intermediate 56C**)
LC/MS(ES+) m/z [MH]⁺= 382.3 (**Intermediate 56D**)

### Intermediate 57

### 6-({2-[(2-aminoethyl)(methyl)amino]ethyl}thio)-1-ethyl-4-oxo-1,4-dihydro-3-quinolinecarboxylic acid bis-trifluoroacetate.

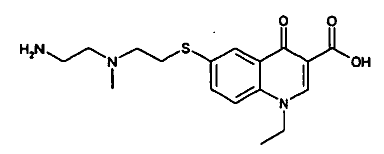

### a) 6-Bromo-1-ethyl-4-oxo-1,4-dihydroquinolin-3-carboxylic acid ethyl ester.

A mixture of potassium carbonate (10.4 g, 74.7 mmol) and 6-bromoquinolone-3-carboxylic acid (10.0 g, 37.3 mmol) in dimethylformamide (100 mL) was heated to 40°C under argon for 10 minutes, iodoethane (12 mL, 150 mmol) was then added. After 14 h the mixture was cooled and the DMF evaporated. The residue was treated with water (40 mL), cooled to 5°C and filtered under vacuum. The resultant cream-coloured solid was dried under vacuum to yield the title compound (11.2 g).
1H-NMR (D6-DMSO) δ: 1.41(3H, t, J = 7.12 Hz); 1.54(3H, J = 7.24 Hz); 4.24(2H, q, J = 7.24 Hz); 4.40(2H, q, J = 7.12 Hz); 7.34(1H, d, J = 9 Hz); 7.76(1H, d x d, J = 2.4 & 9 Hz); 8.65(1H, d, J = 2.4 Hz), 8.49(1H, s).

### b) 6-{[2-({[(1,1-dimethylethyl)oxy]carbonyl}amino)ethyl]thio}-1-ethyl-4-oxo-1,4-dihydro-3-quinolinecarboxylic acid ethyl ester.

A mixture of 1,1-dimethylethyl (2-oxoethyl)carbamate (5.0 g, 30 mmol), potassium carbonate (4.0 g, 30 mmol) and **Intermediate 57a** (5.0 g, 1.5 mmol) in DMSO (50 mL) was heated at 90°C. After 16 h the reaction was cooled and poured into water (200 mL), the organic material extracted into dichloromethane (2 x 50 mL). The combined organic layers were dried and evaporated to yield a brown solid. Chromatography over silica gel eluting with dichloromethane containing an increasing concentration of methanol/ammonium hydroxide gave the title compound as a white solid.
ESMS m/z [MH]⁺= 421.2.

### c) 6-[(2-aminoethyl)thio]-1-ethyl-4-oxo-1,4-dihydro-3-quinolinecarboxylic acid ethyl ester trifluoroacetate.

A solution of **Intermediate 57b** (1.04 g, 2.47 mmol) in trifluoroacetic acid (25 mL) was stirred at room temperature. After 15 minutes the excess trifluoroacetic acid was evaporated to yield the title compound as a light brown solid (1.32 g).
ESMS m/z [MH]⁺= 321.2.

### d) 6-[(2-{[2-({[(1,1-dimethylethyl)oxy]carbonyl}amino)ethyl]amino}ethyl)thio]-1-ethyl-4-oxo-1,4-dihydro-3-quinolinecarboxylic acid ethyl ester.

A mixture of **Intermediate 57c** (1.00, 2.47 mmol), 1,1-dimethylethyl (2-oxoethyl)carbamate (0.47 g, 2.96 mmol) and sodium acetate (0.51 g, 6.8 mmol) in 1% acetic acid in methanol (20 mL) was allowed to stir for 10 mins at room temperature before sodium cyanoborohydride (0.31 g, 4.94 mmol) was added. After 20 h an additional batch (0.18 g, 1.13 mmol) of the aldehyde was added. Atter 3 days the reaction evaporated and the residue chromatographed over silica gel eluting with 0 - 10% [9:1 methanol/20 M ammonia] in dichloromethane to give the desired compound contaminated with the bis-alkylated product (0.80 g). Reverse phase chromatography over C 18 silica eluting with 0.5% aqueous formic acid/0.5% formic acid acetonitrile gave title compound (0.42 g).
ESMS m/z [MH]⁺= 464.1.

### e) 6-({2-[[2-({[(1,1-dimethylethyl)oxy]carbonyl}amino)ethyl](methyl)amino] ethyl}thio)-1-ethyl-4-oxo-1,4-dihydro-3-quinolinecarboxylic acid ethyl ester.

To a mixture of Intermediate 57d (0.356 g, 0.617 mmol) and potassium carbonate (0.13 g, 0.93 mmol) in dry DMF (8 mL) was added iodomethane (0.043 mL, 0.69 mmol). After stirring at room temperature for 48 h the solvent was evaporated and the residue chromatographed over silca gel eluting with 0 - 10% [9:1 methanol/20 M ammonia] in dichloromethane to give the title compound (0.147 g).
ESMS m/z [MH]⁺= 478.2.

### f) 6-({2-[[2-({[(1,1-dimethylethyl)oxy]carbonyl}amino)ethyl](methyl)amino] ethyl}thio)-1-ethyl-4-oxo-1,4-dihydro-3-quinolinecarboxylic acid.

**Intermediate 57e** (0.132 g, 0.27 mmol) in dioxan (5 mL), water (1 mL) and 2M sodium hydroxide (1.3 mL) were stirred at room temperature. After 20 h soli d carbon dioxide was added, pH 9, and the solvent evaporated to yield the title compound a white solid (0.104 g). ESMS m/z [MH]⁺= 450.2.

### g) 6-({2-[(2-aminoethyl)(methyl)amino]ethyl}thio)-1-ethyl-4-oxo-1,4-dihydro-3-quinolinecarboxylic acid bis-trifluoroacetate.

A solution of **Intermediate 57f** (0.104 g, 0.23 mmol) in trifluoroacetic acid (5 mL) was stirred at room temperature. After 15 mins the excess trifluoroacetic acid was evaporated and the resultant crude product subjected to reverse phase chromatography over C18 silica eluting with 1% aqueous trifluoroacetic acid/acetonitrile yield the title compound as a colourless gum ( 0.134 g)
ESMS m/z [MH]⁺= 350.

### Intermediate 58

### 9-({2-[(2-aminoethyl)oxy]ethyl}amino)-5-methyl-1-oxo-6,7-dihydro-1H,5H-pyrido[3,2,1-ij]quinoline-2-carboxylic acid

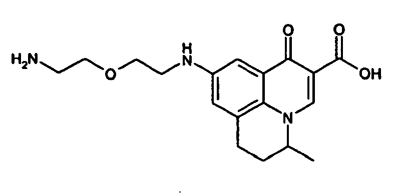

A mixture of 9-fluoro-5-(R*S*)-methyl-1-oxo-6,7-dihydro-1*H*,5*H*-pyrido[3,2,1-*ij*]quinoline-2-carboxylic acid (0.13 g, 0.5 mmol) {2-[(2-aminoethyl)oxy]ethyl}amine (0.25 mL, 2.4 mmol) in *N*-methyl pyrrolidinone (0.25 mL) was subjected to microwave irradiation resulting in an internal temperature of 200°C. After 0.5 h the mixture was cooled and purified by mass directed autoprep eluting with formic acid/water/acetonitrile to yield the title compound as a yellow oil (0.146 g).
ESMS m/z [MH]⁺= 346.4.

### Intermediate 59

### 6-({2-[(2-aminoethyl)thio]ethyl}oxy)-1-ethyl-4-oxo-1,4-dihydro-3-1uinolinecarboxylic acid

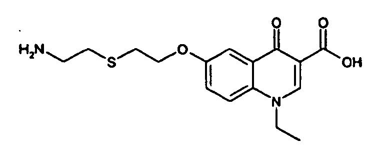

### a) 1-ethyl-6-[(2-hydroxyethyl)oxy]-4-oxo-1,4-dihydro-3-quinolinecarboxylic acid ethyl ester.

A mixture of ethyl 1-ethyl-6-hydroxy-4-oxo-1,4-dihydro-3-quinolinecarboxylate (0.261 g, 1.0 mmol), potassium carbonate (0.345 g, 2.5 mmol) and iodoethanol (0.078 mL, 1.0 mmol) in DMF (3 mL) was heated at 80°C after which time a further portion of potassium carbonate (0.345 g) and iodoethanol (0.078 mL) was added. Heating was continued for 16 h. The mixture was cooled, concentrated and the residue chromatographed over silica gel eluting with with 0 - 20% methanol in ethyl acetate to give the title compound (0.144 g).
ESMS m/z [MH]⁺= 262.2.

### b) 1-ethyl-6-({2-[(methylsulfonyl)oxy]ethyl}oxy)-4-oxo-1,4-dihydro-3-quinolinecarboxylic acid ethyl ester.

A suspension of **Intermediate 59a** (0.117 g, 0.38 mmol) in a mixture of dichloromethane (5 mL) and triethylamine (0.212 mL) was treated with methanesulfonyl chloride (0.065 mL, 0.845 mmol). After 1 h the mixture was concentrated to yield the crude methanesulfonate ester which was used without further purification.
ESMS m/z [MH]⁺= 384.3.

### c) 6-[(2-{[2-({[(1,1-dimethylethyl)oxy]carbonyl}amino)ethyl]thio}ethyl)oxy]-1-ethyl-4-oxo-1,4-dihydro-3-quinolinecarboxylic acid ethyl ester.

**Intermediate 59b** (0.147 g, 0.38 mmol) was suspended in DMF (5mL) and treated with sodium iodide (0.058 g, 0.384 mmol), 1,1-dimethylethyl (2-mercaptoethyl)carbamate (0.047 mL, 0.576 mmol) and potassium carbonate (0.265 g, 1.42 mmol). After 4.5 h at room temperature a further portion of 1,1-dimethylethyl (2-mercaptoethyl)carbamate (0.097 mL, 1.18 mmol), potassium carbonate (0.08 g, 0.57 mmol) and DMSO (5mL) was added. After stirring overnight the mixture diluted with dichloromethane and filtered. The fitlrate was evaporated and subjected to freeze- drying to remove the DMSO. The residue was purified by mass-directed autoprep eluting with formic acid/water/acetonitrile to yield the title compound (0.113 g).
ESMS m/z [MH]⁺= 465.3.

### d) 6-[(2-{[2-({[(1,1-dimethylethyl)oxy]carbonyl}amino)ethyl]thio}ethyl)oxy]-1-ethyl-4-oxo-1,4-dihydro-3-quinolinecarboxylic acid.

To a solution of **Intermediate 59c** (0.113 g, 0.24 mmol) in THF ( 3 mL) was added 2M sodium hydroxide ( 0.134 mL, 0.26 mmol). After stirring at 50°C for 18 h the mixture was cooled and treated with solid carbon dioxide. Evaporation yielded the title compound (0.130 g).
ESMS *m*/*z* [M+H]⁺= 437.3.

### e) 6-({2-[(2-aminoethyl)thio]ethyl}oxy)-1-ethyl-4-oxo-1,4-dihydro-3-quinolinecarboxylic acid trifluoroacetate.

To a solution of **Intermediate 59d** (0.130 g, 0.24 mmol) in dichloromethane (5 mL) was added trifluoroacetic acid (2 mL). After stirring for 0.5 h the mixture was concentrated to yield the title compound (0.11 g).
ESMS *m*/*z* [M+H]⁺ = 337.3.

### Intermediate 60

### 6-{3-[(2-aminoethyl)oxylpropyl}-1-ethyl-4-oxo-1,4-dihydro-3-quinolinecarboxylic acid

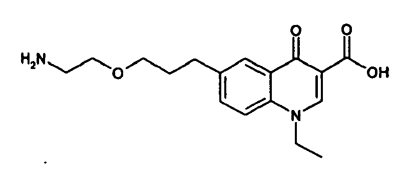

### a) 6-(3-{[2-({[(1,1-dimethylethyl)oxy]carbonyl}amino)ethyl]oxy}-1-propyn-1-yl)-1-ethyl-4-oxo-1,4-dihydro-3-quinolinecarboxylic acid ethyl ester.

A solution of 1-ethyl-6-iodo-4-oxo-1,4-dihydro-3-quinolinecarboxylic acid ethyl ester (4.95 g, 15.6 mmol) and copper iodide (0.03 g) in dry acetonitrile (120 mL) and triethylamine ( 76 mL) was deoxgenated by purging with argon for 20 mins. The resultant mixture was treated with bis-tri-phenylphoshpino palladium dichloride (0.13 g) and 1,1-dimethylethyl [2-(propyloxy)ethyl]carbamate (4.05 g, 20.3 mmol). After 3 h the mixture was concentrated and the residue chromatographed over silica gel eluting with with 0 - 4% [9:1 methanol/20 M ammonia] in dichloromethane to give the title compound ( 6.71 g).
ESMS *m*/*z* [M+H]⁺= 443.2.

### b) 6-(3-{[2-({[(1,1-dimethylethyl)oxy]carbonyl}amino)ethyl]oxy}propyl)-1-ethyl-4-oxo-1,4-dihydro-3-quinolinecarboxylic acid ethyl ester.

A mixture of **Intermediate 60a** (6.76 g, 15.1 mmol) and 10% palladium charcoal (0.7 g) suspended in ethanol (250 mL) was hydrogenated at atmospheric pressure and room temperature. After 4 h the reaction was filtered and the solvent evaporated to give the title compound as a yellow foam (5.95 g).
ESMS *m*/*z* [M+H]⁺= 447.2.

### c) 6-(3-{[2-({[(1,1-dimethylethyl)oxy]carbonyl}amino)ethyl]oxy}propyl)-1-ethyl-4-oxo-1,4-dihydro-3-quinolinecarboxylic acid.

A solution of **Intermediate 60b** (1.07 g, 2.40 mmol) in dioxan (35 mL) and ethanol (10 mL) was treated with 1M sodium hydroxide (27 mL). After 4 h solid carbon dioxide was added the mixture was concentrated to yield the title compound as a white solid (1.0 g).
ESMS *m*/*z* [M+H]⁺ = 419.2.

### d) 6-{3-[(2-aminoethyl)oxy]propyl}-1-ethyl-4-oxo-1,4-dihydro-3-quinolinecarboxylic acid trifluoroacetic acid.

A mixture of **Intermediate 60c** and trifluoroacetic acid was stirred at room temperature. After 0.5 h the solvent was evaporated and the residue subjected to reverse phase chromatography over C18 silica eluting with 1% trifluoroacetic acid/water/acetonitrile. The resultant oil was dissolved in methanol and precipitated with diethyl ether to yield the title compound as a white soild (0.43 g).
ESMS *m*/*z* [M+H]⁺= 319.2.

### Intermediate 61:

### 6-[2-({2-[(2-Aminoethyl)oxy]ethyl}oxy)ethyl]-1-ethyl-4-oxo-1,4-dihydro-3-quinolinecarboxylic acid hydrochloride

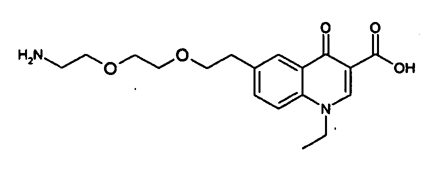

### a) 1-Ethyl-6-[2-({2-[(2-hydroxyethyl)oxy]ethyl}oxy)ethyl]-4-oxo-1,4-dihydro-3-quinolinecarboxylic acid ethyl ester

To a stirred mixture of 1,4-dihydro-6-iodo-4-oxo-quinoline-3-carboxylic acid ethyl ester (8.55 g, 23.0 mmol), triethylamine (4.82 mL, 34.6 mmol), and 2-{[2-(ethenyloxy)ethyl]oxy}ethanol (6.29 mL, 46.1 mmol) in toluene (25 mL) was added 10% palladium on charcoal (0.245 g) and the mixture heated to 100°C. After 3 h the solvent was removed *in vacuo* to give a residue which was taken up in ethyl acetate and filtered through celite. The filtrate was washed with an aqueous solution of sodium dihydrogen phosphate, dried (Na₂SO₄), filtered, and concentrated *in vacuo* to give a mixture containing 1-ethyl-6-[2-({2-[(2-hydroxyethyl)oxy]ethyl}oxy)ethenyl]-4-oxo-1,4-dihydro-3-quinolinecarboxylic acid ethyl ester as a brown oil (12.13 g), ESMS *m*/*z* 376.3 [M+H]⁺ which was used without further purification. A solution of this material in ethyl acetate (50 mL), and dichloromethane (100 mL) was hydrogenated over 10% palladium on charcoal (2 g) at atmospheric pressure, with additional 10% palladium on charcoal (5 g) added during the course of the reaction. After 72 h the catalyst was removed by filtration, and the filtrate concentrated *in vacuo* to give a residue which was purified by flash chromatography (silica gel, 0-7% methanolic ammonia [2M] in dichloromethane) to give a mixture (4.21 g) which was taken up dichloromethane (140 mL) and hydrogenated over 10% palladium on charcoal (2 g) at atmospheric pressure for 14 h. Further 10% palladium on charcoal (2.6 g) was then added and the mixture hydrogenated at 45 p.s.i. for 29 h. The mixture was then filtered and concentrated *in vacuo* to give a residue which was purified by flash chromatography (silica gel, 0-6% methanolic ammonia [2M] in dichloromethane) to give a mixture which was taken up in ethyl acetate, washed with an aqueous solution of sodium dihydrogen phosphate, dried (Na₂SO₄), filtered, and concentrated *in vacuo* to give the title compound as a yellow/brown oil (2.15 g).
ESMS *m*/*z* [M+H]⁺= 378.2.

### b) 6-(10,10-Dioxido-3,6,9-trioxa-10-thiaundec-1-yl)-1-ethyl-4-oxo-1,4-dihydro-3-quinolinecarboxylic acid ethyl ester

A solution of **Intermediate 61a** (1.58 g, 4.17 mmol) in dichloromethane (30 mL) at 0°C was treated with triethylamine (0.99 mL, 7.10 mmol), followed by methanesulfonyl chloride (0.42 mL, 5.43 mmol), and the mixture stirred for 2 h. Saturated sodium hydrogen carbonate solution (20 mL) was then added and the organic solvent removed *in vacuo*. The aqueous mixture was adjusted to pH 11 by the addition of an aqueous solution of sodium carbonate, then extracted with ethyl acetate. The organic layers were combined, dried (Na₂SO₄), filtered, and concentrated *in vacuo* to give the title compound as a pale yellow gum (2.04 g).
ESMS *m*/*z* [M+H]⁺ = 456.3.

### c) 6-[2-({2-[(2-Azidoethyl)oxy]ethyl}oxy)ethyl]-1-ethyl-4-oxo-1,4-dihydro-3-quinolinecarboxylic acid ethyl ester

A solution of **Intermediate 61b** (1.90 g, 4.17 mmol) in dichloromethane (20 mL) was treated with 1,1,3,3-tetramethylguanidinium azide (1.95 g, 12.3 mmol) and stirred at room temperature for 21 h, then at reflux for 27 h. Additional 1,1,3,3-tetramethylguanidinium azide (0.30 g, 1.90 mmol) was added, and the mixture heated at reflux for a further 8 h. The mixture was concentrated *in vacuo* to give a residue which was taken up in ethyl acetate, washed with water, dried (Na₂SO₄), filtered, and concentrated *in vacuo* to give a residue which was purified by flash chromatography (silica gel, 0-6% methanolic ammonia [2M] in dichloromethane) to give the title compound as a colourless gum (1.49 g).
ESMS *m*/*z* [M+H]⁺= 403.3.

### d) 6-[2-({2-[(2-Azidoethyl)oxy]ethyl}oxy)ethyl]-1-ethyl-4-oxo-1,4-dihydro-3-quinolinecarboxylic acid

A solution of **Intermediate 61c** (1.47 g, 3.64 mmol) in 1,4-dioxane (20 mL) was treated with 2 N aqueous sodium hydroxide (3.64 mL). After 20 h the mixture was concentrated *in vacuo* to give a residue which was taken up in water, and treated with excess solid carbon dioxide. The resulting precipitate was removed by filtration and dried *in vacuo* to give the title compound as a cream solid (1.09 g).
ESMS *m*/*z* [M+H]⁺= 375.2.

### e) 6-[2-({2-[(2-Aminoethyl)oxy]ethyl}oxy)ethyl]-1-ethyl-4-oxo-1,4-dihydro-3-quinolinecarboxylic acid hydrochloride

A solution of **Intermediate 61d** (1.07 g, 2.87 mmol) in tetrahydrofuran (30 mL) was treated with triphenylphosphine (1.50 g, 5.73 mmol) and stirred for 20 min. Water (2 mL) was added and stirring continued for 21 h. The solvent was then removed *in vacuo* to give a residue which was taken up in hydrochloric acid (2 N) and washed with ethyl acetate. The aqueous solution was concentrated *in vacuo* to give a residue which was taken up in water and the solution lyophilised to give the title compound as a cream solid (0.63 g).
ESMS *m*/*z* [M+H]⁺= 349.3.

### Intermediate 62

### O-(9E)-Hydroximino-4"-O-propenoyl erythromycin A

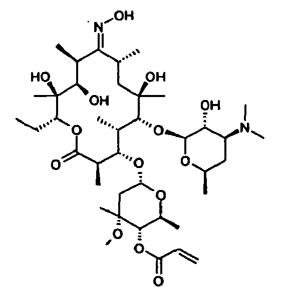

The title compound (1.0 g) was prepared according to procedure described for **Intermediate 14** starting from 2'-*O*-acetyl-*O*-(9*E*)-acetylhydroximino erythromycin A.
ESMS *m*/*z* [M+H]⁺ = 803.5.

### Intermediate 63

### O-(9E)-methoxymethyloximino-4"-O-propenoyl erythromycin A

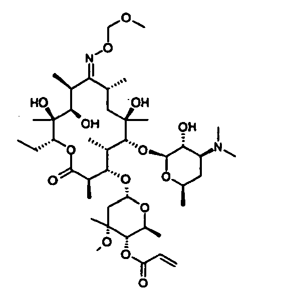

The title compound (1.43 g) was prepared according to procedure described for **Intermediate 14** starting from 2'-*O*-acetyl-(9*E*)-methoxymethyloximino-erythromycin A.
ESMS *m*/*z* [M+H]⁺= 877.7.

### Reference example 1

### 4"-O-(3-{4-[3-(3-Ethoxycarbonyl-1-ethyl-4-oxo-1,4-dihydro-quinolin-6-yl)-prop-2-ynyl]-piperazin-1-yl}-propionyl)-azithromycin

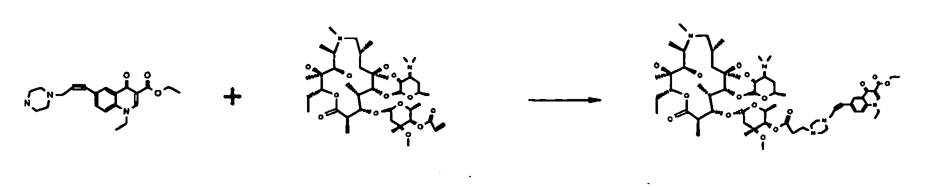

To the solution of 4"-O-acryloyl-azitromycin (0.4916 g, 0.613 mmol) in acetonitrile (20 mL) **Intermediate 5** (0.9 mg, 2.45 mmol), triethylamine (0.273 mL) and water (0.685 mL) were added and suspension was heated at 90 °C for 24 hours. The solvent was evaporated and the residue was extracted with DCM and water (2x50 mL). Organic layer was washed with NaCl and NaHCO₃ (2x50 mL), dried over K₂CO₃ and evaporated in vacuum. The product was precipitated from EtOAc/n-hexane yielding product (0.35 g). Product (0.17 g) was purificated by column chromatography (DCM: MeOH: NH₃ = 90:3:0.5) to yield title product (0.05 g).
MS (ES+) m/z : [MH]⁺ = 1170.6
**¹H-NMR(500 MHz, CDCl₃)** δ: 8.57 (1H, Q), 8.53 (1H, Q), 7.68 (1H, Q), 7.41 (1H, Q), 5.21 (1H, H-1"), 4.70 (2H, H-13+H-4"), 4.56 (1H, H-1'), 4.38 (3H, Q+H-5"), 4.25(3H, Q+H-3), 3.82 (1H, H-5'), 3.68 (1H, H-11), 3.61 (1H, H-5), 3.53 (2H, CH2), 3.31 (3H, H-3"OMe), 3.25 (1H, H-2"), 2.71 (12H, H-2, H-10, CH2, 4xCH2-P), 2.54 (4H, H-9a, H-3', CH2), 2.38 (1H, H-2"a), 2.31 (6H, 3'NMe2), 2.02 (3H, H-4, H-8, H-9b), 1.91 (1H, H-14a), 1.75 (1H, H-7a), 1.68 (1H, H-4'a), 1.62 (1H, H-2"b), 1.55 (3H, Q), 1.48 (1H, H-14b), 1.41 (3H, Q), 1.30 (3H, 6Me), 1.28 (1H, H-2'b), 1.25 (1H, H-7b), 1.19 (6H, 5'Me+2Me), 1.18 (3H, 5"Me), 1.14 (3H, 3"Me), 1.10 (3H, 12e), 1.09 (3H, 10Me), 1.04 (3H, 4Me), 0.89 (6H, 8Me+14Me)
**¹³C-NMR(75 MHz, CDCl₃)** δ: 178.99, 173.57, 172.02, 165.67, 148.56, 138.05, 135.40, 131.57, 129.16, 120.12, 115.69, 111.57, 102.35, 85.98, 84.13, 83.21, 78.81, 77.67, 77.48, 74.24, 73.65, 73.55, 73.06, 70.92, 70.11, 67.84, 65.70, 63.02, 62.57, 61.00, 53.46, 52.80, 52.11, 49.43, 48.93, 47.71, 45.23, 42.29, 42.20, 40.39, 36.23, 34.96, 32.50, 29.70, 29.01, 27.60, 26.80, 21.98, 21.89, 21.35, 21.31, 17.83, 16.22, 14.54, 14.50, 14.45, 11.29, 9.03, 7.34.

### Example 2

### 4"-O-(3-{4-[3-(3-Ethoxycarbonyl-1-ethyl-4-oxo-1,4-dihydro-quinolin-6-yl)-propyl]-piperazin-1-yl}-propionyl)-azithromycin

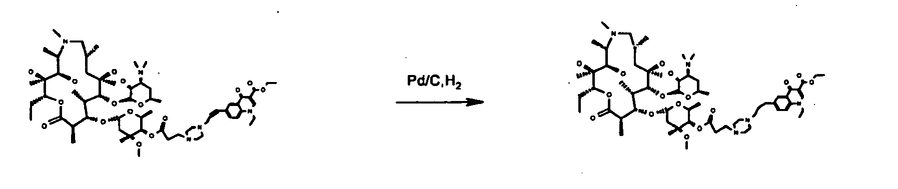

Hydrogenation of **Reference example 1** (0.25 mg) in methanol (20 mL) with addition of 10 % Pd/C (100 mg) at 5 bar for 20 hours yielded the title product.
MS (ES+) m/z : [MH]⁺ = 1174.8
**¹³C-NMR(75 MHz, DMSO)** δ: 177.61, 173.24, 172.11, 165.24, 164.40, 163.56, 158.99, 154.18, 148.98, 139.37, 137.31, 133.76, 129.03, 125.90, 116.94, 110.30, 102.61, 94.91, 83.91, 78.45, 77.97, 76.89, 75.46, 74.11, 73.18, 72.99, 71.03, 69.18, 67.28, 65.40, 62.73, 62.00, 53.13, 49.33, 48.72, 45.13, 72.28, 42.20, 40.86, 40.82, 36.29, 34.84, 32.99, 32.73, 30.69, 28.47, 27.98, 25.93, 22.61, 22.32, 21.48, 21.24, 18.29, 18.22, 15.16, 14.95, 14.85, 11.48, 9.50, 7.30.

### Reference example 3

### 4"-O-(3-{4-[3-(3-Ethoxycarbonyl-1-ethyl-4-oxo-1,4-dihydro-quinolin-6-yl)-prop-2-ynyl]-piperazin-1-yl}-propionyl)-6-O-methyl-erythromycin A

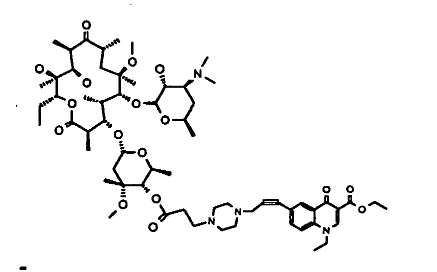

Using the procedure of **Reference example 1, Intermediate 5 and Intermediate 20** gave the title compound.
MS (ES+) m/z : [MH]⁺ = 1169.9
**¹H-NMR(500 MHz, CDCl₃)** δ: 8.58 (1H, Q), 8.48 ((1H, Q), 7.68(1H, Q), 7.39 (1H, Q), 5.07 (1H, H-13), 4.98 (1H, H-1''), 4.68 (1H, H-4"), 4.60 (1H, H-1'), 4.39 (2H, CH₂-Q), 4.34 (1H, H-5"), 4.25 (2H, CH₂-Q), 3.77 (1H, H-3), 3.76 (2H, H-5'+H-11), 3.65 (1H, H-5), 3.53 (2H, CH₂), 3.30 (3H, 3''OMe), 3.22 (1H,H-2'), 3.03 (3H, 6OMe), 2.98 (1H, H-10), 2.91 (1H, H-2), 2.74 (10H, CH₂+4xCH₂-P), 2.57 (4H, CH₂+H-8+H-3'), 2.40 (1H, H-2"a), 2.35 (6H, 3'NMe₂), 1.94 (1H, H-4), 1.89 (1H, H-14a), 1.83 (1H, H-7a), 1.70 (1H, H-4'a), 1.65 (1H, H-2"b), 1.61 (1H, H-7b), 1.55 (3H, CH₃-Q), 1.47 (1H, H-14b), 1.41 (3H, CH₃-Q), 1.37 (3H, 6Me), 1.24 (1H, H-4'b), 1.20 (3H, 5'Me), 1.19 (3H, 2Me), 1.15 (3H, 5"Me), 1.13 (3H, 3"Me), 1.12 (6H, 8Me+10Me), 1.10 (3H, 4Me), 0.84 (3H, 15Me).
**¹³C-NMR(75 MHz, CDCl₃)** δ: 175.11, 172.97, 171.32, 165.00, 147.97, 137.45, 134.76, 130.99, 128.55, 119.49, 115.07, 110.97, 101.38, 95.41, 85.34, 83.53, 79.901, 78.08, 77.67, 77.38, 76.02, 73.70, 72.21, 70.47, 68.51, 67.13, 64.78, 62.42,60.39,39.74, 38.61, 38.16, 36.62, 34.61, 31.93, 29.10, 21.25, 20.54, 20.45, 19.11, 17.78, 17.45, 15.38, 13.93, 13.84, 13.53, 11.77, 10.00, 8.58.

### Reference example 4

### 4"-O-(3-{4-[3-(3-Ethoxycarbonyl-1-ethyl-4-oxo-1,4-dihydro-quinolin-6-yl)-prop-2-ynyl]-piperazin-1-yl}-propionyl)-11-O-methyl-azithromycin

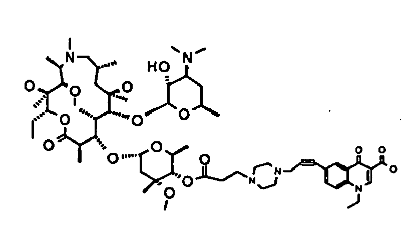

Using the procedure of **Reference example 1, Intermediate 5 and Intermediate 14** gave the title compound.
MS (ES+) m/z : [MH]⁺ = 1156.5
**¹H-NMR(500 MHz, CDCl₃)** δ: 8.77 (1H, Q), 8.59 (1H, Q), 7.85 (1H, Q), 7.59 (1H, Q), 5.15 (1H, H-1"), 4.71 (1H, H-4"), 4.67 (1H, H-13), 4.60 (1H, H-1'), 4.40 (1H, H-5"), 4.39 (2H, CH₂-Q), 4.37 (1H, H-3), 3.82 (1H, H-5'), 3.63 (1H, H-5), 3.58 (3H, 11OM), 3.55, (2H, CH₂),3.40 (1H, H-11), 3.32 (3H, 3''OMe), 3.29 (1H, H-2'), 2.74 (1H, H-2), 2.68 (10H, CH₂+4xCH₂-P), 2.55 (3H, H-9a+CH₂), 2.38 (1H, H-2"a), 2.07 (1H, H-9b), 2.02 (2H, H-4+H-8), 1.92 (1H, H-14a), 1.68 (1H, H-7a), 1.56 (3H, CH₂-Q+H-2''a), 1.43 (1H, H-14b), 1.31 (1H, H-7b), 1.25 (3H, 6Me), 1.21 (6H, 2Me+5'Me), 1.15 (3H, 5"Me), 1.14 (3H, 3"Me), 1.11(3H, 12Me), 1.03 (6H, 4Me+10Me), 0.91 (3H, 8Me), 0.87 (3H, 15Me).
**¹³C-NMR(75 MHz, CDCl₃)** δ: 177.91, 177.88, 172.06, 166.80, 147.98, 138.35, 136.78, 130.64, 126.59, 121.57, 116.45, 109.39, 101.99, 94.79, 87.41, 85.01, 83.77,83.47, 78.96, 78.01, 77.87, 74.42, 73.26, 73.08, 71.09, 70.96, 67.57, 65.52, 62.81, 62.70, 62.13, 53.42, 52.81, 52.05, 49.80, 49.45, 47.64, 45.44, 42.78, 42.55, 35,94, 35.11, 32.48, 29.70, 27.67, 26.75, 22.23, 21.77, 21.70, 21.35, 17.75, 17.09, 14.70, 14.65, 11.30, 9.45, 7.25.

### Example 5

### 4"-O-(3-{4-[3-(3-Ethoxycarbonyl-1-ethyl-4-oxo-1,4-dihydro-quinolin-6-yl)-propyl]-piperazin-1-yl}-propionyl)-11-O-methyl-azithromycin

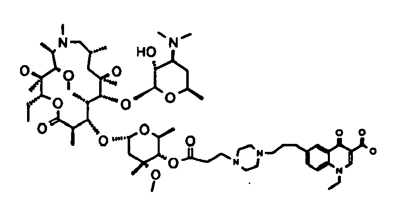

Using the procedure of **Example 2, Reference example 4** gave the title compound.
MS (ES+) m/z : [MH]⁺ = 1161.5
**¹-NMR(500 MHz, CDCl₃)** δ: 8.77 (1H, Q), 8.37 (1H, Q), 7.68 (1H, Q), 7.56 (1H, Q), 5.16 (1H, H-1''), 4.69 (2H, H-13+H-4"), 4.58 (1H, H-1'), 4.39 (4H, H-3+H-5''+CH₂-Q), 3.79 (1H, H-5'), 3.61 (1H, H-5, 3.58 (3H, 11OMe), 3.40 (1H, H-11), 3.32 (4H, 3"OMe-H-2'), 2.83 (2H, CH₂), 2.74 (3H, H-2+CH2), 2.68 (10H, 4xCH₂-P+H-10+H-3'), 2.52 (3H, CH₂+H-9a), 2.41 (1H, H-2"a), 2.36 (8H, 3'NMe₂+CH₂), 2.24 (3H, 9NMe), 2.07 (2H, H-8+H-9b), 2.00 (1H, H-4), 1.89 (3H, H-14a+CH₂), 1.73 (2H, H-7a+H-4'a), 1.60(4H, CH₃-Q+H-2''b), 1.47 (1H, H-14b), 1.28 (2H, H-7b+H-4'b), 1.26 (3H, 6Me), 1.20 (6H, 2Me+5'Me), 1.14 (3H, 5"Me), 1.13 (3H, 3"Me), 1.11 (3H, 12Me), 1.04 (6H, 4Me+10Me), 0.91 (3H, 8Me), 0.90 (3H, 15Me).
**¹³C-NMR(75 MHz, CDCl₃)** δ: 178.39, 177.95, 172.04, 167.29, 147.44, 140.81, 137.49, 134.85, 126.66, 126.35, 116.31, 108.83, 102.23, 94.82, 85.06, 83.67, 78.97, 78.10, 77.88, 74.43, 73.22, 73.17, 71.08, 70.11, 67.51, 65.51, 62.83, 62.70, 62.15, 57.45, 53.48, 53.02, 52.81, 49.70, 49.45, 45.47, 42.77, 42.62, 40.45, 35.93, 35.14, 33.01, 32.54, 29.70, 28.18, 27.67, 26.75, 22.23, 21.78, 2.1.75, 21.36, 17.75, 17.12, 14.74, 14.67, 11.29, 9.37, 7.22.

### Example 6

### 4"-O-(3-{2-[2-(3-Carboxy-7-chloro-1-cyclopropyl-4-oxo-1,4-dihydro-quinolin-6-ylamino)-ethoxy]-ethoxy}-propionyl)-roxythromycin

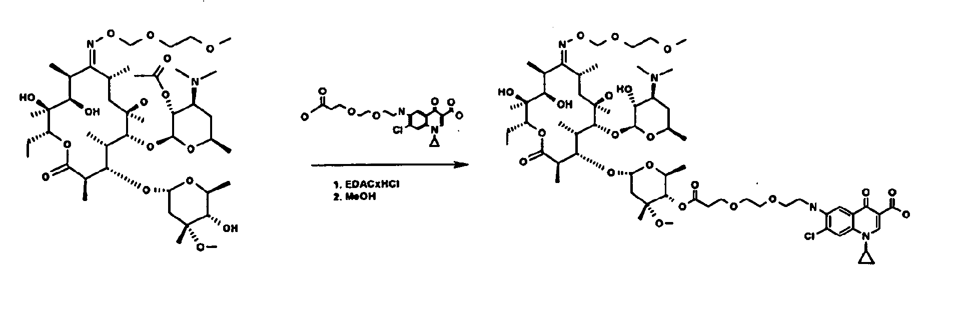

**Intermediate 7** (2.51 g, 5,7 mmol) was dissolved in 10 mL of DMF/4Å, and cooled to 0°C under N₂ atmosphere. In this solution, EDACxHCl (1.1 g, 5.7 mmol) was added and stirred for 5 min. Then, 2'-O-acetyl-roxythromycin (2.5 g, 2.84 mmol) dilluted in 10 mL of DCM/4Å, and DMAP (1.1 g, 8.55 mmol) were added and stirred for 24 h. In the reaction mixture 30 mL of EtOAc was added and it was extracted with 3x20 mL of H₂O. The organic layers were washed with brine and evaporated affording 500 mg g of 2'-O-acetyl protected product which was dissolved in 100 mL MeOH and stirred at 40°C for 24 h. MeOH was evaporated under reduced pressure. The residue was purified by column chromatography in (DCM-MeO-:NH₃ = 90:5:0.5) to yield 250 mg of the title product.
MS (ES+) m/z : [MH]⁺ = 1258.84.

### Example 7

### 4"-O-(3-{2-[2-(3-Carboxy-7-chloro-1-cyclopronyl-4-oxo-1,4-dihydro-quinolin-6-ylamino)-ethoxy]-ethoxy}-propionyl)-6-O-methyl-erythromycin A

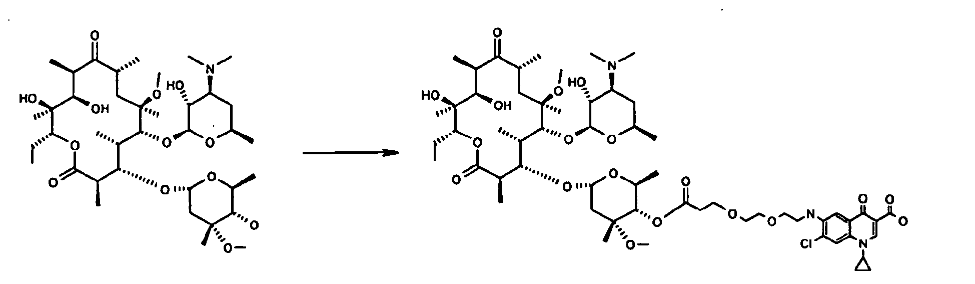

Using the procedure of **Example 6, Intermediate 7** and 2'-O-acetyl-6-O-methyl-erythromycin A gave the title compound.
MS (ES+) m/z : [MH]⁺ = 1169.79.

### Example 8

### 4"-O-(3-{2-[2-(3-Carboxy-7-chloro-1-cyclopropyl-4-oxo-1,4-dihydro-quinolin-6-ylamino)-ethoxy]-ethoxy}-propionyl)-azithromycin

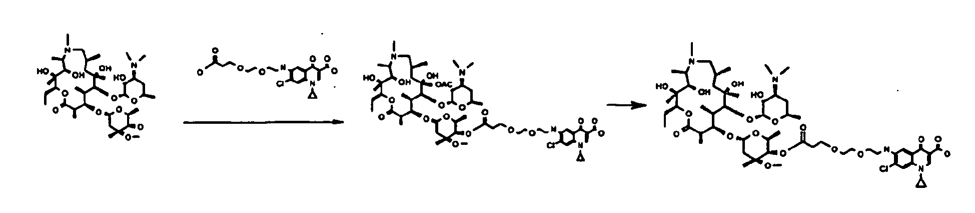

Using the procedure of **Example 6, Intermediate 7** (1.7 g, 3.87 mmol), 2'-O-acetyl-azithromycin (6.1 g, 7.72 mmol), EDACxHCl (1.47 g, 7.72 mmol) and DMAP (943 mg, 7.72 mmol) gave the title compound (500 mg).
MS (ES+) m/z : [MH]⁺ = 1169.37.
**¹H-NMR (500 MHz, CDCl₃)** δ: 8.73 (s, 1H), 8.05 (s, 1H), 7.55 (s, 1H), 5.18 (d, 1H), 5.04 (t, 1H), 4.70 (d, 2H), 4.69 (d, 2H), 4.55 (d, 1H), 4.42 (m, 1H), 4.43 (d, 1H), 4.25 (s, 1H), 3.80 (m, 2H), 3.79(m, 2H), 3.78 (m, 2H), 3.76 (m, 2H), 3.68 (m, 2H), 3.60 (m, 2H), 3.57 (q, 3H), 3.52 (q, 2H), 3.37 (m,3H), 3.31 (m, 2H), 2.74 (m, 3H), 2.70 (m, 2H), 2.68(m, 2H), 2.63 (m, 3H), 2.55 (m, 2H), 2.45 (m, 2H), 2.35 (m, 2H), 2.31 (q, 2H), 2.08 (m, 1H), 2.07 (m, 2H), 2.05 (m, 2H), 1.91 (q, 1H), 1.78 (d, 2H), 1.76 (d, 2H), 1.74 (d, 1H), 1.62 (d, 1H), 1.39 (q, 2H), 1.28 (q, 2H), 1.26 (m, 2H), 1.20 (m, 3H), 1.21 (m, 2H), 1.12 (d, 3H), 1.11 (m, 3H), 1.10 (d, 4H), 1.08 (d, 2H), 1.04 (d, 2H), 0.91 (m, 3H).
**¹³C-NMR(75 MHz, CDCl₃)** δ: 178.29, 176.95, 170.72, 166.70, 145.33, 142.39, 132.08, 127.03, 125.71, 117.48, 107.06, 104.03, 101.62, 94.05, 82.62, 78.35, 77.09, 76.87, 76.45, 76.02, 73.67, 73.05, 73.01, 72.36, 70.36, 69.88, 69.81, 69.47, 68.29, 67.16, 66.10, 65.00, 62.38, 61.95, 48.84, 44.57, 42.72, 41.61, 41.51, 39.83, 35.69, 34.76, 34.54, 34.36, 30.99, 28.85, 26.93, 26.18, 22.06, 21.38, 21.19, 20.71, 20.58, 17.19, 15.60, 13.96, 13.52, 10.69, 8.52, 7.52, 6.84,

### Example 9

### 4"-O-(3-{2-[2-(3-carboxy-7-chloro-1-cyclopropyl-4-oxo-14-dihydro-quinolin-6-ylamino)-ethoxy]-ethoxy}-propionyl)-11-O-methyl-azithromycin

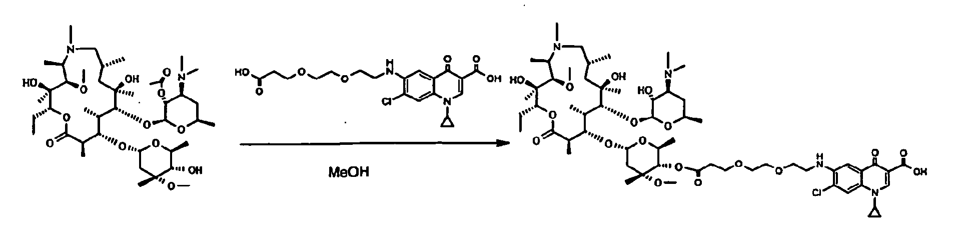

Using the procedure of **Example 6, Intermediate 7** and 2'-O-acetyl-11-*O*-methyl-azithromycin gave the title compound. Product was purified by column chromatography (eluent CH₂Cl₂-MeOH-NH₃:90:15:1.5) and precipitated twice from EtOAc: n- hexane yielding 95.8 % pure the title compound.
MS (ES+) m/z : [MH]⁺ = 1184.1.

### Example 10

### Acetate salt of Example 9

To a solution of **Example 9** (0.3g, 0.25 mmol) in EtOAc (2.5 mL) acetic acid (0.032 mL, 0.56 mmol) was added under stirring in an ice bath. Addition of diisopropylether (15 mL) and n-hexane (30 mL) yielded 0.289g of precipitated acetate salt. (92.5% pure).

### Example 11

### 4"-O-(3-{2-[2-(3-Carboxy-1-cyclopropyl-4-oxo-1,4-dihydro-quinolin-6-ylamino)-ethoxy]-ethoxy}-propionyl)-azithromycin

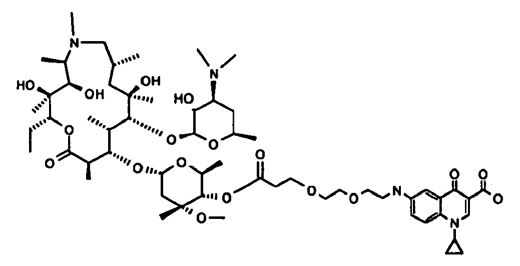

**Example 8** (300 mg, 0.26 mmol) was dissolved in 20 mL of ethanol, 100 mg of 10 % Pd/C was added and the mixture was stired under H₂ pressure (5 bar) for 15 hours.
Coloumn chromatography in (DCM-MeOH-NH₃ = 90:5:0.5) yielded 200 mg of the title compound.
MS (ES+) m/z : [MH]⁺ = 1136.38.

### Example 12

### 4"-O-(2-{3-[2-(3-Carboxy-7-chloro-1-cyclopropyl-4-oxo-1,4-dihydro-quinolin-6-yloxy)-ethoxy]-propionylamino}-acetyl)-azithromycin 11,12-cyclic carbonate

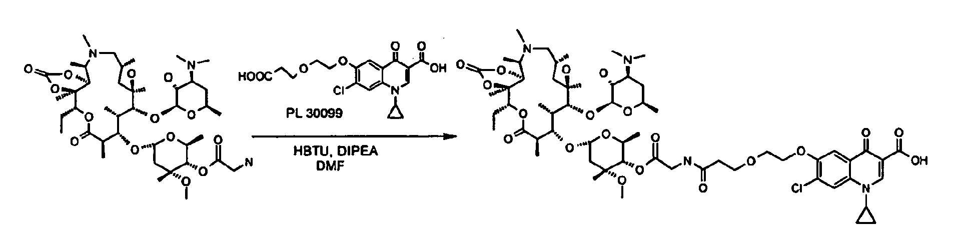

To the solution of **Intermediate 26** (100mg, 0.25mmol) and HBTU (91.04mg, 0.24mmol) in DMF (720 µL) was added DIPEA (61.6 µL, 0.35mmol). Reaction mixture was stirred for 20 min and **Intermediate 11** (147.12 mg, 0.18mmol) was added in portions through 2-3 min. Stirring was continued for 3 days at room temperature. Then, 10 mL of H₂O was added and extracted with 3x40 mL of EtOAc. The organic layer was washed with NaHCO₃ (3x20 mL) and NaCl (3x20 mL). The organic layer was dried over Na₂SO₄ and evaporated in vacuum. The crude product was purified via SPE chromatography in system (CH₂Cl₂-MeOH-NH₄OH = 90:9:0.5) giving 24.17 mg of the title compound (purity 96.3%).
MS (ES+) m/z : [MH]⁺ = 1210.6
**¹³C-NMR(125 MHz, CD₃OD)** δ: 5.7, 8.6, 10.15, 14.56, 20.37, 22.32, 23.31, 23.82, 23.86, 27.46, 27.71, 30.59, 30.87, 31.86, 32.87, 33.19, 35.09, 35.99, 36.76, 37.58, 40.54, 42.15, 43.04, 43.72, 46.53, 48.64, 48.81, 48.98, 49.15, 49.32, 49.49, 49.66, 62.54, 64.21, 64.94, 66.52, 68.47, 68.64, 68.67, 70.35, 70.67, 71.79, 74.56, 75.35, 77.85, 79.26, 81.07, 84.90, 86.65, 88.10, 96.39, 102.29, 103.62, 109.16, 120.41, 128.38, 131.44, 136.96, 154.98, 171.30, 174.54, 177.93, 179.42.

### Example 13

### 4"-O-(2-{3-[2-(3-Carboxy-7-chloro-1-cyclopropyl-4-oxo-1,4-dihydro-quinolin-6-ylamino)-ethoxy]-propionylamino}-acetyl)-azithromycin 11,12-cyclic carbonate

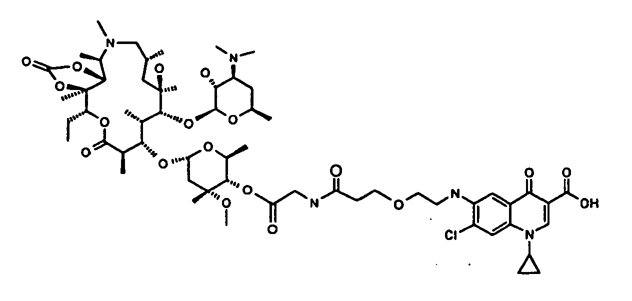

Using the procedure of **Example 12, Intermediate 11** and 6-[2-(2-carboxy-ethoxy)-ethylamino]-7-chloro-1-cyclopropyl-4-oxo-1,4-dihydro-quinoline-3-carboxylic acid gave the title compound.
MS (ES+) m/z : [MH]⁺ = 1209.1
**¹³C-NMR(75 MHz, CD₃OD)** δ: 3.59, 6.43, 7.91, 8.05, 9.01, 11.93, 12.45, 13.34, 16.44, 19.67, 20.21, 20.34, 21.20, 21.75, 25.33, 25.56, 28.48, 28.76, 29.76, 31.08, 32.95, 33.83, 34.92, 35.52, 38.43, 40.05, 40.96, 41.63, 42.39, 44.44, 46.18, 46.46, 46.74, 47.03, 47.31, 47.60, 47.88, 48.20, 52.82, 60.47, 62.08, 64.37, 66.11, 66.41, 66.57, 68.23, 69.69, 72.45, 73.22, 75.47, 77.11, 78.92, 82.76, 84.48, 85.93, 94.26, 101.06, 101.53, 103.12, 117.94, 152.84, 169.13, 172.53, 177.25.

### Example 14

### 4"-O-(2-{3-[2-(3-Carboxy-6-fluoro-1-cyclopropyl-8-methoxy-4-oxo-1,4-dihydro-quinolin-7-ylamino)-ethoxy]-propionylamino}-acetyl)-azithromycin 11,12-cyclic carbonate

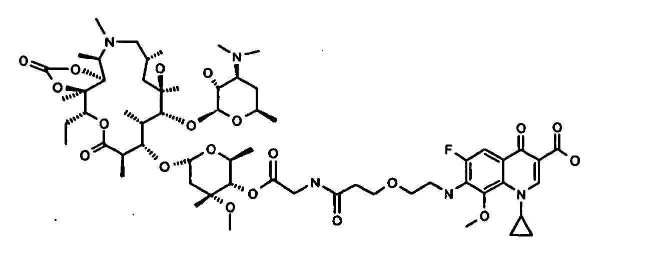

Using the procedure of **Example 12, Intermediate 11** and 7-[2-(2-carboxy-ethoxy)-ethylamino]-6-fluoro-1-cyclopropyl-8-methoxy-4-oxo-1,4-dihydro-quinoline-3-carboxylic acid gave the title compound.
MS (ES+) m/z : [MH]⁺ = 1223.6

### Example 15

### 4"-O-[2-(3-{2-[2-(3-Carboxy-6-fluoro-1-cyclopropyl-4-oxo-1,4-dihydro-quinolin-7-ylamino)-ethoxy]-ethoxy}-propionylamino)-acetyl]-azithromycin 11,12-cyclic carbonate (A)

### and

### 4"-O-[2-(3-{2-[2-(3-Carboxy-7-chloro-1-cyclopropyl-4-oxo-1,4-dihydro-quinolin-6-ylamino)-ethoxy]-ethoxy}-propionylamino)-acetyl]-azithromycin 11,12-cyclic carbonate (B)

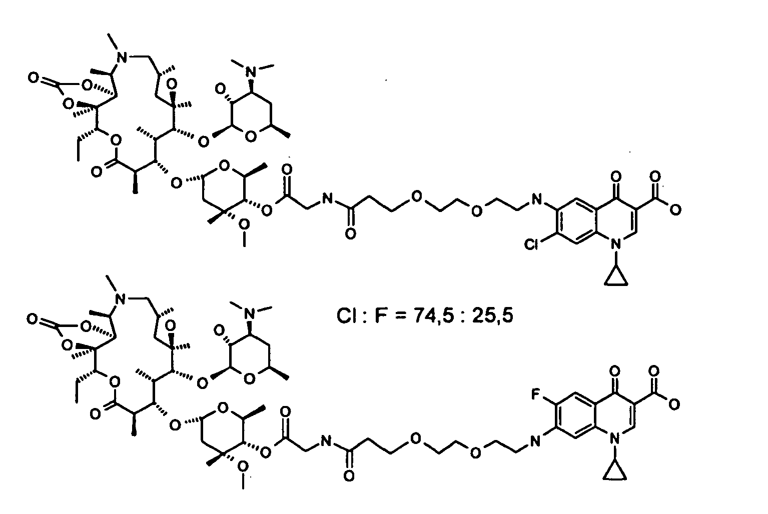

To the solution of **Intermediate 27** (800mg) and HBTU (650mg) in DMF (5.2 mL) DIPEA (450 µL) was added. **Intermediate 11** (1.5 g) was diluted in DMF (3 mL) and added to the reaction mixture. Stirring was continued over 2 days at room temperature. Then, 50 mL of H₂O was added and extracted with EtOAc (3x100 mL). The organic layer was washed with NaHCO₃ (3x100 mL) and NaCl (3x100 mL). The organic layer was dried over Na₂SO₄ and evaporated in vacuum. The product (0.73 g) was purified via SPE chromatography in system (CH₂Cl₂ -MeOH-NH₄OH = 100:6:0.1) and 102 mg of title compound was isolated (purity 93.5%) as a mixture of fluoro and chloro derivatives in a ratio 25.5 : 74.5.
MS (ES+) m/z [MH₂]²⁺ = 618.9 for **(A)**
MS (ES+) m/z [MH₂]²⁺ = 626.9 for **(B)**

### Example 16

### 4"-O-[2-(3-{2-[2-(3-Carboxy-7-chloro-1-cyclopropyl-4-oxo-1,4-dihydro-quinolin-6-ylamino)-ethoxy]-ethoxy}-propionylamino)-acetyl]-azithromycin

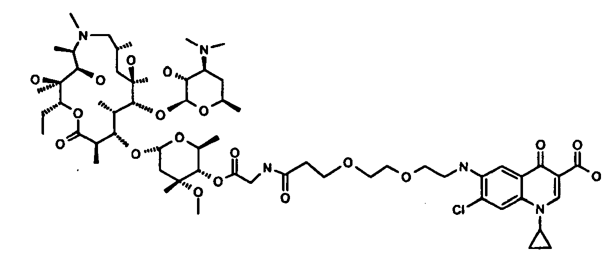

Using the procedure of **Example 15, Intermediate 7** and **Intermediate 12** gave the title compound.
MS (ES+) m/z : [MH]⁺ = 1226.4.

### Reference example 17

### 4"-O-(2-{3-({2-[3-(6-Ethoxycarbonyl-7-oxo-2,3-dihydro-1H,7H-pyrido[3,2,1-ij]quinolin-9-yl)-prop-2-ynylamino] -ethyl}-propyl-amino)-propinoylaminol-acetyl}-azithromycin

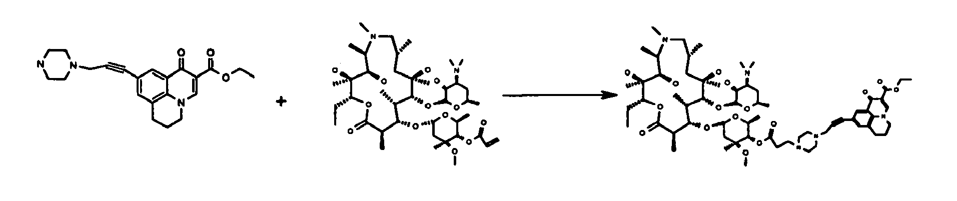

To the solution of 4"-O-acryloyl-azitromycin (116.29 mg, 0.613 mmol) in acetonitrile (20 mL) **Intermediate 15** (0.22 g, 0.58 mmol), triethylamine (0.065 mL) and water (0.162 mL) were added and suspension was heated to 85 °C for 24 hours. The solvent was evaporated and the residue was extracted with DCM and water (2x20 mL). Organic layer was washed with NaCl and NaHCO₃ (2x20 mL), dried over K₂CO₃ and evaporated in vacuum. The product was precipitated from EtOA/n-hexane yielding the crude product (0.35 g). Product (0.17 g) was purified by column chromatography (DCM-MeOH-NH₃ = 90:3:0.5) to yield the title product (0.036 g).
MS (ES+) m/z : [MH]⁺ = 1182.5
**¹H-NMR(500 MHz, CDCl₃)** δ: 8.37 (1H, Q), 8.30 (1H, Q), 7.45 (1H, Q), 5.16 (1H, H-1''), 4.70 (1H, H-4"), 4.57 (1H, H-1'), 4.41 (1H, H-5''), 4.38 (2H, CH₂-Q), 4.26 (1H, H-3), 4.17 (2H, CH₂-Q), 3.80 (1H, H-5'), 3.69 (1H, H-11), 3.63 (1H, H-5), 3.52 (2H, CH₂), 3.31 (3H, 3''OMe), 3.25 (1H, H-2'), 3.02 (2H, CH₂-Q), 2.76 (1H, H-2), 2.72 (11H, CH₂+4xCH₂-P+H-10), 2.54 (4H, CH₂+H-3'+H-9a), 2.37 (1H, H-2''a), 2.24 (2H, CH₂-Q), 2.04 (2H, H-8+H-9b), 1.89 (1H, H-14a), 1.75 (1H, H-7a), 1.67 (1H, H-4'a), 1.61 (1H, H-2"b), 1.49 (1H, H-14b), 1.40 (3H, CH₃-Q), 1.30 (3H, 6Me), 1.26 (2H, H-7b+H-4'b), 1.21 (3H, 5'Me), 1.20 (3H, 2Me), 1.16 (3H, 5"Me), 1.13 (3H, 3"Me), 1.10 (3H, 12Me), 1.09 (3H, 10 Me), 1.05 (3H, 4Me), 0.91 (3H, 8Me), 0.88 )3H, 15Me).
**¹³C-NMR(75 MHz, CDCl₃)** δ: 178.89, 173.73, 172.01, 165.68, 147.81, 135.98134.50, 129.04, 128.77, 126.91, 119.64, 110.80, 102.33, 94.67, 85.51, 84.40, 83.25, 78.83, 77.73, 77.47, 74.27, 73.81, 73.61, 73.05, 70.94, 70.11, 67.83, 65.67, 62.98, 62.50, 60.89, 53.46, 52.80, 52.73, 52.07, 49.42, 47.70, 45.15, 42.24, 42.23, 40.39, 36.30, 35.01, 32.51, 28.98, 27.56, 26.79, 26.45, 22.00, 21.88, 21.32, 21.27, 21.19, 17.86, 16.20, 14.44, 14.12, 11.28, 9.10, 7.43

### Example 18

### 4"O-{2-[3-({2-[3-(6-Ethoxycarbonyl-7-oxo-2,3-dihydro-1H,7H-pyrido[3,2,1-ij]quinolin-9-yl)-propylamino]-ethyl}-propyl-amino)-propionylamino]-acetyl}-azithromycin

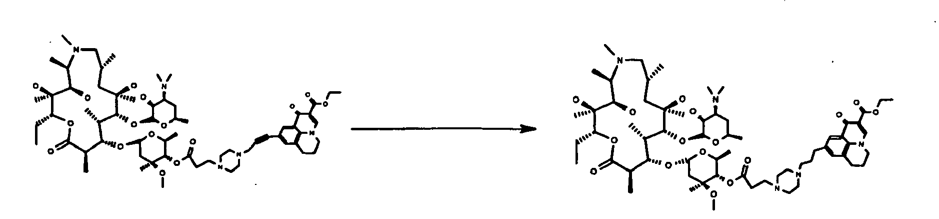

Hydrogenation of **Reference example 17** (0.04 g) in methanol (10 mL) with addition of 10 % Pd/C
(20 mg) at 5 bar for 20 hours affording the title product (0.03 g).
MS (ES+) m/z : [MH]⁺ = 1187.6

### Example 19

### 4"-O-(3-{2-[2-(3-Carboxy-7-chloro-1-cyclopropyl-4-oxo-1,4-dihydro-quinolin-6-ylamino)-ethoxy]-ethoxy}-propionyl)-9(E)-ethoxyimino-erythromycin A

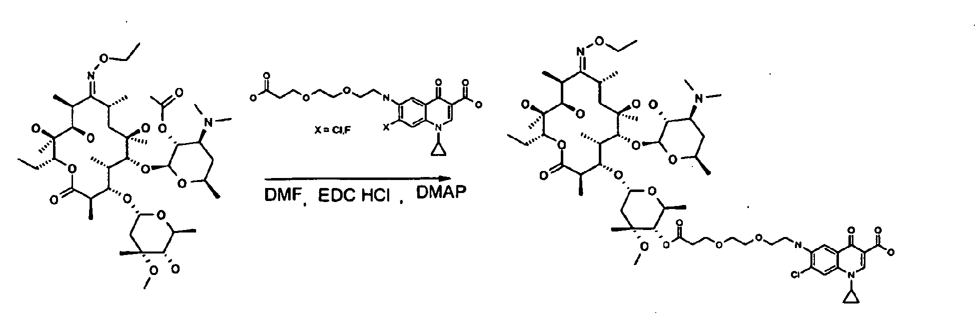

In 4 mL of dry DMF was dissolved 1.5 g of **Intermediate 27** (1 eq) and cooled on ice bath. To the solution was added EDC HCl (1.09 g; 1.5 eq) and the reaction mixture was stirred at 0 °C for 30min under the flow of N₂. Than was added a solution of 2'-*O*-acetyl-(9*E*)-ethoxyimino erythromycin A (2.48 g; 0.8 eq) in 4 mL of DMF and after 1 hour DMAP (2x463 mg; 2 eq). The resulting mixture was stirred over the weekend, during which time the reaction mixture was allowed to worm to ambient temperature. By extraction with water and DCM the layers are separated. The water layer was extracted two times with DCM. Organic layers were collected, dried on Na₂SO₄, filtered off and organic solvent evaporated. The foamy residue (260 mg) was dissolved in MeOH (20 mL) and solution was stirred over the night with heating at 60 °C. The methanol was evaporated under vacuum and the foamy residue was purified by column chromatography on silica gel with DCM-MeOH-NH₄OH = 90:5:0.5 as eluent. Collected fractions with chloro derivative were precipitated from EtOAC / hexane.
MS (ES+) m/z : [MH]⁺ = 1197

### Example 20

### 4"-O-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-6-O-methyl-erythromycin A and

### 4"-O-[3-(2-{2-[2-(3-Carboxy-6-fluoro-1-cyclopropyl-4-oxo-1,4-dihydro-quinolin-7-ylamino)-ethoxy]-ethoxy}-ethylamino)-propionyl]-6-O-methyl-erythromycin A

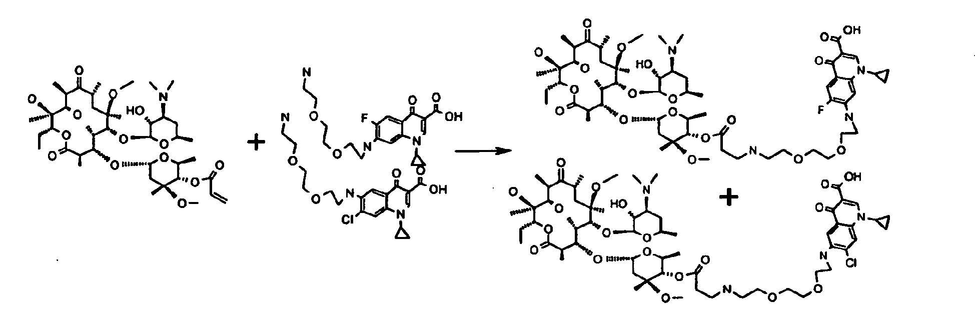

To a solution of **Intermediate 19** (0.26 mg) in CH₃CN (10 mL), H₂O (2 mL) and Et₃N (0.3 mL) was added **Intermediate 28** (0.25 mg, mixture (A) and (B) and the resulting mixture was heated at 70 °C for 17 hours. To the reaction solution was added EtOAc (50 mL) and water (50 mL), pH was adjusted to 10 and the layers were separated. The aqueous phase was washed with EtOAc (50 mL). The combined organic layers were concentrated under reduced pressure and the residue was purified on silica gel using: DCM-MeOH-NH₄OH = 90:9:1.5 affording the title compound (315 mg) as an mixture of two derivatives, 7-chloro and 6-fluoro in ratio 2:1 determined by 1H NMR.
MS (ES+) m/z : [MH]⁺ = 1195 (6-fluoro derivative)
MS (ES+) m/z : [MH]⁺ = 1211 (7-chloro derivative)

### Example 21

### 4"-O-(3-{2-{2-(3-Carboxy-7-chloro-1-cyclopropyl-4-oxo-1,4-dihydro-quinolin-6-ylamino)-ethoxyl-ethylamino}-propionyl)- 6-O-methyl-erythromycin A and

### 4"-O-(3-{2-[2-(3-Carboxy-6-fluoro-1-cyclopropyl-4-oxo-1,4-dihydro-quinolin-7-ylamino)-ethoxy]-ethylamino}-propionyl)- 6-O-methyl-erythromycin A

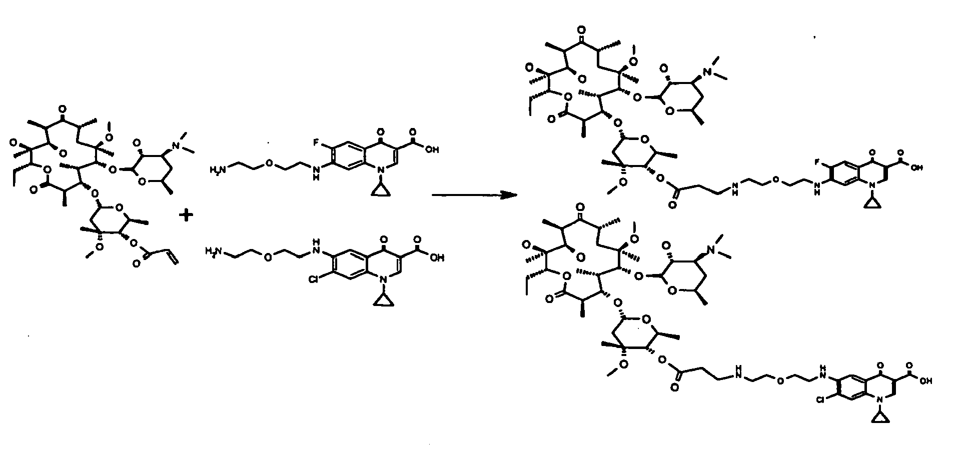

To a solution of **Intermediate 20** (50 mg, 0.06 mmol), and **Intermediate 30** (50 mg, 0.14 mmol, 2eq.) in CH₃CN / H₂O = 10/1 (11 mL), Et₃N (0.25 mL, 0,18 mmol, 3 eq.) was added and the reaction mixture was stirred at 70 °C for 48 hours. Solvents were evaporated, 30 mL of EtOAc was added and extracted with aq. NaHCO₃ (30 mL). Organic layer was evaporated and product was purified by column chromatography (eluent: CH₂Cl₂-MeOH-NH₃ = 90:15:1.5) yielding 18 mg of the title compounds as a mixture chloro and fluoro derivatives.
MS (ES+) m/z : [MH]⁺ = 1151.4 (6-fluoro derivative)
MS (ES+) m/z : [MH]⁺ = 1167.8 (7-chloro derivative

### Example 22

### 4"-O-(3-{2-[2-(10-Carboxy-9-oxo-3,4-dihydro-2H,9H-1-oxa-4a-aza-phenanthren-6-ylamino)-ethoxy]-ethoxy}-propionyl)-azithromycin

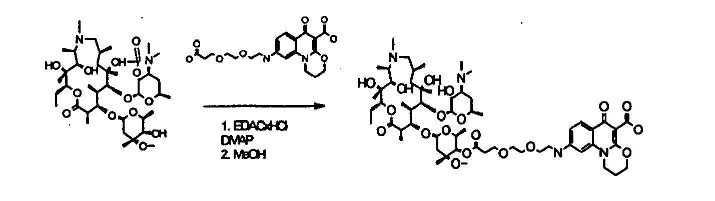

**Intermediate 1f** (470 mg, 1.12 mmol) was dissolved in DMF/4Å (6 mL), and cooled to 0 °C under N₂ atmosphere. In this solution, EDACxHCl (2 eq) was added and stirred for 5 min. Then, 2'-O-acetyl-azithromycin (1.3 g, 1.68 mmol) in 2.5 mL DCM/4Å, and DMAP (3 eq) were added and stirred for 3 days. Water and EtOAc were added and the layers were separated. The water layer was extracted two times with EtOAc. The combined organic layers were dried over K₂CO₃ and evaporated yielding crude 2'-O-acetyl-protected product in a mixture with starting compounds. Obtained product was dissolved in MeOH (60 mL) and the solution was stirred for 24 hours at 55 °C. The methanol was evaporated under vacuo and the residue was precipitated form EtOAc:n-hexane yielding 310 mg of the title compound.
MS (ES+) m/z: [MH]⁺ = 1152

### Reference example 23

### 11-O-Methyl-4''-O-(3-{2-[3-(3-Carboxy-1-ethyl-4-oxo-1,4-dihydro-quinolin-6-yl)-prop-2-ynyloxyl-ethylamino}-propionyl)-azithromycin

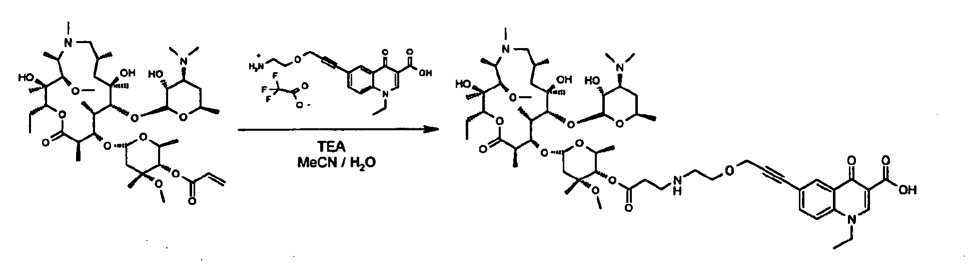

Into a solution of **Intermediate 9** (0.3 g) in MeCN (3 mL) and H₂O (1 mL) triethylamine (0.525 mL) was added. The solution has been stirring at room temperature for 20 minutes. **Intermediate 14b** (0.145 g) solution in MeCN (7 mL) was added and the mixture has been stirring at room temperature for 20 hours. The solvents were evaporated, DCM (10 mL) and H₂O (10 mL) were added, pH was adjusted to 9.2 by 2M NaOH and organic layer was separated and evaporated. The product was purified by column chromatography in system DCM - MeOH - NH₃ = 90 : 13 : 1.8 and precipitated from EtOAc / n-hexane yielding 18 mg of the title compound.
MS (ES+) m/z: [MH]⁺ = 1131.57

### Example 24

### 11-O-Methyl-4''-O-(3-{2-[3-(3-Carboxy-1-ethyl-4-oxo-1,4-dihydro-quinolin-6-yl)-propoxy]-ethylamino}-propionyl)-azithromycin

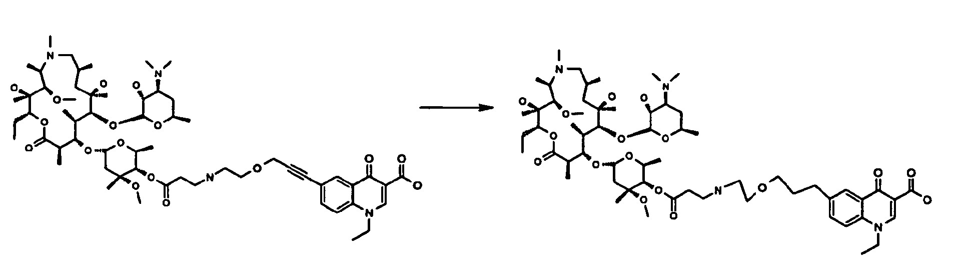

**Reference example 23** (15 mg) was dissolved in MeOH (20 mL) at room temperature and hydrogenated at 4 bar pressure in the presence of 10 % Pd / C (15 mg) for 5 hours. The mixture was filtrated, evaporated and the product was precipitated from EtOAc / n-hexane yielding 2 mg of the title compound.
MS (ES+) m/z: [MH]⁺ = 1135.6

### Example 25

### 4"-O-(3-{2-[2-(3-Carboxy-6-fluoro-1-cyclopropyl-4-oxo-1,4-dihydro-quinolin-7-ylamino)-ethoxy]-ethoxy}-propionyl)-azithromycin

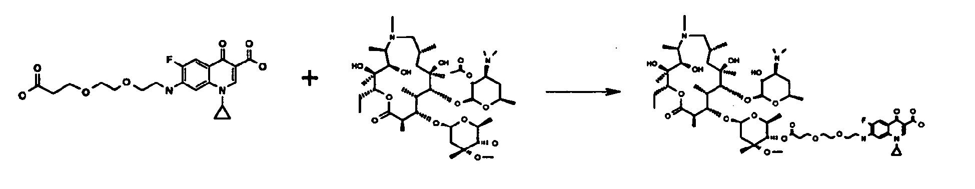

Using the procedure of **Example 6, Intermediate 27A** (600 mg, 1.19 mmol) and **2'-O-acetyl-azithromycin** (1.4 g, 1.8 mmol) gave 200 mg of the title compound.
MS (ES+) m/z: [MH]⁺ = 1153.4

### Example 26

### 4"-O-(3-{2-[2-(3-Carboxy-7-chloro-1-cyclopropyl-4-oxo-1,4-dihydro-quinolin-6-ylamino)-ethoxy]-ethoxy}-propionyl)-6-O-methyl-erythromycin A 11,12-cyclic carbamate

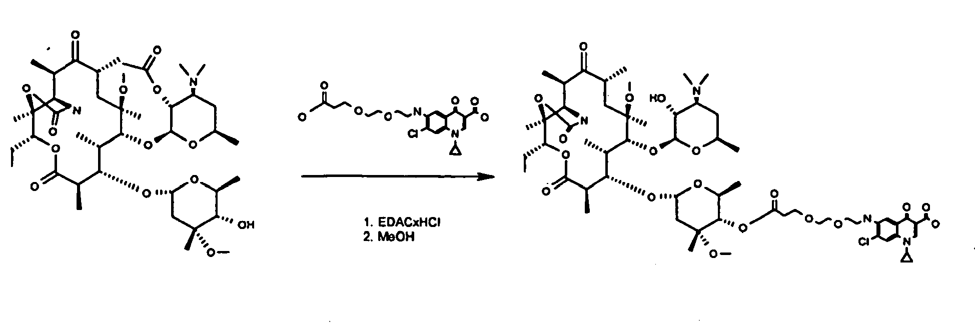

Using the procedure of **Example 6, Intermediate 7** (0.3 g, 0.68 mmol) and **2'-*O*-acetyl-6-*O-*methyl-erythromycin A 11,12-cyclic carbamate** (1.1 g, 1.36 mmol) gave the crude product. Column chromatography (eluent: DCM:MeOH:NH₃ = 90:5:0.5 yielded 200 mg of the title compound.
MS (ES+) m/z: [MH]⁺ = 1194.85

### Example 27

### 4"-O-(3-{2-[2-(3-Carboxy-6-fluoro-1-cyclopropyl-4-oxo-1,4-dihydro-quinolin-7-ylamino)-ethoxy]-ethoxy}-popionyl)-6-O-methyl-erythromycin A

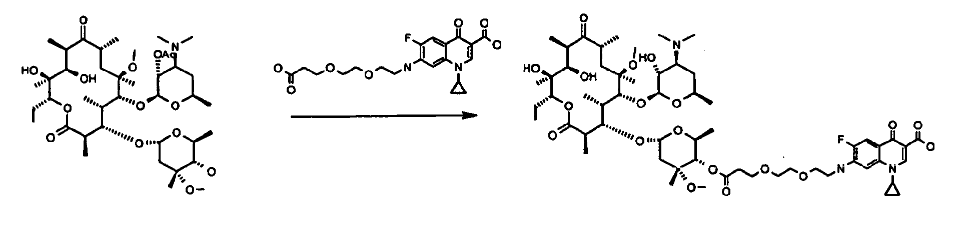

Using the procedure of **Example 6, Intermediate 27A** (500 mg, 1.19 mmol) and **2'-*O-*acetyl-6-*O*-methyl-erythromycin A** (1.87 g, 2.37 mmol) gave the crude product.
Column chromatography in (eluent: DCM-MeOH-NH₃ = 90:5:0.5) yielded 300 mg of the title compound.
MS (ES+) m/z: [MH]⁺ = 1153.35

### Example 28

### 2'-O-Propiony-4"-O-(3-{2-[2-(3-carboxy-7-chloro-1-cyclopropyl-4-oxo-1,4-dihydro-quinolin-6-ylamino)-ethoxy]-ethoxy}-propionyl)-azithromycin

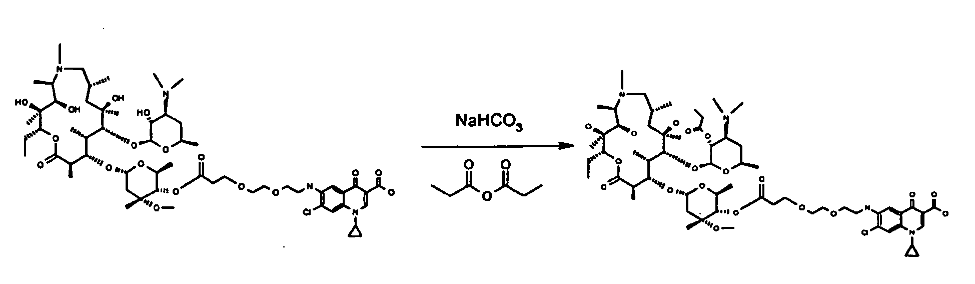

**Example 8** (200 mg, 0.17 mmol) was dissolved in DCM (5 mL), and to that solution NaHCO₃ (67 mg, 0.79 mmol) and propionic acid anhydride (0.27 ml, 0.207 mmol) were added and stirred at R.T. overnight. To the reaction mixture H₂O (15 mL) was added and extracted with DCM (2x 10 mL). Organic layers were separated, washed with brine and solvent was evaporated under pressure affording 150 mg of the title compound
MS (ES+) m/z: [MH]⁺ = 1126.87

### Example 29

### 4"-O-(3-{2-[2-(3-Carboxy-7-chloro-1-ethyl-4-oxo-1,4-dihydro-quinoline-6-ylamino)-ethoxy]-ethoxy}-propionyl)-azithromycin (A), and

### 4"-O-(3-{2-[2-(3-Carboxy-6-fluoro-1-ethyl-4-oxo-1,4-dihydro-quinoline-7-ylamino)-ethoxy]-ethoxy}-propionyl)-azithromycin (B)

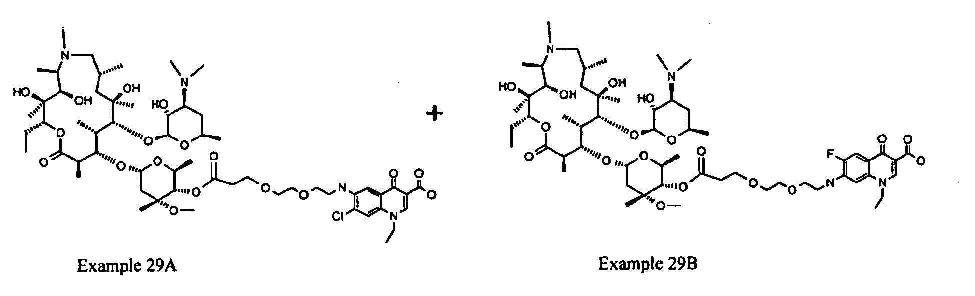

Using the procedure of **Example 6**, mixture of **Intermediates 33A** and **33B** (400 mg, 0.9 mmol) and **2'-*O*-acetyl-azithromycin** (1.4 g, 1.8 mmol) gave the mixture of chloro (A) and fluoro (B) compounds (700 mg).
According to HPLC/MS product contains **(A) : (B)** = 1 : 1
HPLC/MS (ES) m/z: [MH]⁺ = 1158.84 (Example 29A)
[MH]⁺ = 1142.42 (Example 29B)

Column chromatography (eluent: DCM-MeOH-NH₃ = 90:5:0.5) yielded 300 mg of the title compound (A) and 250 mg of the title compound (B).

### Example 30

### 4"-O-(3-{2-[2-(3-Carboxy-1-ethyl-4-oxo-1,4-dihydro-quinoline-6-ylamino)-ethoxy]-ethoxy}-propionyl)-azithromycin

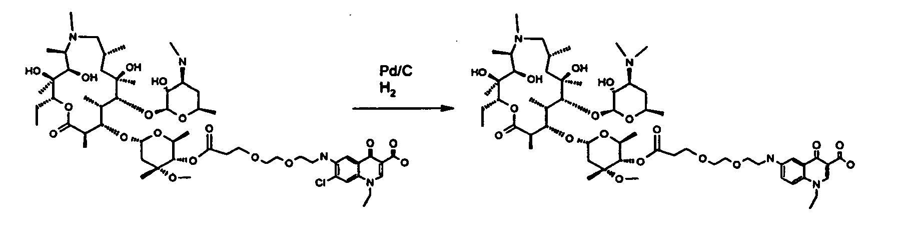

Using the procedure of **Example 11, Example 29A** (100 mg, 0.086 mmol) yielded 90 mg of the title compound.
HPLC/MS (ES) m/z: [NM]⁺ = 1124.5

### Example 31

### 4"-O-(3-{2-[2-(3-carboxy-7-chloro-1-isopropyl-4-oxo-1,4-dihydro-quinoline-6-ylamino)-ethoxy]-ethoxy}-propionyl)-azithromycin

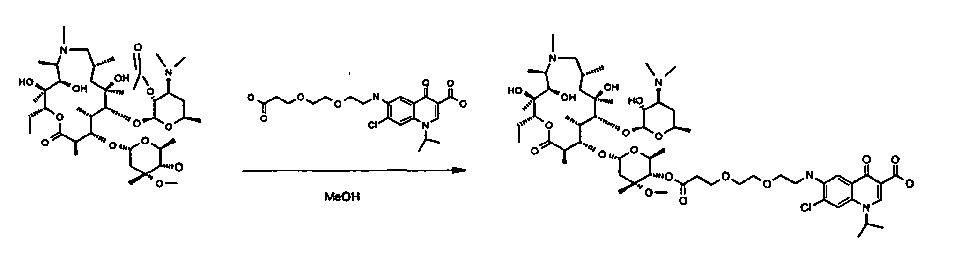

Using the procedure of **Example 6, Intermediate 34** (600 mg, 1.36 mmol) and **2'-O-acetylazythromycin** (1.79 g, 2.26 mmol) gave the title compound. Product was purified by precipitation from EtOAc : n-hexane yielding 700 mg og the title compound.
HPLC/MS (ES) m/z: [MH]⁺ = 1172.9

### Example 32

### 4"-O-(3-{2-[2-(6-Carboxy-7-oxo-2,3-dihydro-1H,7H-pyrido[3,2,1-ij]quinolin-9-ylamino)-ethoxy]-ethoxy}-propionyl)-azithromycin

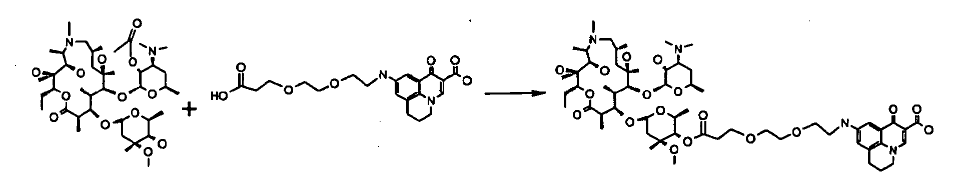

Using the procedure of **Example 6, Intermediate 35** (150 mg, 0.37 mmol) and **2'-O-acetylazythromycin** (295 mg, 0.39 mmol) gave the title compound. Product was purified by precipitation from EtOAc : n-hexane yielding 104.5 mg of the title compound.
**¹H-NMR (500MHz, DMSO-d6)** δ: 0.80 (t, 3H), 0.84 (d, 3H), 0.94 (q, 6H), 1.01 (m, 6H), 1.08 (d, 7H), 1.14 (s, 3H), 1.25 (m, 2H), 1.37 (m, 1H), 1.51 (d, 1H), 1.58 (d, 1H), 1.64 (q, 1H), 1.77 (q, 1H), 1.87 (m, 1H), 1.90 (m, 1H), 2.10 (m, 3H), 2.19 (s, 3H), 2.21 (s, 6H), 2.30 (d, 1H), 2.35 (d, 1H), 2.39 (m, 1H), 2.50 (m, 2H), 2.67 (m, 2H), 2.97 (t, 2H), 3.4 (t, 1H), 3.22 (s, 3H), 3.27 (q, 2H), 3.43 (s, 1H), 3.46 (d, 1H), 3.52 (m, 4H), 3.58 (m, 2H), 3.66 (m, 3H), 4.18 (t, 1H), 4.33 (k, 1H), 4.39 (t, 2H), 4.43 (d, 1H), 4.55 (d, 1H), 4.74 (q, 1H), 4.91 (d, 1H), 6.31 (t, 1H), 7.09 (s, 1H), 7.14 (s, 1H), 8.65 (s, 1H).
**¹³C-NMR (500MHz, DMSO-d6)** δ: 6.67, 8.86, 10.88, 14.55, 17.64, 20.47, 20.74, 20.89, 21.43, 21.54, 22.1, 26.00, 26.20, 27.36, 30.09, 34.22, 34.91, 35.67, 39.95, 41.60, 41.68, 42.44, 44.53, 48.80, 51.21, 52.64, 61.34, 62.15, 64.78, 66.09, 66.76, 68.66, 69.58, 70.47, 72.40, 73.52, 74.90, 76.30, 77.37, 78.02, 82.66, 94.29, 100.22, 102.02, 105.46, 120.43, 126.97, 128.47, 129.91, 143.82, 146.81, 166.87, 170.92, 176.49, 177.3.

### Example 33

### 4"-O-(3-{2-[2-(6-Carboxy-7-oxo-2,3-dihydro-1H,7H-pyrido[3,2,1-ij]quinolin-9-ylamino)-ethoxy]-ethoxy}-propionyl)-6-O-methyl-erythromycin A

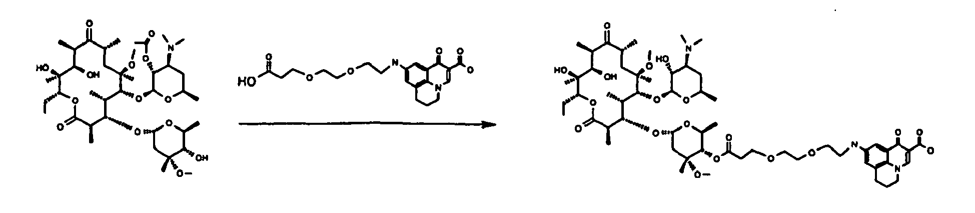

Using the procedure of **Example 6, Intermediate 35** (200 mg) and **2'-O-acetyl-6-*O*-methyl-erythromycin A** (391 mg) gave the title compound. Product was purified by precipitation from EtOAc : n-hexane yielding 150 mg of the title compound.
**¹H-NMR (500MHz, DMSO-d6)** δ: 0.75 (t, 3H), 1.04 (m, 12H), 1.06 (m, 3H), 1.09 (m, 4H), 1.13 (d, 3H), 1.26 (s, 3H), 1.43 (m, 3H), 1.46 (m, 1H), 1.58 (m, 1H), 1.67 (d; 1H), 1.74 (d, 2H), 1.85 (m, 2H), 1.87 (m, 1 H), 2.11 (m, 2H), 2.20 (s, 6H), 2.35 (m, 1 H), 2.39 (m, 1H), 2.50 (m, 2H), 2.60 (m, 1H), 2.82 (m, 1H), 2.92 (s, 3H), 2.97 (m, 3H), 3.04 (t, 1H), 3.21 (s, 3H), 3.27 (q, 2H), 3.52 (m, 4H), 3.53 (m, 1H), 3.56 (m, 2H), 3.59 (m, 1H), 3.62 (m, 1H), 3.66 (m, 2H), 4.28 (k, 1H), 4.43 (d, 1H), 4.56 (d, 1H), 4.85 (d, 1H), 5.05 (d, 1H), 6.28 (m, 1H), 7.8 (s, 1 H), 7.14 (s, 1H), 8.63 (s, 1H).
**¹³C-NMR (500MHz), DMSO-d6):** 8.90, 10.40, 11.85, 15.89, 16.93, 17.62, 18.05, 19.77, 20.26, 20.63, 20.77, 21.52, 26.21, 30.14, 34.25, 34.90, 37.93, 40.26, 42.47, 43.62, 44.25, 48.86, 50.10, 52.56, 62.40, 64.51, 66.07, 66.82, 68.66, 68.85, 69.58, 70.60, 72.07, 74.10, 75.91, 77.03, 77.75, 77.83, 79.20, 95.44, 100.29, 101.88, 120.34, 127.11, 128.48, 129.81, 143.90, 146.71, 166.91, 170.87, 175.02, 176.40, 218.63.

### Example 34

### 4"-O-(3-{2-[2-(3-Carboxy-7-chloro-1-cyclopropyl-4-oxo-1,4-dihydro-quinolin-6-ylamino)-ethoxy]-ethoxy}-propionyl)-6-O-propyl-erythromycin A

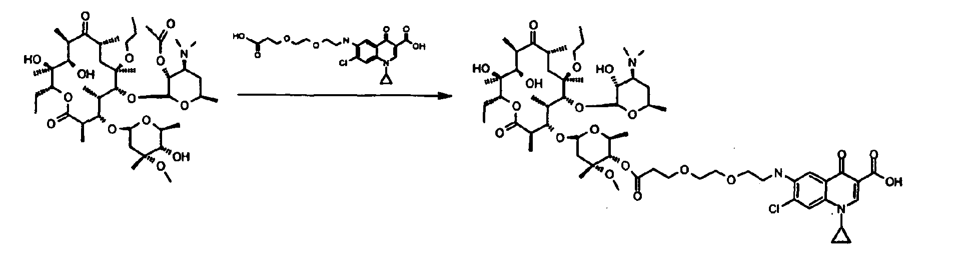

Using the procedure of **Example 6, Intermediate 7** (100 mg) and **2'-O-acetyl-6-*O-*propylerythromycin A** (378 mg, 2 eq) gave the title compound. Product was purified by column chromatography (eluent CH₂Cl₂-MeOH-NH₃ = 90 : 13 : 1.3) yielding the title compound (1 mg).
MS (ES+) m/z [MH₂]²⁺ = 599.10.

### Example 35

### 4"-O-(3-{2-[2-(3-Carboxy-1-cyclopropyl-4-oxo-1,4-dihydro-quinolin-7-yloxy)-ethoxy]-ethoxy}-propionyl)-azithromycin

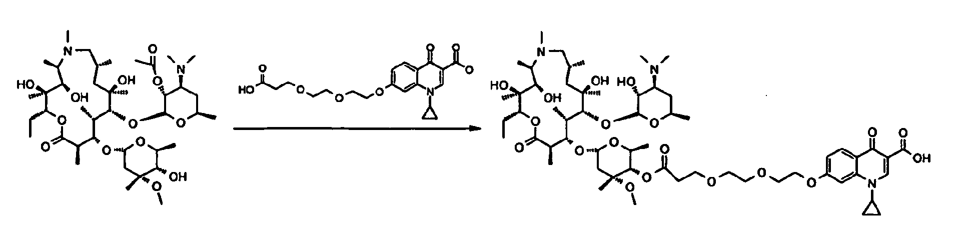

Using the procedure of **Example 6, Intermediate 36** (180 mg) and **2'-*O*-acetyl-azithromycin** (702 mg, 2 eq) gave the title compound. Product was precipitated from EtOAc: n-hexane yielding the title compound (100 mg).
MS (ES+) m/z [MH₂]²⁺ = 569.00

### Reference example 36

### 4"-O-(3-{2-[3-(3-carboxy-1-ethyl-4-oxo-1,4-dihydro-quinolin-6-yl)-prop-2-ynyloxylethylamino}-propionyl)- 6-O-methyl-erythromycin A

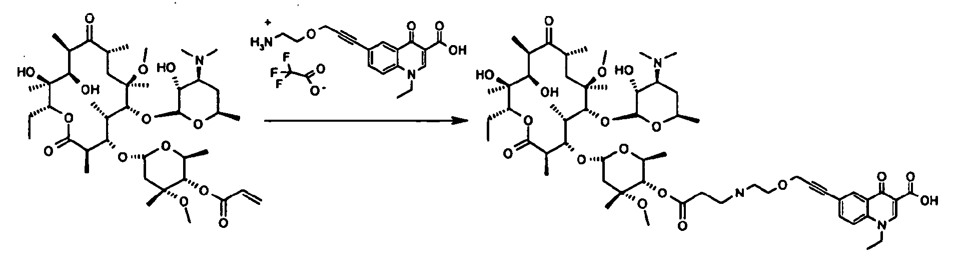

Using the procedure of **Reference example 23, Intermediate 9** (300 mg) and **Intermediate 20** (150 mg, 0.5 eq) gave the title compound. Product was purified by column chromatography (eluent CH₂Cl₂-MeOH-NH₃ = 90 : 13 : 1.3) and precipitated from EtOAc: n-hexane yielding 95 % pure title compound (63 mg).
MS (ES+) m/z [MH]⁺ = 1117.2.

### Example 37

### 4"-O-(3-{2-[3-(3-Carboxy-1-ethyl-4-oxo-1,4-dihydro-quinolin-6-yl)-propoxy]-ethylamino}-propionyl)-6-O-methyl-erythromycin A

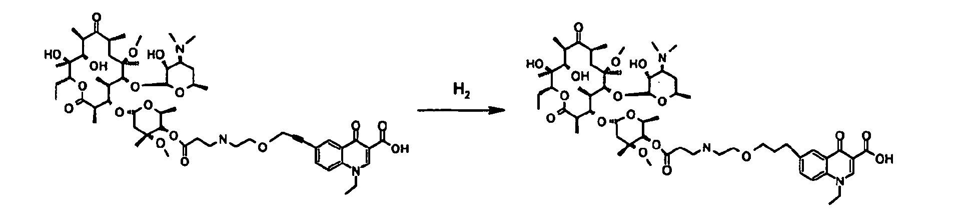

Using the procedure of **Example 24, Reference example 36** (50 mg) gave the title compound. Product was precipitated from EtOAc: n-hexane yielding 80% pure title compound (24 mg).
MS (ES+) m/z [MH]⁺ = 1121.2.

### Example 38

### 4"-O-(3-{2-[2-(3-Carboxy-1-cyclopropyl-4-oxo-1,4-dihydro-quinolin-7-yloxy)-ethoxy]-ethoxy}-propionyl)-6-O-methyl-erythromycin A

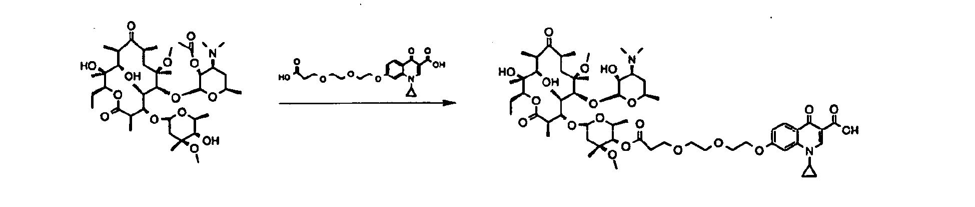

Using the procedure of **Example 6, Intermediate 36** (100 mg, 0.25 mmol) and **2'-O-acetyl-6-*O*-methyl-erythromycin A** (400 mg, 2 eq) gave the title compound. Product was purified by column chromatography (eluent CH₂Cl₂-MeOH-NH₃ = 90 : 13 : 1.7) and precipitated from EtOAc: n-hexane yielding 70% pure title compound (5 mg).
MS (ES+) m/z [MH]⁺ = 1135.56.

### Example 39

### 4"-O-(3-{2-[2-(3-Carboxy-1-cyclopropyl-4-oxo-1,4-dihydro-quinolin-7-yloxy)-ethoxyl-ethoxy}-propionyl)-9-ethyloximino-6-O-methyl-erythromycin A

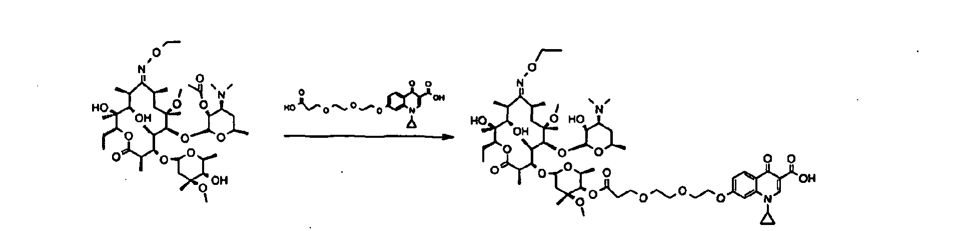

Using the procedure of **Example 6, Intermediate 36** (100 mg, 0.25 mmol) and **2'-O-acetyl-9-ethyloximino-6-*O*-methyl-erythromycin A** (400 mg, 2 eq) gave the title compound. Product was purified by column chromatography (eluent CH₂Cl₂-MeOH-NH₃ = 90 : 13 : 1.7) and precipitated from EtOAc: n-hexane yielding 94% pure title compound (10 mg).
MS (ES+) m/z [MH₂]²⁺ = 590.12.

### Example 40

### 4"-O-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-9-(1-isopropoxy-cyclohexyl)oximinoerythromycin A

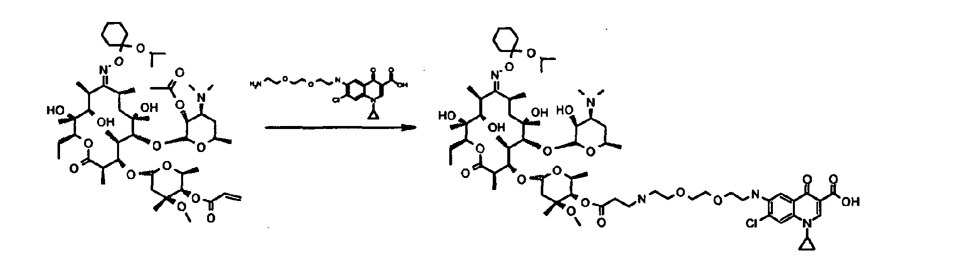

To the solution of **Intermediate 28A** (58 mg, 0.14 mmol) in actonitrile (1.5 mL) and water (0.5 mL) trethylamine (50 µL, 2.5 eq) was added and the mixture was stirred at R.T.. for 20 minutes. Than **Intermediate 37** (70 mg, 0.07 mmol) solution in actonitrile (3 mL) was added and the reaction mixture was stirred at 70 °C for 20 hours. The solvents were evaporated and to residue CH₂Cl₂ (5 mL) was added, filtrated and the filtrate was evaporated once again. The oily residue (105 mg) was dissolved in MeOH (6 mL) and the solution was stirred at 50 °C for 20 hours. MeOH was evaporated and the product was precipitated first from CH₂Cl₂ / n-hexane (76 mg) and from CH₂Cl₂ / di-*i*-propyl ether / n-hexane yielding 75% pure the title compound (69 mg).
MS (ES+) m/z [MH₂]²⁺ = 677.30

### Example 41

### 4"-O-(3-{2-[2-(3-Carboxy-7-chloro-1-cyclopropyl-4-oxo-1,4-dihydro-quinolin-6-ylamino)-ethoxy]-ethoxy}-propionyl)-9-(1-isopropoxy-cyclohexyl)oximino-erythromcin A

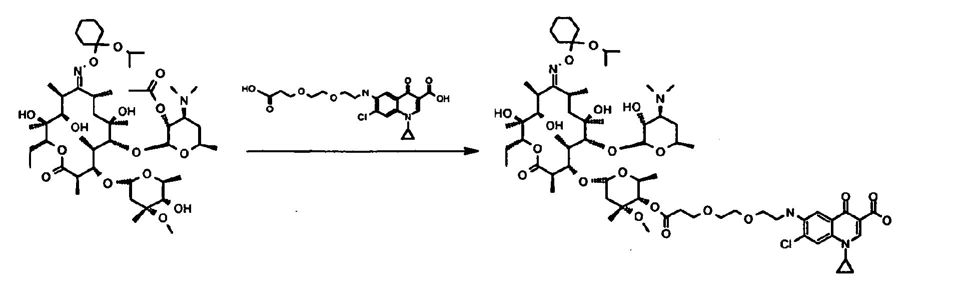

Using the procedure of **Example 6, Intermediate 7** (471 mg, 0.76 mmol) and **2'-*O*-acetyl-9-(1-isopropoxy-cyclohexyl)oximino-erythromycin A** (350 mg, 0.38 mmol) gave the title compound. Product was purified by column chromatography (eluent CH₂Cl₂-MeOH-NH₃ = 90 : 13 : 1.7) and precipitated from EtOAc: di-*i*-propyl ether : n-hexane yielding 90% pure title compound (58 mg).
MS (ES+) m/z [MH]⁺ = 1310.54.

### Example 42

### 4"-O-(3-{2-[2-(3-Carboxy-1-cyclopropyl-4-oxo-1,4-dihydro-quinolin-7-yloxy)-ethoxy]-ethoxy}-propionyl)-9-(1-isopropoxy-cyclohexyl)oximino-erythromycin A

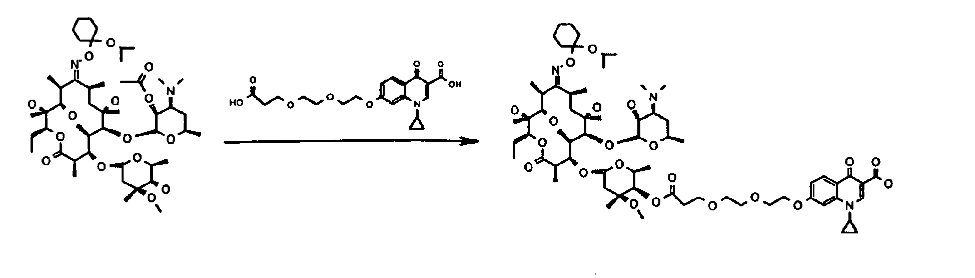

Using the procedure of **Example 6, Intermediate 36** (131 mg, 0.32 mmol) and **2'-*O*-acetyl-9-(1-isopropoxy-cyclohexyl)oximino-erythromycin A** (150 mg, 0.16 mmol) gave the title compound. Product was precipitated from CH₂Cl₂ : di-*i*-propyl ether : n-hexane yielding 70% pure title compound (88 mg).
MS (ES+) m/z [MH₂]²⁺ = 1276.64.

### Example 43

### 4"-O-(3-{2-[2-(3-Carboxy-7-chloro-1-cyclopropyl-4-oxo-1,4-dihydro-quinolin-6-ylamino)-ethoxy]-ethoxyl}-propionyl)-9-oxime erythromycin A

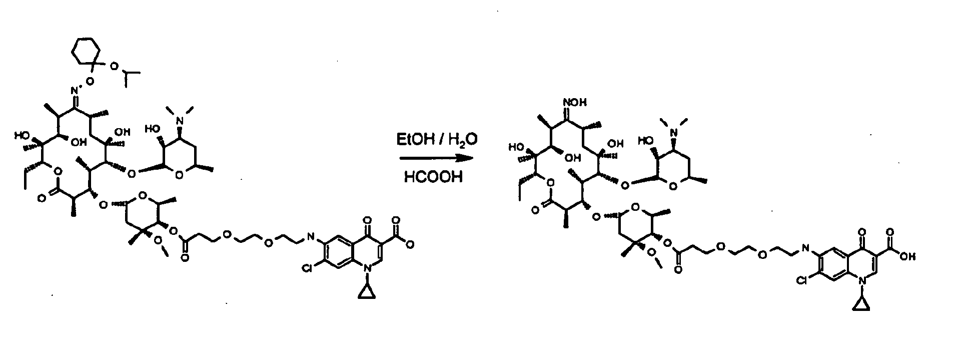

To the solution of **Example 41** (22 mg, 0.017 mmol) in EtOH (1 mL), water (2 mL) and HCOOH (10 µL) were added and the solution was stirred for 20 hours at R.T. After that EtOH was evaporated, CH₂Cl₂ (2 mL) added and pH was adjusted to 9.5. Then, organic layer was separated and evaporated giving oily residue. The pure product was precipitated from CH₂Cl₂ : di-*i*-propyl ether yielding 5 mg of the title compound (91 % Area by LC-MS).
MS (ES+) m/z [MH]⁺ = 1170.2.

### Example 44

### 4"-O-(3-{2-[2-(3-Carboxy-7-chloro-1-cyclopropyl-4-oxo-1,4-dihydro-quinolin-6-ylamino)-ethoxy]-ethoxy}-propionyl)-9-oxime erythromycin A diacetate salt

To the solution of **Example 43** (166 mg) in CH₂Cl₂ (1 mL) at R.T. acetic acid (16 µl, 2 eq) was added and the product was precipitated by addition of di-*i*-propyl ether (10 mL) yielding 148 mg of the title product.

### Example 45

### 4"-O-(3-{2-[2-(3-Carboxy-1-cyclopropyl-4-oxo-1,4-dihydro-quinolin-7-yloxy)-ethoxy]-ethoxy}-propionyl) erythromycin A 9(E)- and 9(Z)-oxime

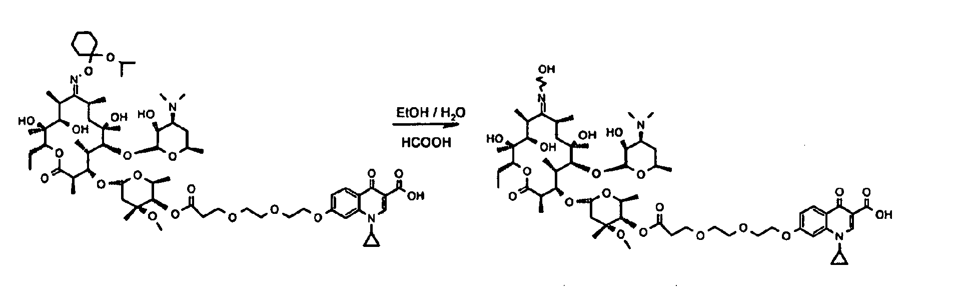

Using the procedure of **Example 43** from **Example 42** (46 mg, 0.036 mmol) after precipitiation from CH₂Cl₂ : di-*i*-propyl ether the title compound (5 mg) was obtained as a mixture of E and Z isomers. According to HPLC/MS product contains *E* and *Z* isomer.
HPLC/MS (ES) m/z: [MH]⁺ = 1137.3.

### Example 46

### 4"-O-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-9-oxime erythromycin A

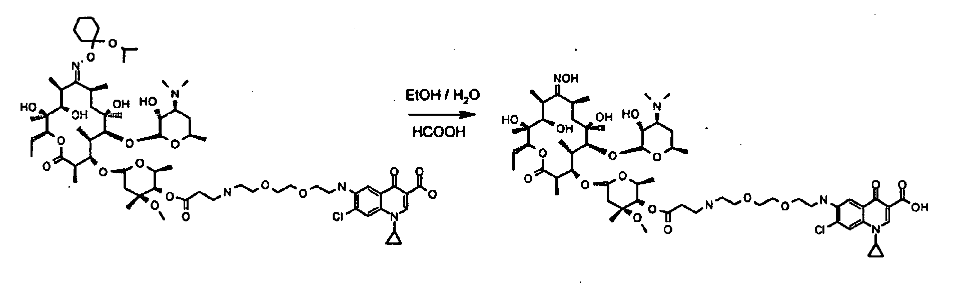

Using the procedure of **Example 43** from **Example 40** (60 mg, 0.043 mmol) after precipitiation from CH₂Cl₂ : di-*i*-propyl ether the title compound (17 mg) was obtained as a 70% pure product.
MS (ES+) m/z [MH]⁺ = 1213.3.

### Example 47

### 4"-O-(3-{2-[2-(3-Carboxy-1-cyclopropyl-7-methoxy-4-oxo-1,4-dihydro-guinolin-6-ylamino)-ethoxy]-ethoxyl}-propionyl)-azithromycin

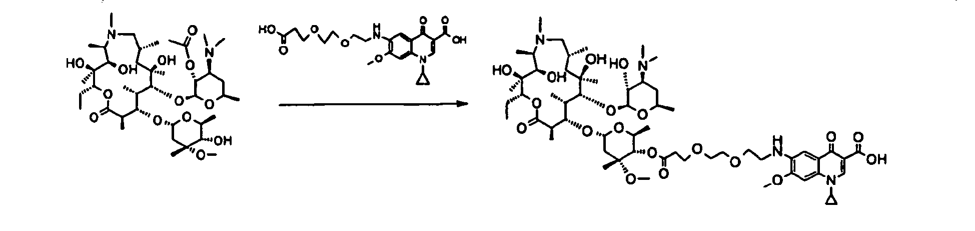

Using the procedure of **Example 6, Intermediate 38** (1.1 g) and **2'-*O*-acetyl-azithromycin** (2.0 g, 3 mmol) gave the title compound. Product was precipitated from EtAc : n-hexane yielding the title compound (230 mg).
MS (ES+) m/z [MH₂]²⁺ = 583.4.

### Example 48

### 4"-O-(3-{2-[2-(3-Carboxy-1-cyclopropyl-7-dimethylamino-4-oxo-1,4-dihydro-quinolin-6-ylamino)-ethoxy]-ethoxy}-propionyl) azithromycin

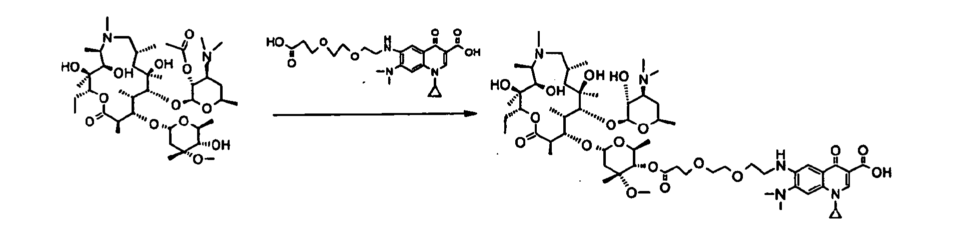

Using the procedure of **Example 6, Intermediate 39 and 2'-*O*-acetyl-azithromycin** (2.5 g, 3 mmol) gave the title compound. Product was precipitated from EtAc : n-hexane yielding the title compound (231 mg).
MS (ES+) m/z [MH₂]²⁺ = 589.4.

### Example 49

### 4"-O-(3-{2-[3-(3-carboxy-1-ethyl-4-oxo-1,4-dihydro-quinolin-6-yl)-propoxy]-ethoxy}-propionyl)-azithromycin

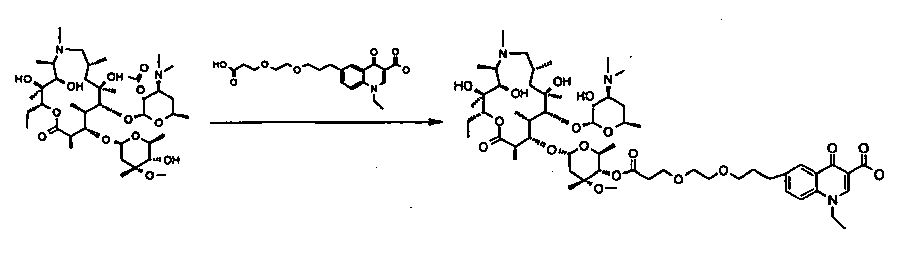

Using the procedure of **Example 6, Intermediate 42** (3.75 g, 0.0096 mol), **2'-*O*-acetyl-azithromycin** (9.11 g, 0.012 mol), EDACxHCl (3.67 g, 0.019 mol)) and DMAP (2.35 g, 0.019 mol) gave the title compound. Product was purified by column chromatography (eluent CH₂Cl₂-MeOH-NH₃ = 90 : 9 : 1.5) and precipitated from EtOAc: n-hexane yielding the title compound (7 g).
LC/MS(ES+) m/z [MH]⁺= 1122.6
**¹H-NMR (300 MHz, DMSO)** δ: 9.03 (s, 1H); 8.18(d, 1H); 7.98(d, 1H); 7.83 (dd, 1H); 4.91 (d, 1H); 4.73 (dd, 1H); 4.59 (t, 2H); 4.55 (d, 1H); 4.43 (d, 1H); 4.33 (m, 1H); 4.17(dd, 1H); 3.66 (m, 1H); 3.64 (m, 2H); 3.50 (ov, 2H), 3.47 (ov, 1H); 3,45 (ov, 1H); 3,43 (ov, 2H); 3,38 (t, 2H); 3.22 (s, 3H); 3.05 (dd, 1H); 2.81 (t, 2H); 2.67 (ov, 1H); 2.67 (ov, 1H); 2.59 (m, 2H); 2.40 (m, 1H); 2.35 (dd, 1H); 2.31 (d, 1H); 2.21 (s, 3H); 2.18 (s, 3H); 2.11 (t, 1H); 1.88 (ov, 1H); 1.85 (ov, 2H); 1.85 (ov, 1H); 1.78 (m, 1H); 1.66 (dd, 1H); 1.59 (m, 1H); 1.51 (d, 1H); 1.42 (t, 3H); 1.37 (m, 1H); 1.27 (dd, 1H); 1.12 (s, 3H); 1.1 (s, 3H); 1.09 (ov, 1H); 1.08 (d, 3H); 1.07 (dd, 3H); 1.03 (d, 3H); 1.01 (s, 3H); 0.96 (d, 3H); 0.94 (d, 3H); 0.84 (d, 3H); 0.79 (t, 3H)
**¹³C-NMR (75 MHz, DMSO)** δ: 177.44; 177.03; 170.92; 166.09; 148.46; 140.23; 137.35; 134.89; 125.45; 124.55; 118.03; 107.47; 102.02; 94.30; 82.66; 78.01; 77.34; 76.29; 74.87; 73.52; 72.40; 72.04; 70.45; 69.37; 69.66; 69.23; 68.61; 66.75; 66.09; 64.78; 62.16; 61.39; 48.79; 44.53; 41.59; 40.23; 35.66; 34.94; 34.24; 31.10; 30.99; 30.56; 27.31; 25.98; 22.00; 21.56; 20.88; 20.49; 17.64; 14.54; 10.88; 8.86; 6.67; 4.,89.

### Example 50

### 4"-O-(3-{2-[3-(3-carboxy-1-ethyl-4-oxo-1,4-dihydro-quinolin-6-yl)-propoxy]-ethoxy}-propionyl)-azithromycin diacetate salt

To a solution of **Example 49** (150 mg, 0.134 mmol) in EtOAc (10 mL) acetic acid (0.017 mL, 0.29 mmol) was added under stirring at R.T. Precipitate occurred after addition of n-hexane (100 mL), it was filtered off yielding the titled product (135 mg).

### Example 51

### 4"-O-(3-{2-[3-(3-carboxy-1-ethyl-4-oxo-1,4-dihydro-quinolin-6-yl)-propoxy]-ethoxy]-propionyl)-6-O-methyl erythromycin A

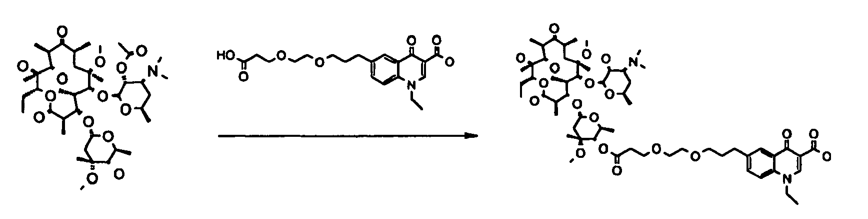

Using the procedure of **Example 6, Intermediate 42** and **2'-*O*-acetyl-6-O-methyl erythromycin A** gave the title compound. Product was precipitated from EtOAc: n-hexane yielding the title compound (112 mg).
LC/MS(ES+) m/z [MH]⁺= 1122.1.

### Example 52

### 9-Ethyloximino-4"-O-(3-{2-[3-(3-carboxy-1-ethyl-4-oxo-1,4-dihydro-quinolin-6-yl)-propoxy]-ethoxy}-propionyl)-erythromycin A

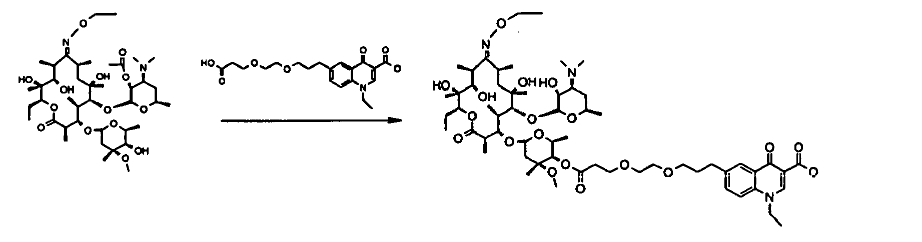

Using the procedure of **Example 6, Intermediate 42** and **2'-*O*-acetyl-9-ethyloximinoerythromycin A** gave the title compound. Product was precipitated from EtOAc: n-hexane yielding the title compound (106 mg).
LC/MS(ES+) m/z [MH]⁺= 1151.1.

### Example 53

### 4"-O-(3-{2-[3-(3-Carboxy-1-ethyl-4-oxo-1,4-dihydro-quinolin-6-yl)-propoxy]-ethoxy}-propionyl)-6-O-methyl-8a-aza-8a-homoerythromycin A

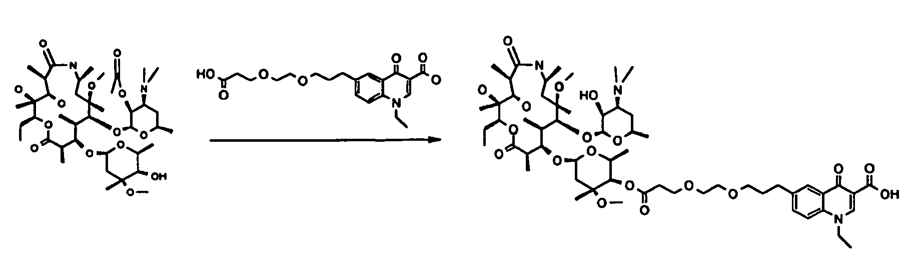

Using the procedure of **Example 6, Intermediate 42d** and **2'-*O*-acetyl-6-O-methyl-8a-aza-8a-homoerythromycin A** gave the title compound. Product was precipitated from EtOAc: n-hexane yielding the title compound (60 mg).
LC/MS(ES+) m/z [MH]⁺= 1136.1.

### Example 54

### 4"-O-(3-{2-[3-(3-Carboxy-1-ethyl-4-oxo-1,4-dihydro-quinolin-6-yl)-propoxy]-ethoxy}-propionyl)-roxythromycin

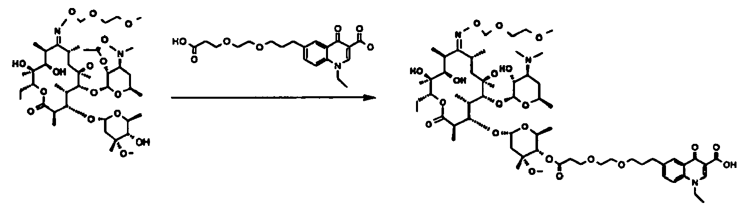

Using the procedure **of Example 6, Intermediate 42 and 2'-*O*-acetyl-roxythromycin** gave the title compound. Product was precipitated from EtOAc: n-hexane yielding the title compound (105.5 mg).
LC/MS(ES+) m/z [MH]⁺= 1210.68.

### Example 55

### 4"-O-(3-{2-[2-(3-Carboxy-7-chloro-1-cyclopropyl-4-oxo-1,4-dihydro-quinolin-6-yloxy)-ethoxy]-ethoxy}-propionyl)-azithromycin

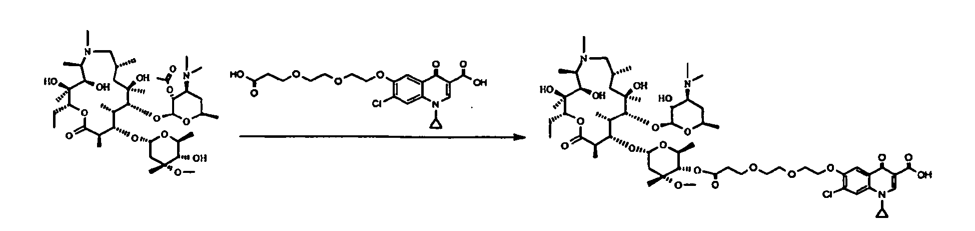

Using the procedure of **Example 6, Intermediate 13** and **2'-*O*-acetyl-azithromycin** gave the title compound. Product was purified by column chromatography (eluent: CH₂Cl₂ : MeOH: NH₃ = 90:9:0.5) yielding the title compound (61 mg).
LC/MS(ES+) m/z [MH]⁺= 1169.9.

### Example 56

### 4"-O-(3-{2-[3-(3-Carboxy-1-ethyl-4-oxo-1,4-dihydro-quinolin-6-yl)-propoxy]-ethoxy}-propionyl)-6-O-methyl-11-desoxy-11-(R)-methylamino-erythromycin A 11,12-carbamate

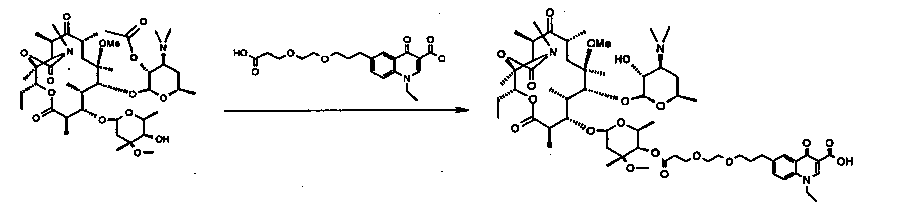

Using the procedure of **Example 6, Intermediate 42** and **2'-*O*-acetyl-6-O-methyl-11-desoxy-11-(R)-methylamino-erythromycin A 11,12-carbamate** reacted according to procedure in J. Org. Chem., 1988, 53(10), 2340-5 gave the title compound. Product was precipitated from EtOAc: n-hexane yielding the title compound (181 mg).
LC/MS(ES+) m/z [MH]⁺= 1161.3.

### Example 57

### 4"-O-(3-{2-[3-(3-Carboxy-1-cycloprooyl-4-oxo-1,4-dihydro-quinolin-6-yl)-propoxy]-ethoxy}-propionyl)-azithromycin

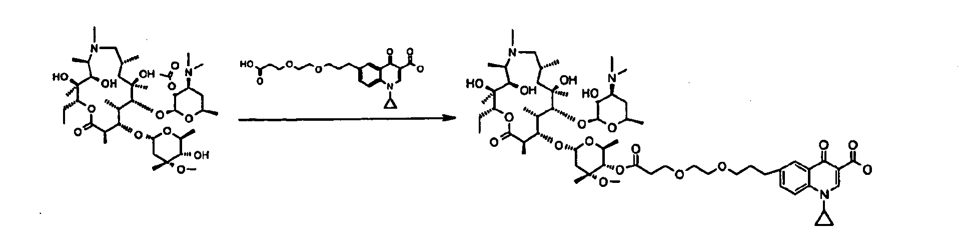

Using the procedure of **Example 6, Intermediate 44** (0.612 g, 1.5 mmol), and **2'-*O*-acetyl-azithromycin** (1.4 g, 1.8 mmol), EDACxHCl (0.574 g, 3 mmol) and DMAP (0.367 g, 3 mmol) gave the title compound. Product was purified by column chromatography (eluent CH₂Cl₂-MeOH-NH₃ = 90 : 9 : 1.5) and precipitated from EtOAc: n-hexane yielding the title compound (640 mg).
LC/MS(ES+) m/z [MH]⁺= 1134.35.
**¹H-NMR (300 MHz, DMSO)** δ: 8.73 (s, 1H); 8.22 (d, 1H); 8.16 (d, 1H); 7.87 (dd, 1H); 4.91 (d, 1H); 4.74 (dd, 1H); 4.56 (d, 1H); 4.43 (d, 1H); 4.34 (m, 1H); 4.17 (dd, 1H); 3.84 (m, 1H); 3.69 (m, 1H); 3.66 (m, 2H); 3.47 (d, 1H); 3.43 (m, 1H); 3.38 (t, 2H); 3.33-3.37 (ov, 4H); 3.23 (s, 3H); 3.04 (t, 1H); 2.83 (t, 2H); 2.67 (m, 1H); 2.58 (t, 2H); 2.40 (m, 1H); 2.38 (d, 1H); 2.33 (d, 1H); 2.21 (s, 6H); 2.19 (s, 3H); 2.11 (t, 1H); 1.89 (m, 1H); 1.83-1.86 (ov, 3H); 1.78 (m, 1H); 1.67 (dd, 1H); 1.59 (m, 1H); 1.51 (s, 1H); 1.36 (m, 1H); 1.32 (m, 2H); 1.25 (m, 1H); 1.18 (m, 2H); 1.13 (s, 3H); 1.09 (d, 3H); 1.08 (d, 3H); 1.07-1.09 (ov, 1H); 1.03 (d, 3H); 1.01 (s, 6H); 0.95 (d, 3H); 0.94 (d, 3H); 0.85 (dd, 3H); 0.79 (t, 3H).
**¹³C-NMR (75 MHz, DMSO)** δ: 177.72; 177.04; 170.93; 165.92; 147.97; 140.43; 139.36; 134.81; 124.92; 124.29; 118.42; 107.14; 102.04; 94.32; 82.69; 78.03; 77.39; 76.32; 74.91; 73.54; 72.43; 70.45; 69.67; 69.39; 69.24; 68.64; 66.77; 66.10; 64.81; 62.18; 61.39; 48.82; 44.55; 41.69; 41.61; 40.27; 35.79; 35.68; 34.96; 34.26; 31.05; 30.65; 30.15; 27.35; 26.01; 22.02; 21.58; 20.51; 20.19; 17.67; 17.64; 14.59; 10.89; 8.89; 7.48; 6.68.

### Example 58

### 4"-O-(3-{2-[3-(3-Carboxy-4-oxo-1-propyl-1,4-dihydro-quinolin-6-yl)-propoxy]-ethoxy}-propionyl)-azithromycin

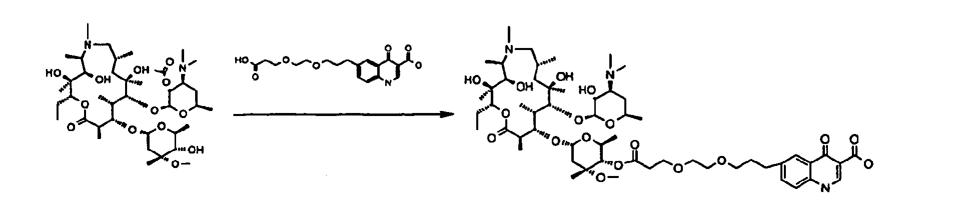

Using the procedure of **Example 6, Intermediate 46** and **2'-*O*-acetyl-azithromycin** gave the title compound. Product was precipitated from EtOAc: n-hexane yielding the title compound (170 mg).
LC/MS(ES+) m/z [MH]⁺= 1094.70.

### Example 59

### 4"-O-(3-{2-[3-(3-Carboxy-4-oxo-1,4-dihydro-quinolin-6-yl)-propoxy]-ethoxy}-propionyl)-azithromycin

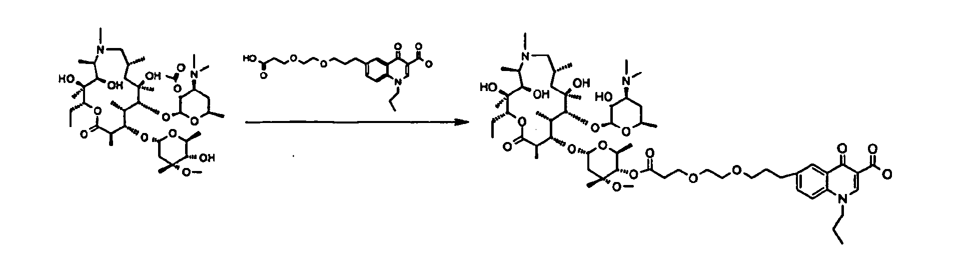

Using the procedure of **Example 6, Intermediate 48** and **2'-*O*-acetyl-azithromycin** gave the title compound. Product was precipitated from EtOAc: n-hexane yielding the title compound (400 mg).
LC/MS(ES+) m/z [MH]⁺= 1136.42.
**¹H-NMR (300 MHz, DMSO-d6)** δ: 9.02 (s, 1H); 8.18 (d, 1H); 7.98 (d, 1H); 7.82 (dd,1H); 4.95 (d, 1H); 4.75 (dd, 1H); 4.56 (t, 2H); 4.53 (d, 1H); 4.43 (d, 1H); 4.34 (m, 1H); 4.17 (d, 1H); 3.69 (m, 1H); 3.66 (t, 2h); 3.53-3.45 (ov, 4H); 3.47 (ov, 1H); 3.45 (ov, 1H); 3.38 (ov, 2H); 3.22 (s, 3H); 3.05 (dd, 1H); 2.81 (t, 2H); 2.67 (m, 2H); 2.58 (t, 2H); 2.41 (m, 1H); 2.34 (dbr, 1H); 2.28 (d, 1H); 2.22 (s, 3H); 2.19 (s, 3H); 2.12 (tbr, 1H); 1.88 ( ov, 1H); 1.86-1.80 (ov, 2H); 1.83 (ov, 1H); 1.77 (m, 1H); 1.67 (dd, 1H); 1.61 (m, 1H); 1.52 (d, 1H); 1.38 (m, 1H); 1.27 (ddbr, 1H); 1.12 (s, 3H); 1.09 (d, 6H); 1.05 (ov, 1H); 1.02 (d, 3H); 1.01 (s, 6H); 0.85 (d, 3H); 0.95 (d, 6H); 0.92 (t, 3H); 0.80 (t, 3H);
**¹³C-NMR (75 MHz, DMSO-d6)** δ: 177.64; 177.24; 171.13; 166.34; 149.02; 140.46; 137.76; 135.06; 125.63; 124.73; 118.38; 107.42; 102.21; 94.51; 82.86; 78.21; 77.57; 76.49; 75.06; 73.73; 72.62; 70.62; 70.62; 69.87; 69.58; 69.45; 68.80; 66.30; 65.00; 32.37; 61.65; 55.03; 49.01; 44.74; 41.8; 41.5; 40.42; 35.88; 35.15; 34.44; 31.21; 30.78; 30.32; 27.51; 26.18; 22.20; 21.76; 21.4; 21.09; 20.7; 17.86; 17.78; 14.78; 11.09; 10.65; 9.07; 6.89.

### Example 60

### 4"-O-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-azithromycin (A)

### 4"-O-[3-(2-{2-[2-(3-Carboxy-6-fluoro-l-cyclopropyl-4-oxo-1,4-dihydro-quinolin-7-ylamino)-ethoxy]-ethoxy}-ethylamino)-propionyl]-azithromycin (B)

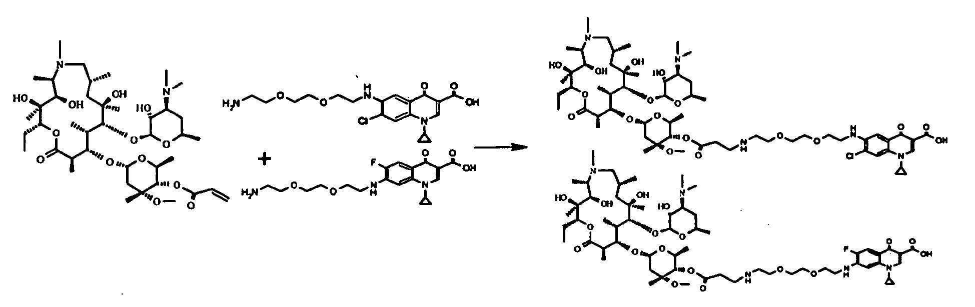

Using the procedure of **Reference example 1, Intermediate 14** and mixture of **Intermediates 28A** and **28B** gave the title compounds (85 mg) as a mixture of chloro and fluoro derivatives in a ratio 3:1.
LC/MS(ES+) m/z [MH]⁺= 1212.9 (Example 60A)
LC/MS(ES+) m/z [MH]⁺= 1196.4 (Example 60B)

### Example 61

### 4"-O-(3-{2-[2-(3-Carboxy-7-chloro-1-cyclopropyl-4-oxo-1,4-dihydro-quinolin-6-ylamino)-ethoxy]-ethylamino}-propionyl)-azithromycin (A)

### 4"-O-(3-{2-[2-(3-Carboxy-6-fluoro-1-cyclopropyl-4-oxo-1,4-dihvdro-quinolin-7-ylamino)-ethoxy]-ethylamiao}-propionyl)-azithromycin (B)

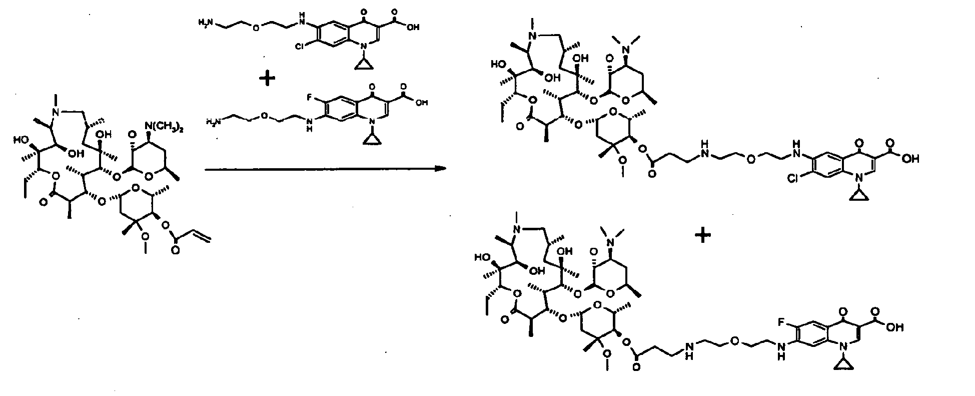

Using the procedure of **Reference example 1, Intermediate 14** and mixture of **Intermediates 30A** and **30B** gave the title compounds (81 mg) as a mixture of chloro and fluoro derivatives in a ratio 3:2.
LC/MS(ES+) m/z [MH]⁺= 1168.4 (Example 61A)
LC/MS(ES+) m/z [MH]⁺= 1152.5 (Example 61B)

### Reference example 62

### 4"-O-[3-(2-{2-[3-(3-Carboxy-1-ethyl-4-oxo-1,4-dihydro-quinolin-6-yl)-prop-2-ynyloxy]-ethoxy}-ethylamino)-propionyl]-azithromycin

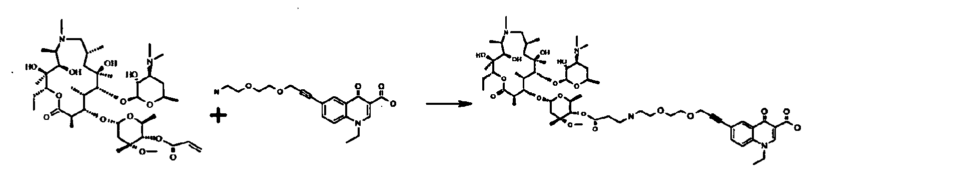

Using the procedure of **Reference example 1, Intermediate 14** and **Intermediates 49** gave the title compound (3 mg).
LC/MS(ES+) m/z [MH]⁺= 1161.4.

### Examaple 63

### 4"-O-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-6-O-methyl erythromycin A

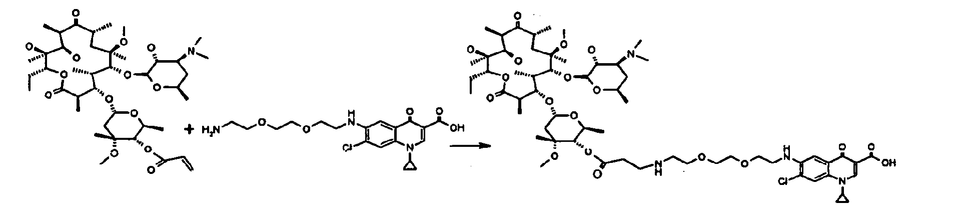

Using the procedure of **Reference example 1, Intermediate 20** and **Intermediates 28A** gave the title compound (49 mg).
LC/MS(ES+) m/z [MH]⁺= 1211.9.

### Example 64

### 4"-O-(3-{2-[2-(3-Carboxy-7-chloro-1-cyclopropyl-4-oxo-1,4-dihydro-quinolin-6-ylamino)-ethoxy]-ethoxy}-propionyl)-6-O-methyl-8a-aza-8a-homoerythromycin A

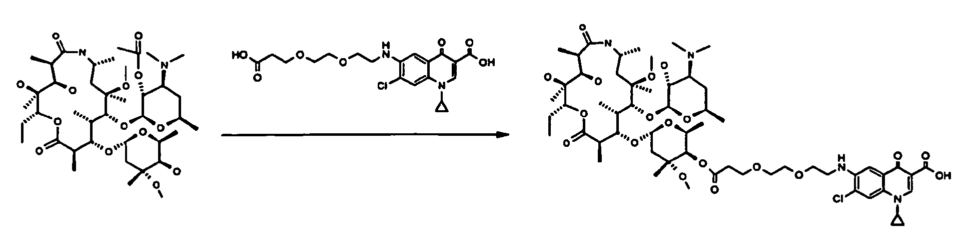

Using the procedure of **Example 6, Intermediate 7** and **2'-*O*-acetyl-6-*O*-methyl-8a-aza-8a-homoerythromycin A** gave the title compound. Product was precipitated from EtOAc: n-hexane yielding the title compound (85 mg).
LC/MS(ES+) m/z [MH]⁺= 1183.8.

### Example 65

### 4"-O-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-azithromycin

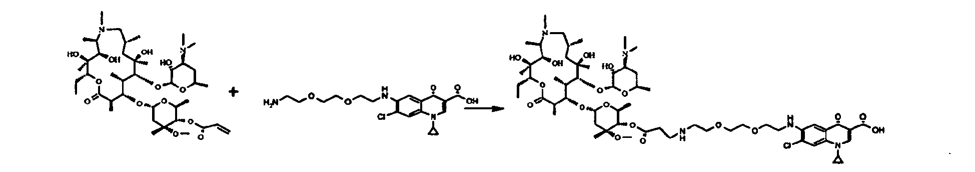

Using the procedure of Reference example 1, Intermediate 14 and Intermediates 28A gave the title compound (152 mg).
LC/MS(ES+) m/z [MH]⁺= 1212.9.

### Example 66

### 4"-O-[3-(2-{[2-(3-Carboxy-7-chloro-1-cyclopropyl-4-oxo-1,4-dihydro-quinolin-6-ylamino)-ethyl]-methyl-amino}-ethoxy)-propionyl]-azithromycin

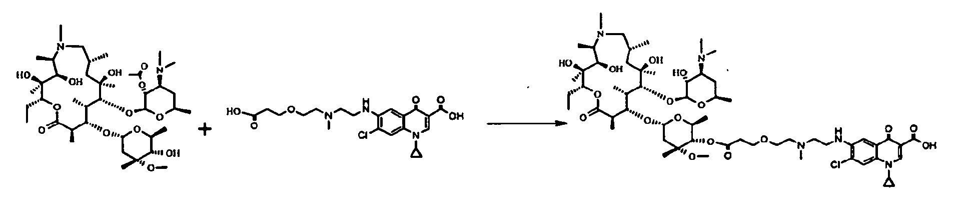

Using the procedure of **Example 6, Intermediate 52** and **2'-*O*-acetyl-azithromycin** gave the title compound. Product was precipitated from EtOAc: n-hexane yielding the title compound (50 mg).
LC/MS(ES+) m/z [MH]⁺= 1182.9.

### Example 67

### 4"-O-(3-{2-[2-(3-Carboxy-1-cyclopropyl-4-oxo-1,4-dihydro-quinolin-6-yloxy)-ethoxy]-ethoxy}-Dropionyl)-azithromycin

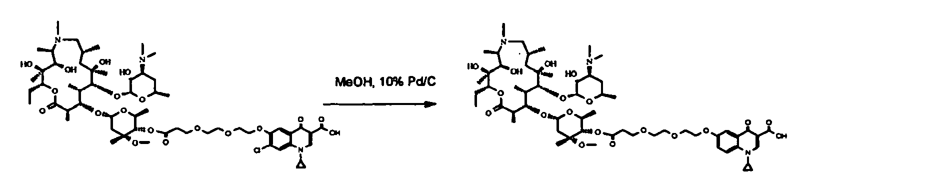

**Example 55** (80 mg, 0.068 mmol) was dissolved in 30 mL of methanol, 40 mg of 10 % Pd/C was added and the mixture was stired under H₂ pressure (5 bar) for 6 hours. The reaction mixture was filtrated and solvent evaporated in vacuum. Crude product was diluted in isopropylacetate
(8 mL) and extracted with aqueous NaHCO₃ (2x5 mL). Organic layer was evaporated in vacuum and crude product precipitated from EtOAc:n-hexane yielding 68 mg of the title product.
LC/MS(ES+) m/z [MH]⁺= 1136.6.
**¹³C-NMR(75 MHz, CDCl₃)** δ: 178.9, 178.0, 171.4, 167.2, 157.3, 146.7, 135.8, 127.4, 125.0, 118.9, 108.1, 106.7, 102.3, 94.7, 83.2, 79.0, 77.8, 77.5, 74.3, 73.8, 73.6, 72.9, 71.0, 70.8, 70.6, 70.1, 69.5, 68.2, 67.9, 66.7, 65.6, 63.0, 62.5, 49.4, 45.2, 42.2, 42.1, 40.4, 36.3, 35.5, 35.2, 35.0, 29.0, 27.5, 26.8, 22.0, 21.9, 21.3, 21.2, 17.8, 16.2, 14.6, 11.3; 9.1, 8.3, 7.5.

### Example 68

### 4"-O-(3-{2-[2-(3-Carboxy-7-chloro-4-oxo-1,4-dihydro-quinolin-6-yloxy)-ethoxy]-ethoxy}-propionyl)-azithromycin

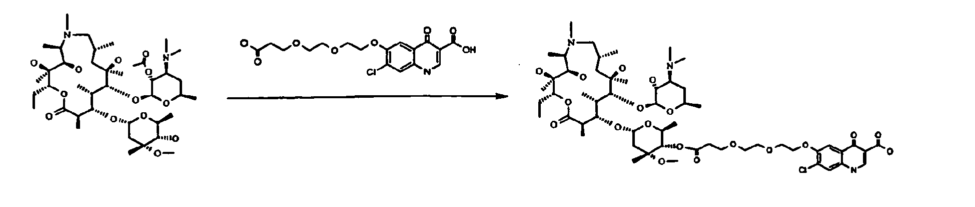

Using the procedure of **Example 6, Intermediate 54** and **2'-*O*-acetyl-azithromycin** gave the title compound. Product was purified by column chromatography (eluent: CH₂Cl₂ MeOH: NH₃ = 90:9:1.5) yielding the title compound (92 mg).
LC/MS(ES+) m/z [MH]⁺= 1130.8.

### Example 69

### 4"-O-(3-{2-[2-(3-Carboxy-7-chloro-4-oxo-1,4-dihydro-quinolin-6-ylamino)-ethoxy]-ethoxy}-propionyl)-azithromycin

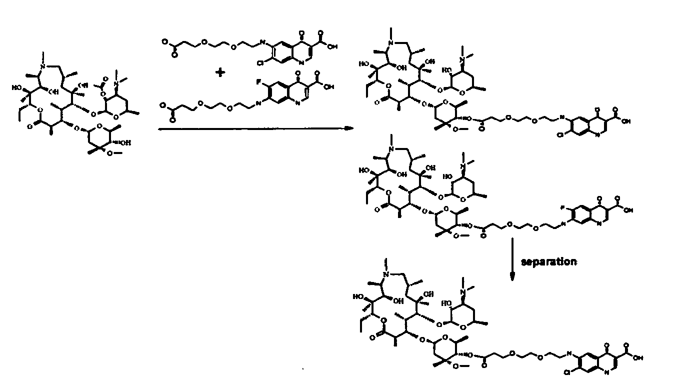

Using the procedure of **Example 6, 2'-*O*-acetyl-azithromycin** and mixture of **Intermediate 56C** and **Intermediate 56D** gave a mixture of chloro and fluoro compounds (670 mg). Purification by column chromatography (eluent: CH₂Cl₂ : MeOH: NH₃ = 90:9:1.5) yield 150 mg of the title compound.
LC/MS(ES+) m/z [MH]⁺= 1129.8.

### Example 70

### 4"-O-{[6-({2-[(2-Aminoethyl)(methyl)aminolethyl}thio)-1-ethyl-4-oxo-1,4-dihydro-3-quinolinecarboxylic acid]propionyl}-6-O-methyl erythromycin A

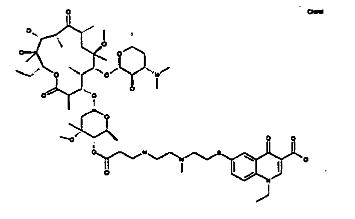

Using the procedure of **Reference example 1, Intermediate 20** and **Intermediate 57** gave the title compound in 43 % yield.
ESMS m/z [MH]⁺= 1151.7.

### Example 71

### 4"-O-{[6-({2-[(2-Aminoethyl)(methyl)amino]ethyl}thio)-1-ethyl-4-oxo-1,4-dihydro-3-quinolinecarboxylic acid]propionyl}-azithromycin

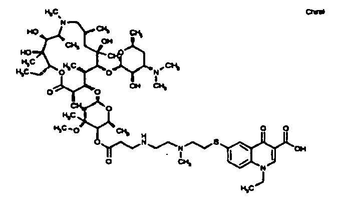

Using the procedure of **Reference example 1, 4"-*O*-propenoyl-azithromycin** and **Intermediate 57** gave the title compound in 20 % yield.
ESMS m/z [MH]⁺= 1152.8.

### Example 72

### 4"-O-{[6-({2-[(2-Aminoethyl)thio]ethyl}oxy)-1-ethyl-4-oxo-1,4-dihydro-3-quinolinecarboxylic acid]propionyl}-6-O-methyl erythromycin A

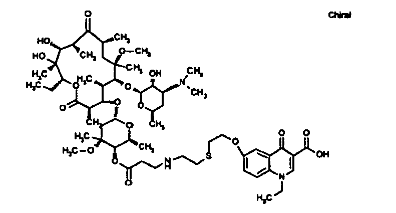

Using the procedure of **Reference example 1, Intermediate 20** and **Intermediate 59** gave the title compound in 62 % yield.
ESMS m/z [MH]⁺=1138.9.

### Example 73

### 4"-O-{[6-({2-[(2-Aminoethyl)thio]ethyl}oxy)-1-ethyl-4-oxo-1,4-dihydro-3-quinolinecarboxylic acid]propionyl}-O-(9E)-methoxymethyloximino erythromycin A

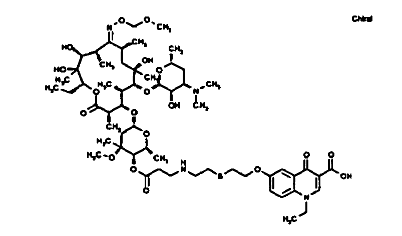

Using the procedure of **Reference example 1, Intermediate 63** and **Intermediate 59** gave the title compound in 44 % yield.
ESMS m/z [MH]⁺= 1184.2.

### Example 74

### 4"-O-{[6-({2-[(2-Aminoethyl)thio]ethyl}oxy)-1-ethyl-4-oxo-1,4-dihydro-3-quinolinecarboxylic acid]propionyl}-O-(9E)-hydroximino erythromycin A

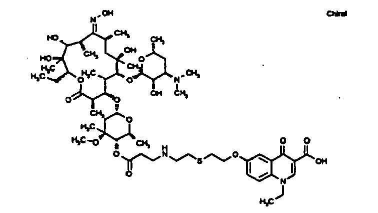

Using the procedure of **Reference example 1, Intermediate 62** and **Intermediate 59** gave the title compound in 48 % yield.
ESMS m/z [MH]⁺= 1140.2.

### Example 75

### 4"-O-{[1-Ethyl-6-(3-{[2-Aminoethyl]oxy}propyl)-4-oxo-1,4-dihydro-3-quinolinecarboxylic acidl]]propionyl}-O-(9E)-hydroximino erythromycin A

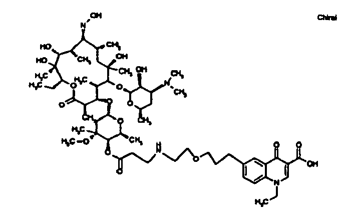

Using the procedure of **Reference example 1, Intermediate 60** and **Intermediate 62** gave the title compound in 52 % yield.
ESMS m/z [MH]⁺= 1122.0.

### Example 76

### 4"-O-{[1-Ethyl-6-(3-{[2-(methylamino)ethyl]oxy}propyl)-4-oxo-1,4-dihydro-3-quinolinecarboxylic acid]]propionyl}-O-(9E)-hydroximino erythromycin A

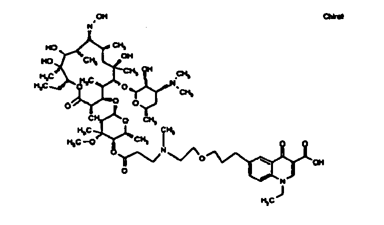

A solution of **Example 75** (0.032 g, 0.049 mmol) dissolved in a mixture of chloroform (0.7 mL), 37% aqueous formaldehyde (7 uL) and formic acid (7 uL) was stirred at 60°C. After 4 h the mixture was cooled, evaporated and the residue subjected to chromatography over silica gel eluting with with 0 - 10% [9:1 methanol/20 M ammonia] in dichloromethane gave the title compound in 58 % yield.
ESMS m/z [MH]⁺= 1137.0.

### Reference example 77

### 4"-O-{[6-({2-[(2-aminoethyl)oxy]ethyl}oxy)-ethyl-4-oxo-1,4-dihydro-3-quinolinecarboxylic acid]propionyl}-6-O-methylerythromycin A

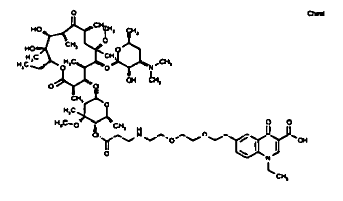

Using the procedure of **Reference example 1, Intermediate 61 and Intermediate 20** gave the title compound.
ESMS m/z [MH]⁺= 1115.2.

### Biological Data

The MIC (µg/ml) of test compounds against various organisms was determined including: *S. aureus* Smith ATCC 0329, *S. pneumoniae* 0541, *S. pyogenes* 0542, *E. faecalis* ATCC 0004, *H. influenzae* ATCC 0529, *M. catarrhalis* ATCC 0324.

Examples and reference examples 3-8, 11, 14, 19, 20, 23, 24, 26, 27, 30-33, 35-38, 48, 49, 51, 55-57, 59, 62, 64 and 65 have an MIC ≤1 µg/ml against *S. aureus* Smith ATCC 0329, S. *pneumoniae* 0541, *S. pyogenes* 0542 and *E. faecalis* ATCC 0004.

Examples and reference examples 3, 4, 8, 11, 14, 15, 19, 23, 24, 26, 27, 30-33, 35-38, 48, 49, 51, 55-57, 59, 62, 64 and 65 have an MIC ≤2 µg/ml against *H. influenzae* ATCC 0529 and *M. catarrhalis* ATCC 0324.

Examples 6, 8, 11, 19, 20, 30-33, 37, 49, 51, 52, 55-57, 59, 64 and 65 have an MIC ≤0.25 µg/ml against erythromycin resistant strains of *Streptococcus pneumoniae* and *Streptococcus pyogenes*.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the following claims:

## Claims

1. A compound of formula (I), 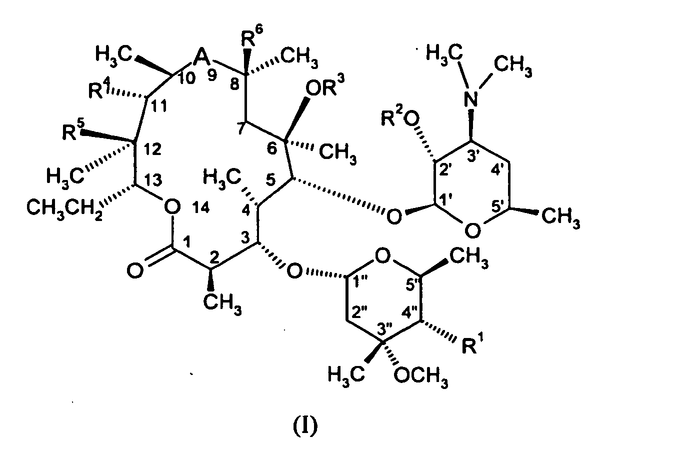 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)(CH₂)_{d}XR¹¹;
R² is hydrogen or a hydroxyl protecting group;
R³ is hydrogen, C₁₋₄alkyl, or C₂₋₆alkenyl optionally substituted by 9 to 10 membered fused bicyclic heteroaryl;
R⁴ is hydroxyl, C₂₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹²,
R⁵ is hydroxyl, or
R⁴ and R⁵ taken together with the intervening atoms form a cyclic group having the following structure: 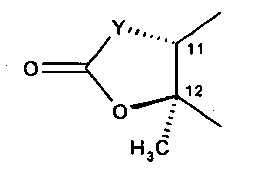
wherein Y is a bivalent radical selected from -CH₂-, -CH(CN)-, -O-, -N(R¹³)- and -CH(SR¹³)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁴R¹⁵ or -C(O)R¹⁴, or
R⁸ and R⁹ together form =CH(CR¹⁴R¹⁵)_{f}aryl, =CH(CR¹⁴R¹⁵)_{f}heterocyclyl, =CR¹⁴R¹⁵ or =C(R¹⁴)C(O)OR¹⁴, wherein the alkyl, aryl and heterocyclyl groups are optionally substituted by up to three groups independently selected from R¹⁶;
R¹⁰ is -OR¹⁷, C₁₋₆alkyl, -(CH₂)_{g}aryl, -(CH₂)_{g}heterocyclyl or -(CH₂)ₕO(CH₂)ᵢOR⁷,
wherein each R¹⁰ group is optionally substituted by up to three groups independently selected from R¹⁶;
R¹¹ is a heterocyclic group having the following structure: 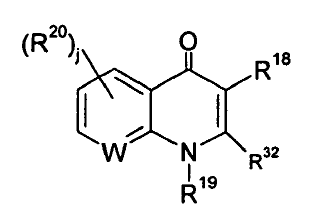 or 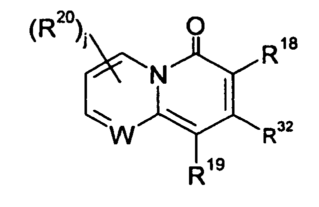
R¹² is hydrogen or C₁₋₆alkyl;
R¹³ is hydrogen or C₁₋₄alkyl substituted by a group selected from optionally substituted phenyl, optionally substituted 5 or 6 membered heteroaryl and optionally substituted 9 to 10 membered fused bicyclic heteroaryl;
R¹⁴ and R¹⁵ are each independently hydrogen or C₁₋₆alkyl;
R¹⁶ is halogen, cyano, nitro, trifluoromethyl, azido, -C(O)R²¹, -C(O)OR²¹, -OC(O)R²¹, -OC(O)OR²¹, -NR²²C(O)R²³, -C(O)NR²²R²³, -NR²²R²³, hydroxyl, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁴R¹⁵, halogen and -OR¹⁴, and the aryl and heteroaryl groups are optionally substituted by up to five groups independently selected from halogen, cyano, nitro, trifluoromethyl, azido, -C(O)R²⁴, -C(O)OR²⁴, -OC(O)OR²⁴, -NR²⁵C(O)R²⁶, -C(O)NR²⁵R²⁶, -NR²⁵R²⁶, hydroxyl, C₁₋₆alkyl and C₁₋₆alkoxy;
R¹⁷ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁷, -S(O)ₙR²⁷, -NR²⁷R²⁸, -CONR²⁷R²⁸, halogen and cyano;
R¹⁸ is hydrogen, -C(O)OR²⁹, -C(O)NHR²⁹, -C(O)CH₂NO₂, or -C(O)CH₂SO₂R⁷;
R¹⁹ is hydrogen; C₁₋₄alkyl optionally substituted by hydroxyl, cyano, NH₂, -NH(C₁₋₄alkyl) or -N(C₁₋₄alkyl)₂; C₂₋₄alkenyl optionally substituted by hydroxyl, cyano, NH₂, -NH(C₁₋₄alkyl) or -N(C₁₋₄alkyl)₂; C₁₋₄alkoxy, C₃₋₇cycloalkyl, -NH₂, -NH(C₁₋₄alkyl) or -N(C₁₋₄alkyl)₂; (C₁₋₄alkyl)OC(O)N(C₁₋₄alkyl) or optionally substituted phenyl or benzyl;
R²⁰ is halogen, C₁₋₄alkyl, C₁₋₄thioalkyl, C₁₋₄alkoxy, -NH₂, -NH(C₁₋₄alkyl) or -N(C₁₋₄alkyl)₂;
R²¹ is hydrogen, C₁₋₁₀alkyl, -(CH₂)ₚaryl or -(CH₂)ₚheteroaryl;
R²² and R²³ are each independently hydrogen, -OR¹⁴, C₁₋₆alkyl, -(CH₂)_{q}aryl or -(CH₂)_{q}heterocyclyl;
R²⁴ is hydrogen, C₁₋₁₀alkyl, -(CH₂)ᵣaryl or -(CH₂)ᵣheteroaryl;
R²⁵ and R²⁶ are each independently hydrogen, -OR¹⁴, C₁₋₆alkyl, -(CH₂)ₛaryl or -(CH₂)ₛheterocyclyl;
R²⁷ and R²⁸ are each independently hydrogen, C₁₋₄alkyl or C₁₋₄alkoxyC₁₋₄alkyl;
R²⁹ is hydrogen or C₁₋₆alkyl optionally substituted by up to three groups independently selected from halogen, C₁₋₄alkoxy, -OC(O)C₁₋₆alkyl and -OC(O)OC₁₋₆alkyl, -(CH₂)_{q}heterocyclyl, -(CH₂)qheteroaryl, -(CH₂)_{q}aryl, or -(CH₂)_{q}C₃₋₇cycloalkyl;
R³⁰ is hydrogen, C₁₋₄alkyl, C₃₋₇cycloalkyl, optionally substituted phenyl or benzyl, acetyl or benzoyl;
R³¹ is hydrogen or R²⁰, or R³¹ and R¹⁹ are linked to form the bivalent radical -O(CH₂)₂-, -(CH₂)ₜ-;-NR⁷(CH₂)ₐ-, -OCH₂NR⁷-, -SCH₂NR⁷-, -CH₂NR⁷CH₂-, -CH₂OCH₂-, -CH₂SCH₂-, -(CH₂)ₐNR⁷- ;
R³² is hydrogen, or R³² and R¹⁹ are linked to form the bivalent radical selected from the group -S(CH₂)_{b}-, -N(R⁷)(CH₂)_{b}-, and -O(CH₂)_{b}-;
R³³ is propyl;
X is -U(CH₂)ᵥB(CH₂)ᵥD-, -U(CH₂)ᵥB-R³³-, -U(CH₂)ᵥB(CH₂)ᵥD(CH₂)ᵥE-, or -U(CH₂)ᵥB(CH₂)ᵥD-R³³-
or X is a group selected from: 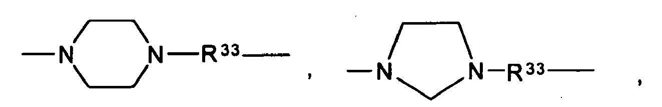 and 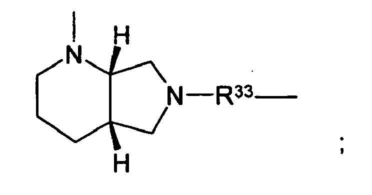
U, B, D and E are independently divalent radicals selected from -N(R³⁰)-, -O-, -S(O)_{z}-, -N(R³⁰)C(O)-, -C(O)N(R³⁰)- and -N[C(O)R³⁰]-;
W is -C(R³¹)- or a nitrogen atom;
a is 1 or 2
b is an integer from 1 to 3;
d is an integer from 1 to 5;
e is an integer from 2 to 4;
f, g, h, m, p, q, r and s are each independently integers from 0 to 4;
i is an integer from 1 to 6;
j, k, n and z are each independently integers from 0 to 2;
t is 2 or 3;
v is an integer from 1 to 8;
or a salt, solvate or ester thereof.

2. A compound according to claim 1, wherein A is -C(O)- or -N(R⁷)-CH₂-.

3. A compound according to claim 1 or claim 2, wherein d is 2.

4. A compound according to any one of the preceding claims, wherein v is 2.

5. A compound according to any one of the preceding claims, wherein R¹¹ is a heterocyclic group of the following formula: 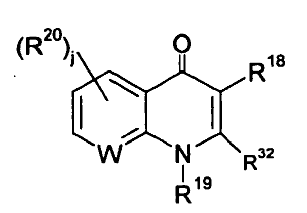 or 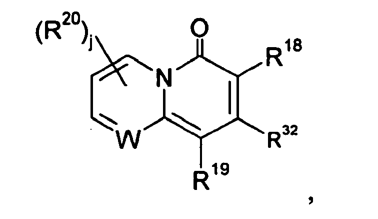 wherein the heterocyclic is linked in the 6 or 7 position and j, R¹⁸, R¹⁹, R²⁰ and R³² are as defined in claim 1.

6. A compound according to claim 5, wherein R¹⁹ is a C₁₋₄alkyl or a C₃₋₇cycloalkyl.

7. A compound according to claim 5 or claim 6, wherein R³² is H.

8. A compound according to any one of claims 5 to 7, wherein R¹⁸ is -C(O)OR²⁹.

9. A compound according to any one of the preceding claims, wherein R³ is hydrogen, R⁴ is hydroxyl and R⁵ is hydroxyl.

10. A compound according to any one of the preceding claims, wherein X is -U(CH₂)ᵥB(CH₂)ᵥD- or -U(CH₂)ᵥB-R³³-.

11. A compound according to claim 10, wherein U is -0- and B is -0-.

12. A compound according to claim 1, selected from:
4"-*O*-(3-{4-[3-(3-Ethoxycarbonyl-1-ethyl-4-oxo-1,4-dihydro-quinolin-6-yl)-propyl]-piperazin-1-yl}-propionyl)-azithromycin,
4"-*O*-(3-{4-[3-(3-Ethoxycarbonyl-1-ethyl-4-oxo-1,4-dihydro-quinolin-6-yl)-propyl]-piperazin-1-yl}-propionyl)-11-*O*-methyl-azithromycin,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-roxythromycin,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-6-*O*-methyl-erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-azithromycin,
4"-*O*-(3-{2-[2-(3-carboxy-7-chloro-1-cyclopropyl-4-oxo-1,4-dihydro-quinolin-6-ylamino)-ethoxy]-ethoxy}-propionyl)-11-O-methyl-azithromycin,
4"-*O*-(3-{2-[2-(3-Carboxy-1-cyclopropyl-4-oxo-1,4-dihydro-quinolin-6-ylamino)-ethoxy]-ethoxy}-propionyl)-azithromycin,
4"-*O*-(2-{3-[2-(3-Carboxy-7-chloro-1-cyclopropyl-4-oxo-1,4-dihydro-quinolin-6-yloxy)-ethoxy]-propionylamino}-acetyl)-azithromycin 11,12-cyclic carbonate,
4"-*O*-(2-{3-[2-(3-Carboxy-7-chloro-1-cyclopropyl-4-oxo-1,4-dihydro-quinolin-6-ylamino)-ethoxy]-propionylamino}-acetyl)-azithromycin 11,12-cyclic carbonate,
4"-*O*-(2-{3-[2-(3-Carboxy-6-fluoro-1-cyclopropyl-8-methoxy-4-oxo-1,4-dihydro-quinolin-7-ylamino)-ethoxy]-propionylamino}-acetyl)-azithromycin 11,12-cyclic carbonate,
4"-*O*-[2-(3-{2-[2-(3-Carboxy-6-fluoro-1-cyclopropyl-4-oxo-1,4-dihydro-quinolin-7-ylamino)-ethoxy]-ethoxy}-propionylamino)-acetyl]-azithromycin 11,12-cyclic carbonate,
4"-*O*-[2-(3-{2-[2-(3-Carboxy-7-chloro-1-cyclopropyl-4-oxo-1,4-dihydro-quinolin-6-ylamino)-ethoxy]-ethoxy}-propionylamino)-acetyl]-azithromycin 11,12-cyclic carbonate,
4"-*O*-[2-(3-{2-[2-(3-Carboxy-7-chloro-1-cyclopropyl-4-oxo-1,4-dihydro-quinolin-6-ylamino)-ethoxy]-ethoxy}-propionylamino)-acetyl]-azithromycin,
4"*O*-{2-[3-({2-[3-(6-Ethoxycarbonyl-7-oxo-2,3-dihydro-1H,7H-pyrido[3,2,1-ij]quinolin-9-yl)-propylamino]-ethyl}-propyl-amino)-propionylamino]-acetyl}-azithromycin,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-9(*E*)-ethoxyimino-erythromycin A,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-6-*O*-methyl-erythromycin A,
4"-*O*-[3-(2-{2-[2-(3-Carboxy-6-fluoro-1-cyclopropyl-4-oxo-1,4-dihydro-quinolin-7-ylamino)-ethoxy]-ethoxy}-ethylamino)-propionyl]-6-*O*-methyl-erythromycin A,
4"-*O*-(3-{2-[2-(3-Carboxy-7-chloro-1-cyclopropyl-4-oxo-1,4-dihydro-quinolin-6-ylamino)-ethoxy]-ethylamino}-propionyl)-6-*O*-methyl-erythromycin A,
4"-*O*-(3-{2-[2-(3-Carboxy-6-fluoro-1-cyclopropyl-4-oxo-1,4-dihydro-quinolin-7-ylamino)-ethoxy]-ethylamino}-propionyl)-6-*O*-methyl-erythromycin A,
4"-*O*-(3-{2-[2-(10-Carboxy-9-oxo-3,4-dihydro-2H,9H-1-oxa-4a-aza-phenanthren-6-ylamino)-ethoxy]-ethoxy}-propionyl)-azithromycin,
11-O-Methyl-4''-O-(3-{2-[3-(3-Carboxy-1-ethyl-4-oxo-1,4-dihydro-quinolin-6-yl)-propoxy]-ethylamino}-propionyl)-azithromycin,
4"-*O*-(3-{2-[2-(3-Carboxy-6-fluoro-1-cyclopropyl-4-oxo-1,4-dihydro-quinolin-7-ylamino)-ethoxy]-ethoxy}-propionyl)-azithromycin,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-6-*O*-methyl-erythromycin A 11,12-cyclic carbamate,
4"-*O*-(3-{2-[2-(3-Carboxy-6-fluoro-1-cyclopropyl-4-oxo-1,4-dihydro-quinolin-7-ylamino)-ethoxy]-ethoxy}-propionyl)-6-*O*-methyl-erythromycin A,
4"-O-(3-{2-[2-(3-Carboxy-7-chloro-1-ethyl-4-oxo-1,4-dihydro-quinoline-6-ylamino)-ethoxy]-ethoxy}-propionyl)-azithromycin,
4"-O-(3-{2-[2-(3-Carboxy-6-fluoro-1-ethyl-4-oxo-1,4-dihydro-quinoline-7-ylamino)-ethoxy]-ethoxy}-propionyl)-azithromycin,
4"-O-(3-{2-[2-(3-Carboxy-1-ethyl-4-oxo-1,4-dihydro-quinoline-6-ylamino)-ethoxy]-ethoxy}-propionyl)-azithromycin,
4"-O-(3-{2-[2-(3-carboxy-7-chloro-1-isopropyl-4-oxo-1,4-dihydro-quinoline-6-ylamino)-ethoxy]-ethoxy}-propionyl)-azithromycin,
4"-O-(3-{2-[2-(6-Carboxy-7-oxo-2,3-dihydro-1H,7H-pyrido[3,2,1-ij]quinolin-9-ylamino)-ethoxy]-ethoxy}-propionyl)-azithromycin,
4"-O-(3-{2-[2-(6-Carboxy-7-oxo-2,3-dihydro-1H,7H-pyrido[3,2,1-ij]quinolin-9-ylamino)-ethoxy]-ethoxy}-propionyl)-6-*O*-methyl-erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-6-*O*-propyl-erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-azithromycin,
4"-*O*-(3-{2-[3-(3-Carboxy-1-ethyl-4-oxo-1,4-dihydro-quinolin-6-yl)-propoxy]-ethylamino}-propionyl)-6-*O*-methyl-erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-6-*O*-methyl-erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-9-ethyloximino-6-*O*-methyl-erythromycin A,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-9-(1-isopropoxy-cyclohexyl)oximino-erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-9-(1-isopropoxy-cyclohexyl)oximino-erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-9-(1-isopropoxy-cyclohexyl)oximino-erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-9-oxime erythromycin A,
4"-*O*-(3-{2-[2-(3-Carboxy-1-cyclopropyl-4-oxo-1,4-dihydro-quinolin-7-yloxy)-ethoxy]-ethoxy}-propionyl)-9-oxime erythromycin A,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-9-oxime erythromycin A,
4"-*O*-(3-{2-[2-(3-Carboxy-1-cyclopropyl-7-methoxy-4-oxo-1,4-dihydro-quinolin-6-ylamino)-ethoxy]-ethoxy}-propionyl)-azithromycin,
4''-*O*-(3-{2-[2-(3-Carboxy-1-cyclopropyl-7-dimethylamino-4-oxo-1,4-dihydro-quinolin-6-ylamino)-ethoxy]-ethoxy}-propionyl) azithromycin,
4"-O-(3-{2-[3-(3-carboxy-1-ethyl-4-oxo-1,4-dihydro-quinolin-6-yl)-propoxy]-ethoxy}-propionyl)-azithromycin,
4"-O-(3-{2-[3-(3-carboxy-1-ethyl-4-oxo-1,4-dihydro-quinolin-6-yl)-propoxy]-ethoxy}-propionyl)-6-O-methyl erythromycin A,
9-Ethyloximino-4"-O-(3-{2-[3-(3-carboxy-1-ethyl-4-oxo-1,4-dihydro-quinolin-6-yl)-propoxy]-ethoxy}-propionyl)-erythromycin A,
4"-O-(3-{2-[3-(3-Carboxy-1-ethyl-4-oxo-1,4-dihydro-quinolin-6-yl)-propoxy]-ethoxy}-propionyl)-6-O-methyl-8a-aza-8a-homoerythromycin A,
4"-O-(3-{2-[3-(3-Carboxy-1-ethyl-4-oxo-1,4-dihydro-quinolin-6-yl)-propoxy]-ethoxy}-propionyl)-roxythromycin,
4"-*O*-(3-{2-[2-(3-Carboxy-7-chloro-1-cyclopropyl-4-oxo-1,4-dihydro-quinolin-6-yloxy)-ethoxy]-ethoxy}-propionyl)-azithromycin,
4"-O-(3-{2-[3-(3-Carboxy-1-ethyl-4-oxo-1,4-dihydro-quinolin-6-yl)-propoxy]-ethoxy}-propionyl)-6-O-methyl-11-desoxy-11-(R)-methylamino-erythromycin A 11,12-carbamate,
4"-O-(3-{2-[3-(3-Carboxy-1-cyclopropyl-4-oxo-1,4-dihydro-quinolin-6-yl)-propoxy]-ethoxy}-propionyl)-azithromycin,
4"-O-(3-{2-[3-(3-Carboxy-4-oxo-1,4-dihydro-quinolin-6-yl)-propoxy]-ethoxy}-propionyl)-azithromycin,
4"-O-(3-{2-[3-(3-Carboxy-4-oxo-1-propyl-1,4-dihydro-quinolin-6-yl)-propoxy]-ethoxy}-propionyl)-azithromycin,
4"-O-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-azithromycin,
4"-O-[3-(2-{2-[2-(3-Carboxy-6-fluoro-1-cyclopropyl-4-oxo-1,4-dihydro-quinolin-7-ylamino)-ethoxy]-ethoxy}-ethylamino)-propionyl]-azithromycin,
4"-O-(3-{2-[2-(3-Carboxy-7-chloro-1-cyclopropyl-4-oxo-1,4-dihydro-quinolin-6-ylamino)-ethoxy]-ethylamino}-propionyl)-azithromycin,
4"-O-(3-{2-[2-(3-Carboxy-6-fluoro-1-cyclopropyl-4-oxo-1,4-dihydro-quinolin-7-ylamino)-ethoxy]-ethylamino}-propionyl)-azithromycin,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-6-*O*-methyl erythromycin A,
4"-*O*-(3-{2-[2-(3-Carboxy-7-chloro-1-cyclopropyl-4-oxo-1,4-dihydro-quinolin-6-ylamino)-ethoxy]-ethoxy}-propionyl)-6-*O*-methyl-8a-aza-8a-homoerythromycin A,
4"-*O*-[3-(2-{2-[2-(3-Carboxy-7-chloro-1-cyclopropyl-4-oxo-1,4-dihydro-quinolin-6-ylamino)-ethoxy]-ethoxy}-ethylamino)-propionyl]-azithromycin,
4"-*O*-[3-(2-{[2-(3-Carboxy-7-chloro-1-cyclopropyl-4-oxo-1,4-dihydro-quinolin-6-ylamino)-ethyl]-methyl-amino}-ethoxy)-propionyl]-azithromycin,
4"-*O*-(3-{2-[2-(3-Carboxy-1-cyclopropyl-4-oxo-1,4-dihydro-quinolin-6-yloxy)-ethoxy]-ethoxy}-propionyl)-azithromycin,
4"-*O*-(3-{2-[2-(3-Carboxy-7-chloro-4-oxo-1,4-dihydro-quinolin-6-yloxy)-ethoxy]-ethoxy}-propionyl)-azithromycin,
4"-*O*-(3-{2-[2-(3-Carboxy-7-chloro-4-oxo-1,4-dihydro-quinolin-6-ylamino)-ethoxy]-ethoxy}-propionyl)-azithromycin,
4"-O-{[6-({2-[(2-Aminoethyl)(methyl)amino]ethyl}thio)-1-ethyl-4-oxo-1,4-dihydro-3-quinolinecarboxylic acid]propionyl}-6-*O*-methylerythromycin A,
4"-O-{[6-({2-[(2-Aminoethyl)(methyl)amino]ethyl}thio)-1-ethyl-4-oxo-1,4-dihydro-3-quinolinecarboxylic acid]propionyl}-azithromycin,
4"-O-{[6-({2-[(2-Aminoethyl)thio]ethyl}oxy)-1-ethyl-4-oxo-1,4-dihydro-3-quinolinecarboxylic acid]propionyl}-6-O-methylerythromycin A,
4"-O-{[6-({2-[(2-Aminoethyl)thio]ethyl}oxy)-1-ethyl-4-oxo-1,4-dihydro-3-quinolinecarboxylic acid]propionyl}-O-(9E)-methoxymethyloximino erythromycin A,
4"-O-{[6-({2-[(2-Aminoethyl)thio]ethyl}oxy)-1-ethyl-4-oxo-1,4-dihydro-3-quinolinecarboxylic acid]propionyl}-O-(9E)-hydroximino erythromycin A,
4"-O-{1-Ethyl-6-(3-{[2-Aminoethyl]oxy}propyl)-4-oxo-1,4-dihydro-3-quinolinecarboxylic acid] ]propionyl}-O-(9E)-hydroximino erythromycin A, and
4"-O-{[1-Ethyl-6-(3-{[2-(methylamino)ethyl]oxy}propyl)-4-oxo-1,4-dihydro-3-quinolinecarboxylic acid] ]propionyl}-O-(9E)-hydroximino erythromycin A,
or a salt, solvate or ester thereof.

13. A compound as claimed in any one of claims 1 to 12, wherein the salt, solvate or ester is a pharmaceutically acceptable salt, solvate or ester.

14. A process for the preparation of a compound as claimed in claim 1 which comprises:
a) reacting a compound of formula (II) 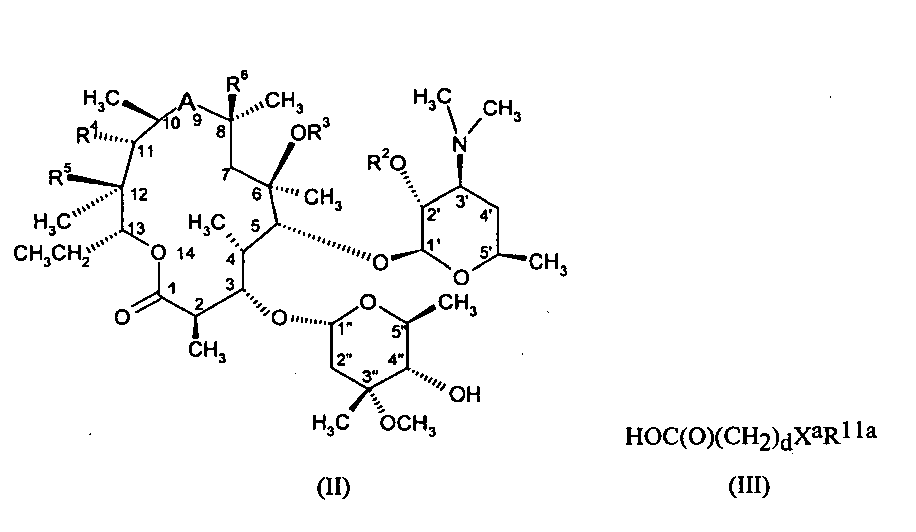 with a suitable activated derivative of the acid (III), wherein X^{a} and R^{11a} are X and R¹¹ as defined in claim 1 or groups convertible to X and R¹¹, to produce a compound of formual (I) wherein d is an integer from 1 to 5;
b) reacting a compound of formula (V) 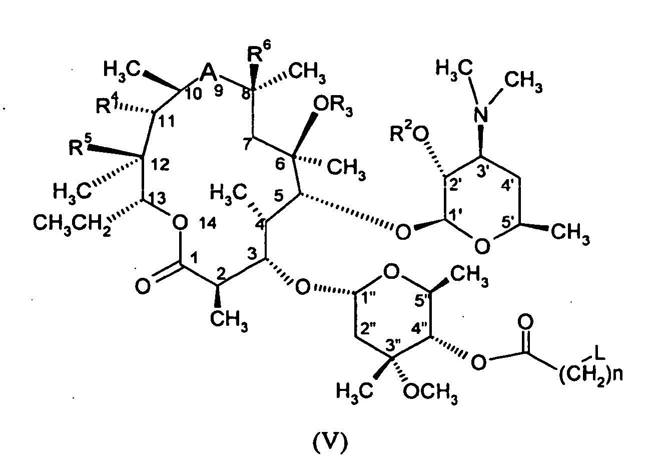 with a compound of formula X^{a}R^{11a} (IV), wherein R^{11a} is R¹¹ as defined in claim 1 or a group convertible to R¹¹ and X^{a} is -U(CH₂)ᵥB- or a group convertible to -U(CH₂)ᵥB- in which U is -N(R³⁰)-, and L is suitable leaving group, to produce a compound of formula (I) wherein U is -N(R³⁰)-; or
c) reacting a compound of formula (VII), with a compound of formula X^{a}R^{11a} (IV), 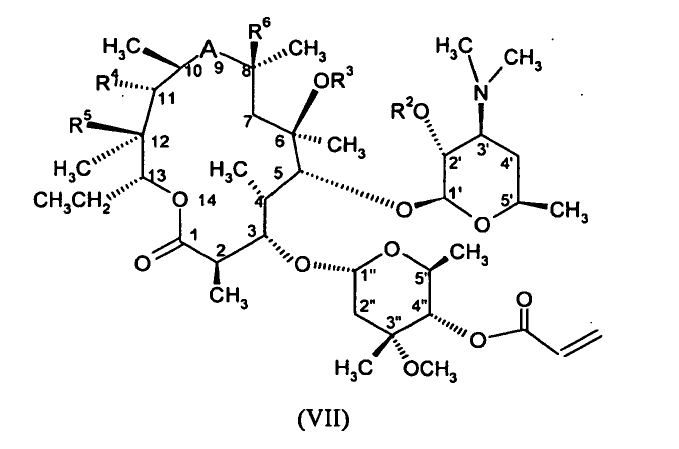 wherein R^{11a} is R¹¹ as defined in claim 1 or a group convertible to R¹¹, and X^{a} is -U(CH₂)ᵥB- or a group convertible to -U(CH₂)ᵥB- in which U is -N(R³⁰)-, to produce a compound of formula (I) wherein d is 2 and U is -N(R³⁰)-, and thereafter, if required, subjecting the resulting compound to one or more of the following operations:
i) removal of the protecting group R²,
ii) conversion of X^{a}R^{11a} to XR¹¹,
iii) conversion of B^{a}R^{11a} to BR¹¹, and
iv) conversion of the resultant compound of formula (I) into a pharmaceutically acceptable salt or solvate thereof.

15. A compound as claimed in any one of claims 1 to 13, or a pharmaceutically acceptable salt, solvate or ester thereof, for use in therapy.

16. Use of a compound as claimed in any one of claims 1 to 13, or a pharmaceutically acceptable salt, solvate or ester thereof, in the manufacture of a medicament for the treatment or prophylaxis of systemic or topical microbial infections in a human or animal body.

17. A compound as claimed in any one of claims 1 to 13, or a pharmaceutically acceptable salt, solvate or ester, for use in the treatment or prophylaxis of systemic or topical microbial infections in a human or animal body.

18. A pharmaceutical composition comprising at least one compound as claimed in any of claims 1 to 13, or a pharmaceutically acceptable salt, solvate or ester thereof, in association with a pharmaceutically acceptable excipient, diluent and/or carrier.

## Patentansprüche

1. Verbindung der Formel (I) 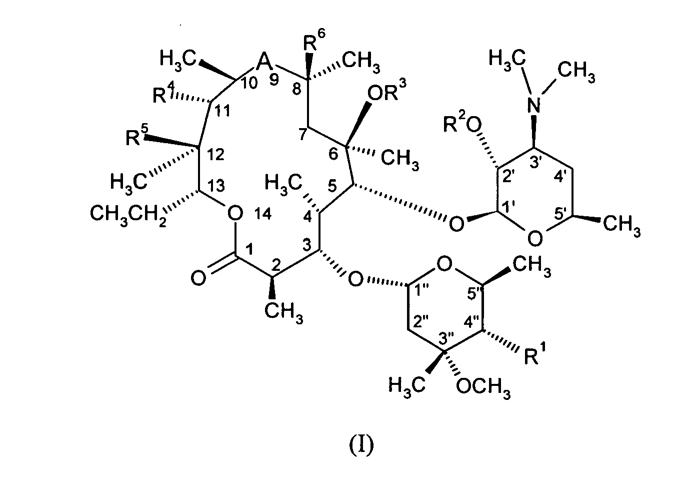 wobei
A ein zweiwertiger Rest, ausgewählt aus -C(O)-, -C(O)NH-, -NHC(O)-, -N(R⁷)-CH₂-, -CH₂-N(R⁷)-, -CH(NR⁸R⁹)- und -C(=NR¹⁰)-, ist;
R¹ für -OC(O)(CH₂)_{d}XR¹¹ steht;
R² Wasserstoff oder eine Hydroxyl-Schutzgruppe ist;
R³ Wasserstoff, C₁₋₄-Alkyl oder C₂₋₆-Alkenyl, gegebenenfalls substituiert mit einem 9- bis 10-gliedrigen kondensierten bicyclischen Heteroaryl, ist;
R⁴ Hydroxyl, C₂₋₆-Alkenyloxy, gegebenenfalls substituiert mit einem 9- bis 10-gliedrigen kondensierten bicyclischen Heteroaryl, oder C₁₋₆-Alkoxy, gegebenenfalls substituiert mit C₁₋₆-Alkoxy oder -O(CH₂)ₑNR⁷R¹², ist;
R⁵ Hydroxyl ist, oder
R⁴ und R⁵ zusammengenommen mit den dazwischenliegenden Atomen einen cyclischen Rest der folgenden Struktur bilden: 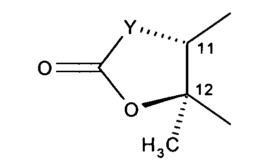
wobei Y ein zweiwertiger Rest, ausgewählt aus -CH₂-, -CH(CN)-, -O-, -N(R¹³)- und -CH(SR¹³)-, ist;
R⁶ Wasserstoff oder Fluor ist;
R⁷ Wasserstoff oder C₁₋₆-Alkyl ist;
R⁸ und R⁹ jeweils unabhängig Wasserstoff, C₁₋₆-Alkyl, -C(=NR¹⁰)NR¹⁴R¹⁵ oder -C(O)R¹⁴ sind, oder
R⁸ und R⁹ zusammen =CH(CR¹⁴R¹⁵)_{f}-Aryl, =CH(CR¹⁴R¹⁵)_{f}-Heterocyclyl, =CR¹⁴R¹⁵ oder =C(R¹⁴)C(O)OR¹⁴ bilden, wobei die Alkyl-, Aryl- und Heterocyclylreste gegebenenfalls mit bis zu drei Resten substituiert sind, die unabhängig aus R¹⁶ ausgewählt sind;
R¹⁰ für -OR¹⁷, C₁₋₆-Alkyl, -(CH₂)_{g}-Aryl, -(CH₂)_{g}-Heterocyclyl oder -(CH₂)ₕO(CH₂)ᵢOR⁷ steht, wobei jeder Rest R¹⁰ gegebenenfalls mit bis zu drei Resten substituiert ist, die unabhängig aus R¹⁶ ausgewählt sind;
R¹¹ ein heterocyclischer Rest der folgenden Struktur: 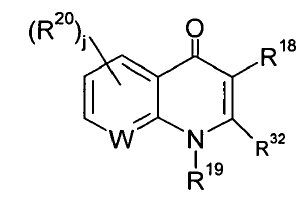 oder 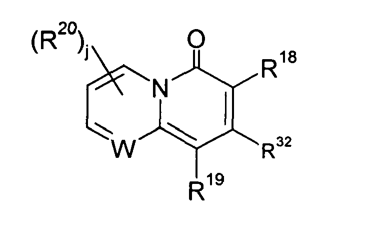 ist;
R¹² Wasserstoff oder C₁₋₆-Alkyl ist;
R¹³ Wasserstoff oder C₁₋₄-Alkyl, substituiert mit einem Rest, ausgewählt aus einem gegebenenfalls substituierten Phenyl, einem gegebenenfalls substituierten 5- oder 6-gliedrigen Heteroaryl und einem gegebenenfalls substituierten 9- bis 10-gliedrigen kondensierten bicyclischen Heteroaryl, ist;
R¹⁴ und R¹⁵ jeweils unabhängig Wasserstoff oder C₁₋₆-Alkyl sind;
R¹⁶ Halogen, Cyano, Nitro, Trifluormethyl, Azido, -C(O)R²¹, -C(O)OR²¹, -OC(O)R²¹, -OC(O)OR²¹, -NR²²C(O)R²³, -C(O)NR²²R²³, -NR²²R²³, Hydroxyl, C₁₋₆-Alkyl, -S(O)ₖC₁₋₆-Alkyl, C₁₋₆-Alkoxy, -(CH₂)ₘ-Aryl oder -(CH₂)ₘ-Heteroaryl ist, wobei der Alkoxyrest gegebenenfalls mit bis zu drei Resten substituiert ist, die unabhängig aus -NR¹⁴R¹⁵, Halogen und -OR¹⁴ ausgewählt sind, und die Aryl- und Heteroarylreste gegebenenfalls mit bis zu fünf Resten substituiert sind, die unabhängig aus Halogen, Cyano, Nitro, Trifluormethyl, Azido, -C(O)R²⁴, -C(O)OR²⁴, -OC(O)OR²⁴, -NR²⁵C(O)R²⁶, -C(O)NR²⁵R²⁶, -NR²⁵R²⁶, Hydroxyl, C₁₋₆-Alkyl und C₁₋₆-Alkoxy ausgewählt sind;
R¹⁷ Wasserstoff, C₁₋₆-Alkyl, C₃₋₇-Cycloalkyl, C₃₋₆-Alkenyl oder ein 5- oder 6-gliedriger heterocyclischer Rest ist, wobei die Alkyl-, Cycloalkyl-, Alkenyl- und heterocyclischen Reste gegebenenfalls mit bis zu drei Substituenten substituiert sind, die unabhängig aus einem gegebenenfalls substituierten 5- oder 6-gliedrigen heterocyclischen Rest, einem gegebenenfalls substituierten 5- oder 6-gliedrigen Heteroaryl, -OR²⁷, -S(O)ₙR²⁷, -NR²⁷R²⁸, -CONR²⁷R²⁸, Halogen und Cyano ausgewählt sind;
R¹⁸ Wasserstoff, -C(O)OR²⁹, -C(O)NHR²⁹, -C(O)CH₂NO₂ oder -C(O)CH₂SO₂R⁷ ist;
R¹⁹ Wasserstoff; C₁₋₄-Alkyl, gegebenenfalls substituiert mit Hydroxyl, Cyano, NH₂, -NH(C₁₋₄-Alkyl) oder -N(C₁₋₄-Alkyl)₂; C₂₋₄-Alkenyl, gegebenenfalls substituiert mit Hydroxyl, Cyano, NH₂, -NH(C₁₋₄-Alkyl) oder -N(C₁₋₄-Alkyl)₂; C₁₋₄-Alkoxy, C₃₋₇-Cycloalkyl, -NH₂, -NH(C₁₋₄-Alkyl) oder -N(C₁₋₄-Alkyl)₂; (C₁₋₄-Alkyl)OC(O)N(C₁₋₄-alkyl) oder gegebenenfalls substituiertes Phenyl oder Benzyl ist;
R²⁰ Halogen, C₁₋₄-Alkyl, C₁₋₄-Thioalkyl, C₁₋₄-Alkoxy, -NH₂, -NH(C₁₋₄-Alkyl) oder -N(C₁₋₄-Alkyl)₂ ist;
R²¹ Wasserstoff, C₁₋₁₀-Alkyl, -(CH₂)ₚ-Aryl oder -(CH₂)ₚ-Heteroaryl ist;
R²² und R²³ jeweils unabhängig Wasserstoff, -OR¹⁴, C₁₋₆-Alkyl, -(CH₂)_{q}-Aryl oder -(CH₂)_{q}-Heterocyclyl sind;
R²⁴ Wasserstoff, C₁₋₁₀-Alkyl, -(CH₂)ᵣ-Aryl oder -(CH₂)ᵣ-Heteroaryl ist;
R²⁵ und R²⁶ jeweils unabhängig Wasserstoff, -OR¹⁴, C₁₋₆-Alkyl, -(CH₂)ₛ-Aryl oder -(CH₂)ₛ-Heterocyclyl sind;
R²⁷ und R²⁸ jeweils unabhängig Wasserstoff, C₁₋₄-Alkyl oder C₁₋₄-Alkoxy-C₁₋₄-alkyl sind;
R²⁹ Wasserstoff oder C₁₋₆-Alkyl, gegebenenfalls substituiert mit bis zu drei Resten, unabhängig ausgewählt aus Halogen, C₁₋₄-Alkoxy, -OC(O)C₁₋₆-Alkyl und -OC(O)OC₁₋₆-Alkyl, -(CH₂)_{q}-Heterocyclyl, -(CH₂)_{q}-Heteroaryl, -(CH₂)_{q}-Aryl oder -(CH₂)_{q}C₃₋₇-Cycloalkyl, ist;
R³⁰ Wasserstoff, C₁₋₄-Alkyl, C₃₋₇-Cycloalkyl, gegebenenfalls substituiertes Phenyl oder Benzyl, Acetyl oder Benzoyl ist;
R³¹ Wasserstoff oder R²⁰ ist, oder R³¹ und R¹⁹ verknüpft sind, um den zweiwertigen Rest -O(CH₂)₂-, -(CH₂)ₜ-; -NR⁷(CH₂)ₐ-, -OCH₂NR⁷-, -SCH₂NR⁷-, -CH₂NR⁷CH₂-, -CH₂OCH₂-, -CH₂SCH₂-, -(CH₂)ₐNR⁷- zu bilden;
R³² Wasserstoff ist, oder R³² und R¹⁹ verknüpft sind, um den zweiwertigen Rest, ausgewählt aus -S(CH₂)_{b}-, -N(R⁷)(CH₂)_{b}- und -O(CH₂)_{b}-, zu bilden;
R³³ Propyl ist;
X für -U(CH₂)ᵥB(CH₂)ᵥD-, -U(CH₂)ᵥB-R³³-, -U(CH₂)ᵥB(CH₂)ᵥD(CH₂)ᵥE- oder -U(CH₂)ᵥB(CH₂)ᵥD-R³³- steht,
oder X ein Rest, ausgewählt aus: 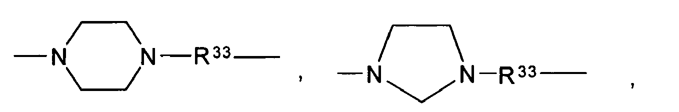 und 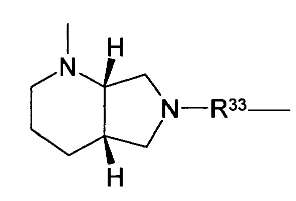 ist;
U, B, D und E unabhängig zweiwertige Reste, ausgewählt aus -N(R³⁰)-, -O-, -S(O)₂-, -N(R³⁰)C(O)-, -C(O)N(R³⁰)- und -N[C(O)R³⁰]-, sind;
W gleich -C(R³¹)- oder ein Stickstoffatom ist;
a gleich 1 oder 2 ist;
b eine ganze Zahl von 1 bis 3 ist;
d eine ganze Zahl von 1 bis 5 ist;
e eine ganze Zahl von 2 bis 4 ist;
f, g, h, m, p, q, r und s jeweils unabhängig ganze Zahlen von 0 bis 4 sind;
i eine ganze Zahl von 1 bis 6 ist;
j, k, n und z jeweils unabhängig ganze Zahlen von 0 bis 2 sind;
t gleich 2 oder 3 ist;
v eine ganze Zahl von 1 bis 8 ist;
oder ein Salz, Solvat oder Ester davon.

2. Verbindung nach Anspruch 1, wobei A gleich -C(O)- oder -N(R⁷)-CH₂- ist.

3. Verbindung nach Anspruch 1 oder Anspruch 2, wobei d gleich 2 ist.

4. Verbindung nach einem der vorhergehenden Ansprüche, wobei v gleich 2 ist.

5. Verbindung nach einem der vorhergehenden Ansprüche, wobei R¹¹ ein heterocyclischer Rest der folgenden Formel: 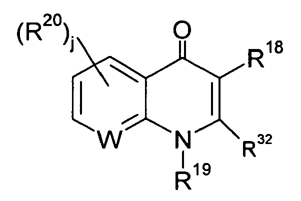 oder 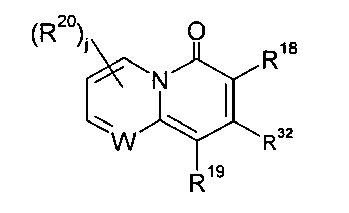 ist,
wobei der heterocyclische Rest an der Position 6 oder 7 verknüpft ist und j, R¹⁸, R¹⁹, R²⁰ und R³² wie in Anspruch 1 definiert sind.

6. Verbindung nach Anspruch 5, wobei R¹⁹ ein C₁₋₄-Alkyl oder ein C₃₋₇-Cycloalkyl ist.

7. Verbindung nach Anspruch 5 oder Anspruch 6, wobei R³² gleich H ist.

8. Verbindung nach einem der Ansprüche 5 bis 7, wobei R¹⁸ gleich -C(O)OR²⁹ ist.

9. Verbindung nach einem der vorhergehenden Ansprüche, wobei R³ Wasserstoff ist, R⁴ Hydroxy ist und R⁵ Hydroxyl ist.

10. Verbindung nach einem der vorhergehenden Ansprüche, wobei X gleich -U(CH₂)ᵥB(CH₂)ᵥD- oder -U(CH₂)ᵥB-R³³- ist.

11. Verbindung nach Anspruch 10, wobei U gleich -O- ist und B gleich -O- ist.

12. Verbindung nach Anspruch 1, ausgewählt aus:
4"-*O*-(3-{4-[3-(3-Ethoxycarbonyl-1-ethyl-4-oxo-1,4-dihydro-chinolin-6-yl)-propyl]-piperazin-1-yl}-propionyl)-azithromycin,
4"-*O*-(3-{4-[3-(3-Ethoxycarbonyl-1-ethyl-4-oxo-1,4-dihydro-chinolin-6-yl)-propyl]-piperazin-1-yl}-propionyl)-11-*O*-methyl-azithromycin,
4"-*O*-(3-{2-[2-(3-Carboxy-7-chlor-1-cyclopropyl-4-oxo-1,4-dihydro-chinolin-6-ylamino)-ethoxy]-ethoxy}-propionyl)-roxithromycin,
4"-*O*-(3-{2-[2-(3-Carboxy-7-chlor-1-cyclopropyl-4-oxo-1,4-dihydro-chinolin-6-ylamino)-ethoxy]-ethoxy}-propionyl)-6-*O*-methyl-erythromycin A,
4"-*O*-(3-{2-[2-(3-Carboxy-7-chlor-1-cyclopropyl-4-oxo-1,4-dihydro-chinolin-6-ylamino)-ethoxy]-ethoxy}-propionyl)-azithromycin,
4"-O-(3-{2-[2-(3-Carboxy-7-chlor-1-cyclopropyl-4-oxo-1,4-dihydro-chinolin-6-ylamino)-ethoxy]-ethoxy}-propionyl)-11-O-methyl-azithromycin,
4"-*O*-(3-{2-[2-(3-Carboxy-1-cyclopropyl-4-oxo-1,4-dihydro-chinolin-6-ylamino)-ethoxy]-ethoxy}-propionyl)-azithromycin,
4"-*O*-(2-{3-[2-(3-Carboxy-7-chlor-1-cyclopropyl-4-oxo-1,4-dihydro-chinolin-6-yloxy)-ethoxy]-propionylamino}-acetyl)-azithromycin-11,12-cyclisches Carbonat,
4"-*O*-(2-{3-[2-(3-Carboxy-7-chlor-1-cyclopropyl-4-oxo-1,4-dihydro-chinolin-6-ylamino)-ethoxy]-propionylamino}-acetyl)-azithromycin-11,12-cyclisches Carbonat,
4"-*O*-(2-{3-[2-(3-Carboxy-6-fluor-1-cyclopropyl-8-methoxy-4-oxo-1,4-dihydro-chinolin-7-ylamino)-ethoxy]-propionylamino}-acetyl)-azithromycin-11,12-cyclisches Carbonat,
4"-*O*-[2-(3-{2-[2-(3-Carboxy-6-fluor-1-cyclopropyl-4-oxo-1,4-dihydro-chinolin-7-ylamino)-ethoxy]-ethoxy}-propionylamino)-acetyl]-azithromycin-11,12-cyclisches Carbonat,
4"-*O*-[2-(3-{2-[2-(3-Carboxy-7-chlor-1-cyclopropyl-4-oxo-1,4-dihydro-chinolin-6-ylamino)-ethoxy]-ethoxy}-propionylamino)-acetyl]-azithromycin-11,12-cyclisches Carbonat,
4"-*O*-[2-(3-{2-[2-(3-Carboxy-7-chlor-1-cyclopropyl-4-oxo-1,4-dihydro-chinolin-6-ylamino)-ethoxy]-ethoxy}-propionylamino)-acetyl]-azithromycin,
4"-*O*-{2-[3-({2-[3-(6-Ethoxycarbonyl-7-oxo-2,3-dihydro-1H,7H-pyrido[3,2,1-ij]-chinolin-9-yl)-propylamino]-ethyl}-propyl-amino)-propionylamino]-acetyl}-azithromycin,
4"-*O*-(3-{2-[2-(3-Carboxy-7-chlor-1-cyclopropyl-4-oxo-1,4-dihydro-chinolin-6-ylamino)-ethoxy]-ethoxy}-propionyl)-9(*E*)-ethoxyimino-erythromycin A,
4"-*O*-[3-(2-{2-[2-(3-Carboxy-7-chlor-1-cyclopropyl-4-oxo-1,4-dihydro-chinolin-6-ylamino)-ethoxy]-ethoxy}-ethylamino)-propionyl]-6-*O*-methyl-erythromycin A,
4"-*O*-[3-(2-{2-[2-(3-Carboxy-6-fluor-1-cyclopropyl-4-oxo-1,4-dihydro-chinolin-7-ylamino)-ethoxy]-ethoxy}-ethylamino)-propionyl]-6-*O*-methyl-erythromycin A,
4"-*O*-(3-{2-[2-(3-Carboxy-7-chlor-1-cyaopropyl-4-oxo-1,4-dihydro-chinolin-6-ylamino)-ethoxy]-ethylamino}-propionyl)-6-*O*-methyl-erythromycin A,
4"-*O*-(3-{2-[2-(3-Carboxy-6-fluor-1-cyclopropyl-4-oxo-1,4-dihydro-chinolin-7-ylamino)-ethoxy]-ethylamino}-propionyl)-6-*O*-methyl-erythromycin A,
4"-*O*-(3-{2-[2-(10-Carboxy-9-oxo-3,4-dihydro-2H,9H-1-oxa-4a-aza-phenanthren-6-ylamino)-ethoxy]-ethoxy}-propionyl)-azithromycin,
11-O-Methyl-4"-O-(3-{2-[3-(3-carboxy-1-ethyl-4-oxo-1,4-dihydro-chinolin-6-yl)-propoxy]-ethylamino}-propionyl)-azithromycin,
4"-*O*-(3-{2-[2-(3-Carboxy-6-fluor-1-cyaopropyl-4-oxo-1,4-dihydro-chinolin-7-ylamino)-ethoxy]-ethoxy}-propionyl)-azithromycin,
4"-*O*-(3-{2-[2-(3-Carboxy-7-chlor-1-cyclopropyl-4-oxo-1,4-dihydro-chinolin-6-ylamino)-ethoxy]-ethoxy}-propionyl)-6-*O*-methyl-erythromycin A-11,12-cyclisches Carbamat,
4"-*O*-(3-{2-[2-(3-Carboxy-6-fluor-1-cyclopropyl-4-oxo-1,4-dihydro-chinolin-7-ylamino)-ethoxy]-ethoxy}-propionyl)-6-*O*-methyl-erythromycin A,
4"-O-(3-{2-[2-(3-Carboxy-7-chlor-1-ethyl-4-oxo-1,4-dihydro-chinolin-6-ylamino)-ethoxy]-ethoxy}-propionyl)-azithromycin,
4"-O-(3-{2-[2-(3-Carboxy-6-fluor-1-ethyl-4-oxo-1,4-dihydro-chinolin-7-ylamino)-ethoxy]-ethoxy}-propionyl)-azithromycin,
4"-O-(3-{2-[2-(3-Carboxy-1-ethyl-4-oxo-1,4-dihydro-chinolin-6-ylamino)-ethoxy]-ethoxy}-propionyl)-azithromycin,
4"-O-(3-{2-[2-(3-Carboxy-7-chlor-1-isopropyl-4-oxo-1,4-dihydro-chinolin-6-ylamino)-ethoxy]-ethoxy}-propionyl)-azithromycin,
4"-O-(3-{2-[2-(6-Carboxy-7-oxo-2,3-dihydro-1H,7H-pyrido[3,2,1-ij]chinolin-9-ylamino)-ethoxy]-ethoxy}-propionyl)-azithromycin,
4"-O-(3-{2-[2-(6-Carboxy-7-oxo-2,3-dihydro-1H,7H-pyrido[3,2,1-ij]chinolin-9-ylamino)-ethoxy]-ethoxy}-propionyl)-6-*O*-methyl-erythromycin A,
4"-*O*-(3-{2-[2-(3-Carboxy-7-chlor-1-cyclopropyl-4-oxo-1,4-dihydro-chinolin-6-ylamino)-ethoxy]-ethoxy}-propionyl)-6-*O*-propyl-erythromycin A,
4"-*O*-(3-{2-[2-(3-Carboxy-1-cyclopropyl-4-oxo-1,4-dihydro-chinolin-7-yloxy)-ethoxy]-ethoxy}-propionyl)-azithromycin,
4"-*O*-(3-{2-[3-(3-Carboxy-1-ethyl-4-oxo-1,4-dihydro-chinolin-6-yl)-propoxy]-ethylamino}-propionyl)-6-*O*-methyl-erythromycin A,
4"-*O*-(3-{2-[2-(3-Carboxy-1-cyclopropyl-4-oxo-1,4-dihydro-chinolin-7-yloxy)-ethoxy]-ethoxy}-propionyl)-6-*O*-methyl-erythromycin A,
4"-*O*-(3-{2-[2-(3-Carboxy-1-cyclopropyl-4-oxo-1,4-dihydro-chinolin-7-yloxy)-ethoxy]-ethoxy}-propionyl)-9-ethyloximino-6-*O*-methyl-erythromycin A,
4"-*O*-[3-(2-{2-[2-(3-Carboxy-7-chlor-1-cyclopropyl-4-oxo-1,4-dihydro-chinolin-6-ylamino)-ethoxy]-ethoxy}-ethylamino)-propionyl]-9-(1-isopropoxy-cyclohexyl)oximino-erythromycin A,
4"-*O*-(3-{2-[2-(3-Carboxy-7-chlor-1-cyclopropyl-4-oxo-1,4-dihydro-chinolin-6-ylamino)-ethoxy]-ethoxy}-propionyl)-9-(1-isopropoxy-cyclohexyl)oximino-erythromycin A,
4"-*O*-(3-{2-[2-(3-Carboxy-1-cyclopropyl-4-oxo-1,4-dihydro-chinolin-7-yloxy)-ethoxy]-ethoxy}-propionyl}-9-(1-isopropoxy-cyclohexyl)oximino-erythromycin A,
4"-*O*-(3-{2-[2-(3-Carboxy-7-chlor-1-cyclopropyl-4-oxo-1,4-dihydro-chinolin-6-ylamino)-ethoxy]-ethoxy}-propionyl)-9-oxim-erythromycin A,
4"-*O*-(3-{2-[2-(3-Carboxy-1-cyclopropyl-4-oxo-1,4-dihydro-chinolin-7-yloxy)-ethoxy]-ethoxy}-propionyl)-9-oxim-erythromycin A,
4"-*O*-[3-(2-{2-[2-(3-Carboxy-7-chlor-1-cyclopropyl-4-oxo-1,4-dihydro-chinolin-6-ylamino)-ethoxy]-ethoxy}-ethylamino)-propionyl]-9-oxim-erythromycin A,
4"-*O*-(3-{2-[2-(3-Carboxy-1-cyclopropyl-7-methoxy-4-oxo-1,4-dihydro-chinolin-6-ylamino)-ethoxy]-ethoxy}-propionyl)-azithromycin,
4"-*O*-(3-{2-[2-(3-Carboxy-1-cyclopropyl-7-dimethylamino-4-oxo-1,4-dihydro-chinolin-6-ylamino)-ethoxy]-ethoxy}-propionyl)-azithromycin,
4"-O-(3-{2-[3-(3-Carboxy-1-ethyl-4-oxo-1,4-dihydro-chinolin-6-yl)-propoxy]-ethoxy}-propionyl)-azithromycin,
4"-O-(3-{2-[3-(3-Carboxy-1-ethyl-4-oxo-1,4-dihydro-chinolin-6-yl)-propoxy]-ethoxy}-propionyl)-6-O-methyl-erythromycin A,
9-Ethyloximino-4"-O-(3-{2-[3-(3-carboxy-1-ethyl-4-oxo-1,4-dihydro-chinolin-6-yl)-propoxy]-ethoxy}-propionyl)-erythromycin A,
4"-O-(3-{2-[3-(3-Carboxy-1-ethyl-4-oxo-1,4-dihydro-chinolin-6-yl)-propoxy]-ethoxy}-propionyl)-6-O-methyl-8a-aza-8a-homoerythromycin A,
4"-O-(3-{2-[3-(3-Carboxy-1-ethyl-4-oxo-1,4-dihydro-chinolin-6-yl)-propoxy]-ethoxy}-propionyl)-roxithromycin,
4"-*O*-(3-{2-[2-(3-Carboxy-7-chlor-1-cyclopropyl-4-oxo-1,4-dihydro-chinolin-6-yloxy)-ethoxy]-ethoxy}-propionyl)-azithromycin,
4"-O-(3-{2-[3-(3-Carboxy-1-ethyl-4-oxo-1,4-dihydro-chinolin-6-yl)-propoxy]-ethoxy}-propionyl)-6-O-methyl-11-desoxy-11-(R)-methylamino-erythromycin A-11,12-carbamat,
4"-O-(3-{2-[3-(3-Carboxy-1-cyclopropyl-4-oxo-1,4-dihydro-chinolin-6-yl)-propoxy]-ethoxy}-propionyl)-azithromycin,
4"-O-(3-{2-[3-(3-Carboxy-4-oxo-1,4-dihydro-chinolin-6-yl)-propoxy]-ethoxy}-propionyl)-azithromycin,
4"-O-(3-{2-[3-(3-Carboxy-4-oxo-1-propyl-1,4-dihydro-chinolin-6-yl)-propoxy]-ethoxy}-propionyl)-azithromycin,
4"-O-[3-(2-{2-[2-(3-Carboxy-7-chlor-1-cyclopropyl-4-oxo-1,4-dihydro-chinolin-6-ylamino)-ethoxy]-ethoxy}-ethylamino)-propionyl]-azithromycin,
4"-O-[3-(2-{2-[2-(3-Carboxy-6-fluor-1-cyclopropyl-4-oxo-1,4-dihydro-chinolin-7-ylamino)-ethoxy]-ethoxy}-ethylamino)-propionyl]-azithromycin,
4"-O-(3-{2-[2-(3-Carboxy-7-chlor-1-cyclopropyl-4-oxo-1,4-dihydro-chinolin-6-ylamino)-ethoxy]-ethylamino}-propionyl)-azithromycin,
4"-O-(3-{2-[2-(3-Carboxy-6-fluor-1-cyclopropyl-4-oxo-1,4-dihydro-chinolin-7-ylamino)-ethoxy]-ethylamino}-propionyl)-azithromycin,
4"-*O*-[3-(2-{2-[2-(3-Carboxy-7-chlor-1-cyclopropyl-4-oxo-1,4-dihydro-chinolin-6-ylamino)-ethoxy]-ethoxy}-ethylamino)-propionyl]-6-*O*-methyl-erythromycin A,
4"-*O*-(3-{2-[2-(3-Carboxy-7-chlor-1-cyclopropyl-4-oxo-1,4-dihydro-chinolin-6-ylamino)-ethoxy]-ethoxy}-propionyl)-6-*O*-methyl-8a-aza-8a-homoerythromycin A,
4"-*O*-[3-(2-{2-[2-(3-Carboxy-7-chlor-1-cyclopropyl-4-oxo-1,4-dihydro-chinolin-6-ylamino)-ethoxy]-ethoxy}-ethylamino)-propionyl]-azithromycin,
4"-*O*-[3-(2-{[2-(3-Carboxy-7-chlor-1-cyclopropyl-4-oxo-1,4-dihydro-chinolin-6-ylamino)-ethyl]-methyl-amino}-ethoxy)-propionyl]-azithromycin,
4"-*O*-(3-{2-[2-(3-Carboxy-1-cyclopropyl-4-oxo-1,4-dihydro-chinolin-6-yloxy)-ethoxy]-ethoxy} -propionyl)-azithromycin,
4"-*O*-(3-{2-{2-(3-Carboxy-7-chlor-4-oxo-1,4-dihydro-chinolin-6-yloxy)-ethoxy]-ethoxy}-propionyl)-azithromycin,
4"-*O*-(3-{2-[2-(3-Carboxy-7-chlor-4-oxo-1,4-dihydro-chinolin-6-ylamino)-ethoxy]-ethoxy}-propionyl)-azithromycin,
4"-O-{[6-({2-[(2-Aminoethyl)(methyl)amino]ethy}thio)-1-ethyl-4-oxo-1,4-dihydro-3-chinolincarbonsäure]propionyl}-6-*O*-methylerythromycin A,
4"-O-{[6-({2-[(2-Aminoethyl)(methyl)amino]ethyl}thio)-1-ethyl-4-oxo-1,4-dihydro-3-chinolincarbonsäure]propionyl}-azithromycin,
4"-O-{[6-({2-[(2-Aminoethyl)thio]ethyl}oxy)-1-ethyl-4-oxo-1,4-dihydro-3-chinolincarbonsäure]propionyl}-6-O-methyl-erythromycin A,
4"-O-{[6-({2-[(2-Aminoethyl)thio]ethyl}oxy)-1-ethyl-4-oxo-1,4-dihydro-3-chinolincarbonsäure]propionyl}-O-(9E)-methoxymethyloximino-erythromycin A,
4"-O-{[6-({2-[(2-Aminoethyl)thio]ethyl}oxy)-1-ethyl-4-oxo-1,4-dihydro-3-chinolincarbonsäure]propionyl}-O-(9E)-hydroximino-erythromycin A,
4"-O-{[1-Ethyl-6-(3-{[2-aminoethyl]oxy}propyl)-4-oxo-1,4-dihydro-3-chinolincarbonsäure]propionyl}-O-(9E)-hydroximino-erythromycin A, und
4"-O-{[1-Ethyl-6-(3-{[2-(methylamino)ethyl]oxy}propyl)-4-oxo-1,4-dihydro-3-chinolincarbonsäure]propionyl}-O-(9E)-hydroximino-erythromycin A,
oder ein Salz, Solvat oder Ester davon.

13. Verbindung nach einem der Ansprüche 1 bis 12, wobei das Salz, Solvat oder Ester ein pharmazeutisch verträgliches Salz, Solvat oder ein pharmazeutisch verträglicher Ester ist.

14. Verfahren zur Herstellung einer Verbindung nach Anspruch 1, umfassend:
a) Umsetzen einer Verbindung der Formel (II) 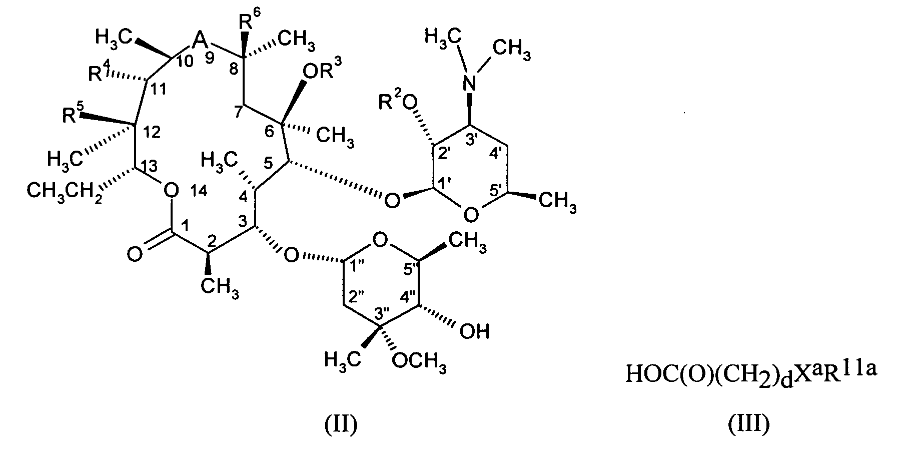 mit einem geeigneten aktivierten Derivat der Säure (III), wobei X^{a} und R^{11a} gleich X und R¹¹, wie in Anspruch 1 definiert, oder in X und R¹¹ umwandelbare Reste sind, um eine Verbindung der Formel (I) herzustellen, wobei d eine ganze Zahl von 1 bis 5 ist;
b) Umsetzen einer Verbindung der Formel (V) 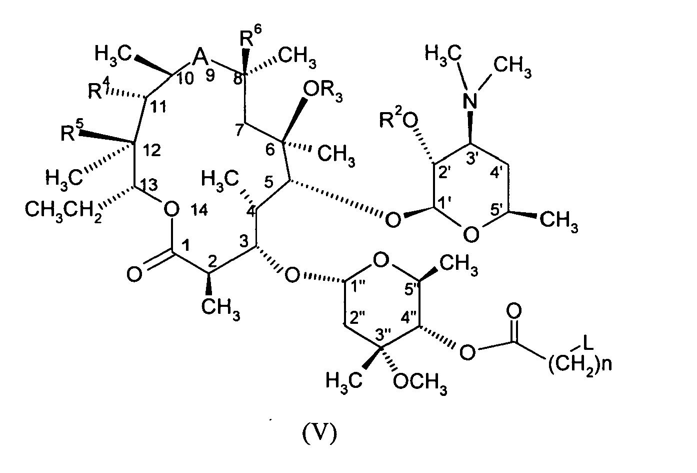 mit einer Verbindung der Formel X^{a}R^{11a} (IV), wobei R^{11a} gleich R¹¹, wie in Anspruch 11 definiert, oder ein in R¹¹ umwandelbarer Rest ist und X^{a} gleich -U(CH₂)ᵥB- oder ein in -U(CH₂)ᵥB- umwandelbarer Rest ist, wobei U gleich -N(R³⁰)- ist und L eine geeignete Abgangsgruppe ist, um eine Verbindung der Formel (I) herzustellen, wobei U gleich -N(R³⁰)- ist; oder
c) Umsetzen einer Verbindung der Formel (VII) mit einer Verbindung der Formel X^{a}R^{11a} (IV), 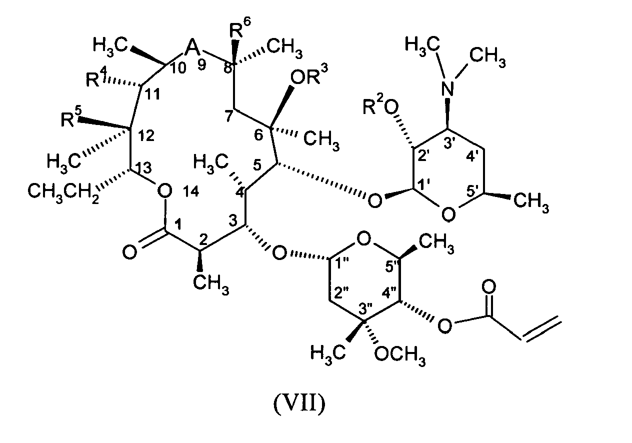 wobei R^{11a} gleich R¹¹, wie in Anspruch 1 definiert, oder ein in R¹¹ umwandelbarer Rest ist und X^{a} gleich -U(CH₂)ᵥB- oder ein in -U(CH₂)ᵥB-umwandelbarer Rest ist, wobei U gleich -N(R³⁰)- ist, um eine Verbindung der Formel (I) herzustellen, wobei d gleich 2 ist und U gleich -N(R³⁰)- ist, und anschließend, falls erforderlich, Unterziehen der resultierenden Verbindung einem oder mehreren der folgenden Schritte:
i) Entfernen der Schutzgruppe R²,
ii) Umwandeln von X^{a}R^{11a} in XR¹¹,
iii) Umwandeln von B^{a}R^{11a} in BR¹¹ und
iv) Umwandeln der resultierenden Verbindung der Formel (I) in ein pharmazeutisch verträgliches Salz oder Solvat davon.

15. Verbindung nach einem der Ansprüche 1 bis 13 oder ein pharmazeutisch verträgliches Salz, Solvat oder ein pharmazeutisch verträglicher Ester davon zur Verwendung bei der Therapie.

16. Verwendung einer Verbindung nach einem der Ansprüche 1 bis 13 oder eines pharmazeutisch verträglichen Salzes, Solvats oder Esters davon bei der Herstellung eines Medikaments zur Behandlung oder Prophylaxe von systemischen oder topischen mikrobiellen Infektionen im Körper eines Menschen oder Tiers.

17. Verbindung nach einem der Ansprüche 1 bis 13 oder ein pharmazeutisch verträgliches Salz, Solvat oder ein pharmazeutisch verträglicher Ester davon zur Verwendung bei der Behandlung oder Prophylaxe von systemischen oder topischen mikrobiellen Infektionen im Körper eines Menschen oder Tiers.

18. Arzneimittel, umfassend mindestens eine Verbindung nach einem der Ansprüche 1 bis 13 oder ein pharmazeutisch verträgliches Salz, Solvat oder einen pharmazeutisch verträglichen Ester davon in Verbindung mit einem pharmazeutisch verträglichen Exzipienten, Verdünnungsmittel und/oder Träger.

## Revendications

1. Composé de formule (I), 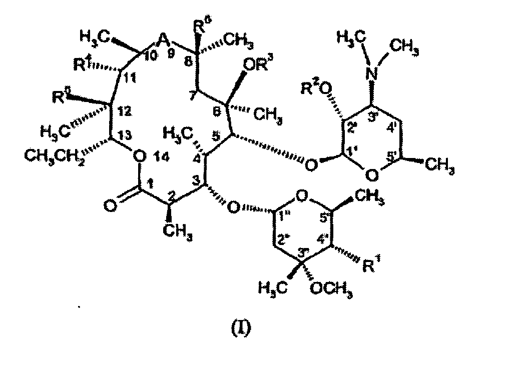 dans laquelle
A représente un radical bivalent choisi entre des radicaux -C(O)-, -C(O)NH-, -NHC(O)-, -N(R⁷)-CH₂-, -CH₂-N(R⁷)-, -CH(NR⁸R⁹)- et -C(=NR¹⁰)- ;
R¹ représente un groupe -OC(O)(CH₂)_{d}XR¹¹;
R² représente un atome d'hydrogène ou un groupe protecteur de la fonction hydroxyle ;
R³ représente un atome d'hydrogène, un groupe alkyle en C₁ à C₄, ou alcényle en C₂ à C₆ facultativement substitué avec un groupe hétéroaryle bicyclique condensé nona- ou décagonal ;
R⁴ représente un groupe hydroxyle, alcényloxy en C₂ à C₆ facultativement substitué avec un groupe hétéroaryle bicyclique condensé nona- ou décagonal, ou alkoxy en C₁ à C₆ facultativement substitué avec un groupe alkoxy en C₁ à C₆ ou -O(CH₂)ₑNR⁷R¹²,
R⁵ représente un groupe hydroxyle, ou bien
R⁴ et R⁵, pris conjointement avec les atomes intermédiaires, forment un groupe cyclique ayant la structure suivante : 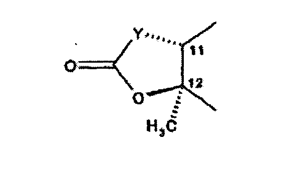 dans laquelle Y représente un radical bivalent choisi entre des radicaux -CH₂-, -CH(CN)-, -O-, -N(R¹³)- et -CH(SR¹³)- ;
R⁶ représente un atome d'hydrogène ou de fluor ;
R⁷ représente un atome d'hydrogène ou un groupe alkyle en C₁ à C₆ ;
R⁸ et R⁹ représentent chacun indépendamment un atome d'hydrogène, un groupe alkyle en C₁ à C₆, -C(=NR¹⁰)NR¹⁴R¹⁵ ou -C(O)R¹⁴, ou bien
R⁸ et R⁹ forment conjointement un groupe -CH(CR¹⁴R¹⁵)_{f}aryle, =CH(CR¹⁴R¹⁵)_{f}hétérocyclyle, =CR¹⁴R¹⁵ ou =C(R¹⁴)C(O)OR¹⁴, dans lequel les groupes alkyle, aryle et hétérocyclyle sont facultativement substitués avec jusqu'à trois groupes choisis indépendamment parmi des groupes R¹⁶;
R¹⁰ représente un groupe -OR¹⁷, alkyle en C₁ à C₆, -(CH₂)_{g}aryle, -(CH₂)_{g}hétérocyclyle ou - (CH₂)ₕO(CH₂)ᵢOR⁷, chaque groupe R¹⁰ étant facultativement substitué avec jusqu'à trois groupes choisis indépendamment parmi des groupes R¹⁶ ;
R¹¹ représente un groupe hétérocyclique ayant la structure suivante : 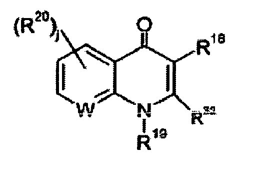 ou 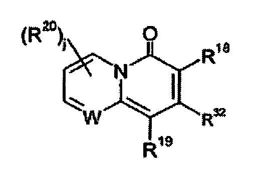
R¹² représente un atome d'hydrogène ou un groupe alkyle en C₁ à C₆ ;
R¹³ représente un atome d'hydrogène ou un groupe alkyle en C₁ à C₄ substitué avec un groupe choisi entre des groupes phényle facultativement substitué, hétéroaryle penta- ou hexagonal facultativement substitué et hétéroaryle bicyclique condensé nona- ou décagonal facultativement substitué ;
R¹⁴ et R¹⁵ représentent chacun indépendamment un atome d'hydrogène ou un groupe alkyle en C₁ à C₆ ;
R¹⁶ représente un groupe halogéno, cyano, nitro, trifluorométhyle, azido, -C(O)R²¹, -C(O)OR²¹, -OC(O)R²¹, -OC(O)OR²¹, -NR²²C(O)R²³, -C(O)NR²²R²³, -NR²²R²³, hydroxy, alkyle en C₁ à C₆, -S(O)ₖ-(alkyle en C₁ à C₆), alkoxy en C₁ à C₆, -(CH₂)ₘaryle, ou -(CH₂)ₘhétéroaryle, dans lequel le groupe alkoxy est facultativement substitué avec jusqu'à trois groupes choisis indépendamment entre des groupes -NR¹⁴R¹⁵, halogéno et -OR¹⁴, et les groupes aryle et hétéroaryle sont facultativement substitués avec jusqu'à cinq groupes choisis indépendamment entre des groupes halogéno, cyano, nitro, trifluorométhyle, azido, -C(O)R²⁴, -C(O)OR²⁴, -OC(O)OR²⁴, -NR²⁵C(O)R²⁶, -C(O)NR²⁵R²⁶, -NR²⁵R²⁶, hydroxyle, alkyle en C₁ à C₆ et alkoxy en C₁ à C₆ ;
R¹⁷ représente un atome d'hydrogène, un groupe alkyle en C₁ à C₆, cycloalkyle en C₃ à C₇, alcényle en C₃ à C₆ ou un groupe hétérocyclique penta- ou hexagonal, dans lequel les groupes alkyle, cycloalkyle, alcényle et hétérocycliques sont facultativement substitués avec jusqu'à trois substituants choisis indépendamment entre un groupe hétérocyclique penta- ou hexagonal facultativement substitué, un groupe hétéroaryle penta- ou hexagonal facultativement substitué, un groupe -OR²⁷, un groupe -S(O)ₙR²⁷, un groupe -NR²⁷R²⁸, un groupe -CONR²⁷R²⁸, un groupe halogéno et un groupe cyano ;
R¹⁸ représente un atome d'hydrogène, un groupe -C(O)OR²⁹, -C(O)NHR²⁹, -C(O)CH₂NO₂ ou -C(O)CH₂SO₂R⁷ ;
R¹⁹ représente un atome d'hydrogène ; un groupe alkyle en C₁ à C₄ facultativement substitué avec un groupe hydroxyle, cyano, NH₂, -NH-(alkyle en C₁ à C₄) ou -N-(alkyle en C₁ à C₄)₂ ; alcényle en C₂ à C₄ facultativement substitué avec un substituant hydroxyle, cyano, NH₂, -NH-(alkyle en C₁ à C₄) ou -N-(alkyle en C₁ à C₄)₂ ; alkoxy en C₁ à C₄, cycloalkyle en C₃ à C₇, -NH₂, -NH-(alkyle en C₁ à C₄) ou -N-(alkyle en C₁ à C₄)₂ ; (alkyle en C₁ à C₄)-OC(O)N-(alkyle en C₁ à C₄) ou un groupe phényle ou benzyle facultativement substitué ;
R²⁰ représente un groupe halogéno, alkyle en C₁ à C₄, thioalkyle en C₁ à C₄, alkoxy en C₁ à C₄, -NH₂, -NH-(alkyle en C₁ à C₄) ou -N-(alkyle en C₁ à C₄)₂ ;
R²¹ représente un atome d'hydrogène, un groupe alkyle en C₁ à C₁₀, -(CH₂)ₚaryle ou -(CH₂)ₚhétéroaryle ;
R²² et R²³ représentent chacun indépendamment un atome d'hydrogène, un groupe -OR¹⁴, alkyle en C₁ à C₆, -(CH₂)_{q}aryle ou -(CH₂)_{q}hétérocyclyle ;
R²⁴ représente un atome d'hydrogène, un groupe alkyle en C₁ à C₁₀, -(CH₂)ᵣaryle ou -(CH₂)ᵣhétéroaryle ;
R²⁵ et R²⁶ représentent chacun indépendamment un atome d'hydrogène, un groupe -OR¹⁴, alkyle en C₁ à C₆, -(CH₂)ₛaryle ou -(CH₂)ₛhétérocyclyle ;
R²⁷ et R²⁸ représentent chacun indépendamment un atome d'hydrogène, un groupe alkyle en C₁ à C₄ ou (alkoxy en C₁ à C₄)-(alkyle en C₁ à C₄) ;
R²⁹ représente un atome d'hydrogène ou un groupe alkyle en C₁ à C₆ facultativement substitué avec jusqu'à trois groupes choisis indépendamment entre des groupes halogéno, alkoxy en C₁ à C₄, -OC(O)-(alkyle en C₁ à C₆) et -OC(O)O-(alkyle en C₁ à C₆) , -(CH₂)_{q}hétérocyclyle, (CH₂)_{q}hétéroaryle, -(CH₂)_{q}aryle, ou -(CH₂)_{q}-(cycloalkyle en C₃ à C₇) ;
R³⁰ représente un atome d'hydrogène, un groupe alkyle en C₁ à C₄, un groupe cycloalkyle en C₃ à C₇, un groupe phényle ou benzyle facultativement substitué, un groupe acétyle ou benzoyle ;
R³¹ représente un atome d'hydrogène ou bien R²⁰, ou R³¹ et R¹⁹ sont liés pour former le radical bivalent -O(CH₂)₂-, -(CH₂)ₜ- ; -NR⁷(CH₂)ₐ-, -OCH₂NR⁷-, -SCH₂NR⁷-, -CH₂NR⁷CH₂-, -CH₂OCH₂-, -CH₂SCH₂-, -(CH₂)ₐNR⁷- ;
R³² représente un atome d'hydrogène, ou bien R³² et R¹⁹ sont liés pour former un radical bivalent choisi dans le groupe consistant en -S(CH₂)_{b}-, -N(R⁷)(CH₂)_{b}-, et -O(CH₂)_{b}- ;
R³³ représente un groupe propyle ;
X représente un groupe -U(CH₂)ᵥB(CH₂)ᵥD-, -U(CH₂)ᵥB-R³³-, -U(CH₂)ᵥB(CH₂)ᵥD(CH₂)ᵥE-, ou -U(CH₂)ᵥB(CH₂)ᵥD-R³³-
ou bien X représente un groupe choisi entre des groupes : 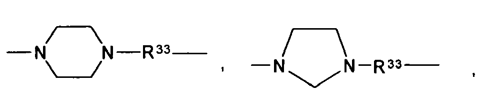 et 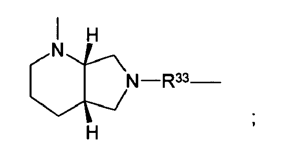
U, B, D et E représentent indépendamment des radicaux divalents choisis entre des radicaux -N(R³⁰)-, -O-, -S(O)_{z}-, -N(R³⁰)C(O)-, -C(O)N(R³⁰)- et -N[C(O)R³⁰]- ;
W représente un groupe -C(R³¹)- ou un atome d'azote ;
a est égal à 1 ou 2 ;
b représente un nombre entier de 1 à 3 ;
d représente un nombre entier de 1 à 5 ;
e représente un nombre entier de 2 à 4 ;
f, g, h, m, p, q, r et s représentent chacun indépendamment des nombres entiers de 0 à 4 ;
i représente un nombre entier de 1 à 6 ;
j, k, n et z représentent chacun indépendamment des nombres entiers de 0 à 2 ;
t est égal à 2 ou 3 ;
v représente un nombre entier de 1 à 8 ;
ou un de ses sels, produits de solvatation ou esters.

2. Composé suivant la revendication 1, dans lequel A représente un groupe -C(O)- ou -N(R⁷)-CH₂-.

3. Composé suivant la revendication 1 ou la revendication 2, dans lequel d est égal à 2.

4. Composé suivant l'une quelconque des revendications précédentes, dans lequel v est égal à 2.

5. Composé suivant l'une quelconque des revendications précédentes, dans lequel R¹¹ représente un groupe hétérocyclique répondant à la formule suivante : 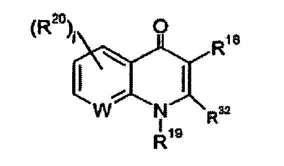 ou 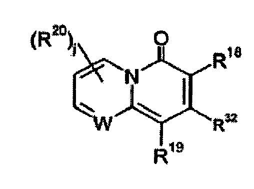 dans laquelle
le groupe hétérocyclique est lié en position 6 ou 7 et j, R¹⁸, R¹⁹, R²⁰ et R³² répondent aux définitions figurant dans la revendication 1.

6. Composé suivant la revendication 5, dans lequel R¹⁹ représente un groupe alkyle en C₁ à C₄ ou cycloalkyle en C₃ à C₇.

7. Composé suivant la revendication 5 ou la revendication 6, dans lequel R³² représente H.

8. Composé suivant l'une quelconque des revendications 5 à 7, dans lequel R¹⁸ représente un groupe -C(O)OR²⁹.

9. Composé suivant l'une quelconque des revendications précédentes, dans lequel R³ représente un atome d'hydrogène, R⁴ représente un groupe hydroxy et R⁵ représente un groupe hydroxyle.

10. Composé suivant l'une quelconque des revendications précédentes, dans lequel X représente un groupe -U(CH₂)ᵥB(CH₂)ᵥD- ou -U(CH₂)ᵥB-R³³-.

11. Composé suivant la revendication 10, dans lequel U représente un groupe -O- et B représente un groupe -O-.

12. Composé suivant la revendication 1, choisi entre :
la 4"-*O*-(3-{4-[3-(3-éthoxycarbonyl-1-éthyl-4-oxo-1,4-dihydro-quinoléine-6-yl)-propyl]-pipérazine-1-yl}-propionyl)-azithromycine,
la 4"-*O*-(3-{4-[3-(3-éthoxycarbonyl-1-éthyl-4-oxo-1,4-dihydro-quinoléine-6-yl)-propyl]-pipérazine-1-yl}-propionyl)-11-*O*-méthyl-azithromycine,
la 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-roxythromycine,
la 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-6-*O*-méthyl-érythromycine A,
la 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-azithromycine,
la 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-11-*O*-méthyl-azithromycine,
la 4"-*O*-(3-{2-[2-(3-carboxy-1-cyclopropyl-4-oxo-1,4-dihydro-quinoléine-6-ylamino)-éthoxy]-éthoxy}-propionyl)-azithromycine,
le 11,12-carbonate cyclique de 4"-*O*-(2-{3-[2-(3-carboxy-7-chloro-1-cyclopropyl-4-oxo-1,4-dihydro-quinoléine-6-yloxy)-éthoxy]-propionylamino}-acétyl)-azithromycine,
le 11,12-carbonate cyclique de 4"-*O*-(2-{3-[2-(3-carboxy-7-chloro-1-cyclopropyl-4-oxo-1,4-dihydro-quinoléine-6-ylamino)-éthoxy]-propionylamino}-acétyl)-azithromycine,
le 11,12-carbonate cyclique de 4"-*O*-(2-{3-[2-(3-carboxy-6-fluoro-1-cyclopropyl-8-méthoxy-4-oxo-1,4-dihydroquinoléine-7-ylamino)-éthoxy]-propionylamino}-acétyl)-azithromycine,
le 11,12-carbonate cyclique de 4"-*O*-[2-(3-{2-[2-(3-carboxy-6-fluoro-1-cyclopropyl-4-oxo-1,4-dihydro-quinoléine-7-ylamino)-éthoxy]-éthoxy}-propionylamino)-acétyl]-azithromycine,
le 11,12-carbonate cyclique de 4"-*O*-[2-(3-{2-[2-(3-carboxy-7-chloro-1-cyclopropyl-4-oxo-1,4-dihydro-quinoléine-6-ylamino)-éthoxy]-éthoxy}-propionylamino)-acétyl]-azithromycine,
la 4"-*O*-[2-(3-{2-[2-(3-carboxy-7-chloro-1-cyclopropyl-4-oxo-1,4-dihydro-quinoléine-6-ylamino)-éthoxy]-éthoxy}-propionylamino)-acétyl]-azithromycine,
la 4"-*O*-{2-[3-({2-[3-(6-éthoxycarbonyl-7-oxo-2,3-dihydro-1H,7H-pyrido[3,2,1-ij]quinoléine-9-yl)-propylamino]-éthyl}-propylamino)-propionylamino]-acétyl}-azithromycine,
la 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-9(E)-éthoxyimino-érythromycine A,
la 4"-*O*-[3-(2-{2-[2-(3-carboxy-7-chloro-1-cyclopropyl-4-oxo-1,4-dihydro-quinoléine-6-ylamino)-éthoxy]-éthoxy}-éthylamino)-propionyl]-6-*O*-méthyl-érythromycine A,
la 4"-*O*-[3-(2-{2-[2-(3-carboxy-6-fluoro-1-cyclopropyl-4-oxo-1,4-dihydro-quinoléine-7-ylamino)-éthoxy]-éthoxy}-éthylamino)-propionyl]-6-*O*-méthyl-érythromycine A,
la 4"-*O*-(3-{2-[2-(3-carboxy-7-chloro-1-cyclopropyl-4-oxo-1,4-dihydro-quinoléine-6-ylamino)-éthoxy]-éthylamino}-propionyl)-6-*O*-méthyl-érythromycine A,
la 4"-*O*-(3-{2-[2-(3-carboxy-6-fluoro-1-cyclopropyl-4-oxo-1,4-dihydro-quinoléine-7-ylamino)-éthoxy]-éthylamino}-propionyl)-6-*O*-méthyl-érythromycine A,
la 4"-*O*-(3-{2-[2-(10-carboxy-9-oxo-3,4-dihydro-2H,9H-1-oxa-4a-aza-phénanthrène-6-ylamino)-éthoxy]-éthoxy}-propionyl)-azithromycine,
la 11-O-méthyl-4"-O-(3-{2-[3-(3-carboxy-1-éthyl-4-oxo-1,4-dihydro-quinoléine-6-yl)-propoxy]-éthylamino}-propionyl)-azithromycine, la 4"-*O*-(3-{2-[2-(3-carboxy-6-fluoro-1-cyclopropyl-4-oxo-1,4-dihydro-quinoléine-7-ylamino)-éthoxy]-éthoxy}-propionyl)-azithromycine,
le 11,12-carbamate cyclique de 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-6-*O*-méthyl-érythromycine A,
la 4"-*O*-(3-{2-[2-(3-carboxy-6-fluoro-1-cyclopropyl-4-oxo-1,4-dihydro-quinoléine-7-ylamino)-éthoxy]-éthoxy}-propionyl)-6-*O*-méthyl-érythromycine A,
la 4"-O-(3-{2-[2-(3-carboxy-7-chloro-1-éthyl-4-oxo-1,4-dihydro-quinoléine-6-ylamino)-éthoxy]-éthoxy}-propionyl)-azithromycine,
la 4"-O-(3-{2-[2-(3-carboxy-6-fluoro-1-éthyl-4-oxo-1,4-dihydro-quinoléine-7-ylamino)-éthoxy]-éthoxy}-propionyl)-azithromycine,
la 4"-O-(3-{2-[2-(3-carboxy-1-éthyl-4-oxo-1,4-dihydroquinoléine-6-ylamino)-éthoxy]-éthoxy}-propionyl)-azithromycine,
la 4"-O-(3-{2-[2-(3-carboxy-7-chloro-1-isopropyl-4-oxo-1,4-dihydro-quinoléine-6-ylamino)-éthoxy]-éthoxy}-propionyl)-azithromycine,
la 4"-O-(3-{2-[2-(3-carboxy-7-oxo-2,3-dihydro-1H,7H-pyrido[3.2-1-ij]quinoléine-9-ylamino)-éthoxy]-éthoxy}-propionyl)-azithromycine,
la 4"-O-(3-{2-[2-(6-carboxy-7-oxo-2,3-dihydro-1H,7H-pyrido[3.2.1-ij]quinoléine-9-ylamino)-éthoxy]-éthoxy}-propionyl)-6-*O*-méthyl-érythromycine A,
la 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-6-*O*-propyl-érythromycine A,
la 4"-*O*-(3-{2-[2-(3-carboxy-1-cyclopropyl-4-oxo-1,4-dihydro-quinoléine-7-yloxy)-éthoxy]-éthoxy}-propionyl)-azithromycine,
la 4"-*O*-(3-{2-[2-(3-carboxy-1-éthyl-4-oxo-1,4-dihydroquinoléine-6-yl)-propoxy]-éthylamino}-propionyl)-6-*O-*méthyl-érythromycine A,
la 4"-*O*-(3-{2-[2-(3-carboxy-1-cyclopropyl-4-oxo-1,4-dihydro-quinoléine-7-yloxy)-éthoxy]-éthoxy}-propionyl)-6-*O-*méthyl-érythromycine A,
la 4"-*O*-(3-{2-[2-(3-carboxy-l-cyclopropyl-4-oxo-1,4-dihydro-quinoléine-7-yloxy)-éthoxy]-éthoxy}-propionyl)-9-éthyloximino-6-O-méthyl-érythromycine A,
la 4"-*O*-[3-(2-{2-[2-(3-carboxy-7-chloro-1-cyclopropyl-4-oxo-1,4-dihydro-quinoléine-6-ylamino)-éthoxy]-éthoxy}-éthylamino)-propionyl]-9-(1-isopropoxy-cyclohexyl)-oximino-érythromycine A,
la 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-9-(1-isopropoxy-cyclohexyl)-oximino-érythromycine A,
la 4"-*O*-(3-{2-[2-(3-carboxy-1-cyclopropyl-4-oxo-1,4-dihydro-quinoléine-7-yloxy)-éthoxy]-éthoxy}-propionyl)-9-(1-isopropoxy-cyclohexyl)-oximino-érythromycine A,
la 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-9-oxime-érythromycine A,
la 4"-*O*-(3-{2-[2-(3-carboxy-1-cyclopropyl-4-oxo-1,4-dihydro-quinoléine-7-yloxy)-éthoxy]-éthoxy}-propionyl)-9-oxime-érythromycine A,
la 4"-*O*-[3-(2-{2-(3-carboxy-7-chloro-1-cyclopropyl-4-oxo-1,4-dihydro-quinoléine-6-ylamino)-éthoxy]-éthoxy}-éthylamino)-propionyl]-9-oxime-érythromycine A,
la 4"-*O*-(3-{2-[2-(3-carboxy-1-cyclopropyl-7-méthoxy-4-oxo-1,4-dihydro-quinoléine-6-ylamino)-éthoxy]-éthoxy}-propionyl)-azithromycine,
la 4"-*O*-(3-{2-[2-(3-carboxy-1-cyclopropyl-7-diméthylamino-4-oxo-1,4-dihydro-quinoléine-6-ylamino)-éthoxy]-éthoxy}-propionyl)-azithromycine,
la 4"-O-(3-{2-[3-(3-carboxy-1-éthyl-4-oxo-1,4-dihydroquinoléine-6-yl)-propoxy]-éthoxy}-propionyl)-azithromycine,
la 4"-O-(3-{2-[3-(3-carboxy-1-éthyl-4-oxo-1,4-dihydroquinoléine-6-yl)-propoxy]-éthoxy}-propionyl)-6-O-méthyl-érythromycine A,
la 9-éthyloximino-4"-O-(3-{2-[3-(3-carboxy-1-éthyl-4-oxo-1,4-dihydro-quinoléine-6-yl)-propoxy]-éthoxy}-propionyl)-érythromycine A,
la 4"-O-(3-{2-[3-(3-carboxy-1-éthyl-4-oxo-1,4-dihydroquinoléine-6-yl)-propoxy]-éthoxy}-propionyl)-6-O-méthyl-8a-aza-8a-homoérythromycine A,
la 4"-O-(3-{2-[3-(3-carboxy-1-éthyl-4-oxo-1,4-dihydroquinoléine-6-yl)-propoxy]-éthoxy}-propionyl)-roxythromycine,
la 4"-*O*-(3-{2-[3-carboxy-7-chloro-1-cyclopropyl-4-oxo-1,4-dihydro-quinoléine-6-yloxy)-éthoxy]-éthoxy}-propionyl)-azithromycine,
le 11,12-carbamate de 4"-O-(3-{2-[3-(3-carboxy-1-éthyl-4-oxo-1,4-dihydro-quinoléine-6-yl)-propoxy]-éthoxy}-propionyl)-6-O-méthyl-11-désoxy-11-(R)-méthylamino-érythromycine A,
la 4"-O-(3-{2-[3-(3-carboxy-1-cyclopropyl-4-oxo-1,4-dihydro-quinoléine-6-yl)-propoxy]-éthoxy}-propionyl)-azithromycine,
la 4"-O-(3-{2-[3-(3-carboxy-4-oxo-1,4-dihydro-quinoléine-6-yl)-propoxy]-éthoxy}-propionyl)-azithromycine,
la 4"-O-(3-{2-[3-(3-carboxy-4-oxo-1-propyl-1,4-dihydroquinoléine-6-yl)-propoxy]-éthoxy}-propionyl)-azithromycine,
la 4"-O-[3-(2-{2-[2-(3-carboxy-7-chloro-1-cyclopropyl-4-oxo-1,4-dihydro-quinoléine-6-ylamino)-éthoxy]-éthoxy}-éthylamino)-propionyl]-azithromycine,
la 4"-O-[3-(2-{2-[2-(3-carboxy-6-fluoro-1-cyclopropyl-4-oxo-1,4-dihydro-quinoléine-7-ylamino)-éthoxy]-éthoxy}-éthylamino)-propionyl]-azithromycine,
la 4"-O-(3-{2-[2-(3-carboxy-7-chloro-1-cyclopropyl-4-oxo-1,4-dihydro-quinoléine-6-ylamino)-éthoxy]-éthylamino}-propionyl)-azithromycine,
la 4"-O-(3-{2-[2-(3-carboxy-6-fluoro-1-cyclopropyl-4-oxo-1,4-dihydro-quinoléine-7-ylamino)-éthoxy]-éthylamino}-propionyl)-azithromycine,
la 4"-*O*-[3-(2-{2-[2-(3-carboxy-7-chloro-1-cyclopropyl-4-oxo-1,4-dihydro-quinoléine-6-ylamino)-éthoxy]-éthoxy}-éthylamino)-propionyl]-6-*O*-méthyl-érythromycine A,
la 4"-*O*-(3-{2-[2-(3-carboxy-7-chloro-1-cyclopropyl-4-oxo-1,4-dihydro-quinoléine-6-ylamino)-éthoxy]-éthoxy}-propionyl)-6-*O*-méthyl-8a-aza-8a-homoérythromycine A,
la 4"-*O*-[3-(2-{2-(3-carboxy-7-chloro-1-cyclopropy]-4-oxo-1,4-dihydro-quinoléine-6-ylamino)-éthoxy]-éthoxy}-éthylamino)-propionyl]-azithromycine,
la 4"-*O*-[3-(2-{[2-(3-carboxy-7-chloro-1-cyclopropyl-4-oxo-1,4-dihydro-quinoléine-6-ylamino)-éthyl]-méthylamino}-éthoxy)-propionyl]-azithromycine,
la 4"-*O*-(3-{2-[2-(3-carboxy-1-cyclopropyl-4-oxo-1,4-dihydro-quinoléine-6-yloxy)-éthoxy]-éthoxy}-propionyl)-azithromycine,
la 4"-*O*-(3-{2-[2-(3-carboxy-7-chloro-4-oxo-1,4-dihydroquinoléine-6-yloxy)-éthoxy]-éthoxy}-propionyl)-azithromycine,
la 4"-*O*-(3-{2-[2-(3-carboxy-7-chloro-4-oxo-1,4-dihydroquinoléine-6-ylamino)-éthoxy]-éthoxy}-propionyl)-azithromycine,
la 4"-O-{[acide6-({2-[(2-aminoéthyl)-(méthyl)-amino)-éthyl}-thio)-1-éthyl-4-oxo-1,4-dihydro-3-quinoléine-carboxylique]-propionyl}-6-*O*-méthyérythromycine A,
la 4"-O-{[6-({2-[acide (2-aminoéthyl)-(méthyl)-amino)-éthyl}-thio)-1-éthyl-4-oxo-1,4-dihydro-3-quinoléinecarboxylique]-propionyl}-azithromycine,
la 4"-O-{[acide 6-({2-[(2-aminoéthyl)-thio]-éthyl}-oxy)-1-éthyl-4-oxo-1,4-dihydro-3-quinoléine-carboxylique]-propionyl}-6-O-méthylérythromycine A,
la 4"-O-{[acide 6-({2-[(2-aminoéthyl)-thio]-éthyl}-oxy)-1-éthyl-4-oxo-1,4-dihydro-3-quinoléine-carboxylique]-propionyl}-O-(9E)-méthoxyméthyloximino-érythomycine A,
la 4"-O-{[acide 6-({2-[(2-aminoéthyl)-thio]-éthyl}-oxy)-1-éthyl-4-oxo-1,4-dihydro-3-quinoléine-carboxylique]-propionyl}-O-(9E)-hydroximino-érythomycine A,
la 4"-O-{[acide 1-éthyl-6-(3-{[2-aminoéthyl]-oxy}-propyl)-4-oxo-1,4-dihydro-3-quinoléine-carboxylique]]-propionyl}-O-(9E)-hydroximino-érythromycine A, et
la 4"-O-{[acide 1-éthyl-6-(3-{[2-(méthylamino)-éthyl]-oxy}-propyl)-4-oxo-1,4-dihydro-3-quinoléine-carboxylique]]-propionyl}-O-(9E)-hydroximino-érythromycine A,
ou un de ses sels, produits de solvatation ou esters.

13. Composé suivant l'une quelconque des revendications 1 à 12, dans lequel le sel, produit de solvatation ou ester est un sel, produit de solvatation ou ester pharmaceutiquement acceptable.

14. Procédé pour la préparation d'un composé suivant la revendication 1, qui comprend :
a) la réaction d'un composé de formule (II) 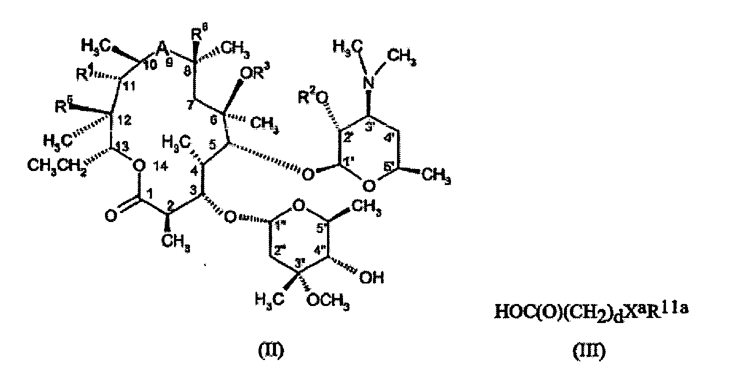 avec un dérivé activé convenable de l'acide (III), dans lequel X^{a} et R^{11a} représentent des groupes X et R¹¹ tels que définis dans la revendication 1 ou des groupes pouvant être convertis en des groupes X et R¹¹, pour produire un composé de formule (I) dans laquelle d représente un nombre entier de 1 à 5 ;
b) la réaction d'un composé de formule (V) 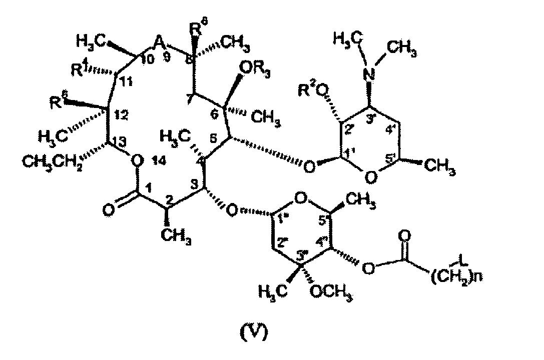 avec un composé de formule X^{a}R^{11a} (IV), dans laquelle R^{11a} représente un groupe R¹¹ tel que défini dans la revendication 1 ou un groupe pouvant être converti en un groupe R¹¹, et X^{a} représente un groupe -U(CH₂)ᵥB- ou un groupe pouvant être converti en un groupe -U(CH₂)ᵥB- dans lequel U représente un groupe -N(R³⁰)-, et L représente un groupe partant convenable, pour produire un composé de formule (I) dans laquelle U représente un groupe -N(R³⁰)- ; ou
c) la réaction d'un composé de formule (VII) avec un composé de formule X^{a}R^{11a} (IV), 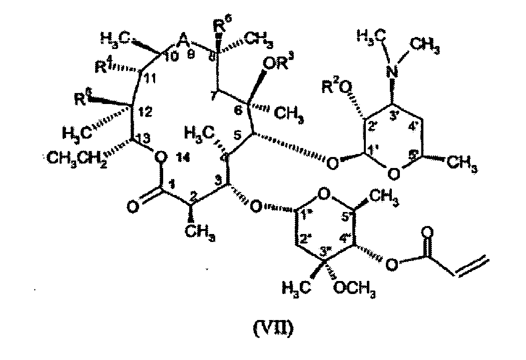 dans laquelle R^{11a} représente un groupe R¹¹ tel que défini dans la revendication 1 ou un groupe pouvant être converti en un groupe R¹¹, et X^{a} représente
un groupe -U(CH₂₎ᵥB- ou un groupe pouvant être converti en un groupe -U(CH₂)ᵥB- dans lequel U représente un groupe -N(R³⁰)-, pour produire un composé de formule (I) dans laquelle d est égal à 2 et U représente un groupe -N(R³⁰)-, et ensuite, si besoin, l'étape consistant à soumettre le composé résultant à une ou plusieurs des opérations suivantes :
i) élimination du groupe protecteur R²,
ii) conversion du groupe X^{a}R^{11a} en un groupe XR¹¹,
iii) conversion du groupe B^{a}R^{11a} en un groupe BR¹¹, et
iv) conversion du composé résultant de formule (I) en un de ses sels ou produits de solvatation pharmaceutiquement acceptables.

15. Composé suivant l'une quelconque des revendications 1 à 13, ou un de ses sels, produits de solvatation ou esters pharmaceutiquement acceptables, destiné à être utilisé en thérapie.

16. Utilisation d'un composé suivant l'une quelconque des revendications 1 à 13, ou d'un de ses sels, produits de solvatation ou esters pharmaceutiquement acceptables, dans la production d'un médicament destiné au traitement ou à la prophylaxie d'infections microbiennes systémiques ou topiques dans l'organisme d'un être humain ou d'un animal.

17. Composé suivant l'une quelconque des revendications 1 à 13, ou un de ses sels, produits de solvatation ou esters pharmaceutiquement acceptables, destiné à être utilisé dans le traitement ou la prophylaxie d'infections microbiennes systémiques ou topiques dans l'organisme d'un être humain ou d'un animal.

18. Composition pharmaceutique comprenant au moins un composé suivant l'une quelconque des revendications 1 à 13, ou un de ses sels, produits de solvatation ou esters pharmaceutiquement acceptables, en association avec un excipient, diluant et/ou support pharmaceutiquement acceptable.
